# EUROPEAN PATENT APPLICATION

(11) **EP 3 950 059 A1**
(43) Date of publication of application: **09.02.2022**
(21) Application number: 20783736.0
(22) Date of filing: 26.03.2020
(51) Int. Cl.: A61P 19/02, A61P 29/00, A61P 43/00, C07D 207/14, C07D 401/04, C07D 401/12, C07D 401/14, C07D 403/04, C07D 403/12, C07D 403/14, C07D 409/04, C07D 413/04, C07D 413/14, C07D 417/04, C07D 417/14, C07D 471/04, C07D 491/08, C07D 491/107, C07D 491/113, A61P 17/04, C07F 7/12

(54) **USE OF T-TYPE CALCIUM CHANNEL BLOCKER FOR TREATING PRURITUS**

(30) Priority: 29.03.2019 JP 2019068811
(71) Applicant: Nippon Chemiphar Co., Ltd., Tokyo 101-0032 (JP); UTI Limited Partnership, Calgary, AB T2L 1Y8 (CA); Kinki University, Higashi-Osaka-shi Osaka 577-8502 (JP)
(72) Inventor: ZAMPONI, Gerald W., Calgary Alberta T2N 4N1 (CA); GADOTTI, Vinicius de Maria, Calgary¨ Alberta T2N 4N1 (CA); KAWABATA, Atsufumi, Higashiosaka-shi Osaka 577-8502 (JP); OGAWA, Toru, Misato-shi Saitama 341-0005 (JP); TANAKA, Hiroto, Misato-shi Saitama 341-0005 (JP); OOI, Isao, Misato-shi Saitama 341-0005 (JP); SAITO, Daisuke, Misato-shi Saitama 341-0005 (JP); HAYASHIDA, Kohei, Misato-shi Saitama 341-0005 (JP); YAMAMOTO, Kohei, Misato-shi Saitama 341-0005 (JP)
(74) Representative: Beckmann, Claus
(86) International application number: PCT/JP2020/013542
(87) International publication number: WO 2020/203609

(57) **Abstract**

A medicament for treating or preventing pruritus is provided. For the medicament for treating or preventing pruritus, a compound having a blocking action on Cav3.2T-type calcium channels represented by General Formulas (I) to (VI), a tautomer of the compound, a stereoisomer of the compound, a pharmaceutically acceptable salt thereof, or a solvate thereof is used as an active ingredient.

## Description

### Technical Field

The present invention relates to a Cav3.2 channel blocker useful for preventing or treating pruritus and use thereof.

### Background Art

Itch (pruritus) is an unpleasant sensation that makes you want to scratch the mucous membranes of the skin. A physiological role of itch is not clear, but is said to be a defense mechanism that removes a foreign enemy such as a parasite attached to the skin by scratching behavior or informs a living body of information such as skin inflammation.

Conventionally, itch has been perceived as a simply mild pain. However, it has been reported that a gastrin-releasing peptide (GRP) expressed in the C fibers of the primary afferent sensory nerve that transmits sensory information from the skin to the spinal cord specifically induces itch (Non Patent Literature 1) and that the dorsal horn nerve of the spinal cord that expresses its receptor GPCR is also specific to itch (Non Patent Literature 2). It is now known that pain and itch are different concepts. In addition, by examination of a reaction in the brain when itch is caused by electrical stimulation using functional MRI and magnetoencephalography, it has been reported that the reaction occurred in the precuneus in the parietal lobe, which does not respond to a pain. From this result, it has been clarified that itch has a different mechanism in the brain from a pain.

The mechanism by which itch occurs has not yet been elucidated. However, it is known that histamine secreted from immune cells (mast cells or basophil granules) induces acute itch. That is, in peripheral tissues, histamine excites the primary afferent nerve C fibers by opening TRPV1 channels via a histamine H1 receptor, and this signal is transmitted from the C fibers to the spinal cord and further to the brain via a neuropeptide such as GRP in the dorsal horn of the spinal cord, which leads to recognition of itch.

In recent years, hydrogen sulfide (H₂S) has been reported as an itch mediator (Non Patent Literature 3). According to this report, H₂S is involved in opioid receptor-mediated itch, but appears to be unrelated to histamine-induced itch. It is indicated that activation of Cav3.2T-type calcium channels is involved in causing this H₂S-induced itch.

An antihistamine, an antiallergic agent, a corticosteroid, a non-steroidal anti-inflammatory drug, and the like are generally used to treat itch. In Japan, nalfurafine, which is a selective κ-opioid receptor agonist, was approved for application to itch of a hemodialysis patient in 2009. However, no itch treatment agent targeting Cav3.2 channels has been known so far.

### Citation List

### Non Patent Literature

Non Patent Literature 1: Sun YG et al., A gastrin-releasing peptide receptor mediates the itch sensation in the spinal cord., Nature, 2007, 448 (7154), 700-703.
Non Patent Literature 2: Sun YG et al., Cellular basis of itch sensation., Science, 2009, 325 (5947), 1531-1534.
Non Patent Literature 3: Wang XL et al., Hydrogen sulfide-induced itch requires activation of Cav3.2 T-type calcium channels in mice., Scientific Reports, 2015, 5:16768

### Summary of Invention

### Technical Problem

One object of the present invention is to provide a medicament for treating or preventing pruritus useful for mammals including humans.

### Solution to Problem

The present inventors have found that a cyclic amine compound having an amide bond has an excellent blocking action on T-type calcium channels, particularly Cav3.2 channels. Then, the present inventors have found that prevention or treatment of pruritus can be achieved by using the Cav3.2 channel blocker, and have completed the present invention.

That is, the present invention provides the following (1) to (84).
(1) A medicament for treating or preventing pruritus, the medicament containing a compound represented by the following General Formula (I), a tautomer of the compound, a stereoisomer of the compound, a pharmaceutically acceptable salt thereof, or a solvate thereof as an active ingredient. 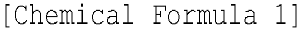 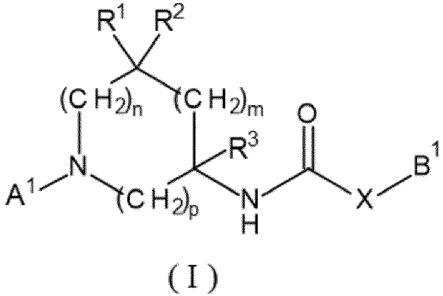
   wherein A¹ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one or two nitrogen atoms and carbon atoms, while herein, the heteroaryl ring or the heterocondensed ring may have a substituent and is bonded to a nitrogen atom of the adjacent cyclic amine by means of a carbon atom constituting these rings;
   B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one or two nitrogen atoms and carbon atoms, while herein, the heteroaryl ring or the heterocondensed ring may have a substituent and is bonded to adjacent X by means of a carbon atom constituting these rings;
   R¹ and R², which may be identical or different, each represent a hydrogen atom, a halogen atom, a hydroxy group, a C₁₋₈ alkyl group, or a C₁₋₈ alkyl group substituted with one to three halogen atoms; or
   R¹, R², and the carbon atom to which R¹ and R² are bonded may be joined together and form a 3- to 5-membered cycloalkyl group;
   R³ represents a hydrogen atom, a halogen atom, a carboxyl group, a cyano group, a carbamoyl group, a C₁₋₈ alkyl group, a C₁₋₈ alkoxycarbonyl group, a C₁₋₈ alkyl group substituted with a C₁₋₈ alkoxy group, a C₁₋₈ alkyl group substituted with a hydroxy group, a C₁₋₈ alkyl group substituted with one to three halogen atoms, or a C₁₋₈ alkyl group substituted with an acyloxy group; or
   R² and R³ may be joined together and form methylene or ethylene;
   X represents: 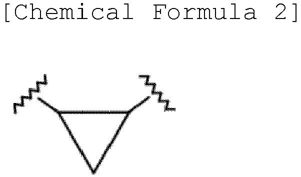
   or 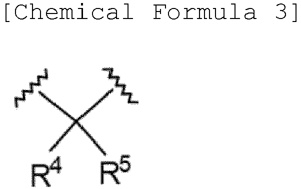
   here, the wavy line represents a bonding position;
   R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group; or
   R⁴, R⁵, and the carbon atom to which R⁴ and R⁵ are bonded may be joined together and form a 3- to 5-membered cycloalkyl group;
   n and m, which may be identical or different, each represent 0 or 1; and
   p represents 1 or 2;
   further, the substituent that may be carried by the phenyl of A¹, the heteroaryl ring of A¹, and the heterocondensed ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
   the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
   here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group.
(2) The medicament according to (1) above, in which X represents: 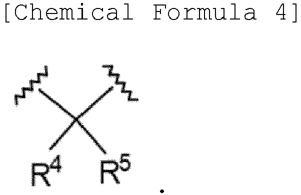
(3) The medicament according to (1) or (2) above, in which A¹ represents a phenyl which may have a substituent.
(4) The medicament according to (1) or (2) above, in which A¹ represents a phenyl which may have a substituent, or a 4-membered to 6-membered heteroaryl ring composed of one to three identical or different heteroatoms selected from an oxygen atom, a sulfur atom, and a nitrogen atom and carbon atoms as ring-constituting atoms which may have a substituent.
(5) The medicament according to (4) above, in which the heteroaryl ring of A¹ is thiophene, oxazole, thiazole, pyridine, pyrazine, pyrimidine, or pyridazine.
(6) The medicament according to (4) above, in which the heteroaryl ring of A¹ represents pyridine.
(7) The medicament according to (1) or (2) above, in which A¹ represents a heterocondensed ring composed of a 4-membered to 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.
(8) The medicament according to (7) above, in which the heterocondensed ring of A¹ is quinoline or benzo[d]thiazole.
(9) The medicament according to any one of (1) to (8) above, in which B¹ represents a phenyl which may have a substituent.
(10) The medicament according to any one of (1) to (8) above, in which B¹ represents a phenyl which may have a substituent, or a 5-membered or 6-membered heteroaryl ring composed of one to three identical or different heteroatoms selected from an oxygen atom, a sulfur atom, and a nitrogen atom and carbon atoms as ring-constituting atoms which may have a substituent.
(11) The medicament according to (10) above, in which the heteroaryl ring of B¹ represents pyridine.
(12) The medicament according to any one of (9) to (11) above, in which when B¹ represents a phenyl which may have a substituent or a 6-membered heteroaryl ring which may have a substituent, the substituent is substituted at a 4-position (para-position).
(13) The medicament according to any one of (1) to (8) above, in which B¹ represents a heterocondensed ring composed of a 5-membered or 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.
(14) The medicament according to (13) above, in which the heterocondensed ring of B¹ represents indole, benzimidazole, indazole, benzoxazole, benzothiazole, benzo[d][1,2,3] triazole, or quinoline.
(15) The medicament according to (13) above, in which the heterocondensed ring of B¹ represents indazole.
(16) The medicament according to any one of (1) to (15) above, in which n represents 1 and m represents 0.
(17) The medicament according to any one of (1) to (16) above, in which p represents 1.
(18) The medicament according to any one of (1) to (17) above, in which R¹ and R² each represent a hydrogen atom.
(19) The medicament according to any one of (1) to (18) above, in which R³ represents a hydrogen atom.
(20) A medicament for treating or preventing pruritus, the medicament containing a compound represented by the following General Formula (II), a tautomer of the compound, a stereoisomer of the compound, a pharmaceutically acceptable salt thereof, or a solvate thereof as an active ingredient. 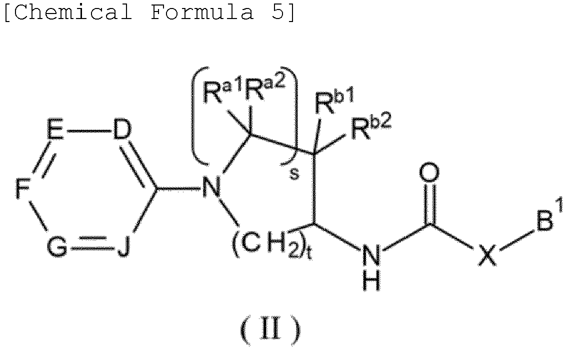
   wherein D, E, F, G, and J are such that any two of them each represent N while the others represent CRs which may be identical or different, or any one of them represents N while the others represent CRs which may be identical or different, or all of them are CRs which may be identical or different; here, R represents a hydrogen atom, a C₁₋₈ alkyl group, a C₁₋₈ alkoxy group, a C₁₋₈ alkyl group substituted with one to three halogen atoms, a C₁₋₈ alkoxy group which may be substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, or a C₁₋₈ alkoxy group substituted with a C₁₋₈ alkoxycarbonyl group; R^{a1}, R^{a2}, R^{b1}, and R^{b2}, which may be identical or different, each represent a hydrogen atom, a halogen atom, a hydroxy group, a C₁₋₈ alkyl group, or a C₁₋₈ alkyl group substituted with one to three halogen atoms; or R^{a1}, R^{a2}, and the carbon atom to which R^{a1} and R^{a2} are bonded may be joined together and form a 3- to 5-membered cycloalkyl group, or R^{b1}, R^{b2}, and the carbon atom to which R^{b1} and R^{b2} are bonded may be joined together and form a 3- to 5-membered cycloalkyl group;
   s represents 0, 1, or 2;
   t represents 1 or 2;
   B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to adjacent X by mean of a carbon atom constituting these rings;
   X represents: 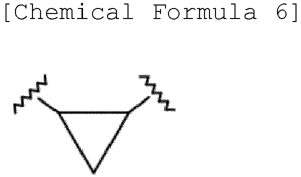
   or 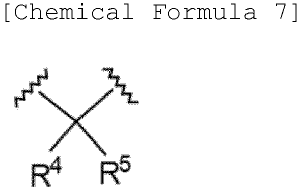
   here, the wavy line represents a bonding position;
   R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group; or
   R⁴, R⁵, and the carbon atom to which R⁴ and R⁵ are bonded may be joined together and form a 3- to 5-membered cycloalkyl group;
   further, the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri(C₁₋₈ alkyl)silyl group, an acylamino group, an (N-acyl) (N-C₁₋₈ alkyl) amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
   here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group.
(21) A medicament for treating or preventing pruritus, the medicament containing a compound represented by the following General Formula (II), a tautomer of the compound, a stereoisomer of the compound, a pharmaceutically acceptable salt thereof, or a solvate thereof as an active ingredient. 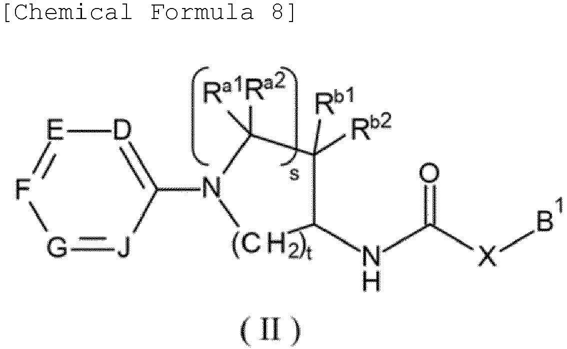
   wherein D, E, F, G, and J are such that any two of them each represent N while the others represent CRs which may be identical or different, or any one of them represents N while the others represent CRs which may be identical or different, or all of them are CRs which may be identical or different;
   here, R represents a hydrogen atom, a C₁₋₈ alkyl group, a C₁₋₈ alkoxy group, a C₁₋₈ alkyl group substituted with one to three halogen atoms, a C₁₋₈ alkoxy group which may be substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, or a C₁₋₈ alkoxy group substituted with a C₁₋₈ alkoxycarbonyl group;
   R^{a1}, R^{a2}, R^{b1}, and R^{b2}, which may be identical or different, each represent a hydrogen atom, a halogen atom, a hydroxy group, a C₁₋₈ alkyl group, or a C₁₋₈ alkyl group substituted with one to three halogen atoms; or
   R^{a1}, R^{a2}, and the carbon atom to which R^{a1} and R^{a2} are bonded may be joined together and form a 3- to 5-membered cycloalkyl group, or R^{b1}, R^{b2}, and the carbon atom to which R^{b1} and R^{b2} are bonded may be joined together and form a 3-to 5-membered cycloalkyl group;
   s represents 0, 1, or 2;
   t represents 1 or 2;
   B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to adjacent X by mean of a carbon atom constituting these rings;
   X represents: 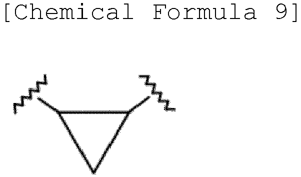
   or 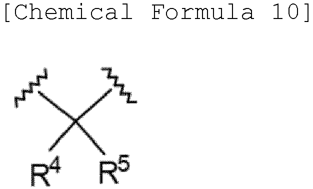
   here, the wavy line represents a bonding position;
   R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group; or R⁴, R⁵, and the carbon atom to which R⁴ and R⁵ are bonded may be joined together and form a 3- to 5-membered cycloalkyl group;
   further, the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri(C₁₋₈ alkyl)silyl group, an acylamino group, an (N-acyl) (N-C₁₋₈ alkyl) amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
   here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group;
   provided that when a 6-membered ring composed of D to J is a phenyl which may have a substituent, or a pyridazine which may have a substituent, and X represents 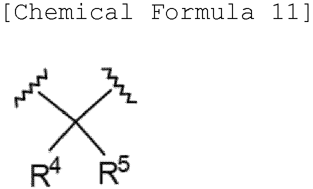
   B¹ represents a heterocondensed ring which may have the above-mentioned substituent.
(22) The medicament according to (20) or (21) above, in which
   X represents 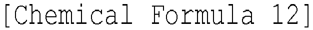 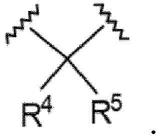
(23) The medicament according to any one of (20) to (22) above, in which D and J each represent CH.
(24) The medicament according to any one of (20) to (22) above, in which any one of D, E, F, G, and J represents N and the others each represent CR.
(25) The medicament according to any one of (20) to (22) above, in which D, E, F, and J represent CRs which may be identical or different, and G represents N.
(26) The medicament according to any one of (20) to (25) above, in which B¹ represents a phenyl which may have a substituent.
(27) The medicament according to any one of (20) to (25) above, in which B¹ represents a phenyl which may have a substituent, or a 5-membered or 6-membered heteroaryl ring composed of one to three identical or different heteroatoms selected from an oxygen atom, a sulfur atom, and a nitrogen atom and carbon atoms as ring-constituting atoms which may have a substituent.
(28) The medicament according to (27) above, in which the heteroaryl ring of B¹ represents pyridine.
(29) The medicament according to any one of (26) to (28) above, in which when B¹ represents a phenyl which may have a substituent or a 6-membered heteroaryl ring which may have a substituent, the substituent is substituted at a 4-position (para-position).
(30) The medicament according to any one of (20) to (25) above, in which B¹ represents a heterocondensed ring composed of a 5-membered or 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.
(31) The medicament according to (30) above, in which the heterocondensed ring of B¹ represents indole, benzimidazole, indazole, benzoxazole, benzothiazole, benzo[d][1,2,3] triazole, or quinoline.
(32) The medicament according to (30) above, in which the heterocondensed ring of B^{a} represents indazole.
(33) The medicament according to any one of (20) to (32) above, in which s represents 1.
(34) The medicament according to any one of (20) to (33) above, in which t represents 1.
(35) The medicament according to any one of (20) to (34) above, in which R^{a1}, R^{a2}, R^{b1}, and R^{b2} each represent a hydrogen atom.
(36) A medicament for treating or preventing pruritus, the medicament containing a compound represented by the following General Formula (III), a tautomer of the compound, a stereoisomer of the compound, a pharmaceutically acceptable salt thereof, or a solvate thereof as an active ingredient. 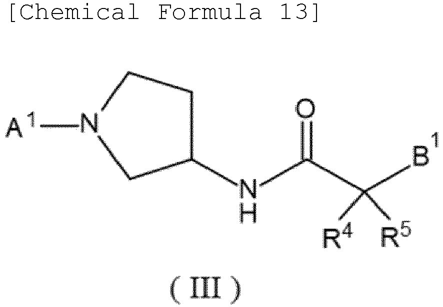
   wherein A¹ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to a nitrogen atom of the adjacent pyrrolidine by means of a carbon atom constituting these rings; and
   B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to adjacent C(R⁴)(R⁵) by means of a carbon atom constituting these rings;
   R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group; or
   R⁴, R⁵, and the carbon atom to which R⁴ and R⁵ are bonded may be joined together and form a 3- to 5-membered cycloalkyl group;
   further, the substituent that may be carried by the phenyl of A¹, the heteroaryl ring of A¹, and the heterocondensed ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
   the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
   here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group.
(37) A medicament for treating or preventing pruritus, the medicament containing a compound represented by the following General Formula (III), a tautomer of the compound, a stereoisomer of the compound, a pharmaceutically acceptable salt thereof, or a solvate thereof as an active ingredient. 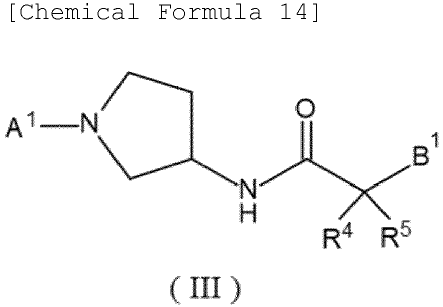
   wherein A¹ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to a nitrogen atom of the adjacent pyrrolidine by means of a carbon atom constituting these rings; and
   B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to adjacent C(R⁴)(R⁵) by means of a carbon atom constituting these rings;
   R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group; or
   R⁴, R⁵, and the carbon atom to which R⁴ and R⁵ are bonded may be joined together and form a 3- to 5-membered cycloalkyl group;
   further, the substituent that may be carried by the phenyl of A¹, the heteroaryl ring of A¹, and the heterocondensed ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
   the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl)(C₁₋₈ alkoxy-substituted C₁₋₈ alkyl) amino group, a tri (C₁₋₈ alkyl)silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
   here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group;
   provided that when A¹ represents a phenyl which may have a substituent, a pyridazine which may have a substituent, and a quinazoline which may have a substituent, B¹ represents a heterocondensed ring which may have the above-mentioned substituent.
(38) The medicament according to (36) or (37) above, in which A¹ represents a phenyl which may have a substituent.
(39) The medicament according to (36) or (37) above, in which A¹ represents a phenyl which may have a substituent, or a 4-membered to 6-membered heteroaryl ring composed of one to three identical or different heteroatoms selected from an oxygen atom, a sulfur atom, and a nitrogen atom and carbon atoms as ring-constituting atoms which may have a substituent.
(40) The medicament according to (39) above, in which the heteroaryl ring of A¹ is thiophene, oxazole, thiazole, pyridine, pyrazine, pyrimidine, or pyridazine.
(41) The medicament according to (39) above, in which the heteroaryl ring of A¹ represents pyridine.
(42) The medicament according to (36) or (37) above, in which A¹ represents a heterocondensed ring composed of a 4-membered to 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.
(43) The medicament according to (42) above, in which the heterocondensed ring of A¹ is quinoline or benzo[d]thiazole.
(44) The medicament according to any one of (36) to (43) above, in which B¹ represents a phenyl which may have a substituent.
(45) The medicament according to any one of (36) to (43) above, in which B¹ represents a phenyl which may have a substituent, or a 5-membered or 6-membered heteroaryl ring composed of one to three identical or different heteroatoms selected from an oxygen atom, a sulfur atom, and a nitrogen atom and carbon atoms as ring-constituting atoms which may have a substituent.
(46) The medicament according to (45) above, in which the heteroaryl ring of B¹ represents pyridine.
(47) The medicament according to any one of (44) to (46) above, in which when B¹ represents a phenyl which may have a substituent or a 6-membered heteroaryl ring which may have a substituent, the substituent is substituted at a 4-position (para-position).
(48) The medicament according to any one of (36) to (43) above, in which B¹ represents a heterocondensed ring composed of a 5-membered or 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.
(49) The medicament according to (48) above, in which the heterocondensed ring of B¹ represents indole, benzimidazole, indazole, benzoxazole, benzothiazole, benzo[d][1,2,3] triazole, or quinoline.
(50) The medicament according to (48) above, in which the heterocondensed ring of B¹ represents indazole.
(51) The medicament according to any one of (36) to (50) above, in which R⁴ and R⁵ each represent a hydrogen atom.
(52) A medicament for treating or preventing pruritus, the medicament containing a compound represented by the following General Formula (IV), a tautomer of the compound, a stereoisomer of the compound, a pharmaceutically acceptable salt thereof, or a solvate thereof as an active ingredient. 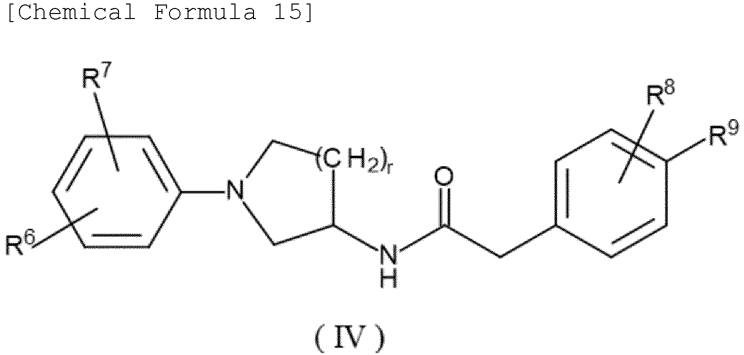
   wherein R⁶, R⁷, and R⁸, which are identical or different, each represent a hydrogen atom, a halogen atom, a C₁₋₈ alkyl group, a C₁₋₈ alkyl group substituted with one to three halogen atoms, a C₁₋₈ alkoxy group, a C₁₋₈ alkoxy group substituted with one to three halogen atoms, a hydroxy group, or a C₁₋₈ alkoxy group substituted with a C₁₋₈ alkoxycarbonyl group;
   R⁹ represents a C₁₋₈ alkyl substituted with one to three halogen atoms, a tert-butyl, or a cyclopropyl; and
   r represents 0, 1, or 2.
(53) The medicament according to (52) above, in which R⁶, R⁷, and R⁸, which are different, each represent a hydrogen atom, a halogen atom, a C₁₋₈ alkyl group, a C₁₋₈ alkyl group substituted with one to three halogen atoms, or a C₁₋₈ alkoxy group.
(54) The medicament according (52) or (53) above, in which R⁹ represents trifluoromethyl or cyclopropyl.
(55) The medicament according to any one of (52) to (54) above, in which r represents 1.
(56) A medicament for treating or preventing pruritus, the medicament containing a compound represented by the following General Formula (V), a tautomer of the compound, a stereoisomer of the compound, a pharmaceutically acceptable salt thereof, or a solvate thereof as an active ingredient. 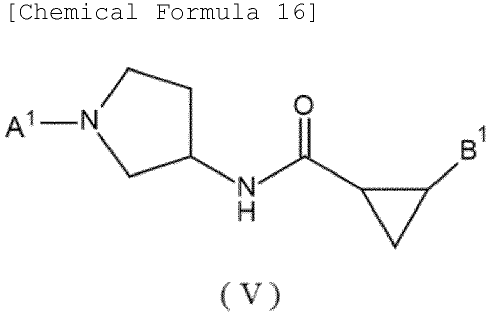
   wherein A¹ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to a nitrogen atom of the adjacent pyrrolidine by means of a carbon atom constituting these rings; and
   B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to the adjacent cyclopropyl by means of a carbon atom constituting these rings;
   further, the substituent that may be carried by the phenyl of A¹, the heteroaryl ring of A¹, and the heterocondensed ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
   the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, a 3- to 5-membered cycloalkyl group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri(C₁₋₈ alkyl)silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
   here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopiperidino, 2-, 3-, or 4-oxopiperidino, morpholino, 1,1-dioxide thiomorpholino, 1,4-dioxo-8-azaspiro[4.5]decan-8-yl, 2-oxo-6-azaspiro[3.3]heptan-6-yl, 3-oxo-8-azabicyclo[3.2.1]octan-8-yl, 2-oxo-5-azabicyclo[2.2.2]octan-5-yl, and 2-oxo-5-azabicyclo[2.2.1]heptan-5-yl, while such a cyclic amino group may be further substituted with a C₁₋₈ alkyl group, a halogen atom, or an acyl group.
(57) The medicament according to (56) above, in which A¹ represents a phenyl which may have a substituent.
(58) The medicament according to (56) above, in which A¹ represents a 4-membered to 6-membered heteroaryl ring composed of one to three identical or different heteroatoms selected from an oxygen atom, a sulfur atom, and a nitrogen atom and carbon atoms as ring-constituting atoms which may have a substituent.
(59) The medicament according to (58) above, in which the heteroaryl ring of A¹ is thiophene, oxazole, thiazole, pyridine, pyrazine, pyrimidine, or pyridazine.
(60) The medicament according to (58) above, in which the heteroaryl ring of A¹ represents pyridine.
(61) The medicament according to (56) above, in which A¹ represents a heterocondensed ring composed of a 4-membered to 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.
(62) The medicament according to (61) above, in which the heterocondensed ring of A¹ is quinoline or benzo[d]thiazole.
(63) The medicament according to any one of (56) to (62) above, in which B¹ represents a phenyl which may have a substituent.
(64) The medicament according to any one of (56) to (62) above, in which B¹ represents a 5-membered or 6-membered heteroaryl ring composed of one to three identical or different heteroatoms selected from an oxygen atom, a sulfur atom, and a nitrogen atom and carbon atoms as ring-constituting atoms which may have a substituent.
(65) The medicament according to (63) above, in which the heteroaryl ring of B¹ represents pyridine.
(66) The medicament according to any one of (56) to (62) above, in which B¹ represents a heterocondensed ring composed of a 5-membered or 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.
(67) The medicament according to (66) above, in which the heterocondensed ring of B¹ represents indole, benzimidazole, indazole, benzoxazole, benzothiazole, benzo[d][1,2,3] triazole, or quinoline.
(68) The medicament according to (66) above, in which the heterocondensed ring of B¹ represents benzo[d]oxazole.
(69) A medicament for treating or preventing pruritus, the medicament containing a compound represented by the following General Formula (VI), a tautomer of the compound, a stereoisomer of the compound, a pharmaceutically acceptable salt thereof, or a solvate thereof as an active ingredient. 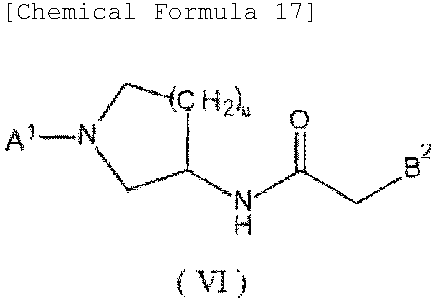
   wherein A¹ represents a phenyl which may have a substituent or a 4- to 6-membered heteroaryl ring composed of one to three identical or different heteroatoms selected from an oxygen atom, a sulfur atom, and a nitrogen atom and carbon atoms as ring-constituting atoms, while here, the heteroaryl ring or the heterocondensed ring may have a substituent and is bonded to a nitrogen atom of the adjacent cyclic amine by means of a carbon atom constituting these rings; and
   B² represents a heterocondensed ring composed of a 5- or 6-membered heteroaryl ring composed of one to three identical or different heteroatoms selected from an oxygen atom, a sulfur atom, and a nitrogen atom and carbon atoms as ring-constituting atoms, and either a benzene ring or a 6-membered heteroaryl ring composed of one to two nitrogen atoms and carbon atoms, while here, the heterocondensed ring may have a substituent and is bonded to the adjacent methylene group by means of a carbon atom constituting these rings;
   further, the substituent that may be carried by the phenyl of A¹ and the heteroaryl ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
   the substituent that may be carried by the heterocondensed ring of B² is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri(C₁₋₈ alkyl)silyl group, an acylamino group, an (N-acyl) (N-C₁₋₈ alkyl) amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
   here, the cyclic amino group of the substituent that may be carried by the heterocondensed ring of B² is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group, and
   u represents 0, 1, or 2.
(70) The medicament according to (69) above, in which A¹ represents a phenyl which may have a substituent, or a 4-membered to 6-membered heteroaryl ring composed of one to three identical or different heteroatoms selected from an oxygen atom, a sulfur atom, and a nitrogen atom and carbon atoms as ring-constituting atoms which may have a substituent.
(71) The medicament according to (70) above, in which the heteroaryl ring of A¹ is thiophene, oxazole, thiazole, pyridine, pyrazine, pyrimidine, or pyridazine.
(72) The medicament according to (70) above, in which the heteroaryl ring of A¹ represents pyridine.
(73) A medicament for treating or preventing pruritus, the medicament containing a compound selected from the following compounds, a tautomer of the compound, a stereoisomer of the compound, a pharmaceutically acceptable salt thereof, or a solvate thereof as an active ingredient:
   (R)-2-(4-cyclopropylphenyl)-N-(1-(5-(trifluoromethyl) pyridin-3-yl) pyrrolidin-3-yl) acetamide,
   (R)-2-(5-(trifluoromethyl) pyridin-2-yl)-N-(1-(5-(trifluoromethyl) pyridin-3-yl) pyrrolidin-3-yl) acetamide,
   (R)-2-(4-isopropylphenyl)-N-(1-(2-(trifluoromethyl) pyrimidin-5-yl) pyrrolidin-3-yl) acetamide,
   (R)-2-(4-isopropylphenyl)-N-(1-(5-(trifluoromethyl) pyridin-3-yl) pyrrolidin-3-yl) acetamide,
   2-(4-cyclopropylphenyl)-N-((3S,4S)-4-hydroxy-1-(5-(trifluoromethyl) pyridin-3-yl) pyrrolidin-3-yl) acetamide,
   (R)-2-(4-cyclopropylphenyl)-N-(5-(5-(trifluoromethyl) pyridin-3-yl)-5-azaspiro[2.4]heptan-7-yl) acetamide,
   (R)-2-(4-cyclopropylphenyl)-N-(5-(6-(trifluoromethyl) pyridin-3-yl)-5-azaspiro[2.4]heptan-7-yl) acetamide,
   (R)-2-(4-cyclopropylphenyl)-N-(5-(4-(trifluoromethyl) thiazol-2-yl)-5-azaspiro[2.4]heptan-7-yl) acetamide,
   2-(4-cyclopropylphenyl)-N-((3R,5S)-5-methyl-1-(5-(trifluoromethyl) pyridin-3-yl) pyrrolidin-3-yl) acetamide,
   2-(4-cyclopropylphenyl)-N-((1S,5R)-3-(5-(trifluoromethyl) pyridin-3-yl)-3-azabicyclo[3.1.0]hexan-1-yl) acetamide,
   2-(4-cyclopropylphenyl)-N-((1R,5S)-3-(5-(trifluoromethyl) pyridin-3-yl)-3-azabicyclo[3.1.0]hexan-1-yl) acetamide,
   (R)-2-(4-(trifluoromethyl)phenyl)-N-(1-(6-(trifluoromethyl) pyrazin-2-yl) pyrrolidin-3-yl) acetamide,
   (R)-N-(1-(6-cyano-5-(trifluoromethyl) pyridin-3-yl) pyrrolidin-3-yl)-2-(4-cyclopropylphenyl) acetamide,
   (R)-3-(2-(4-cyclopropylphenyl) acetamide)-1-(5-(trifluoromethyl) pyridin-3-yl) pyrrolidine-3-methyl carboxylate,
   (S)-3-(2-(4-cyclopropylphenyl) acetamide)-1-(5-(trifluoromethyl) pyridin-3-yl) pyrrolidine-3-methyl carboxylate,
   (R)-3-(2-(4-cyclopropylphenyl) acetamide)-1-(5-(trifluoromethyl) pyridin-3-yl) pyrrolidine-3-carboxylic acid,
   (S)-3-(2-(4-cyclopropylphenyl) acetamide)-1-(5-(trifluoromethyl) pyridin-3-yl) pyrrolidine-3-carboxylic acid,
   (R)-2-(1H-indazol-6-yl)-N-(1-(5-(trifluoromethyl) pyridin-3-yl) pyrrolidin-3-yl) acetamide,
   (R)-2-(4-cyclopropylphenyl)-N-(1-(4-(trifluoromethyl) thiazol-2-yl) pyrrolidin-3-yl) acetamide, (R)-2-(4-isobutylphenyl)-N-((R)-1-(5-(trifluoromethyl) pyridin-3-yl) pyrrolidin-3-yl) propanamide,
   (S)-2-(4-isobutylphenyl)-N-((R)-1-(5-(trifluoromethyl) pyridin-3-yl) pyrrolidin-3-yl) propanamide,
   (R)-2-(4-cyclopropylphenyl)-N-(1-6-methyl-5-(trifluoromethyl) pyridin-3-yl) pyrrolidin-3-yl) acetamide,
   (R)-2-(4-cyclopropylphenyl)-N-(1-(5-(2-hydroxypropan-2-yl) pyridin-3-yl) pyrrolidin-3-yl) acetamide,
   (R)-2-(4-cyclopropylphenyl)-N-(1-(8-(trifluoromethyl) imidazo[1,2-a] pyridin-6-yl) pyrrolidin-3-yl) acetamide,
   (R)-3-(2-(4-cyclopropylphenyl) acetamide)-1-(5-(trifluoromethyl) pyridin-3-yl) pyrrolidine-3-carboxyamide,
   (S)-3-(2-(4-cyclopropylphenyl) acetamide)-1-(5-(trifluoromethyl) pyridin-3-yl) pyrrolidine-3-carboxyamide,
   (R)-2-(4-hydroxyphenyl)-N-(1-(5-(trifluoromethyl) pyridin-3-yl) pyrrolidin-3-yl) acetamide,
   (R)-2-(4-(1,1-dioxidethiomorpholino) phenyl)-N-(1-(5-(trifluoromethyl) pyridin-3-yl) pyrrolidin-3-yl) acetamide,
   (R)-1-(4-chlorophenyl)-N-(1-(5-(trifluoromethyl) pyridin-3-yl) pyrrolidin-3-yl) cyclopropane-1-carboxyamide,
   (R)-2-(4-bromophenyl)-2-hydroxy-N-((R)-1-(5-(trifluoromethyl) pyridin-3-yl) pyrrolidin-3-yl) acetamide,
   (S)-2-(4-bromophenyl)-2-hydroxy-N-((R)-1-(5-(trifluoromethyl) pyridin-3-yl) pyrrolidin-3-yl) acetamide,
   (R)-N-(1-(5-(trifluoromethyl) pyridin-3-yl) pyrrolidin-3-yl)-2-(4-(trimethylsilyl) phenyl) acetamide,
   (R)-2-(4-(2-hydroxy-2-methylpropoxy) phenyl)-N-(1-(5-(trifluoromethyl) pyridin-3-yl) pyrrolidin-3-yl) acetamide,
   2-(4-cyclopropylphenyl)-N-((3S,4S)-4-fluoro-1-(5-(trifluoromethyl) pyridin-3-yl) pyrrolidin-3-yl) acetamide,
   (R)-2-(4-(dimethylamino) phenyl)-N-(1-(5-(trifluoromethyl) pyridin-3-yl) pyrrolidin-3-yl) acetamide,
   (R)-2-(4-nitrophenyl)-N-(1-(5-(trifluoromethyl) pyridin-3-yl) pyrrolidin-3-yl) acetamide,
   (S)-2-(4-isobutylphenyl)-N-((R)-1-(5-(trifluoromethyl) pyridin-3-yl) pyrrolidin-3-yl) propanamide,
   (R)-2-(3-fluoro-4-(trifluoromethyl) phenyl)-N-(1-(5-(trifluoromethyl) pyridin-3-yl) pyrrolidin-3-yl) acetamide,
   (R)-2-(4-aminophenyl)-N-(1-(5-(trifluoromethyl) pyridin-3-yl) pyrrolidin-3-yl) acetamide,
   (R)-1-(4-(trifluoromethyl) phenyl)-N-(1-(5-(trifluoromethyl) pyridin-3-yl) pyrrolidin-3-yl) cyclopropane-1-carboxyamide,
   (R)-2-(4-acetamide phenyl)-N-(1-(5-(trifluoromethyl) pyridin-3-yl) pyrrolidin-3-yl) acetamide,
   (R)-N-(3-cyano-1-(5-(trifluoromethyl) pyridin-3-yl) pyrrolidin-3-yl)-2-(4-cyclopropylphenyl) acetamide,
   (S)-N-(3-cyano-1-(5-(trifluoromethyl) pyridin-3-yl) pyrrolidin-3-yl)-2-(4-cyclopropylphenyl) acetamide,
   (R)-3-(2-(1H-indol-6-yl) acetamide)-1-(5-(trifluoromethyl) pyridin-3-yl) pyrrolidine-3-methyl carboxylate,
   (S)-3-(2-(1H-indol-6-yl) acetamide)-1-(5-(trifluoromethyl) pyridin-3-yl) pyrrolidine-3-methyl carboxylate,
   (R)-3-(2-(4-(trifluoromethyl) phenyl) acetamide)-1-(5-(trifluoromethyl) pyridin-3-yl) pyrrolidine-3-methyl carboxylate,
   (S)-3-(2-(4-(trifluoromethyl) phenyl) acetamide)-1-(5-(trifluoromethyl) pyridin-3-yl) pyrrolidine-3-methyl carboxylate,
   (1S,2S)-2-(benzo[d]oxazol-2-yl)-N-((R)-1-(5-(trifluoromethyl) pyridin-3-yl) pyrrolidin-3-yl) cyclopropane-1-carboxyamide,
   (R)-2-(4-morpholinophenyl)-N-(1-(4-(trifluoromethyl) thiazol-2-yl) pyrrolidin-3-yl) acetamide,
   3-(2-(4-morpholinophenyl) acetamide)-1-(5-(trifluoromethyl) pyridin-3-yl) pyrrolidine-3-methyl carboxylate,
   (R)-2-(4-morpholinophenyl)-N-(1-(6-(trifluoromethyl) pyridin-3-yl) pyrrolidin-3-yl) acetamide,
   (R)-2-(4-cyclopropylphenyl)-N-(3-ethyl-1-(5-(trifluoromethyl) pyridin-3-yl) pyrrolidin-3-yl) acetamide,
   (S)-2-(4-cyclopropylphenyl)-N-(3-ethyl-1-(5-(trifluoromethyl) pyridin-3-yl) pyrrolidin-3-yl) acetamide,
   (1S,2S)-2-(6-fluorobenzo[d]oxazol-2-yl)-N-((R)-1-(5-(trifluoromethyl) pyridin-3-yl) pyrrolidin-3-yl) cyclopropane-1-carboxyamide,
   (1S,2S)-2-(5,6-difluorobenzo[d]oxazol-2-yl)-N-((R)-1-(5-(trifluoromethyl) pyridin-3-yl) pyrrolidin-3-yl) cyclopropane-1-carboxyamide,
   (R)-2-(4-(2-oxa-6-azaspiro[3.3]heptan-6-yl) phenyl)-N-(1-(5-(trifluoromethyl) pyridin-3-yl) pyrrolidin-3-yl) acetamide,
   (1S,2S)-2-(benzo[d]oxazol-2-yl)-N-((R)-1-(4-(trifluoromethyl) thiazol-2-yl) pyrrolidin-3-yl) cyclopropane-1-carboxyamide,
   (1S,2S)-2-(6-fluorobenzo[d]oxazol-2-yl)-N-((R)-1-(6-(trifluoromethyl) pyridin-3-yl) pyrrolidin-3-yl) cyclopropane-1-carboxyamide,
   (1S,2S)-2-(5-fluorobenzo[d]oxazol-2-yl)-N-((R)-1-(5-(trifluoromethyl) pyridin-3-yl) pyrrolidin-3-yl) cyclopropane-1-carboxyamide,
   (1R,2R)-2-(benzo[d]oxazol-2-yl)-N-((R)-1-(5-(trifluoromethyl) pyridin-3-yl) pyrrolidin-3-yl) cyclopropane-1-carboxyamide,
   (1S,2S)-2-(benzo[d]oxazol-2-yl)-N-((R)-1-(5-(trifluoromethyl) pyridin-3-yl) pyrrolidin-3-yl) cyclopropane-1-carboxyamide,
   (1S,2S)-2-(1H-benzo[d]imidazol-2-yl)-N-((R)-1-(4-(trifluoromethyl) phenyl) pyrrolidin-3-yl) cyclopropane-1-carboxyamide,
   (1R,2R)-2-(1H-benzo[d]imidazol-2-yl)-N-((R)-1-(4-(trifluoromethyl) phenyl) pyrrolidin-3-yl) cyclopropane-1-carboxyamide,
   (1S,2S)-2-(1H-benzo[d]imidazol-2-yl)-N-((S)-1-(4-(trifluoromethyl) phenyl) pyrrolidin-3-yl) cyclopropane-1-carboxyamide,
   (1R,2R)-2-(1H-benzo[d]imidazol-2-yl)-N-((R)-1-(6-(trifluoromethyl) pyridin-3-yl) pyrrolidin-3-yl)) cyclopropane-1-carboxyamide,
   (1S,2S)-2-(1H-benzo[d]imidazol-2-yl)-N-((S)-1-(6-(trifluoromethyl) pyridin-3-yl) pyrrolidin-3-yl) cyclopropane-1-carboxyamide,
   (1S,2S)-2-(1H-benzo[d]imidazol-2-yl)-N-((R)-1-(5-(trifluoromethyl) pyrimidin-2-yl) pyrrolidin-3-yl)) cyclopropane-1-carboxyamide,
   (1S,2S)-2-(1H-benzo[d]imidazol-2-yl)-N-((R)-1-(4-fluoro-3-(trifluoromethyl) phenyl) pyrrolidin-3-yl)) cyclopropane-1-carboxyamide,
   (1S,2S)-N-((R)-1-(4-fluoro-3-(trifluoromethyl) phenyl) pyrrolidin-3-yl)-2-(quinazolin-2-yl) cyclopropane-1-carboxamide,
   (1S,2S)-2-(1H-benzo[d]imidazol-2-yl)-N-((R)-1-(3-(methylsulfonyl) phenyl) pyrrolidin-3-yl) cyclopropane-1-carboxamide,
   (1S,2S)-2-(1H-benzo[d]imidazol-2-yl)-N-((R)-1-(3-cyanophenyl) pyrrolidin-3-yl) cyclopropane-1-carboxyamide,
   (1S,2S)-2-(5-cyano-1H-benzo[d]imidazol-2-yl)-N-((R)-1-(6-(trifluoromethyl) pyridin-3-yl) pyrrolidin-3-yl) cyclopropane-1-carboxyamide,
   (1R,2R)-2-(5-cyano-1H-benzo[d]imidazol-2-yl)-N-((R)-1-(6-(trifluoromethyl) pyridin-3-yl) pyrrolidin-3-yl) cyclopropane-1-carboxyamide,
   (1S,2S)-2-(1H-benzo[d]imidazol-2-yl)-N-((R)-1-(2-(trifluoromethyl) pyridin-4-yl) pyrrolidin-3-yl)) cyclopropane-1-carboxyamide,
   (1S,2S)-2-(1H-benzo[d]imidazol-2-yl)-N-((R)-1-(6-(trifluoromethyl) pyridin-3-yl) piperidin-3-yl)) cyclopropane-1-carboxyamide,
   (1R,2R)-2-(1H-benzo[d]imidazol-2-yl)-N-((R)-1-(6-(trifluoromethyl) pyridin-3-yl) piperidin-3-yl)) cyclopropane-1-carboxyamide,
   (1S,2S)-2-(1H-benzo[d]imidazol-2-yl)-N-((R)-1-(4-(trifluoromethyl) thiazol-2-yl) pyrrolidin-3-yl)) cyclopropane-1-carboxyamide,
   (1S,2S)-2-(1H-benzo[d]imidazol-2-yl)-N-((R)-1-(4-(trifluoromethyl) phenyl) piperidin-3-yl) cyclopropane-1-carboxyamide,
   (1S,2S)-2-(1H-indol-3-yl)-N-((R)-1-(6-(trifluoromethyl) pyridin-3-yl) pyrrolidin-3-yl) cyclopropane-1-carboxyamide,
   (1R,2R)-2-(1H-indol-3-yl)-N-((R)-1-(6-(trifluoromethyl) pyridin-3-yl) pyrrolidin-3-yl) cyclopropane-1-carboxyamide,
   (1S,2S)-2-(1H-benzo[d]imidazol-2-yl)-N-((R)-1-(6-methoxy-5-(trifluoromethyl) pyridin-3-yl) pyrrolidin-3-yl) cyclopropane-1-carboxyamide, (1S,2S)-2-(1H-benzo[d]imidazol-2-yl)-N-((R)-1-(4-(tert-butyl) thiazol-2-yl) pyrrolidin-3-yl) cyclopropane-1-carboxyamide,
   (1S,2S)-2-(1H-benzo[d]imidazol-2-yl)-N-((R)-1-(6-(trifluoromethyl) pyridazin-3-yl) pyrrolidin-3-yl)) cyclopropane-1-carboxyamide,
   (1S,2S)-2-(1H-benzo[d]imidazol-2-yl)-N-((R)-1-(2-(trifluoromethyl) pyrimidin-5-yl) pyrrolidin-3-yl)) cyclopropane-1-carboxyamide,
   (1S,2S)-2-(1H-benzo[d]imidazol-2-yl)-N-((R)-1-(5-(trifluoromethyl) pyridin-3-yl) pyrrolidin-3-yl)) cyclopropane-1-carboxyamide,
   (1S,2S)-2-(1H-benzo[d]imidazol-2-yl)-N-((R)-1-(6-fluorobenzo[d]thiazol-2-yl) pyrrolidin-3-yl) cyclopropane-1-carboxyamide,
   (1S,2S)-2-(1-cyclopropyl-1H-benzo[d]imidazol-2-yl)-N-((R)-1-(6-(trifluoromethyl) pyridin-3-yl) pyrrolidin-3-yl) cyclopropane-1-carboxyamide,
   (1S,2S)-2-(1H-benzo[d]imidazol-2-yl)-N-((R)-1-(6-fluoroquinolin-2-yl) pyrrolidin-3-yl) cyclopropane-1-carboxyamide,
   (1S,2S)-2-(1-cyclopropyl-1H-benzo[d]imidazol-2-yl)-N-((R)-1-(3-trifluoromethyl) phenyl) pyrrolidin-3-yl) cyclopropane-1-carboxyamide,
   (1S,2S)-2-(1H-benzo[d]imidazol-2-yl)-N-((R)-1-(5-bromopyridin-3-yl) pyrrolidin-3-yl) cyclopropane-1-carboxyamide,
   (R)-2-(quinolin-6-yl)-N-(1-(5-(trifluoromethyl) pyridin-3-yl) pyrrolidin-3-yl) acetamide,
   (R)-2-(1H-indol-3-yl)-N-(1-(5-(trifluoromethyl) pyridin-3-yl) pyrrolidin-3-yl) acetamide,
   (R)-2-(quinolin-7-yl)-N-(1-(5-(trifluoromethyl) pyridin-3-yl) pyrrolidin-3-yl) acetamide,
   (R)-2-(4-cyclopropylphenyl)-N-(1-(4-(trifluoromethyl) oxazol-2-yl) pyrrolidin-3-yl) acetamide,
   (R)-2-(1-methyl-5-(trifluoromethyl)-1H-pyrazol-3-yl)-N-(1-(5-(trifluoromethyl) pyridin-3-yl) pyrrolidin-3-yl) acetamide,
   (R)-2-(6-(trifluoromethyl) pyridin-3-yl)-N-(1-(5-(trifluoromethyl) pyridin-3-yl) pyrrolidin-3-yl) acetamide,
   (R)-2-(4-(trifluoromethyl) phenyl)-N-(1-(5-(trifluoromethyl) pyridin-3-yl) pyrrolidin-3-yl) acetamide-2,2-d2,
   (R)-2-(4-(3,3-difluoropyrrolidin-1-yl) phenyl)-N-(1-(6-(trifluoromethyl) pyridin-3-yl) pyrrolidin-3-yl) acetamide,
   (R)-2-(1H-indol-6-yl)-N-(1-(6-(trifluoromethyl) pyridin-3-yl) pyrrolidin-3-yl) acetamide,
   2-(4-((2R,6S)-2,6-dimethylmorpholino) phenyl)-N-((R)-1-(5-(trifluoromethyl) pyridin-3-yl) pyrrolidin-3-yl) acetamide,
   (R)-2-(4-(pyrrolidin-1-yl) phenyl)-N-(1-(5-(trifluoromethyl) pyridin-3-yl) pyrrolidin-3-yl) acetamide,
   (R)-2-(4-((2-methoxyethyl) (methyl) amino) phenyl)-N-(1-(5-(trifluoromethyl) pyridin-3-yl) pyrrolidin-3-yl) acetamide,
   2-(4-(trifluoromethyl) phenyl)-N-(1-(5-(trifluoromethyl) pyridin-3-yl) azetidin-3-yl) acetamide,
   2-(1H-indol-6-yl)-N-(1-(5-(trifluoromethyl) pyridin-3-yl) azetidin-3-yl) acetamide,
   (R)-2-(1-methyl-1H-indol-6-yl)-N-(1-(5-(trifluoromethyl) pyridin-3-yl) pyrrolidin-3-yl) acetamide,
   (R)-2-(1-methyl-1H-indol-6-yl)-N-(1-(6-(trifluoromethyl) pyridin-3-yl) pyrrolidin-3-yl) acetamide,
   (R)-2-(4-(4-methylpiperazin-1-yl) phenyl)-N-(1-(5-(trifluoromethyl) pyridin-3-yl) pyrrolidin-3-yl) acetamide,
   (R)-2-(4-(piperazin-1-yl) phenyl)-N-(1-(5-(trifluoromethyl) pyridin-3-yl) pyrrolidin-3-yl) acetamide,
   (R)-2-(4-(4-acetylpiperazin-1-yl) phenyl)-N-(1-(5-(trifluoromethyl) pyridin-3-yl) pyrrolidin-3-yl) acetamide,
   (R)-2-(4-(2-oxopiperidin-1-yl) phenyl)-N-(1-(5-(trifluoromethyl) pyridin-3-yl) pyrrolidin-3-yl) acetamide,
   (R)-2-(6-chlorobenzo[d]oxazol-2-yl)-N-(1-(5-(trifluoromethyl) pyridin-3-yl) pyrrolidin-3-yl) acetamide,
   (R)-2-(benzo[d]oxazol-2-yl)-N-(1-(5-(trifluoromethyl) pyridin-3-yl) pyrrolidin-3-yl) acetamide,
   (1S,2S)-2-(3,5-dichlorophenyl)-N-((R)-1-(5-(trifluoromethyl) pyridin-3-yl) pyrrolidin-3-yl) cyclopropane-1-carboxamide,
   (1R,2R)-2-(3,5-dichlorophenyl)-N-((R)-1-(5-(trifluoromethyl) pyridin-3-yl) pyrrolidin-3-yl) cyclopropane-1-carboxamide,
   (1S,2S)-2-(4-bromophenyl)-N-((R)-1-(5-(trifluoromethyl) pyridin-3-yl) pyrrolidin-3-yl) cyclopropane-1-carboxyamide,
   (1R,2R)-2-(4-bromophenyl)-N-((R)-1-(5-(trifluoromethyl) pyridin-3-yl) pyrrolidin-3-yl) cyclopropane-1-carboxyamide,
   (R)-2-(4-(1,4-dioxa-8-azaspiro[4.5]decan-8-yl) phenyl)-N-(1-(5-(trifluoromethyl) pyridin-3-yl) pyrrolidin-3-yl) acetamide,
   (R)-2-(4-(4-oxopiperidin-1-yl) phenyl)-N-(1-(5-(trifluoromethyl) pyridin-3-yl) pyrrolidin-3-yl) acetamide,
   (R)-2-(4-cyclopropylphenyl)-N-(3-(hydroxymethyl)-1-(5-(trifluoromethyl) pyridin-3-yl) pyrrolidin-3-yl) acetamide,
   (S)-2-(4-cyclopropylphenyl)-N-(3-(hydroxymethyl)-1-(5-(trifluoromethyl) pyridin-3-yl) pyrrolidin-3-yl) acetamide,
   (R)-(3-(2-(4-cyclopropylphenyl)acetamido)-1-(5-(trifluoromethyl)pyridin-3-yl)pyrrolidin-3-yl)methyl acetate,
   (S)-(3-(2-(4-cyclopropylphenyl)acetamido)-1-(5-(trifluoromethyl)pyridin-3-yl)pyrrolidin-3-yl)methyl acetate,
   (R)-2-(4-cyclopropylphenyl)-N-(3-(methoxymethyl)-1-(5-(trifluoromethyl) pyridin-3-yl) pyrrolidin-3-yl) acetamide,
   (S)-2-(4-cyclopropylphenyl)-N-(3-(methoxymethyl)-1-(5-(trifluoromethyl) pyridin-3-yl) pyrrolidin-3-yl) acetamide,
   (1S,2S)-2-(4-cyclopropylphenyl)-N-((R)-1-(5-(trifluoromethyl) pyridin-3-yl) pyrrolidin-3-yl) cyclopropane-1-carboxamide,
   (1R,2R)-2-(4-cyclopropylphenyl)-N-((R)-1-(5-(trifluoromethyl) pyridin-3-yl) pyrrolidin-3-yl) cyclopropane-1-carboxamide,
   (1S,2S)-2-(3,4-difluorophenyl)-N-((R)-1-(5-(trifluoromethyl) pyridin-3-yl) pyrrolidin-3-yl) cyclopropane-1-carboxamide,
   (1R,2R)-2-(3,4-difluorophenyl)-N-((R)-1-(5-(trifluoromethyl) pyridin-3-yl) pyrrolidin-3-yl) cyclopropane-1-carboxamide,
   (1S,2S)-2-(4-chlorophenyl)-N-((R)-1-(5-(trifluoromethyl) pyridin-3-yl) pyrrolidin-3-yl) cyclopropane-1-carboxyamide,
   (1R,2R)-2-(4-chlorophenyl)-N-((R)-1-(5-(trifluoromethyl) pyridin-3-yl) pyrrolidin-3-yl) cyclopropane-1-carboxyamide,
   2-(4-isopropylphenyl)-N-(1-(5-(trifluoromethyl) pyridin-3-yl) piperidin-4-yl) acetamide,
   (R)-2-(4-isopropylphenyl)-N-(1-(6-methoxy-5-(trifluoromethyl) pyridin-3-yl) pyrrolidin-3-yl) acetamide, (R)-2-(4-isopropylphenyl)-N-(1-(5-(trifluoromethyl) pyrimidin-2-yl) pyrrolidin-3-yl) acetamide,
   (R)-2-(4-isopropylphenyl)-N-(1-(4-(trifluoromethyl) thiazol-2-yl) pyrrolidin-3-yl) acetamide,
   (R)-2-(4-isopropylphenyl)-N-(1-(6-(trifluoromethyl) pyridin-3-yl) pyrrolidin-3-yl) acetamide,
   (S)-2-(4-isopropylphenyl)-N-(1-(5-(trifluoromethyl) pyridin-3-yl) pyrrolidin-3-yl) acetamide,
   (R)-2-(4-(trifluoromethoxy) phenyl)-N-(1-(5-(trifluoromethyl) pyridin-3-yl) pyrrolidin-3-yl) acetamide,
   (R)-2-(4-(2,2,2-trifluoroethoxy) phenyl)-N-(1-(5-(trifluoromethyl) pyridin-3-yl) pyrrolidin-3-yl) acetamide,
   2-(4-isopropylphenyl)-N-(1-(5-(trifluoromethyl) pyridin-3-yl) azetidin-3-yl) acetamide,
   2-(4-isopropylphenyl)-N-(1-(6-(trifluoromethyl) pyridin-3-yl) azetidin-3-yl) acetamide,
   (R)-2-(3,5-dichlorophenyl)-N-(1-(5-(trifluoromethyl) pyridin-3-yl) pyrrolidin-3-yl) acetamide,
   (R)-2-(3-chloro-5-fluorophenyl)-N-(1-(5-(trifluoromethyl) pyridin-3-yl) pyrrolidin-3-yl) acetamide,
   (R)-2-(4-cyclopropylphenyl)-N-(1-(6-(trifluoromethyl) pyridin-3-yl) pyrrolidin-3-yl) acetamide,
   (R)-2-(4-isopropylphenyl)-N-(1-(5-(trifluoromethyl) thiophen-2-yl) pyrrolidin-3-yl) acetamide,
   (R)-2-(4-(trifluoromethyl) phenyl)-N-(1-(5-(trifluoromethyl) pyridin-3-yl) pyrrolidin-3-yl) acetamide,
   (R)-2-(1H-indol-6-yl)-N-(1-(5-(trifluoromethyl) pyridin-3-yl) pyrrolidin-3-yl) acetamide,
   (R)-2-(4-cyclopropylphenyl)-N-(1-(6-(2,2,2-trifluoroethoxy) pyridin-3-yl) pyrrolidin-3-yl) acetamide,
   (R)-2-(4-cyclopropylphenyl)-N-(1-(5-(2,2,2-trifluoroethoxy) pyridin-3-yl) pyrrolidin-3-yl) acetamide,
   (R)-2-(4-cyclopropylphenyl)-N-(1-(4-(2,2,2-trifluoroethoxy) pyridin-2-yl) pyrrolidin-3-yl) acetamide,
   (R)-2-(4-cyclopropylphenyl)-N-(1-(2-methoxy-5-(trifluoromethyl) pyridin-3-yl) pyrrolidin-3-yl) acetamide,
   (R)-2-(4-cyclopropylphenyl)-N-(1-(5-(trifluoromethyl) pyridin-3-yl) piperidin-3-yl) acetamide,
   (R)-2-(4-cyclopropylphenyl)-N-(1-(6-(trifluoromethyl) pyridin-3-yl) piperidin-3-yl) acetamide,
   (R)-2-(4-(2-hydroxypropan-2-yl) phenyl)-N-(1-(5-(trifluoromethyl) pyridin-3-yl) pyrrolidin-3-yl) acetamide,
   (R)-2-(4-(3-methylpyrazin-2-yl) phenyl)-N-(1-(5-(trifluoromethyl) pyridin-3-yl) pyrrolidin-3-yl) acetamide,
   (R)-2-(4-(4-methyloxazol-5-yl) phenyl)-N-(1-(5-(trifluoromethyl) pyridin-3-yl) pyrrolidin-3-yl) acetamide,
   (R)-3-(3-(2-(4-cyclopropylphenyl) acetamide) pyrrolidin-1-yl)-5-(trifluoromethyl) pyridine 1-oxide,
   (3R)-3-(2-(4-cyclopropylphenyl) acetamide)-1-(5-(trifluoromethyl) pyridin-3-yl) pyrrolidine 1-oxide,
   (R)-2-(4-cyclopropylphenyl)-2-methyl-N-(1-(5-(trifluoromethyl) pyridin-3-yl) pyrrolidin-3-yl) propanamide,
   (R)-2-(4-cyclopropylphenyl)-N-(1-(3-fluoro-5-(trifluoromethyl) phenyl) pyrrolidin-3-yl) acetamide,
   (R)-2-amino-2-(4-cyclopropylphenyl)-N-((R)-1-(5-(trifluoromethyl) pyridin-3-yl) pyrrolidin-3-yl) acetamide,
   (S)-2-amino-2-(4-cyclopropylphenyl)-N-((R)-1-(5-(trifluoromethyl) pyridin-3-yl) pyrrolidin-3-yl) acetamide,
   (R)-2-(4-cyclopropylphenyl)-2-(dimethylamino)-N-((R)-1-(5-(trifluoromethyl) pyridin-3-yl) pyrrolidin-3-yl) acetamide,
   (S)-2-(4-cyclopropylphenyl)-2-(dimethylamino)-N-((R)-1-(5-(trifluoromethyl) pyridin-3-yl) pyrrolidin-3-yl) acetamide,
   (R)-2-(1-methyl-1H-indol-5-yl)-N-(1-(5-(trifluoromethyl) pyridin-3-yl) pyrrolidin-3-yl) acetamide,
   (R)-2-(2,4-bis(trifluoromethyl) phenyl)-N-(1-(5-(trifluoromethyl) pyridin-3-yl) pyrrolidin-3-yl) acetamide,
   (R)-2-(2-fluoro-4-(trifluoromethyl) phenyl)-N-(1-(5-(trifluoromethyl) pyridin-3-yl) pyrrolidin-3-yl) acetamide,
   (R)-2-(1-methyl-1H-indazol-5-yl)-N-(1-(5-(trifluoromethyl) pyridin-3-yl) pyrrolidin-3-yl) acetamide,
   (R)-2-(1-methyl-1H-indazol-5-yl)-N-(1-(4-(trifluoromethyl) thiazol-2-yl) pyrrolidin-3-yl) acetamide,
   (R)-2-(1-methyl-1H-indazol-5-yl)-N-(1-(6-(trifluoromethyl) pyridin-3-yl) pyrrolidin-3-yl) acetamide,
   (R)-2-(4-(2,2-dimethylmorpholino) phenyl)-N-(1-(5-(trifluoromethyl) pyridin-3-yl) pyrrolidin-3-yl) acetamide,
   (R)-2-(4-(1H-pyrazol-1-yl) phenyl)-N-(1-(5-(trifluoromethyl) pyridin-3-yl) pyrrolidin-3-yl) acetamide,
   (R)-N-(3-methyl-1-(5-(trifluoromethyl) pyridin-3-yl) pyrrolidin-3-yl)-2-(4-morpholinophenyl) acetamide,
   (R)-2-(4-cyclopropylphenyl)-N-(3-methyl-1-(5-(trifluoromethyl) pyridin-3-yl) pyrrolidin-3-yl) acetamide,
   2-(4-(3-oxa-8-azabicyclo[3.2.1]octan-8-yl) phenyl)-N-((R)-1-(5-(trifluoromethyl) pyridin-3-yl) pyrrolidin-3-yl) acetamide,
   2-(4-(3-oxa-8-azabicyclo[3.2.1]octan-8-yl) phenyl)-N-((R)-1-(6-(trifluoromethyl) pyridin-3-yl) pyrrolidin-3-yl) acetamide,
   (R)-2-(benzo[d]oxazol-5-yl)-N-(1-(6-(trifluoromethyl) pyridin-3-yl) pyrrolidin-3-yl) acetamide,
   (R)-2-(benzo[d]oxazol-5-yl)-N-(1-(5-(trifluoromethyl) pyridin-3-yl) pyrrolidin-3-yl) acetamide,
   (R)-2-(2-methylbenzo[d]oxazol-5-yl)-N-(1-(5-(trifluoromethyl) pyridin-3-yl) pyrrolidin-3-yl) acetamide,
   (R)-2-(2-methylbenzo[d]oxazol-5-yl)-N-(1-(6-(trifluoromethyl) pyridin-3-yl) pyrrolidin-3-yl) acetamide,
   2-(4-((1S,4S)-2-oxa-5-azabicyclo[2.2.2]octan-5-yl) phenyl)-N-((R)-1-(5-(trifluoromethyl)) pyridin-3-yl) pyrrolidin-3-yl) acetamide,
   2-(4-((1S,4S)-2-oxa-5-azabicyclo[2.2.2]octan-5-yl) phenyl)-N-((R)-1-(6-(trifluoromethyl)) pyridin-3-yl) pyrrolidin-3-yl) acetamide,
   (R)-2-(benzo[d]thiazol-6-yl)-N-(1-(5-(trifluoromethyl) pyridin-3-yl) pyrrolidin-3-yl) acetamide,
   (R)-2-(benzo[d]thiazol-6-yl)-N-(1-(6-(trifluoromethyl) pyridin-3-yl) pyrrolidin-3-yl) acetamide,
   (R)-2-(1H-indazol-5-yl)-N-(1-(5-(trifluoromethyl) pyridin-3-yl) pyrrolidin-3-yl) acetamide,
   (R)-2-(1H-indazol-5-yl)-N-(1-(6-(trifluoromethyl) pyridin-3-yl) pyrrolidin-3-yl) acetamide,
   2-(4-cyclopropylphenyl)-N-(3-(trifluoromethyl)-1-(5-(trifluoromethyl) pyridin-3-yl) pyrrolidin-3-yl) acetamide,
   (R)-N-(3-(trifluoromethyl)-1-(5-(trifluoromethyl) pyridin-3-yl) pyrrolidin-3-yl)-2-(4-(trifluoromethyl) phenyl) acetamide,
   (S)-N-(3-(trifluoromethyl)-1-(5-(trifluoromethyl) pyridin-3-yl) pyrrolidin-3-yl)-2-(4-(trifluoromethyl) phenyl) acetamide,
   (R)-2-(1H-benzo[d][1,2,3]triazol-5-yl)-N-(1-(5-(trifluoromethyl) pyridin-3-yl) pyrrolidin-3-yl) acetamide,
   (R)-2-(1H-benzo[d][1,2,3]triazol-5-yl)-N-(1-(6-(trifluoromethyl) pyridin-3-yl) pyrrolidin-3-yl) acetamide,
   2-(4-((1R,4R)-2-oxa-5-azabicyclo[2.2.1]heptan-5-yl) phenyl)-N-((R)-1-(5-(trifluoromethyl)) pyridin-3-yl) pyrrolidin-3-yl) acetamide,
   2-(4-((1S,4S)-2-oxa-5-azabicyclo[2.2.1]heptan-5-yl) phenyl)-N-((R)-1-(5-(trifluoromethyl)) pyridin-3-yl) pyrrolidin-3-yl) acetamide,
   2-(4-((1R,4R)-2-oxa-5-azabicyclo[2.2.1]heptan-5-yl) phenyl)-N-((R)-1-(6-(trifluoromethyl)) pyridin-3-yl) pyrrolidin-3-yl) acetamide,
   2-(4-((1S,4S)-2-oxa-5-azabicyclo[2.2.1]heptan-5-yl) phenyl)-N-((R)-1-(6-(trifluoromethyl)) pyridin-3-yl) pyrrolidin-3-yl) acetamide,
   (R)-2-(1-methyl-1H-benzo[d][1,2,3]triazol-5-yl)-N-(1-(5-(trifluoromethyl) pyridin-3-yl) pyrrolidin-3-yl) acetamide,
   (R)-2-(1-methyl-1H-benzo[d] [1,2,3]triazol-5-yl)-N-(1-(6-(trifluoromethyl) pyridin-3-yl) pyrrolidin-3-yl) acetamide,
   2-(4-cyclopropylphenyl)-N-(1-(4-(trifluoromethyl) phenyl) azetidin-3-yl) acetamide,
   2-(4-cyclopropylphenyl)-N-(1-(4-fluoro-3-(trifluoromethyl) phenyl) azetidin-3-yl) acetamide,
   (R)-2-(4-bromophenyl)-N-(1-(5-(trifluoromethyl) pyridin-3-yl) pyrrolidin-3-yl) acetamide,
   (R)-2-(4-cyclopropylphenyl)-N-(1-(6-fluorobenzo[d]thiazol-2-yl) pyrrolidin-3-yl) acetamide,
   (R)-N-(1-(5-bromothiazol-2-yl) pyrrolidin-3-yl)-2-(4-cyclopropylphenyl) acetamide,
   (R)-2-(4-cyclopropylphenyl)-N-(1-(4-fluoro-3-(trifluoromethyl) phenyl) pyrrolidin-3-yl) acetamide,
   (R)-2-(4-cyclopropylphenyl)-N-(1-(3-methoxyphenyl) pyrrolidin-3-yl) acetamide,
   (R)-2-(4-(3,3-difluoropyrrolidin-1-yl) phenyl)-N-(1-(5-(trifluoromethyl) pyridin-3-yl) pyrrolidin-3-yl) acetamide,
   ethyl (R)-2-(3-(3-(2-(4-cyclopropylphenyl)acetamido)pyrrolidin-1-yl)phenoxy)-2-methylpropanoate,
   (R)-2-(4-cyclopropylphenyl)-N-(1-(3-hydroxyphenyl)pyrrolidin-3-yl) acetamide,
   (R)-2-(4-cyclopropylphenyl)-N-(1-(6-(trifluoromethyl)pyridin-2-yl)pyrrolidin-3-yl)acetamide,
   (R)-2-(4-cyclopropylphenyl)-N-(1-(4,6-dimethoxypyrimidin-2-yl)pyrrolidin-3-yl) acetamide,
   (R)-2-(4-morpholinophenyl)-N-(1-(5-(trifluoromethyl)pyridin-3-yl)pyrrolidin-3-yl)acetamide,
   (R)-2-(4-cyclopropylphenyl)-N-(1-(4-(trifluoromethyl)pyridin-2-yl)pyrrolidin-3-yl)acetamide,
   (R)-2-(4-trifluoromethyl) phenyl-N-(1-(6-(trifluoromethyl)pyridin-3-yl)pyrrolidin-3-yl)acetamide,
   (R)-N-(1-(4-fluoro-3-(trifluoromethyl)phenyl)pyrrolidin-3-yl)-2-(4-(trifluoromethyl)phenyl)acetamide,
   (R)-2-(4-(trifluoromethyl)phenyl)-N-((R)-1-(6-(trifluoromethyl)pyridin-3-yl)pyrrolidin-3-yl) propanamide,
   (S)-2-(4-(trifluoromethyl)phenyl)-N-((R)-1-(6-(trifluoromethyl)pyridin-3-yl)pyrrolidin-3-yl) propanamide,
   (1S,2S)-2-(1H-benzo[d]imidazol-2-yl)-N-((R)-1-(5-(trifluoromethyl)pyridin-3-yl) piperidin-3-yl)cyclopropane-1-carboxyamide,
   (R)-2-(1H-indol-6-yl)-N-(1-(4-(trifluoromethyl) thiazol-2-yl)pyrrolidin-3-yl)acetamide, and
   (R)-2-(1-methyl-1H-indol-5-yl)-N-(1-(6-(trifluoromethyl)pyridin-3-yl)pyrrolidin-3-yl)acetamide.
(74) The medicament according to any one of (1) to (73) above, in which the pruritus is histaminergic pruritus.
(75) The medicament according to any one of (1) to (73) above, in which the pruritus is non-histaminergic pruritus.
(76) Use of the compound according to any one of (1) to (73) above, a tautomer of the compound, a stereoisomer of the compound, a pharmaceutically acceptable salt thereof, or a solvate thereof for producing a medicament for preventing or treating pruritus.
(77) The use according to (76) above, in which the pruritus is histaminergic pruritus.
(78) The use according to (76) above, in which the pruritus is non-histaminergic pruritus.
(79) The compound according to any one of (1) to (73) above, a tautomer of the compound, a stereoisomer of the compound, a pharmaceutically acceptable salt thereof, or a solvate thereof, used for preventing or treating pruritus.
(80) Use of the compound according to any one of (1) to (73) above, a tautomer of the compound, a stereoisomer of the compound, a pharmaceutically acceptable salt thereof, or a solvate thereof, used for preventing or treating histaminergic pruritus.
(81) Use of the compound according to any one of (1) to (73) above, a tautomer of the compound, a stereoisomer of the compound, a pharmaceutically acceptable salt thereof, or a solvate thereof, used for preventing or treating non-histaminergic pruritus.
(82) A method for treating pruritus in a human, the method including a step of administering an effective amount of the compound according to any one of (1) to (73) above, a tautomer of the compound, a stereoisomer of the compound, a pharmaceutically acceptable salt thereof, or a solvate thereof to a subject in need thereof.
(83) The method according to (82) above, in which the pruritus is histaminergic pruritus.
(84) The method according to (82) above, in which the pruritus is non-histaminergic pruritus.

### Advantageous Effects of Invention

The compound of the present invention blocks Cav3.2 channels and has an effect of reducing pruritus.

### Brief Description of Drawings

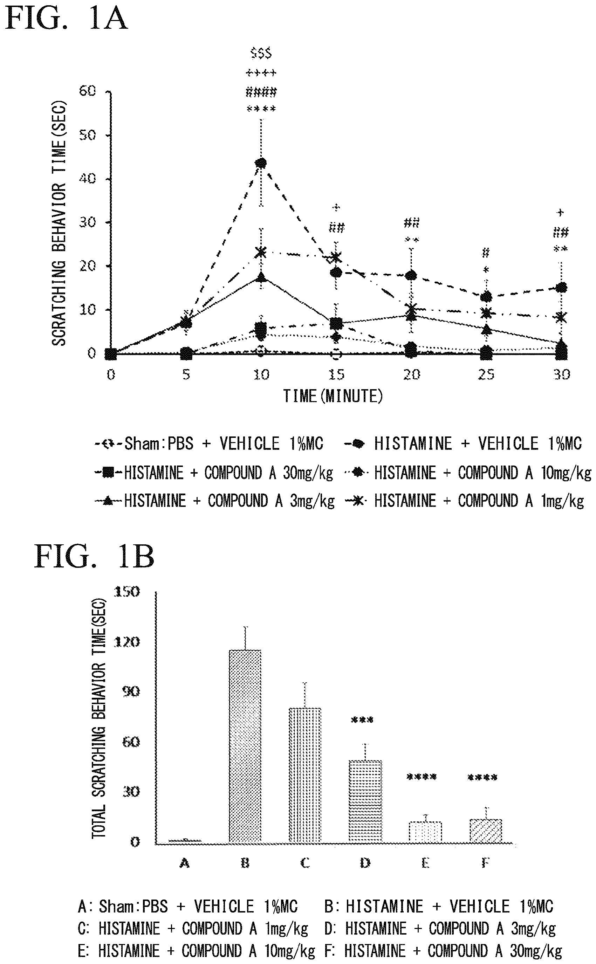

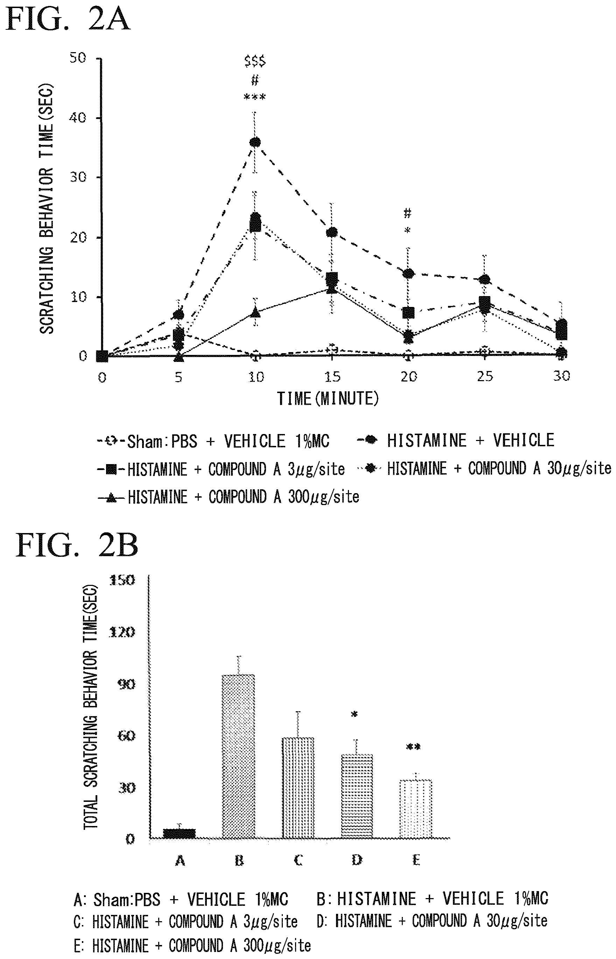

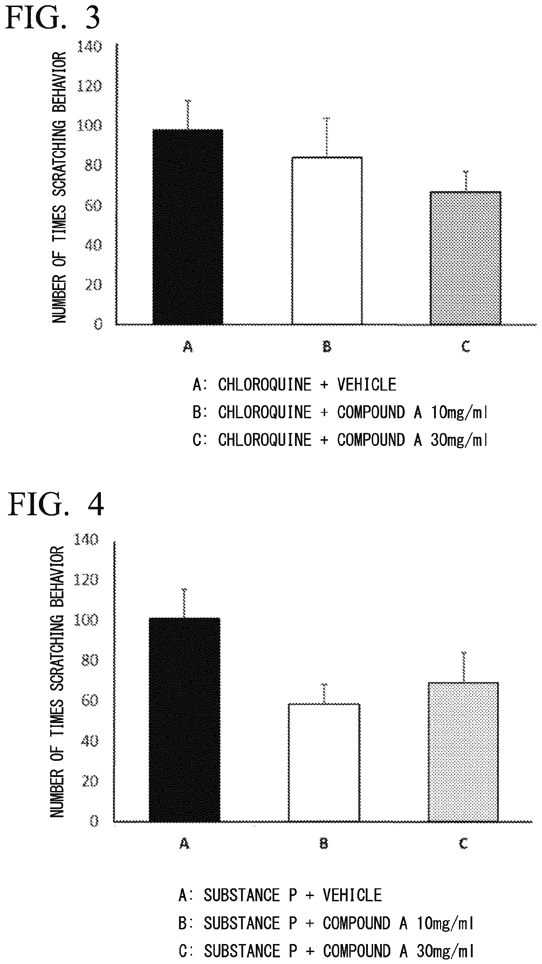

Fig. 1A is a graph illustrating results of pharmacological test 2 and illustrates scratching time every five minutes for each administration. After analysis with two-way ANOVA, a significant difference test was performed by a Bonferroni test. *: p < 0.05, **: p < 0.01, ****: p < 0.0001, histamine + compound A 30 mg/kg vs histamine + vehicle, #: p < 0.05, ##: p < 0.01, ####: p < 0.0001, histamine + compound A 10 mg/kg vs histamine + vehicle, +: p < 0.05, ++++: p < 0.0001, histamine + compound A 3 mg/kg vs histamine + vehicle, $$$: p < 0.01, histamine + compound A 1 mg/kg vs histamine + vehicle
Fig. 1B is a graph illustrating results of pharmacological test 2 and illustrates total scratching time in 30 minutes for each administration. After one-way ANOVA analysis, a significant difference test was performed by a Dunnett's test. ***: p < 0.001, ****: p < 0.0001, histamine + vehicle
Fig. 2A is a graph illustrating results of pharmacological test 3 and illustrates scratching time every five minutes for each administration. After analysis with two-way ANOVA, a significant difference test was performed by a Bonferroni test. $$: p < 0.05, histamine + compound A 3 µg/site vs histamine + vehicle, #: p < 0.05, histamine + compound A 30 µg/site vs histamine + vehicle, *: p < 0.05, ***: p < 0.001, histamine + compound A 300 µg/site vs histamine + vehicle
Fig. 2B is a graph illustrating results of pharmacological test 3 and illustrates total scratching time in 30 minutes for each administration. After one-way ANOVA analysis, a significant difference test was performed by a Dunnett's test. *: p < 0.05, **: p < 0.01, histamine + compound A vs histamine + vehicle
Fig. 3 is a graph illustrating results of pharmacological test 4 and illustrates the number of scratching behaviors in 30 seconds for each administration.
Fig. 4 is a graph illustrating results of pharmacological test 5 and illustrates the number of scratching behaviors in 30 seconds for each administration.

### Description of Embodiments

Next, the present invention will be described in more detail.

According to the present specification, the C₁-₈ alkyl group may be a linear, branched, or cyclic alkyl group such as a methyl group, an ethyl group, a propyl group, an isopropyl group, a butyl group, a cyclopropyl group, a cyclopentyl group, a cyclohexyl group, an isobutyl group, a tert-butyl group, a pentyl group, or a hexyl group.

The C₁-₈ alkyl group substituted with one to three halogen atoms may be a methyl group, an ethyl group, a propyl group, an isopropyl group, a butyl group, a tert-butyl group, or the like, each group being substituted with one to three of a halogen atom such as a fluorine atom, a chlorine atom, or a bromine atom, and preferred examples include a trifluoromethyl group, a chloromethyl group, a 2-chloroethyl group, a 2-bromoethyl group, a 2-fluoroethyl group, and the like.

The C₁-₈ alkoxy group may be a methoxy group, an ethoxy group, a propoxy group, an isopropoxy group, a butoxy group, an isobutoxy group, a tert-butoxy group, a pentyloxy group, a hexyloxy group, or the like.

The C₁-₈ alkoxy group substituted with one to three halogen atoms may be a methoxy group, an ethoxy group, a propoxy group, an isopropoxy group, a butoxy group, a tert-butoxy group, or the like, each group being substituted with one to three of a halogen atom such as a fluorine atom, a chlorine atom, or a bromine atom, and preferred examples include a trifluoromethoxy group, a chloromethoxy group, a 2-chloroethoxy group, a 2-bromoethoxy group, a 2-fluoroethoxy group, a 2,2,2-trifluoroethoxy group, and the like.

The halogen atom may be a fluorine atom, a chlorine atom, a bromine atom, an iodine atom, or the like.

The C₁-₈ alkoxycarbonyl group may be a methoxycarbonyl group, an ethoxycarbonyl group, a butoxycarbonyl group, a tert-butoxycarbonyl group, or the like.

The acyl group may be preferably an acyl group having 1 to 7 carbon atoms, and more preferred examples include an acetyl group, a propionyl group, a benzoyl group, and the like.

The C₁₋₈ alkyl group substituted with a hydroxy group may be a hydroxymethyl group, a 2-hydroxypropan-2-yl, or the like.

The C₁₋₈ alkyl group substituted with an acyloxy group may be an acetyloxymethyl group, or the like.

The C₁₋₈ alkoxy group substituted with a hydroxy group may be a 2-hydroxyethoxy group, or the like.

The C₁₋₈ alkylsulfonyl group may be a methanesulfonyl group or the like.

The (C₁₋₈ alkoxy-substituted C₁₋₈ alkyl) amino group may be a (2-methoxyethyl)amino group, or the like.

The 3-membered to 5-membered cycloalkyl group may be a cyclopropyl group, a cyclobutyl group, or the like.

The C₁₋₈ alkylamino group may be an ethylamino group, or the like.

The C₂₋₁₂ dialkylamino group may be a dimethylamino group, a diethylamino group, or the like.

The (C₁₋₈ alkyl) (C₁₋₈ alkoxy-substituted C₁₋₈ alkyl)amino group may be an N-ethyl-N-(2-methoxyethyl)amino group, or the like.

The tri (C₁₋₈ alkyl) silyl group may be a trimethylsilyl group, a triethylsilyl group, or the like. The acylamino group may be an acetylamino group, or the like.

The (N-acyl) (N-C₁₋₈ alkyl)amino group may be an (N-acetyl)(N-ethyl)amino group, or the like.

The 3-membered to 6-membered cyclic ether may be THF, oxetane, or the like.

The C₁₋₈ alkyl group substituted with a C₁₋₈ alkoxy group may be a linear, branched, or cyclic alkyl group such as a methyl group, an ethyl group, a propyl group, an isopropyl group, a butyl group, a cyclopropyl group, a cyclopentyl group, a cyclohexyl group, an isobutyl group, a tert-butyl group, a pentyl group, or a hexyl group, each group being substituted with an alkoxy group having 1 to 6 carbon atoms, such as a methoxy group, an ethoxy group, a propoxy group, an isopropoxy group, a butoxy group, an isobutoxy group, a tert-butoxy group, a pentyloxy group, or a hexyloxy group.

The C₁₋₈ alkoxy group substituted with a C₁₋₈ alkoxycarbonyl group may be an isopropyloxy group substituted with ethoxycarbonyl, or the like.

The "tautomer" includes interconversion by proton transfer, such as keto-enol isomerization or imine-enamine isomerization.

The "stereoisomers" refer to compounds which have the same chemical structure but are different in terms of spatial arrangement of atoms or groups. For example, stereoisomers such as cis/trans isomers, optically active substances, and racemates may be present; however, all of these are included in the present invention, and mixtures of enantiomers or diastereomers are also included in the present invention.

Furthermore, the compounds of the present invention also include a compound in which the pyridine in Example 134 that will be described below is pyridine 1-oxide, a compound in which the pyridine is pyrrolidine 1-oxide or piperidine 1-oxide, and the like.

As the "pharmacologically acceptable salt" of the compound represented by General Formula (I), for example, a mineral acid salt of hydrochloric acid, sulfuric acid, nitric acid, or phosphoric acid, and an organic acid salt of formic acid, acetic acid, citric acid, tartaric acid, methanesulfonic acid, p-toluenesulfonic acid, oxalic acid, or malic acid can be used as an acid addition salt, but are not limited thereto. Examples of a base addition salt include a salt with an inorganic base such as a lithium salt, a sodium salt, a potassium salt, a magnesium salt, or a calcium salt, an ammonium salt, and an addition salt with an organic base such as a triethylamine salt or an ethanolamine salt, but are not limited thereto.

The "solvate" means a form of a molecular complex containing the compound of the present invention and one or more pharmaceutically acceptable solvent molecules of a chemical quantity, and examples of the solvent molecule include an alcohol such as ethanol and water. When the solvent molecule is water, the solvate may be called a hydrate, and examples of the hydrate include a monohydrate and a dihydrate.

The compound of the present invention also includes a prodrug and a derivative substituted with a stable isotope. Examples of the isotope include hydrogen, carbon, nitrogen, oxygen, phosphorus, sulfur, fluorine, and chlorine, such as ²H, ³H, ¹³C, ¹⁴C, ¹⁵N, ¹⁸O, ¹⁷O ³¹P, ³²P ³⁵S, ¹⁸F, or ³⁶Cl.

"Pruritus" is an unpleasant sensation that makes you want to scratch the skin or mucous membranes, and is roughly divided into pruritus mediated by histamine and pruritus not mediated by histamine (refractory pruritus). Examples of the pruritus mediated by histamine include pruritus caused by a skin disease, such as seborrheic dermatitis, self-sensitizing dermatitis, caterpillar dermatitis, insect bites, Photosensitivity disorder, prurigo, herpes, impetigo, eczema, ringworm, lichen, scabies, or acne vulgaris.

Meanwhile, examples of the refractory pruritus not mediated by histamine include pruritus associated with atopic dermatitis, psoriasis, bile stagnation, uremia, allergic conjunctivitis, acute conjunctivitis, or chronic conjunctivitis, and pruritus associated with hemodialysis.

As an endogenous substance that induces pruritus, histamine, serotonin, substance P, and the like are known. Histamine is a classic itch factor released mainly from mast cells. It is known that substance P acts on an NK-1 receptor on mast cells to induce degranulation, and also releases another pruritus-inducing substance or an itchenhancing substance via the NK1 receptor on keratinocyte, or acts on the C fibers to cause pruritus.

The "histaminergic pruritus" means pruritus mediated by histamine, and is generally pruritus that can be treated with an antihistamine agent. Meanwhile, the "non-histaminergic pruritus" means pruritus not mediated by histamine, and includes refractory pruritus. This is pruritus resistant to an antihistamine agent.

When the compound of the present invention, a tautomer of the compound, a stereoisomer of the compound, a pharmaceutically acceptable salt thereof, or a solvate thereof is used as a medicament, the compound, a stereoisomer of the compound, a pharmaceutically acceptable salt thereof, or a solvate thereof itself can be formulated, or can be mixed with an appropriate pharmaceutically acceptable carrier and formulated. Here, examples of the pharmaceutically acceptable carrier include a diluent, a binder, an excipient, a lubricant, a disintegrant, a wetting agent, an emulsifier, a surfactant, an antioxidant, a preservative, a colorant, a flavoring agent, and an odorant.

Examples of a dosage form include an oral preparation such as a powder, a granule, a tablet, or a capsule, an external preparation such as an eye drop, a nasal drop, a coating agent, a patch, a spray, a gel, or a cream. The medicament can be administered orally or parenterally.

A dose of the medicament of the present invention is not particularly limited, and can be appropriately increased or decreased according to age, sex, body weight, symptoms, therapeutic effect, administration method, type of substance or salt, sensitivity to medicament, therapeutic effect, and the like. However, in general, for an adult, in a case of an oral preparation, the medicament is administered one to six times a day in a range of 0.01 mg to 1000 mg, preferably 0.1 mg to 500 mg, more preferably 0.1 mg to 300 mg at a time. In a case of an external preparation, the medicament is used one to six times a day in a range of 0.01 mg to 2000 mg, preferably 0.05 mg to 1000 mg, more preferably 0.1 mg to 500 mg. In a case of an injection, the medicament is used one to six times a day in a range of 0.001 mg to 1000 mg, preferably 0.1 mg to 100 mg, more preferably 0.1 mg to 50 mg at a time.

### General synthesis method

The compounds of the present invention can be each produced using commercially available compounds as raw materials and using any known method or the method described below. Examples of the known method include the methods described in Lectures on Experimental Chemistry, 5th Edition (Maruzen Publishing Co., Ltd.), New Edition Heterocyclic Compounds (KODANSHA LTD.), Protective Groups in Organic Synthesis (Wiley), and the like.

Depending on the compounds produced using the present production method, protection or deprotection and conversion or introduction of functional groups may be effective in the various stages of production. In such a case, the operation or procedure is not limited to the operation or procedure of the described production method, and any appropriate operation or procedure can be applied using known methods.

A prodrug of a compound of the present invention can be produced by applying any known method such as amidation, esterification, or alkylation in the various stages of production.

Depending on the compound produced using the present production method, various salts, hydrates, and crystal polymorphisms may be included. Furthermore, in a case in which an optical isomer, a geometric isomer, or a tautomer may exist, unless particularly limited, a mixture at any ratio may be included. A mixture of these isomers can be separated by any known method.

A method for producing the compounds of the present invention will be described below; however, the method for producing the compound of the present invention is not limited to the following method.

In the present specification, the following abbreviations may be used.

M: molar concentration, N: normality, MS: mass spectrum, [M+H]⁺: protonated molecule ion peak, [M+Na]⁺: sodium ion addition molecule ion peak, [M-H]⁻: deprotonated molecule Ion peak, CDCl₃: deuterated chloroform, DMSO-d₆: deuterated dimethyl sulfoxide, CD₃OD: deuterated methanol, ¹H NMR: proton nuclear magnetic resonance, Me: methyl group, Et: ethyl group, t-Bu: tert-butyl group, CN: cyano group, CF₃: trifluoromethyl group, Ts: p-toluenesulfonyl group, Boc: tert-butoxycarbonyl group, DMF: N,N-dimethylformamide, THF: tetrahydrofuran, DME: 1,2-dimethoxy ethane, HATU: 0-(7-azabenzotriazol-1-yl)-N,N,N',N'-tetramethyluronium hexafluorophosphate, HOBT: 1-hydroxybenzotriazole, WSC: 1-ethyl-3-(dimethylaminopropyl) carbodiimide, DMT-MM: 4-(4,6-dimethoxy-1,3,5,-triazin-2-yl)-4-methylmorpholinium chloride, DMAP: N,N-dimethyl-4-aminopyridine, DIBAL-H: diisobutylaluminum hydride, L-selectride: tri(sec-butyl) boron lithium hydride, DIPEA: N,N-diisopropylethylamine, BINAP: 2,2'-bis(diphenylphosphino)-1,1'-binaphthyl, DMSO: dimethyl sulfoxide, FBS: fetal bovine serum, DMEM: Dulbecco's modified Eagle medium, CO₂: carbon dioxide, NaCl: sodium chloride, KCl: potassium chloride, MgCl₂: magnesium chloride, CaCl₂: calcium chloride, CsCl: cesium chloride, CsF: cesium fluoride, HEPES: 4-(2-hydroxyethyl)-1-piperazine ethanesulfonic acid, EGTA: glycol etherdiamine 4 acetate

A method for producing a compound represented by General Formula (Z) is described below.

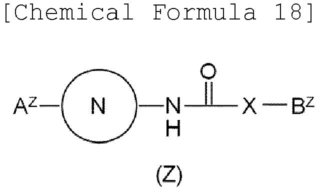

In the general formula, A^{Z} represents, for example, A¹ in the compound represented by General Formula (I) or
the following in the compound represented by General Formula (II):

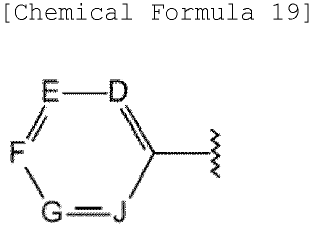

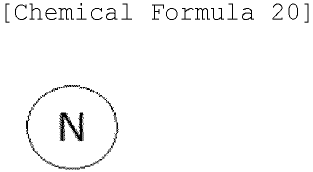

The above represents, for example, the following in the compound represented by General Formula (I): 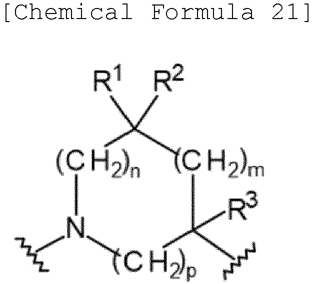 or the following in the compound represented by General Formula (II):

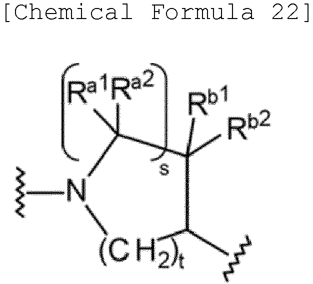

X represents the following in the compound represented by General Formula (I) or the compound represented by General Formula (II): 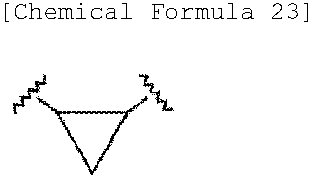 or 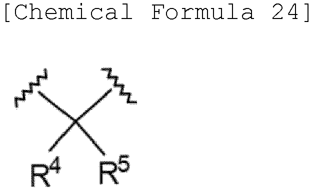 and B^{Z} represents, for example, B¹ in the compound represented by General Formula (I) or B² in the compound represented by General Formula (VI).

Compounds represented by General Formulas (III) to (VI) and the like can also be produced in a similar manner.

Method for producing compound (Z)

The above-described compound (Z) can be produced by, for example, the method illustrated below.

### (Production method 1)

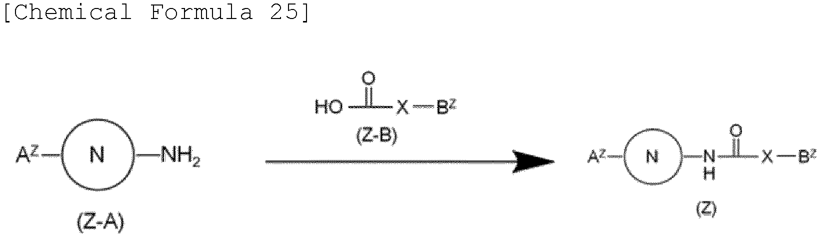

wherein the reference symbols have the same meanings as described above.

Compound (Z) can be produced by causing compound (Z-A) to react with compound (Z-B) in an appropriate solvent such as DMF in the presence of a condensing agent such as HATU or WSC, and if necessary, in the presence of an additive such as HOBT or DMAP and if necessary, a base such as triethylamine or DIPEA, at 0°C to 100°C.

Furthermore, the compound can be produced by causing compound (Z-A) to react with a carboxylic acid chloride or carboxylic acid anhydride corresponding to compound (Z-B) in an appropriate solvent such as tetrahydrofuran, and if necessary, in the presence of a base such as triethylamine, DIPEA, or pyridine, at 0°C to 100°C

In addition to that, the compound can be produced from compound (Z-A) and compound (Z-B), or compounds respectively equivalent thereto, using the condensation reaction described in Christian A.G.N. Montalbetti, et al, Tetrahedron, 61(46), 2005, 10827-10852, or a condensation reaction equivalent thereto.

Compound (Z-A) can be produced by, for example, the method described below. 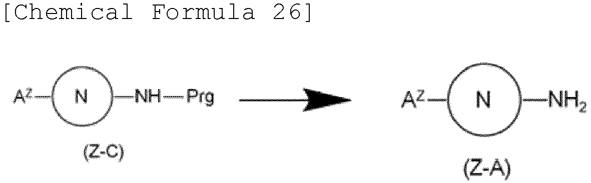 wherein Prg means a protective group and represents any arbitrary functional group that can be converted to hydrogen by a deprotection reaction; the other reference symbols have the same meanings as described above.

Compound (Z-A) can be produced by causing compound (Z-C) to react with an acid such as hydrogen chloride or trifluoroacetic acid at -10°C to 100°C in an appropriate solvent such as methanol, or without solvent.

Furthermore, compound (Z-A) can be produced by subjecting compound (Z-C) to a hydrogenation reaction using a catalyst such as palladium-carbon or palladium hydroxide at 0°C to 100°C in an appropriate solvent such as methanol.

In addition to that, compound (Z-A) can be produced from compound (Z-C) or a compound equivalent thereto using a deprotection reaction for a protective group, which is described in Protective Groups in Organic Synthesis (Wiley) or the like, or a deprotection reaction equivalent thereto.

Compound (Z-C) can be produced by, for example, the method described below. 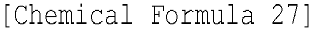 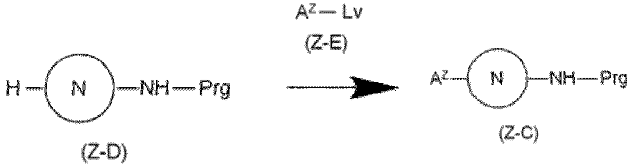 wherein Prg means a protective group and represents any arbitrary functional group that can be converted to hydrogen by a deprotection reaction; Lv represents a leaving group; and the other reference symbols have the same meanings as described above.

Compound (Z-C) can be produced by causing compound (Z-D) to react with compound (Z-E) in an appropriate solvent such as DMF in the presence of a metal catalyst such as tris(dibenzylidene acetone)dipalladium or copper iodide, and if necessary, in the presence of a ligand such as BINAP or ethylenediamine, and if necessary, a base such as triethylamine or DIPEA, at 0°C to 200°C. Regarding the leaving group, an appropriate functional group such as a halogen or a tosyl group is used.

In addition to that, compound (Z-C) can be produced by causing compound (Z-D) to react with compound (Z-E) in an appropriate solvent such as DMF, in the presence of a base such as sodium hydride or cesium carbonate, at 0°C to 200°C.

Furthermore, the compound can be produced from compound (Z-D) and compound (Z-E), or compounds respectively equivalent thereto, using reactions equivalent to these reactions.

Compound (Z-D) can be a commercially available product or can be produced from a commercially available product according to a known method. For example, the compound can be produced from appropriate starting raw materials by combining known methods according to the synthesis methods described in known patent literatures such as WO 2014/159969, WO 2012/154274, WO 2017/190609, and WO 2016/027195, or according to synthesis methods equivalent to those.

Compound (Z-E) can be a commercially available product or can be produced from a commercially available product according to a known method. For example, the compound can be produced from appropriate starting raw materials by combining known methods according to the synthesis methods described in a known academic literature, Cottet Fabrice, et al, Eur. J. Org. Chem. (2), 2002, 327-330, or known patent literatures such as WO 2013/088404 and WO 2010/064707, or according to synthesis methods equivalent to those.

Compound (Z-B) can be produced by, for example, the method described below. 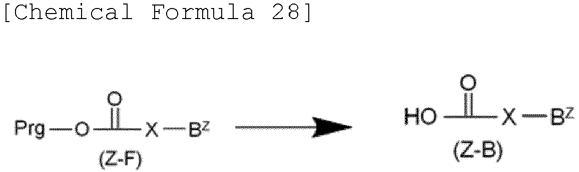 wherein Prg means a protective group and represents any arbitrary functional group that can be converted to hydrogen by a deprotection reaction; the other reference symbols have the same meanings as described above.

Compound (Z-B) can be produced from compound (Z-F) by causing the compound to react with a base such as lithium hydroxide or sodium hydroxide in an appropriate solvent such as a mixed solvent of water and methanol or tetrahydrofuran, at 0°C to 100°C.

In addition to that, the compound can be produced from compound (Z-F) or a compound equivalent thereto using a deprotection reaction for a protective group, which is described in Protective Groups in Organic Synthesis (Wiley) or the like, or a deprotection reaction equivalent thereto.

Compound (Z-F) can be a commercially available product or can be produced from a commercially available product according to a known method. For example, the compound can be produced from appropriate starting raw materials by combining known methods according to the synthesis methods described in known patent literatures such as WO 2017/122754, WO 2013/026914, and WO 2015/073528 and academic literatures such as Radoslaw Laufer, Bioorg. Med. Chem, 22(17), 2014, 4968-4997, or according to synthesis methods equivalent to those.

Furthermore, compound (Z-B) can be a commercially available product or can be produced from a commercially available product according to a known method. For example, the compound can be produced from appropriate starting raw materials by combining known methods according to the synthesis methods described in known patent literatures such as WO 2013/144295, WO 2014/010748, and WO 2008/125337, or according to synthesis methods equivalent to those.

### (Production method 2)

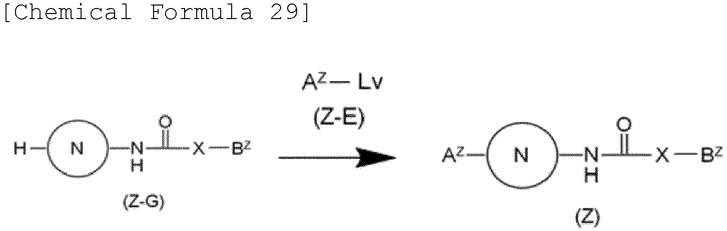

wherein Lv represents a leaving group; and the other reference symbols have the same meanings as described above.

Compound (Z) of the present invention can be produced by causing compound (Z-E) to react with compound (Z-G) in an appropriate solvent such as DMF in the presence of a metal catalyst such as tris(dibenzylidene acetone)dipalladium or copper iodide, and if necessary, in the presence of a ligand such as BINAP or ethylenediamine, and if necessary, a base such as triethylamine or DIPEA, at 0°C to 200°C. Regarding the leaving group, an appropriate functional group such as a halogen or a tosyl group is used.

In addition to that, compound (Z) can be produced by causing compound (Z-E) to react with compound (Z-G) in an appropriate solvent such as DMF in the presence of a base such as sodium hydride or cesium carbonate at 0°C to 200°C.

Furthermore, the compound can be produced from compound (Z-E) and compound (G -G), or compounds respectively equivalent thereto, using reactions equivalent to these reactions.

Compound (Z-G) can be produced by, for example, the method described below. 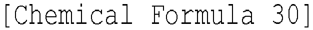 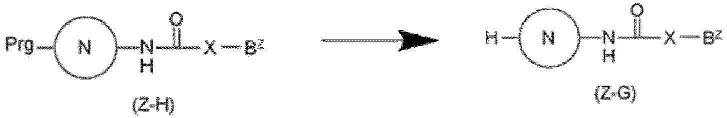 wherein Prg means a protective group and represents any arbitrary functional group that can be converted to hydrogen by a deprotection reaction; the other reference symbols have the same meanings as described above.

Compound (Z-G) can be produced by causing compound (Z-H) to react with an acid such as hydrochloric acid or trifluoroacetic acid at -10°C to 100°C in an appropriate solvent such as methanol or chloroform, or without solvent.

Furthermore, compound (Z-G) can be produced by subjecting compound (Z-H) to a hydrogenation reaction using a catalyst such as palladium-carbon or palladium hydroxide at 0°C to 100°C in an appropriate solvent such as methanol or THF.

In addition to that, the compound can be produced from compound (Z-H) or a compound equivalent thereto using a deprotection reaction for a protective group, which is described in Protective Groups in Organic Synthesis (Wiley) or the like, or a deprotection reaction equivalent thereto.

Compound (Z-H) can be produced by, for example, the method described below. 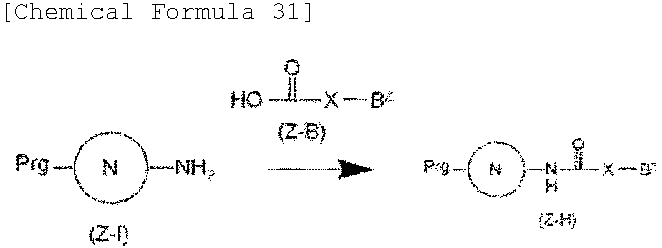 wherein Prg means a protective group and represents any arbitrary functional group that can be converted to hydrogen by a deprotection reaction; the other reference symbols have the same meanings as described above.

Compound (Z-H) can be produced by causing compound (Z-B) to react with compound (Z-I) in an appropriate solvent such as DMF in the presence of a condensing agent such as HATU or WSC, and if necessary, in the presence of an additive such as HOBT or DMAP and if necessary, a base such as triethylamine or DIPEA, at 0°C to 100°C.

Furthermore, the compound can be produced by causing compound (Z-I) to react with a carboxylic acid chloride or carboxylic acid anhydride corresponding to compound (Z-B) in an appropriate solvent such as tetrahydrofuran, and if necessary, in the presence of a base such as triethylamine, DIPEA, or pyridine, at 0°C to 100°C.

In addition to that, the compound can be produced from compound (Z-B) and compound (Z-I), or compounds respectively equivalent thereto, using the condensation reaction described in Christian A.G.N. Montalbetti, et al, Tetrahedron, 61(46), 2005, 10827-10852, or a condensation reaction equivalent thereto.

Compound (Z-I) can be a commercially available product or can be produced from a commercially available product according to a known method. For example, the compound can be produced from appropriate starting raw materials by combining known methods according to the synthesis methods described in known patent literatures such as WO 2016/100154, WO 2012/125893, and WO 2008/013130 and academic literatures such as Kyoji Tomita, J. Med. Chem, 45(25), 2002, 5564-5575, or according to synthesis methods equivalent to those.

### Examples

Hereinafter, the present invention will be specifically described with reference to Examples. However, the present invention is not limited only to these Examples.

Regarding the column, PLC, and TLC used in Reference Examples and Examples, unless particularly stated otherwise, either silica gel or NH silica gel, or both of them were used. For an analysis of a synthesized compound, ¹H NMR (400 MHz), atmospheric pressure ionization high-resolution time-of-flight mass spectrometry (ESI), and other appropriate analysis methods were used.

Compounds in Reference Examples and compounds in Examples were named based on names obtained by converting a structural formula drawn using ChemDraw ver. 13, 14, or 15 manufactured by Cambridge Software Corporation by a naming algorithm mounted on the software.

### Reference Example 1-1

Tert-butyl (R)-(1-(5-(trifluoromethyl)pyridin-3-yl)pyrrolidin-3-yl) carbamate

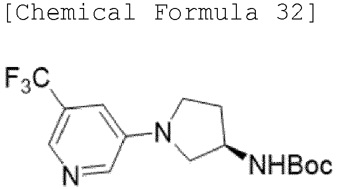

3-Bromo-5-(trifluoromethyl)pyridine (230 mg, 1.02 mmol), tert-butyl (R)-pyrrolidin-3-ylcarbamate (190 mg, 1.02 mmol), tris(dibenzylidene acetone)dipalladium(0) (93 mg, 0.10 mmol), BINAP (67 mg, 0.22 mmol), and sodium tert-butoxide (196 mg, 2.04 mmol) were suspended in toluene (4.0 mL), and the suspension was stirred for 3 hours at 85°C under a nitrogen gas stream. To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 0 to 100%), and thus the title compound (white amorphous, 116 mg, 34%) was obtained.

¹H NMR (CDCl₃, 400 MHz): δ = 1.46 (s, 9H), 1.9 - 2.1 (m, 1H), 2.2 - 2.4 (m, 1H), 3.23 (dd, 1H, J = 4, 10 Hz), 3.3 - 3.6 (m, 2H), 3.65 (dd, 1H, J = 6, 10 Hz), 4.40 (br s, 1H), 4.69 (br s, 1H), 6.9 - 7.0 (m, 1H), 8.12 (d, 1H, J = 3 Hz), 8.21 (s, 1H).

### Reference Example 1-2

(R)-1-(5-(trifluoromethyl)pyridin-3-yl)pyrrolidin-3-amine

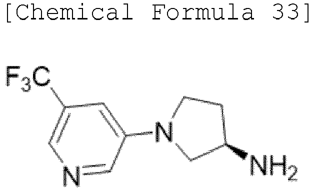

To tert-butyl (R)-(1-(5-(trifluoromethyl)pyridin-3-yl)pyrrolidin-3-yl)carbamate (116 mg, 0.35 mmol) synthesized in Reference Example 1-1, trifluoroacetic acid (2.0 mL) was added under ice cooling, and the mixture was stirred for 1 hour at room temperature. Under ice cooling, a saturated aqueous solution of sodium hydrogen carbonate and ethyl acetate were added to the reaction liquid, the mixture was stirred for a while, subsequently the organic layer was dried over anhydrous sodium sulfate, insoluble materials were filtered, subsequently the solvent was distilled off under reduced pressure, and thus the title compound (brown oily material, 60 mg, 74%) was obtained.

¹H NMR (CDCl₃, 400 MHz): δ = 1.8 - 1.9 (m, 1H), 2.2 - 2.3 (m, 1H), 3.08 (dd, 1H, J = 4, 10 Hz), 3.3 - 3.5 (m, 1H), 3.5 - 3.6 (m, 2H), 3.7 - 3.9 (m, 1H), 6.92 (dd, 1H, J = 2, 2 Hz), 8.11 (d, 1H, J = 3 Hz), 8.17 (s, 1H). The 2H content is not observable.

### Reference Example 2

Tert-butyl (R)-(1-(2-(trifluoromethyl)pyrimidin-5-yl)pyrrolidin-3-yl) carbamate

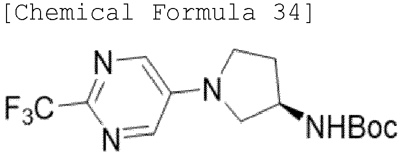

According to a technique similar to Reference Example 1-1, the title compound (white powder, 41 mg, 3.0%) was obtained using 5-bromo-2-(trifluoromethyl)pyrimidine (940 mg, 4.14 mmol) and tert-butyl (R)-pyrrolidin-3-ylcarbamate (950 mg, 5.10 mmol).

¹H NMR (CDCl₃, 400 MHz): δ = 1.46 (s, 9H), 2.0 - 2.1 (m, 1H), 2.3 - 2.5 (m, 1H), 3.28 (dd, 1H, J = 4, 10 Hz), 3.4 - 3.6 (m, 2H), 3.70 (dd, 1H, J = 6, 10 Hz), 4.3 - 4.5 (m, 1H), 4.68 (br s, 1H), 8.10 (s, 2H).

### Reference Example 3

Tert-butyl ((3S,4S)-4-hydroxy-1-(5-(trifluoromethyl)pyridin-3-yl)pyrrolidin-3-yl)carbamate

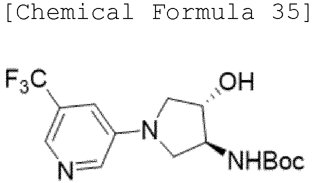

3-Bromo-5-(trifluoromethyl)pyridine (134 mg, 0.59 mmol), tert-butyl ((3S,4S)-4-hydroxypyrrolidin-3-yl)carbamate (100 mg, 0.49 mmol), tris(dibenzylidene acetone)dipalladium(0) (45 mg, 0.05 mmol), XantPhos (57 mg, 0.10 mmol), and potassium carbonate (137 mg, 0.99 mmol) were suspended in toluene (5.0 mL), and the suspension was stirred for 16 hours at 85°C under a nitrogen gas stream. To the reaction liquid that had been left to cool to room temperature, ethyl acetate was added, insoluble materials were filtered through Celite, and the solvent was distilled off under reduced pressure. A residue thus obtained was purified by silica gel column chromatography, and the title compound (white crystals, 106 mg, 62%) was obtained.

¹H NMR (CDCl₃, 400 MHz): δ = 1.47 (s, 9H), 3.24 (dd, 1H, J = 5, 10 Hz), 3.3 - 3.4 (m, 2H), 3.7 - 3.9 (m, 2H), 4.1 - 4.2 (m, 1H), 4.4 - 4.5 (m, 1H), 4.76 (br s, 1H), 6.94 (dd, 1H, J = 2, 2 Hz), 8.12 (d, 1H, J = 3 Hz), 8.24 (s, 1H) .

### Reference Example 4

Tert-butyl (R)-(5-(5-(trifluoromethyl)pyridin-3-yl)-5-azaspiro[2.4]heptan-7-yl) carbamate

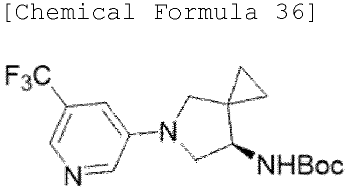

3-Bromo-5-(trifluoromethyl)pyridine (383 mg, 1.70 mmol), tert-butyl (R)-(5-azaspiro[2.4]heptan-7-yl)carbamate (300 mg, 1.41 mmol), tris(dibenzylidene acetone)dipalladium(0) (93 mg, 0.10 mmol), XantPhos (164 mg, 0.28 mmol), and sodium tert-butoxide (272 mg, 2.83 mmol) were suspended in toluene (14 mL), and the suspension was stirred for 16 hours at 85°C under a nitrogen gas stream. To the reaction liquid that had been left to cool to room temperature, ethyl acetate was added, insoluble materials were filtered through Celite, and the solvent was distilled off under reduced pressure. A residue thus obtained was purified by silica gel column chromatography (heptane: ethyl acetate) (concentration gradient: 0 to 100%), and the title compound (yellow crystals, 336 mg, 67%) was obtained.

¹H NMR (CDCl₃, 400 MHz): δ = 0.7 - 0.8 (m, 2H), 0.8 - 1.0 (m, 2H), 1.44 (s, 9H), 3.10 (d, 1H, J = 10 Hz), 3.5 - 3.6 (m, 1H), 3.63 (d, 1H, J = 9 Hz), 3.7 - 3.8 (m, 1H), 3.8 - 3.9 (m, 1H), 4.78 (br s, 1H), 6.9 - 7.0 (m, 1H), 8.09 (d, 1H, J = 3 Hz), 8.21 (s, 1H).

### Reference Example 5

Tert-butyl (R)-(5-(6-(trifluoromethyl)pyridin-3-yl)-5-azaspiro[2.4]heptan-7-yl) carbamate

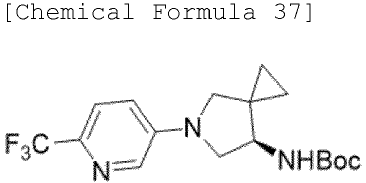

According to a technique similar to Reference Example 4, the title compound (yellow crystals, 271 mg, 80%) was obtained using tert-butyl (R)-(5-azaspiro[2.4]heptan-7-yl)carbamate (200 mg, 0.94 mmol) and 5-bromo-2-(trifluoromethyl)pyridine (255 mg, 1.13 mmol).

¹H NMR (CDCl₃, 400 MHz): δ = 0.7 - 0.8 (m, 2H), 0.8 - 1.0 (m, 2H), 1.44 (s, 9H), 3.11 (d, 1H, J = 10 Hz), 3.5 - 3.6 (m, 1H), 3.63 (d, 1H, J = 9 Hz), 3.7 - 3.8 (m, 1H), 3.8 - 3.9 (m, 1H), 4.76 (br s, 1H), 6.80 (dd, 1H, J = 3, 9 Hz), 7.49 (d, 1H, J = 9 Hz), 7.98 (d, 1H, J = 3 Hz).

### Reference Example 6

Tert-butyl (R)-(5-(4-(trifluoromethyl)thiazol-2-yl)-5-azaspiro[2.4]heptan-7-yl) carbamate

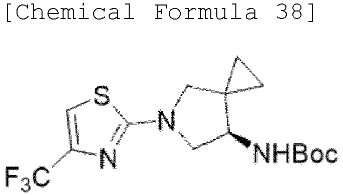

According to a technique similar to Reference Example 4, the title compound (yellow crystals, 192 mg, 56%) was obtained using tert-butyl (R)-(5-azaspiro[2.4]heptan-7-yl)carbamate (200 mg, 0.94 mmol) and 2-bromo-4-(trifluoromethyl)thiazole (255 mg, 1.10 mmol).

¹H NMR (CDCl₃, 400 MHz): δ = 0.6 - 0.8 (m, 2H), 0.8 - 1.0 (m, 2H), 1.44 (s, 9H), 3.32 (d, 1H, J = 10 Hz), 3.57 (d, 1H, J = 8 Hz), 3.74 (d, 1H, J = 11 Hz), 3.8 - 3.9 (m, 2H), 4.77 (br s, 1H), 6.93 (d, 1H, J = 0.8 Hz).

### Reference Example 7-1

Tert-butyl (2S,4R)-4-(2-(4-cyclopropylphenyl)acetamido)-2-methylpyrrolidine-1-carboxylate

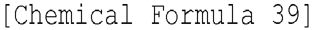

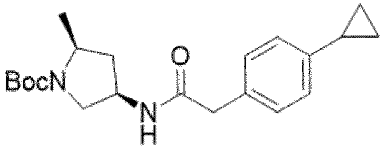

Tert-butyl (2S,4R)-4-amino-2-methylpyrrolidine-1-carboxylate (250 mg, 1.25 mmol) and 2-(4-cyclopropylphenyl)acetic acid (264 mg, 1.50 mmol) synthesized in Reference Example 33-2 were dissolved in DMF (13 mL), subsequently DIPEA (691 µL, 4.01 mmol) and HATU (570 mg, 1.50 mmol) were added thereto, and the mixture was stirred overnight at room temperature. Water was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 0 to 100%), and the title compound (colorless oily material, 365 mg, 82%) was obtained.

¹H NMR (CDCl₃, 400 MHz): δ = 0.6 - 0.7 (m, 2H), 0.9 - 1.0 (m, 2H), 1.1 - 1.2 (m, 3H), 1.43 (s, 9H), 1.7 - 1.9 (m, 3H), 3.0 - 3.1 (m, 1H), 3.51 (s, 2H), 3.5 - 3.7 (m, 1H), 3.7 - 4.0 (m, 1H), 4.4 - 4.6 (m, 1H), 5.31 (d, 1H, J = 7 Hz), 7.04 (d, 2H, J = 8 Hz), 7.10 (d, 2H, J = 8 Hz).

### Reference Example 7-2

2-(4-cyclopropylphenyl)-N-((3R,5S)-5-methylpyrrolidin-3-yl) acetamide

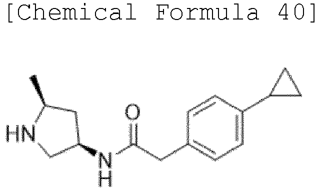

A 2N hydrochloric acid-methanol solution (15 mL) was added to tert-butyl (2S,4R)-4-(2-(4-cyclopropylphenyl)acetamido)-2-methylpyrrolidine-1-carboxylate (365 mg, 1.02 mmol) synthesized in Reference Example 7-1 under ice cooling, the mixture was stirred for 2 hours at room temperature, and then the solvent was distilled off under reduced pressure. A residue thus obtained was purified by silica gel column chromatography (ethyl acetate: methanol) (concentration gradient: 0 to 40%), and the title compound (white crystals, 270 mg, 103%) was obtained.

¹H NMR (CDCl₃, 400 MHz): δ = 0.6 - 0.7 (m, 2H) , 0.9 - 1.0 (m, 2H), 1.13 (d, 3H, J = 6 Hz), 1.5 - 1.7 (m, 3H), 1.8 - 1.9 (m, 1H), 2.51 (dd, 1H, J = 5, 12 Hz), 3.1 - 3.2 (m, 1H), 3.38 (dd, 1H, J = 7, 12 Hz), 3.50 (s, 2H), 4.3 - 4.5 (m, 1H), 5.50 (br s, 1H), 7.05 (d, 2H, J = 8 Hz), 7.12 (d, 2H, J = 8 Hz).

### Reference Example 8

Tert-butyl (3-(5-(trifluoromethyl)pyridin-3-yl)-3-azabicyclo[3.1.0]hexan-1-yl) carbamate

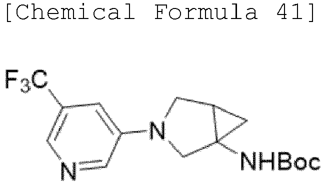

According to a technique similar to Reference Example 4, the title compound (colorless oily material, 103 mg, 23%) was obtained using tert-butyl (3-azabicyclo[3.1.0]hexan-1-yl)carbamate (250 mg, 1.26 mmol) and 3-bromo-5-(trifluoromethyl)pyridine (342 mg, 1.51 mmol) .

¹H NMR (CDCl₃, 400 MHz): δ = 1.1 - 1.2 (m, 1H), 1.2 - 1.3 (m, 1H), 1.46 (s, 9H), 1.8 - 2.0 (m, 1H), 3.4 - 3.7 (m, 3H), 3.75 (d, 1H, J = 8 Hz), 5.13 (br s, 1H), 6.93 (dd, 1H, J = 2, 2 Hz), 8.09 (d, 1H, J = 2 Hz), 8.20 (s, 1H).

### Reference Example 9

Tert-butyl (R)-(1-(6-(trifluoromethyl)pyridin-2-yl)pyrrolidin-3-yl) carbamate

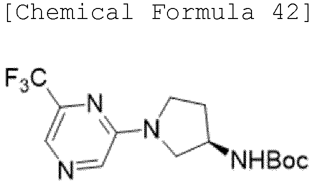

Tert-butyl (R)-pyrrolidin-3-ylcarbamate (250 mg, 1.34 mmol) was dissolved in DMF (13 mL), subsequently 2-chloro-6-(trifluoromethyl)pyrazine (294 mg, 1.61 mmol) and potassium carbonate (371 mg, 2.68 mmol) were added thereto, and the mixture was stirred for 20 hours at 120°C

To the reaction liquid that had been left to cool to room temperature, water was added, the mixture was stirred for a while, and then the mixture was extracted with a mixed liquid of chloroform and methanol. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 0 to 100%), and the title compound (white crystals, 292 mg, 65%) was obtained.

¹H NMR (CDCl₃, 400 MHz): δ = 1.46 (s, 9H), 1.9 - 2.1 (m, 1H), 2.2 - 2.4 (m, 1H), 3.43 (dd, 1H, J = 4, 11 Hz), 3.5 - 3.7 (m, 2H), 3.82 (dd, 1H, J = 6, 11 Hz), 4.39 (br s, 1H), 4.74 (d, 1H, J = 7 Hz), 8.03 (s, 1H), 8.14 (s, 1H).

### Reference Example 10

Tert-butyl (R)-(1-(6-cyano-5-(trifluoromethyl)pyridin-3-yl)pyrrolidin-3-yl)carbamate

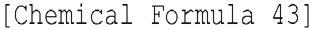

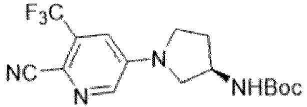

According to a technique similar to Reference Example 4, the title compound (yellow oily material, 139 mg, 30%) was obtained using tert-butyl (R)-pyrrolidin-3-ylcarbamate (200 mg, 1.07 mmol) and 5-bromo-3-(trifluoromethyl)picolinonitrile (323 mg, 1.29 mmol).

¹H NMR (CDCl₃, 400 MHz): δ = 1.46 (s, 9H), 2.0 - 2.2 (m, 1H), 2.3 - 2.5 (m, 1H), 3.33 (dd, 1H, J = 5, 10 Hz), 3.4 - 3.6 (m, 2H), 3.7 - 3.8 (m, 1H), 4.3 - 4.5 (m, 1H), 4.84 (br s, 1H), 6.95 (d, 1H, J = 3 Hz), 8.07 (d, 1H, J = 3 Hz) .

### Reference Example 11-1

Methyl 1-benzyl-3-(2-(4-cyclopropylphenyl)acetamido)pyrrolidine-3-carboxylate

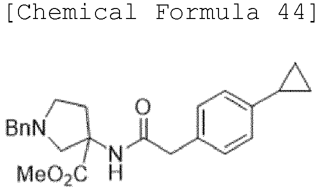

According to a technique similar to Reference Example 7-1, the title compound (white crystals, 362 mg, 86%) was obtained using methyl 3-(azanyl)-1-benzylpyrrolidine-3-carboxylate (250 mg, 1.07 mmol) and 2-(4-cyclopropylphenyl)acetic acid (226 mg, 1.28 mmol) synthesized in Reference Example 33-2.

¹H NMR (CDCl₃, 400 MHz): δ = 0.6 - 0.8 (m, 2H), 0.9 - 1.0 (m, 2H), 1.8 - 2.0 (m, 2H), 2.4 - 2.5 (m, 1H), 2.5 - 2.7 (m, 1H), 2.75 (d, 1H, J = 10 Hz), 2.79 (d, 1H, J = 10 Hz), 2.8 - 2.9 (m, 1H), 3.50 (s, 2H), 3.54 (d, 1H, J = 13 Hz), 3.62 (d, 1H, J = 13 Hz), 3.70 (s, 3H), 6.03 (br s, 1H), 7.0 - 7.1 (m, 2H), 7.1 - 7.2 (m, 2H), 7.2 - 7.3 (m, 5H) .

### Reference Example 11-2

Methyl 3-(2-(4-cyclopropylphenyl)acetamido)pyrrolidine-3-carboxylate

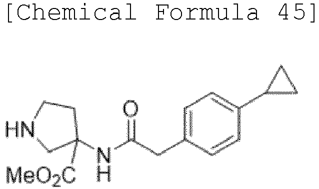

Methyl 1-benzyl-3-(2-(4-cyclopropylphenyl)acetamido)pyrrolidine-3-carboxylate (362 mg, 0.92 mmol) synthesized in Reference Example 11-1 was dissolved in methanol (20 mL), palladium-carbon (36 mg) was added thereto, and the mixture was stirred for 4 hours at room temperature under a hydrogen gas stream. Insoluble materials were filtered, subsequently the solvent was distilled off under reduced pressure, a residue thus obtained was purified by silica gel column chromatography (ethyl acetate: methanol) (concentration gradient: 0 to 40%), and the title compound (94 mg, 34%) was obtained.

¹H NMR (CDCl₃, 400 MHz): δ = 0.6 - 0.7 (m, 2H), 0.9 - 1.0 (m, 2H), 1.8 - 1.9 (m, 1H), 2.0 - 2.2 (m, 2H), 2.2 - 2.3 (m, 1H), 2.9 - 3.0 (m, 1H), 3.10 (d, 1H, J = 12 Hz), 3.1 - 3.2 (m, 1H), 3.33 (d, 1H, J = 12 Hz), 3.51 (s, 2H), 3.73 (s, 3H), 6.35 (br s, 1H), 7.0 - 7.1 (m, 2H), 7.1 - 7.2 (m, 2H).

### Reference Example 12-1

Tert-butyl (R)-(1-(4-(trifluoromethyl)thiazol-2-yl)pyrrolidin-3-yl)carbamate

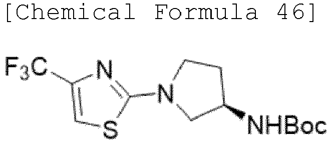

According to a technique similar to Reference Example 9, the title compound (white crystals, 706 mg, 90%) was obtained using 2-bromo-4-(trifluoromethyl)thiazole (540 mg, 2.33 mmol) and tert-butyl (R)-pyrrolidin-3-ylcarbamate (520 mg, 2.79 mmol) .

¹H NMR (CDCl₃, 400 MHz): δ = 1.45 (s, 9H), 1.9 - 2.1 (m, 1H), 2.2 - 2.4 (m, 1H), 3.35 (dd, 1H, J = 4, 10 Hz), 3.5 - 3.7 (m, 2H), 3.75 (dd, 1H, J = 6, 10 Hz), 4.38 (br s, 1H), 4.68 (br s, 1H), 6.92 (d, 1H, J = 0.7 Hz).

### Reference Example 12-2

(R)-1-(4-(trifluoromethyl)thiazol-2-yl)pyrrolidin-3-amine

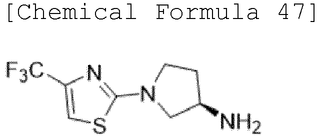

According to a technique similar to Reference Example 7-2, the title compound (white crystals, 480 mg, 97%) was obtained using tert-butyl (R)-(1-(4-(trifluoromethyl)thiazol-2-yl)pyrrolidin-3-yl)carbamate (706 mg, 2.09 mmol) synthesized in Reference Example 12-1.

¹H NMR (CDCl₃, 400 MHz): δ = 1.34 (br s, 2H), 1.8 - 1.9 (m, 1H), 2.2 - 2.3 (m, 1H), 3.23 (dd, 1H, J = 4, 10 Hz), 3.5 - 3.6 (m, 1H), 3.6 - 3.7 (m, 2H), 3.7 - 3.9 (m, 1H), 6.90 (br s, 1H).

### Reference Example 13

Tert-butyl (R)-(1-(6-methyl-5-(trifluoromethyl)pyridin-3-yl)pyrrolidin-3-yl)carbamate

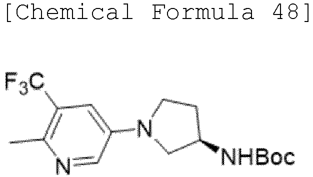

According to a technique similar to Reference Example 4, the title compound (white crystals, 22 mg, 4.9%) was obtained using 5-bromo-2-methyl-3-(trifluoromethyl)pyridine (310 mg, 1.29 mmol) and tert-butyl (R)-pyrrolidin-3-ylcarbamate (289 mg, 1.55 mmol).

¹H NMR (CDCl₃, 400 MHz): δ = 1.46 (s, 9H), 1.9 - 2.1 (m, 1H), 2.2 - 2.4 (m, 1H), 2.5 - 2.6 (m, 3H), 3.20 (dd, 1H, J = 4, 10 Hz), 3.3 - 3.4 (m, 1H), 3.4 - 3.5 (m, 1H), 3.61 (dd, 1H, J = 6, 10 Hz), 4.40 (br s, 1H), 4.70 (br s, 1H), 7.00 (d, 1H, J = 3 Hz), 7.98 (d, 1H, J = 3 Hz).

### Reference Example 14-1

Tert-butyl (R)-(1-(5-(2-hydroxypropan-2-yl)pyridin-3-yl)pyrrolidin-3-yl)-carbamate

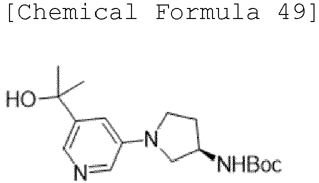

According to a technique similar to Reference Example 3, the title compound (brown amorphous, 187 mg, 60%) was obtained using 2-(5-bromopyridin-3-yl)propan-2-ol (210 mg, 0.97 mmol) and tert-butyl (R)-pyrrolidin-3-ylcarbamate (217 mg, 1.17 mmol).

¹H NMR (CDCl₃, 400 MHz): δ = 1.46 (s, 9H), 1.58 (s, 6H), 1.9 - 2.0 (m, 1H), 2.2 - 2.4 (m, 1H), 2.80 (br s, 1H), 3.17 (dd, 1H, J = 4, 10 Hz), 3.3 - 3.4 (m, 1H), 3.4 - 3.5 (m, 1H), 3.56 (dd, 1H, J = 6, 10 Hz), 4.36 (br s, 1H), 4.96

(br s, 1H), 6.99 (dd, 1H, J = 2, 2 Hz), 7.78 (d, 1H, J = 3 Hz), 8.01 (d, 1H, J = 2 Hz).

### Reference Example 14-2

(R)-2-(5-(3-aminopyrrolidin-1-yl)pyridin-3-yl)propan-2-ol

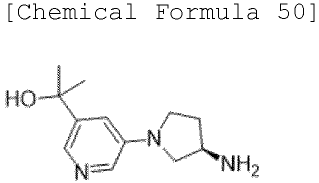

According to a technique similar to Reference Example 7-2, the title compound (brown oily material, 114 mg, 88%) was obtained using tert-butyl (R)-(1-(5-(2-hydroxypropan-2-yl)pyridin-3-yl)pyrrolidin-3-yl)-carbamate (187 mg, 0.58 mmol) synthesized in Reference Example 14-1.

¹H NMR (CDCl₃, 400 MHz): δ = 1.59 (s, 6H), 1.7 - 1.9 (m, 1H), 2.1 - 2.3 (m, 1H), 3.03 (dd, 1H, J = 4, 10 Hz), 3.3 - 3.4 (m, 1H), 3.4 - 3.6 (m, 4H), 3.7 - 3.8 (m, 1H), 6.99 (dd, 1H, J = 2, 2 Hz), 7.80 (d, 1H, J = 3 Hz), 8.00 (d, 1H, J = 2 Hz). The 1H content is not observable.

### Reference Example 15

Tert-butyl (R)-(1-(8-(trifluoromethyl)imidazo[1,2-a]pyridin-6-yl)pyrrolidin-3-yl)-carbamate

### [Chemical Formula 51]

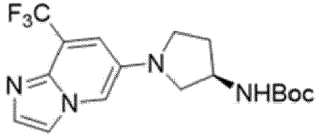

According to a technique similar to Reference Example 4, the title compound (brown oily material, 90 mg, 26%) was obtained using 6-bromo-8-(trifluoromethyl)imidazo[1,2-a]pyridine (250 mg, 0.94 mmol) and tert-butyl (R)-pyrrolidin-3-ylcarbamate (211 mg, 1.13 mmol) .

¹H NMR (CDCl₃, 400 MHz): δ = 1.46 (s, 9H), 1.9 - 2.1 (m, 1H), 2.3 - 2.4 (m, 1H), 3.17 (dd, 1H, J = 4, 9 Hz), 3.2 - 3.4 (m, 1H), 3.4 - 3.5 (m, 1H), 3.5 - 3.6 (m, 1H), 4.40 (br s, 1H), 4.79 (br s, 1H), 7.19 (d, 1H, J = 0.8 Hz), 7.40 (d, 1H, J = 1 Hz), 7.56 (d, 1H, J = 0.8 Hz), 7.64 (d, 1H, J = 1 Hz).

### Reference Example 16

Ethyl 2-(4-(1,1-dioxide thiomorpholino)phenyl)acetate

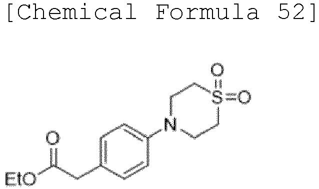

According to a technique similar to Reference Example 3, the title compound (pale yellow oily material, 30 mg, 8.2%) was obtained using ethyl 2-(4-bromophenyl)acetate (300 mg, 1.23 mmol) and 1,1-dioxide thiomorpholine (334 mg, 2.47 mmol).

¹H NMR (CDCl₃, 400 MHz): δ = 1.26 (t, 3H, J = 7 Hz), 3.0 - 3.1 (m, 4H), 3.54 (s, 2H), 3.8 - 3.9 (m, 4H), 4.12 (q, 2H, J = 7 Hz), 6.8 - 6.9 (m, 2H), 7.2 - 7.3 (m, 2H).

### Reference Example 17

Ethyl 2-(4-(trimethylsilyl)phenyl)acetate

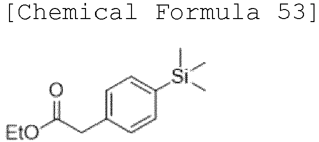

In a nitrogen atmosphere, a small amount of iodine was introduced into a round-bottom flask containing magnesium (168 mg, 6.98 mmol) and THF (1.0 mL), subsequently ethyl 2-(4-bromophenyl)acetate (500 mg, 2.06 mmol) dissolved in THF (3.4 mL) was added thereto, and the mixture was heated to reflux for 30 minutes. The reaction liquid was left to cool to room temperature, chlorotrimethylsilane (520 µL, 4.11 mmol) was added thereto, and the mixture was heated to reflux for 2 hours. To the reaction liquid that had been left to cool to room temperature, a saturated aqueous solution of ammonium chloride was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 0 to 100%), and the title compound (colorless oily material, 30 mg, 6.2%) was obtained.

¹H NMR (CD₃OD, 400 MHz): δ = 0.25 (s, 9H), 1.24 (t, 3H, J = 7 Hz), 3.59 (s, 2H), 4.10 (q, 2H, J = 7 Hz), 7.2 - 7.3 (m, 2H), 7.4 - 7.5 (m, 2H).

### Reference Example 18

Tert-butyl (3S,4S)-3-(2-(4-cyclopropylphenyl)acetamido)-4-fluoropyrrolidine-1-carboxylate

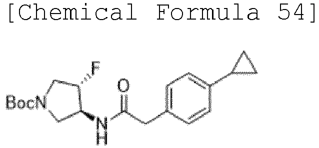

According to a technique similar to Reference Example 7-1, the title compound (yellow powder, 369 mg, 83%) was obtained using tert-butyl (3S,4S)-3-amino-4-fluoropyrrolidine-1-carboxylate (250 mg, 1.22 mmol) and 2-(4-cyclopropylphenyl)acetic acid (259 mg, 1.47 mmol) synthesized in Reference Example 33-2.

¹H NMR (CDCl₃, 400 MHz): δ = 0.6 - 0.7 (m, 2H), 0.9 - 1.0 (m, 2H), 1.45 (s, 9H), 1.8 - 1.9 (m, 1H), 3.1 - 3.7 (m, 6H), 4.43 (br s, 1H), 4.8 - 5.1 (m, 1H), 5.32 (br s, 1H), 7.0 - 7.1 (m, 2H), 7.1 - 7.2 (m, 2H).

### Reference Example 19

Tert-butyl 3-cyano-3-(2-(4-cyclopropylphenyl)acetamido)pyrrolidine-1-carboxylate

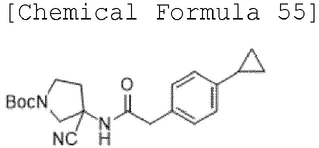

According to a technique similar to Reference Example 7-1, the title compound (yellow oily material, 454 mg, 86%) was obtained using tert-butyl 3-amino-3-cyanopyrrolidine-1-carboxylate (300 mg, 1.42 mmol) and 2-(4-cyclopropylphenyl)acetic acid (300 mg, 1.70 mmol) synthesized in Reference Example 33-2.

¹H NMR (CDCl₃, 400 MHz): δ = 0.6 - 0.7 (m, 2H), 0.9 - 1.0 (m, 2H), 1.44 (s, 9H), 1.8 - 1.9 (m, 1H), 2.2 - 2.6 (m, 2H), 3.3 - 3.4 (m, 1H), 3.4 - 3.6 (m, 4H), 3.9 - 4.0 (m, 1H), 6.3 - 6.4 (m, 1H), 7.04 (d, 2H, J = 8 Hz), 7.10 (d, 2H, J = 8 Hz).

### Reference Example 20-1

Methyl 1-benzyl-3-((tert-butoxycarbonyl)amino)pyrrolidine-3-carboxylate

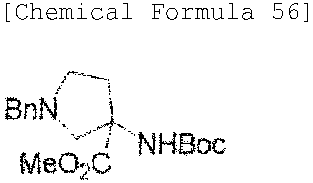

Methyl 3-amino-1-benzylpyrrolidine-3-carboxylate (500 mg, 2.13 mmol), DIPEA (788 µL, 4.57 mmol), and Boc anhydride (931 mg, 4.27 mmol) were dissolved in chloroform (21 mL), and the solution was stirred for 16 hours at room temperature. Water was added to the reaction liquid, the mixture was stirred for a while, and then the mixture was extracted with chloroform. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 0 to 100%), and the title compound (colorless oily material, 522 mg, 73%) was obtained.

¹H NMR (CDCl₃, 400 MHz): δ = 1.42 (s, 9H), 1.9 - 2.0 (m, 1H), 2.5 - 2.7 (m, 2H), 2.7 - 3.0 (m, 3H), 3.61 (d, 1H, J = 13 Hz), 3.68 (d, 1H, J = 13 Hz), 3.74 (s, 3H), 5.12 (br s, 1H), 7.2 - 7.4 (m, 5H).

### Reference Example 20-2

Methyl 3-((tert-butoxycarbonyl)amino)-1-(5-(trifluoromethyl)pyridin-3-yl)pyrrolidine-3-carboxylate

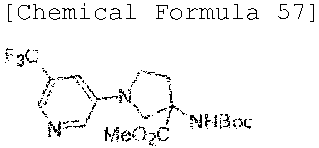

According to a technique similar to Reference Example 11-2, a crude form of methyl 3-((tert-butoxycarbonyl)amino)pyrrolidine-3-carboxylate (colorless oily material, 402 mg) was obtained using methyl 1-benzyl-3-((tert-butoxycarbonyl)amino)pyrrolidine-3-carboxylate (522 mg, 1.56 mmol) synthesized in Example 20-1. According to a technique similar to Reference Example 4, the title compound (34 mg, 5.8%) was obtained using the crude form of methyl 3-((tert-butoxycarbonyl)amino)pyrrolidine-3-carboxylate (402 mg) thus obtained and (3-bromo-5-(trifluoromethyl)pyridine (423 mg, 1.87 mmol).

¹H NMR (CDCl₃, 400 MHz): δ = 1.45 (s, 9H), 2.3 - 2.5 (m, 1H), 2.5 - 2.6 (m, 1H), 3.5 - 3.6 (m, 2H), 3.6 - 3.7 (m, 1H), 3.79 (s, 3H), 4.01 (d, 1H, J = 10 Hz), 5.17 (br s, 1H), 6.9 - 7.0 (m, 1H), 8.11 (d, 1H, J = 3 Hz), 8.22 (s, 1H) .

### Reference Example 21-1

Tert-butyl 3-cyano-3-(2-(4-(trifluoromethyl)phenyl)acetamido)pyrrolidine-1-carboxylate

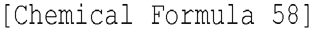

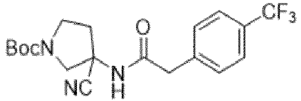

According to a technique similar to Reference Example 7-1, the title compound (white powder, 352 mg, 62%) was obtained using tert-butyl 3-amino-3-cyanopyrrolidine-1-carboxylate (300 mg, 1.42 mmol) and 2-(4-(trifluoromethyl)phenyl)acetic acid (348 mg, 1.70 mmol).

¹H NMR (CDCl₃, 400 MHz): δ = 1.45 (s, 9H), 2.2 - 2.6 (m, 2H), 3.3 - 3.7 (m, 3H), 3.68 (s, 2H), 3.9 - 4.1 (m, 1H), 5.7 - 5.8 (m, 1H), 7.40 (d, 2H, J = 8 Hz), 7.64 (d, 2H, J = 8 Hz).

### Reference Example 21-2

Methyl 3-(2-(4-(trifluoromethyl)phenyl)acetamido)pyrrolidine-3-carboxylate

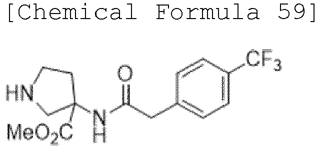

According to a technique similar to Reference Example 7-2, the title compound (white powder, 213 mg, 73%) was obtained using tert-butyl 3-cyano-3-(2-(4-(trifluoromethyl)phenyl)acetamido)pyrrolidine-1-carboxylate (352 mg, 0.89 mmol) synthesized in Reference Example 21-1.

¹H NMR (CDCl₃, 400 MHz): δ = 2.0 - 2.1 (m, 1H), 2.2 - 2.4 (m, 2H), 3.0 - 3.1 (m, 1H), 3.14 (d, 1H, J = 12 Hz), 3.1 - 3.2 (m, 1H), 3.35 (d, 1H, J = 12 Hz), 3.61 (s, 2H), 3.75 (s, 3H), 6.52 (br s, 1H), 7.40 (d, 2H, J = 8 Hz), 7.61 (d, 2H, J = 8 Hz).

### Reference Example 22-1

Dimethyl (1S,2S)-cyclopropane-1,2-dicarboxylate 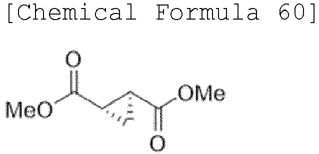 (1S,2S)-cyclopropane-1,2-dicarboxylic acid (2.0g, 15.4 mmol) was dissolved in methanol (30 mL), thionyl chloride (2.79 mL, 38.4 mmol) and a small amount of DMF were added thereto, the mixture was stirred for 4 hours at room temperature, and then the solvent was distilled off under reduced pressure. Water and ethyl acetate were added to a residue thus obtained, the mixture was stirred for a while, subsequently an organic layer thus separated was washed with a saturated aqueous solution of sodium hydrogen carbonate, and the organic layer was dried over anhydrous sodium sulfate. Insoluble materials were filtered, subsequently the solvent was distilled off under reduced pressure, and the title compound (brown oily material, 2.09g, 86%) was obtained.

¹H NMR (CDCl₃, 400 MHz): δ = 1.4 - 1.5 (m, 2H), 2.1 - 2.2 (m, 2H), 3.71 (s, 6H).

### Reference Example 22-2

(1S,2S)-2-(methoxycarbonyl)cyclopropane-1-carboxylic acid

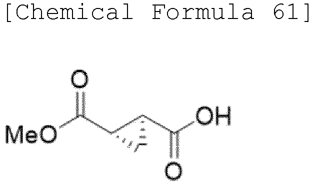

Dimethyl (1S,2S)-cyclopropane-1,2-dicarboxylate (2.09g, 13.2 mmol) synthesized in Reference Example 22-1 was dissolved in methanol (13 mL) and water (2.0 mL), a 2N aqueous solution of sodium hydroxide (6.28 mL) was added thereto, the mixture was stirred for 16 hours at room temperature, and then the solvent was distilled off under reduced pressure. Water was added to a residue thus obtained, the mixture was washed with chloroform, a 2N aqueous solution of hydrochloric acid was added thereto to adjust the pH of the aqueous layer to 2 to 3, and then the aqueous layer was extracted with a mixed liquid of chloroform and methanol. An organic layer thus separated was dried over anhydrous sodium sulfate, insoluble materials were filtered, subsequently the solvent was distilled off under reduced pressure, and thereby the title compound (brown oily material, 1.45g, 76%) was obtained.

¹H NMR (CDCl₃, 400 MHz): δ = 1.4 - 1.6 (m, 2H), 2.1 - 2.2 (m, 1H), 2.2 - 2.3 (m, 1H), 3.72 (s, 3H). The 1H content is not observable.

### Reference Example 22-3

Methyl (1S,2S)-2-((2-bromo-4-fluorophenyl)carbamoyl)cyclopropane-1-carboxylate

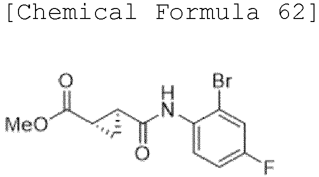

According to a technique similar to Reference Example 7-1, the title compound (515 mg, 65%) was obtained using (1S,2S)-2-(methoxycarbonyl)cyclopropane-1-carboxylic acid (363 mg, 2.52 mmol) synthesized in Reference Example 22-2 and 2-bromo-4-fluoroaniline (574 mg, 3.02 mmol).

¹H NMR (CDCl₃, 400 MHz): δ = 1.4 - 1.5 (m, 1H), 1.5 - 1.6 (m, 1H), 2.1 - 2.2 (m, 1H), 2.2 - 2.3 (m, 1H), 3.75 (s, 3H), 7.05 (ddd, 1H, J = 6, 8, 9 Hz), 7.31 (dd, 1H, J = 3, 8 Hz), 7.76 (br s, 1H), 8.28 (dd, 1H, J = 6, 9 Hz).

### Reference Example 22-4

Methyl (1S,2S)-2-(6-fluorobenzo[d]oxazol-2-yl)cyclopropane-1-carboxylate

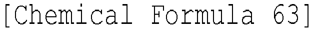

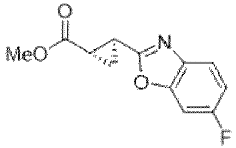

Methyl (1S,2S)-2-((2-bromo-4-fluorophenyl)carbamoyl)cyclopropane-1-carboxylate (515 mg, 1.63 mmol) synthesized in Reference Example 22-3, copper(I) iodide (31 mg, 0.16 mmol), 1,10-phenanthroline (59 mg, 0.33 mmol), and cesium carbonate (796 mg, 2.44 mmol) were suspended in DME (5.0 mL), and the suspension was heated to reflux for 16 hours under a nitrogen gas stream. The reaction liquid that had been left to cool to room temperature was filtered through Celite, and the solvent was distilled off under reduced pressure. A residue thus obtained was purified by silica gel column chromatography (heptane: ethyl acetate) (concentration gradient: 0 to 100%), and the title compound (pale yellow crystals, 226 mg, 59%) was obtained.

¹H NMR (CDCl₃, 400 MHz): δ = 1.7 - 1.8 (m, 2H), 2.4 - 2.5 (m, 1H), 2.7 - 2.8 (m, 1H), 3.75 (s, 3H), 7.0 - 7.1 (m, 1H), 7.19 (dd, 1H, J = 2, 8 Hz), 7.55 (dd, 1H, J = 5, 9

Hz) .

### Reference Example 23-1

Ethyl 2-(4-morpholinophenyl)acetate

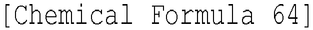

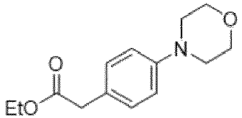

Ethyl 2-(4-bromophenyl)acetate (1.28g, 5.27 mmol), morpholine (917 µL, 10.5 mmol), palladium acetate (118 mg, 0.53 mmol), tert-butyl XPhos (447 mg, 1.05 mmol), and cesium carbonate (3.43g, 10.5 mmol) were suspended in toluene (25 mL), and the suspension was stirred for 16 hours at 110°C under a nitrogen gas stream. The reaction liquid that had been left to cool to room temperature was filtered through Celite, and the solvent was distilled off under reduced pressure. A residue thus obtained was purified by silica gel column chromatography (heptane: ethyl acetate) (concentration gradient: 0 to 100%), and the title compound (yellow crystals, 760 mg, 58%) was obtained.

¹H NMR (CDCl₃, 400 MHz): δ = 1.25 (t, 3H, J = 7 Hz), 3.1 - 3.2 (m, 4H), 3.53 (s, 2H), 3.8 - 3.9 (m, 4H), 4.14 (q, 2H, J = 7 Hz), 6.8 - 6.9 (m, 2H), 7.1 - 7.2 (m, 2H).

### Reference Example 23-2

2-(4-Morpholinophenyl)acetic acid

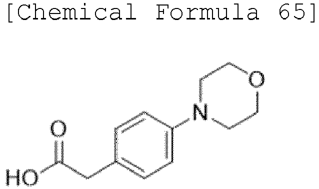

Ethyl 2-(4-morpholinophenyl)acetate (760 mg, 3.05 mmol) synthesized in Reference Example 23-1 was dissolved in methanol (5.0 mL), a 2N aqueous solution of sodium hydroxide (4.6 mL) was added thereto, the mixture was stirred for 16 hours at room temperature, and the solvent was distilled off under reduced pressure. Water was added to a residue thus obtained, the mixture was washed with chloroform, a 2N aqueous solution of hydrochloric acid was added thereto to neutralize the mixture, and then the solvent was distilled off under reduced pressure. A mixed liquid of chloroform and methanol was added to a residue thus obtained, the mixture was stirred for a while, insoluble materials were filtered, subsequently the solvent was distilled off under reduced pressure, and a crude form of the title compound (white powder, 733 mg) was obtained.

¹H NMR (CD₃OD, 400 MHz): δ = 3.0 - 3.1 (m, 4H), 3.38 (s, 2H), 3.8 - 3.9 (m, 4H), 6.8 - 6.9 (m, 2H), 7.2 - 7.3 (m, 2H). The 1H content is not observable.

### Reference Example 24

Tert-butyl 3-(2-(4-cyclopropylphenyl)acetamido)-3-ethylpyrrolidine-1-carboxylate

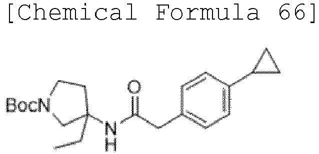

According to a technique similar to Reference Example 7-1, the title compound (white crystals, 782 mg, 90%) was obtained using tert-butyl 3-amino-3-ethylpyrrolidine-1-carboxylate (500 mg, 2.33 mmol) and 2-(4-cyclopropylphenyl)acetic acid (493 mg, 2.80 mmol) synthesized in Reference Example 33-2.

¹H NMR (CDCl₃, 400 MHz): δ = 0.6 - 0.7 (m, 2H), 0.7 - 0.8 (m, 3H), 0.9 - 1.0 (m, 2H), 1.44 (s, 9H), 1.5 - 2.5 (m, 5H), 3.1 - 3.6 (m, 6H), 5.14 (d, 1H, J = 14 Hz), 7.0 - 7.1 (m, 2H), 7.1 - 7.2 (m, 2H).

### Reference Example 25-1

Methyl (1S,2S)-2-((2-bromophenyl)carbamoyl)cyclopropane-1-carboxylate

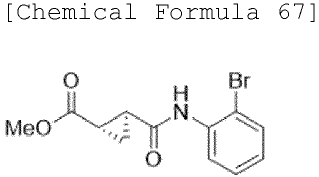

According to a technique similar to Reference Example 22-1, a crude form of dimethyl (1S,2S)-cyclopropane-1,2-dicarboxylate (brown oily material, 695 mg) was obtained using (1S,2S)-cyclopropane-1,2-dicarboxylic acid (500 mg, 3.84 mmol). According to a technique similar to Reference Example 22-2, (1S,2S)-2-(methoxycarbonyl)cyclopropane-1-carboxylic acid (427 mg, 77%) was obtained using the crude form of dimethyl (1S,2S)-cyclopropane-1,2-dicarboxylate (695 mg). According to a technique similar to Reference Example 7-1, the title compound (yellow powder, 397 mg, 45%) was obtained using (1S,2S)-2-(methoxycarbonyl)cyclopropane-1-carboxylic acid (427 mg, 2.96 mmol) thus obtained and 2-bromoaniline (612 mg, 3.56 mmol).

¹H NMR (CDCl₃, 400 MHz): δ = 1.4 - 1.5 (m, 1H), 1.5 - 1.6 (m, 1H), 2.1 - 2.2 (m, 1H), 2.2 - 2.3 (m, 1H), 3.74 (s, 3H), 6.9 - 7.0 (m, 1H), 7.2 - 7.3 (m, 1H), 7.54 (dd, 1H, J = 1, 8 Hz), 7.93 (br s, 1H), 8.30 (d, 1H, J = 8 Hz).

### Reference Example 25-2

Methyl (1S,2S)-2-(benzo[d]oxazol-2-yl)cyclopropane-1-carboxylate

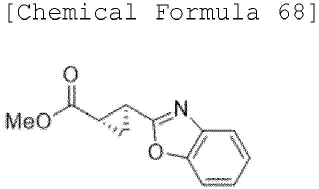

According to a technique similar to Reference Example 22-4, the title compound (95 mg, 33%) was obtained using methyl (1S,2S)-2-((2-bromophenyl)carbamoyl)cyclopropane-1-carboxylate (397 mg, 1.33 mmol) synthesized in Reference Example 25-1.

¹H NMR (CDCl₃, 400 MHz): δ = 1.7 - 1.8 (m, 2H), 2.4 - 2.5 (m, 1H), 2.7 - 2.8 (m, 1H), 3.75 (s, 3H), 7.2 - 7.3 (m, 2H), 7.4 - 7.5 (m, 1H), 7.6 - 7.7 (m, 1H).

Reference Example 26-1

Methyl (1S,2S)-2-((2-bromo-4,5-difluorophenyl)carbamoyl)cyclopropane-1-carboxylate

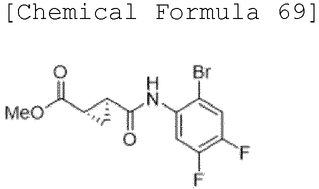

According to a technique similar to Reference Example 7-1, the title compound (313 mg, 39%) was obtained using (1S,2S)-2-(methoxycarbonyl)cyclopropane-1-carboxylic acid (344 mg, 2.39 mmol) synthesized in Reference Example 22-2 and 2-bromo-4,5-difluoroaniline (596 mg, 2.87 mmol).

¹H NMR (CDCl₃, 400 MHz): δ = 1.4 - 1.6 (m, 2H), 2.1 - 2.2 (m, 1H), 2.3 - 2.4 (m, 1H), 3.75 (s, 3H), 7.3 - 7.4 (m, 1H), 7.79 (br s, 1H), 8.3 - 8.4 (m, 1H).

### Reference Example 26-2

Methyl (1S,2S)-2-(5,6-difluorobenzo[d]oxazol-2-yl)cyclopropane-1-carboxylate

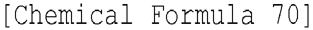

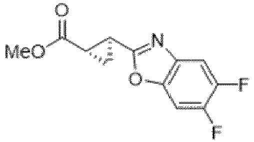

According to a technique similar to Reference Example 22-4, the title compound (pale yellow oily material, 68 mg, 29%) was obtained using methyl (1S,2S)-2-((2-bromo-4,5-difluorophenyl)carbamoyl)cyclopropane-1-carboxylate (313 mg, 0.94 mmol) synthesized in Reference Example 26-1.

¹H NMR (CDCl₃, 400 MHz): δ = 1.7 - 1.8 (m, 2H), 2.4 - 2.5 (m, 1H), 2.7 - 2.8 (m, 1H), 3.75 (s, 3H), 7.3 - 7.4 (m, 1H), 7.4 - 7.5 (m, 1H).

### Reference Example 27

Ethyl 2-(4-(2-oxa-6-azaspiro[3.3]heptan-6-yl)phenyl)acetate

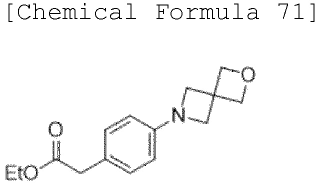

Ethyl 2-(4-bromophenyl)acetate (1.0g, 4.11 mmol), 2-oxa-6-azaspiro[3.3]heptane (816 mg, 8.23 mmol), palladium acetate (92 mg, 0.41 mmol), XPhos (270 mg, 0.57 mmol), and cesium carbonate (2.68g, 8.23 mmol) were suspended in toluene (30 mL), and the suspension was stirred for 16 hours at 110°C under a nitrogen gas stream. The suspension was left to cool to room temperature, insoluble materials were filtered through Celite, and the solvent was distilled off under reduced pressure. A residue thus obtained was purified by silica gel column chromatography (heptane: ethyl acetate) (concentration gradient: 0 to 100%), and the title compound (colorless oily material, 1.26g, 117%) was obtained.

¹H NMR (CDCl₃, 400 MHz): δ = 1.24 (t, 3H, J = 7 Hz), 3.50 (s, 2H), 4.01 (br s, 4H), 4.13 (q, 2H, J = 7 Hz), 4.83 (br s, 4H), 6.4 - 6.5 (m, 2H), 7.1 - 7.2 (m, 2H).

### Reference Example 28-1

Methyl (1S,2S)-2-((2-bromo-5-fluorophenyl)carbamoyl)cyclopropane-1-carboxylate

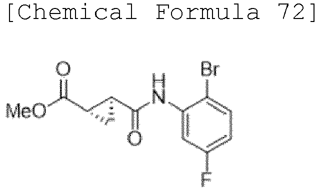

(1S,2S)-2-(methoxycarbonyl)cyclopropane-1-carboxylic acid (294 mg, 2.04 mmol) synthesized in Reference Example 22-2 and 2-bromo-5-fluoroaniline (465 mg, 2.45 mmol) were dissolved in DMF (15 mL), subsequently DIPEA (1.13 mL, 6.56 mmol) and HATU (931 mg, 2.45 mmol) were added thereto, and the mixture was stirred for 16 hours at 50°C

To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 0 to 100%), and the title compound (ivory-colored powder, 210 mg, 33%) was obtained.

¹H NMR (CDCl₃, 400 MHz): δ = 1.4 - 1.5 (m, 1H), 1.5 - 1.6 (m, 1H), 2.1 - 2.2 (m, 1H), 2.3 - 2.4 (m, 1H), 3.74 (s, 3H), 6.73 (ddd, 1H, J = 3, 8, 9 Hz), 7.49 (dd, 1H, J = 6, 9 Hz), 7.95 (br s, 1H), 8.21 (dd, 1H, J = 2, 11 Hz).

### Reference Example 28-2

Methyl (1S,2S)-2-(5-fluorobenzo[d]oxazol-2-yl)cyclopropane-1-carboxylate

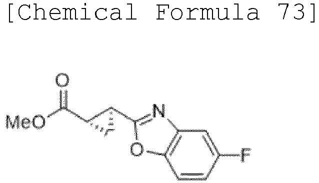

According to a technique similar to Reference Example 22-4, the title compound (ivory-colored powder, 47 mg, 30%) was obtained using methyl (1S,2S)-2-((2-bromo-5-fluorophenyl)carbamoyl)cyclopropane-1-carboxylate (210 mg, 0.66 mmol) synthesized in Reference Example 28-1.

¹H NMR (CDCl₃, 400 MHz): δ = 1.7 - 1.8 (m, 2H), 2.4 - 2.5 (m, 1H), 2.7 - 2.8 (m, 1H), 3.75 (s, 3H), 7.0 - 7.1 (m, 1H), 7.31 (dd, 1H, J = 2, 8 Hz), 7.38 (dd, 1H, J = 4, 9 Hz).

### Reference Example 29-1

Trans-2-(ethoxycarbonyl)cyclopropane-1-carboxylic acid

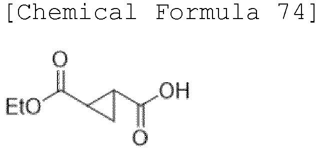

According to a technique similar to Reference Example 22-2, the title compound (white crystals, 5.91g, 95%) was obtained using diethyl trans-cyclopropane-1,2-dicarboxylate (7.3g, 39.2 mmol).

¹H NMR (CDCl₃, 400 MHz): δ = 1.28 (t, 3H, J = 7 Hz), 1.4 - 1.6 (m, 2H), 2.1 - 2.3 (m, 2H), 4.16 (q, 2H, J = 7 Hz). The 1H content is not observable.

### Reference Example 29-2

### Ethyl trans-2-((2-bromophenyl)carbamoyl)cyclopropane-1-carboxylate

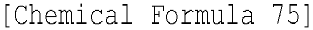

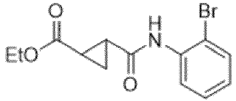

According to a technique similar to Reference

Example 28-1, the title compound (white powder, 3.42g, 87%) was obtained using trans-2-(ethoxycarbonyl)cyclopropane-1-carboxylic acid (2.0g, 12.6 mmol) synthesized in Reference Example 29-1 and 2-bromoaniline (2.61g, 15.2 mmol).

¹H NMR (CDCl₃, 400 MHz): δ = 1.30 (t, 3H, J = 7 Hz), 1.4 - 1.5 (m, 1H), 1.5 - 1.6 (m, 1H), 2.1 - 2.2 (m, 1H), 2.2 - 2.3 (m, 1H), 4.19 (q, 2H, J = 7 Hz), 6.9 - 7.0 (m, 1H), 7.2 - 7.3 (m, 1H), 7.55 (dd, 1H, J = 1, 8 Hz), 7.90 (br s, 1H), 8.33 (d, 1H, J = 8 Hz).

### Reference Example 29-3

Ethyl trans-2-(benzo[d]oxazol-2-yl)cyclopropane-1-carboxylate

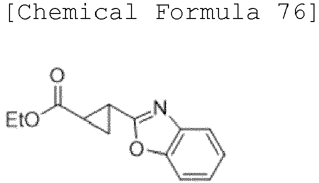

According to a technique similar to Reference Example 22-4, the title compound (pale yellow oily material, 1.22g, 48%) was obtained using ethyl trans-2-((2-bromophenyl)carbamoyl)cyclopropane-1-carboxylate (3.42g, 11.0 mmol) synthesized in Reference Example 29-2.

¹H NMR (CDCl₃, 400 MHz): δ = 1.29 (t, 3H, J = 7 Hz), 1.7 - 1.8 (m, 2H), 2.4 - 2.5 (m, 1H), 2.7 - 2.8 (m, 1H), 4.20 (q, 2H, J = 7 Hz), 7.2 - 7.3 (m, 2H), 7.4 - 7.5 (m, 1H), 7.6 - 7.7 (m, 1H).

### Reference Example 30-1

(1S,2S)-2-(ethoxycarbonyl)cyclopropane-1-carboxylic acid

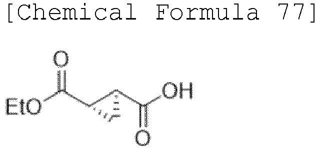

(1S,2S)-cyclopropane-1,2-dicarboxylic acid (2.5g, 19.2 mmol) was dissolved in ethanol (38 mL), thionyl chloride (3.48 mL, 48.0 mmol) and a small amount of DMF were added thereto, the mixture was stirred for 4 hours at room temperature, and then the solvent was distilled off under reduced pressure. Water and ethyl acetate were added to a residue thus obtained, the mixture was stirred for a while, subsequently an organic layer thus separated was washed with a saturated aqueous solution of sodium hydrogen carbonate, and the organic layer was dried over anhydrous sodium sulfate. Insoluble materials were filtered, subsequently the solvent was distilled off under reduced pressure, and thereby a crude form of (1S,2S)-2-(ethoxycarbonyl)cyclopropane-1-carboxylic acid (pale ivory-colored oily material, 3.66g) was obtained.

According to a technique similar to Reference Example 22-2, the title compound (yellow oily material, 2.31g, 76%) was obtained using the crude form of (1S,2S)-2-(ethoxycarbonyl)cyclopropane-1-carboxylic acid (3.66g) thus obtained.

¹H NMR (CDCl₃, 400 MHz): δ = 1.28 (t, 3H, J = 7 Hz), 1.4 - 1.6 (m, 2H), 2.1 - 2.3 (m, 2H), 4.16 (q, 2H, J = 7 Hz). The 1H content is not observable.

### Reference Example 30-2

### Ethyl (1S,2S)-2-((2-bromo-5-fluorophenyl)carbamoyl)cyclopropane-1-carboxylate

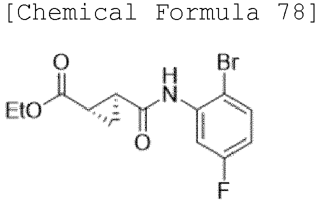

According to a technique similar to Reference Example 28-1, the title compound (ivory-colored powder, 530g, 51%) was obtained using (1S,2S)-2-(ethoxycarbonyl)cyclopropane-1-carboxylic acid (500 mg, 3.16 mmol) synthesized in Reference Example 30-1 and 2-bromoaniline (721 mg, 3.79 mmol).

¹H NMR (CDCl₃, 400 MHz): δ = 1.2 - 1.3 (m, 3H), 1.4 - 1.5 (m, 1H), 1.5 - 1.6 (m, 1H), 2.1 - 2.2 (m, 1H), 2.2 -

2.3 (m, 1H), 4.19 (q, 2H, J = 7 Hz), 6.7 - 6.8 (m, 1H), 7.49 (dd, 1H, J = 6, 8 Hz), 7.93 (br s, 1H), 8.23 (dd, 1H, J = 3, 11 Hz).

### Reference Example 30-3

Ethyl (1S,2S)-2-(5-fluorobenzo[d]oxazol-2-yl)cyclopropane-1-carboxylate

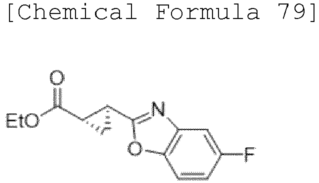

According to a technique similar to Reference Example 22-4, the title compound (brown oily material, 141 mg, 35%) was obtained using ethyl (1S,2S)-2-((2-bromo-5-fluorophenyl)carbamoyl)cyclopropane-1-carboxylate (530 mg, 1.61 mmol) synthesized in Reference Example 30-2.

¹H NMR (CDCl₃, 400 MHz): δ = 1.29 (t, 3H, J = 7 Hz), 1.7 - 1.8 (m, 2H), 2.4 - 2.5 (m, 1H), 2.7 - 2.8 (m, 1H), 4.20 (q, 2H, J = 7 Hz), 7.02 (ddd, 1H, J = 2, 9, 9 Hz), 7.31 (dd, 1H, J = 3, 8 Hz), 7.38 (dd, 1H, J = 4, 9 Hz).

### Reference Example 31-1

Dimethyl 2-(5-(trifluoromethyl)pyridin-2-yl)malonate

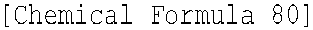

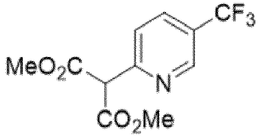

2-Chloro-5-(trifluoromethyl)pyridine (5.00g, 27.5 mmol), dimethyl malonate (5.46g, 41.3 mmol), and cesium carbonate (18.0g, 55.1 mmol) were dissolved in dimethyl sulfoxide (10.0 mL), and the solution was stirred overnight at 110°C under a nitrogen gas stream. To the reaction liquid that had been left to cool to room temperature, water was added, the mixture was stirred for a while, and then the mixture was extracted with ethyl acetate. An organic layer thus separated was washed with saturated brine and dried over anhydrous sodium sulfate, insoluble materials were filtered, and then the solvent was distilled off under reduced pressure. A residue thus obtained was purified by silica gel column chromatography (heptane: ethyl acetate) (concentration gradient: 25 to 75%), and the title compound (yellow oily material, 2.46g, 32%) was obtained.

¹H NMR (CDCl₃, 400 MHz): δ = 3.81 (s, 6H), 5.05 (s, 1H), 7.67 (d, 1H, J = 8 Hz), 7.97 (d, 1H, J = 8 Hz), 8.84 (s, 1H).

### Reference Example 31-2

2-(5-(Trifluoromethyl)pyridin-2-yl)acetic acid

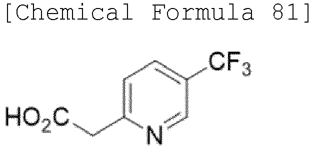

Dimethyl 2-(5-(trifluoromethyl)pyridin-2-yl)malonate (790 mg, 2.85 mmol) synthesized in Reference Example 31-1 was dissolved in methanol (18 mL), a 2N aqueous solution of sodium hydroxide (3.6 mL) was added thereto, and then the mixture was stirred overnight at 50°C

To the reaction liquid that had been left to cool to room temperature, a 2N aqueous solution of hydrochloric acid (3.5 mL) was added to adjust the pH of the aqueous layer to 6 to 7, subsequently the solvent was distilled off under reduced pressure, and thereby the title compound (pale yellow crystals, 864 mg) including sodium chloride was obtained.

¹H NMR (DMSO - d₆, 400 MHz): δ = 3.85 (s, 2H), 7.60 (d, 1H, J = 8 Hz), 8.15 (dd, 1H, J = 2, 8 Hz), 8.86 (s, 1H). The 1H content is not observable.

### Reference Example 32-1

Tert-butyl (R)-(1-(6-(trifluoromethyl)pyridin-3-yl)pyrrolidin-3-yl)carbamate

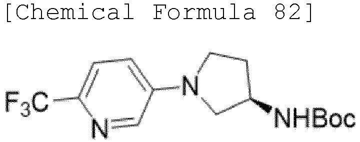

According to a technique similar to Reference Example 4, the title compound (pale yellow amorphous, 2.20g, 89%) was obtained using 5-bromo-2-(trifluoromethyl)pyridine (2.55g, 11.3 mmol) and tert-butyl (R)-pyrrolidin-3-ylcarbamate (1.50g, 7.49 mmol).

¹H NMR (CDCl₃, 400 MHz): δ = 1.46 (s, 9H), 1.9 - 2.0 (m, 1H), 2.2 - 2.4 (m, 1H), 3.2 - 3.3 (m, 1H), 3.3 - 3.6 (m, 2H), 3.6 - 3.7 (m, 1H), 4.3 - 4.5 (m, 1H), 4.6 - 4.8 (m, 1H), 6.82 (dd, 1H, J = 3, 9 Hz), 7.48 (d, 1H, J = 9 Hz), 8.01 (d, 1H, J = 3 Hz).

### Reference Example 32-2

(R)-1-(6-(trifluoromethyl)pyridin-3-yl)pyrrolidin-3-amine

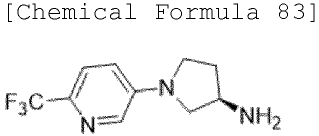

According to a technique similar to Reference Example 1-2, the title compound (pale yellow crystals, 1.50g, 97%) was obtained using tert-butyl (R)-(1-(6-(trifluoromethyl)pyridin-3-yl)pyrrolidin-3-yl)carbamate (2.20g, 6.64 mmol) synthesized in Reference Example 32-1.

¹H NMR (CDCl₃, 400 MHz): δ = 1.40 (br s, 2H), 1.8 - 2.0 (m, 1H), 2.2 - 2.3 (m, 1H), 3.0 - 3.1 (m, 1H), 3.3 - 3.5 (m, 1H), 3.5 - 3.6 (m, 2H), 3.7 - 3.9 (m, 1H), 6.80

(dd, 1H, J = 3, 9 Hz), 7.47 (d, 1H, J = 9 Hz), 8.00 (d, 1H, J = 3 Hz) .

### Reference Example 33-1

Ethyl 2-(4-cyclopropylphenyl)acetate

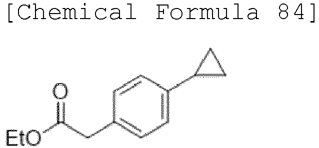

Ethyl 4-bromophenylacetate (6.0g, 24.7 mmol), cyclopropylboronic acid (2.76g, 32.1 mmol), palladium acetate (276 mg, 1.23 mmol), tricyclohexylphosphine (0.6M toluene solution, 4.2 mL, 2.46 mmol), and potassium phosphate monohydrate (19.9g, 86.4 mmol) were suspended in toluene (60.0 mL) and water (3.0 mL), and the suspension was stirred for 16 hours at 100°C under a nitrogen gas stream. The reaction liquid that had been left to cool to room temperature was filtered through a Celite pad, and then the solvent of the filtrate was distilled off under reduced pressure. A residue thus obtained was diluted with ethyl acetate, an organic layer was washed with an aqueous solution of sodium hydrogen carbonate, water, and saturated brine and then was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 0 to 30%), and the title compound (yellow oily material, 4.90g, 97%) was obtained.

¹H NMR (CDCl₃, 400 MHz): δ = 0.6 - 0.7 (m, 2H), 0.9 - 1.0 (m, 2H), 1.24 (t, 3H, J = 8 Hz), 1.8 - 1.9 (m, 1H), 3.52 (s, 2H), 4.12 (q, 2H, J = 8 Hz), 7.01 (d, 2H, J = 8 Hz), 7.15 (d, 2H, J = 8 Hz).

### Reference Example 33-2

2-(4-cyclopropylphenyl)acetic acid

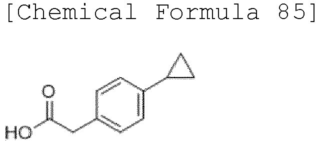

According to a technique similar to Reference Example 22-2, the title compound (white crystals, 3.80g, 90%) was obtained using ethyl 2-(4-cyclopropylphenyl)acetate (4.90g, 24.0 mmol) synthesized in Reference Example 33-1.

¹H NMR (CDCl₃, 400 MHz): δ = 0.6 - 0.7 (m, 2H), 0.9 - 1.0 (m, 2H), 1.8 - 2.0 (m, 1H), 3.60 (s, 2H), 7.03 (d, 2H, J = 8 Hz), 7.16 (d, 2H, J = 8 Hz). The 1H content is not observable.

### Reference Example 34

Tert-butyl (R)-(1-(4-(trifluoromethyl)phenyl)pyrrolidin-3-yl)carbamate

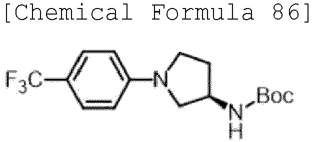

According to a technique similar to Reference Example 1-1, the title compound (pale yellow syrup, 273 mg, 77%) was obtained using 1-bromo-4-(trifluoromethyl)benzene (260 mg, 1.61 mmol) and tert-butyl (R)-pyrrolidin-3-ylcarbamate (200 mg, 1.07 mmol).

¹H NMR (CDCl₃, 400 MHz): δ = 1.45 (s, 9H), 1.9 - 2.1 (m, 1H), 2.2 - 2.4 (m, 1H), 3.20 (dd, 1H, J = 3, 10 Hz), 3.3 - 3.5 (m, 2H), 3.62 (dd, 1H, J = 6, 10 Hz), 4.38 (br s, 1H), 4.69 (br s, 1H), 6.55 (d, 2H, J = 9 Hz), 7.45 (d, 2H, J = 9 Hz).

### Reference Example 35

Tert-butyl (S)-(1-(4-(trifluoromethyl)phenyl)pyrrolidin-3-yl)carbamate

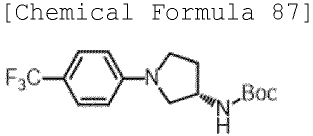

According to a technique similar to Reference Example 1-1, the title compound (pale yellow solid, 236 mg, 67%) was obtained using 1-bromo-4-(trifluoromethyl)benzene (260 mg, 1.61 mmol) and tert-butyl (S)-pyrrolidin-3-ylcarbamate (200 mg, 1.07 mmol).

¹H NMR (CDCl₃, 400 MHz): δ = 1.46 (s, 9H), 1.9 - 2.1 (m, 1H), 2.2 - 2.4 (m, 1H), 3.20 (dd, 1H, J = 3, 10 Hz), 3.3 - 3.5 (m, 2H), 3.62 (dd, 1H, J = 6, 10 Hz), 4.37 (br s, 1H), 4.69 (br s, 1H), 6.55 (d, 2H, J = 9 Hz), 7.45 (d, 2H, J = 9 Hz).

### Reference Example 36

Tert-butyl (R)-(1-(6-(trifluoromethyl)pyridin-3-yl)pyrrolidin-3-yl)carbamate

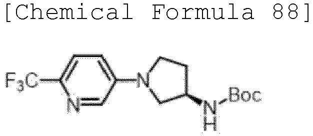

According to a technique similar to Reference Example 1-1, the title compound (pale yellow solid, 53 mg, 59%) was obtained using 5-bromo-2-(trifluoromethyl)pyridine (67 mg, 0.29 mmol) and tert-butyl (R)-pyrrolidin-3-ylcarbamate (50 mg, 0.27 mmol).

¹H NMR (CDCl₃, 400 MHz): δ = 1.45 (s, 9H), 1.9 - 2.1 (m, 1H), 2.2 - 2.4 (m, 1H), 3.23 (dd, 1H, J = 4, 10 Hz), 3.3 - 3.6 (m, 2H), 3.65 (dd, 1H, J = 6, 10 Hz), 4.40 (br s, 1H), 4.81 (br s, 1H), 6.82 (dd, 1H, J = 3, 9 Hz), 7.47 (d, 1H, J = 9 Hz), 7.99 (d, 1H, J = 3 Hz).

Reference Example 37

Tert-butyl (S)-(1-(6-(trifluoromethyl)pyridin-3-yl)pyrrolidin-3-yl)carbamate

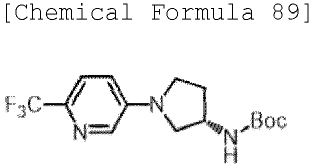

According to a technique similar to Reference Example 1-1, the title compound (pale yellow solid, 59 mg, 66%) was obtained using 5-bromo-2-(trifluoromethyl)pyridine (67 mg, 0.29 mmol) and tert-butyl (S)-pyrrolidin-3-ylcarbamate (50 mg, 0.27 mmol).

¹H NMR (CDCl₃, 400 MHz): δ = 1.45 (s, 9H), 1.9 - 2.1 (m, 1H), 2.2 - 2.4 (m, 1H), 3.24 (dd, 1H, J = 4, 10 Hz), 3.3 - 3.6 (m, 2H), 3.65 (dd, 1H, J = 6, 10 Hz), 4.40 (br s, 1H), 4.89 (br s, 1H), 6.82 (dd, 1H, J = 3, 9 Hz), 7.47 (d, 1H, J = 9 Hz), 7.99 (d, 1H, J = 3 Hz).

### Reference Example 38-1

Tert-butyl (R)-(1-(5-(trifluoromethyl)pyrimidin-2-yl)pyrrolidin-3-yl)carbamate

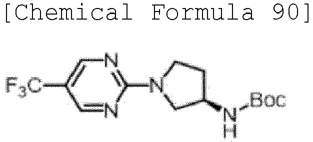

According to a technique similar to Reference Example 9, the title compound (white solid, 86 mg, 97%) was obtained using 2-chloro-5-(trifluoromethyl)pyrimidine (49 mg, 0.27 mmol) and tert-butyl (R)-pyrrolidin-3-ylcarbamate (50 mg, 0.27 mmol).

¹H NMR (CDCl₃, 400 MHz): δ = 1.45 (s, 9H), 1.9 - 2.1 (m, 1H), 2.2 - 2.4 (m, 1H), 3.50 (dd, 1H, J = 5, 12 Hz), 3.6 - 3.8 (m, 2H), 3.88 (dd, 1H, J = 6, 12 Hz), 4.36 (br s, 1H), 4.73 (d, 1H, J = 7 Hz), 8.50 (s, 2H).

### Reference Example 38-2

(R)-1-(5-(trifluoromethyl)pyrimidin-2-yl)pyrrolidin-3-amine

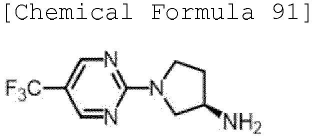

According to a technique similar to Reference Example 1-2, the title compound (white solid, 45 mg, 75%) was obtained using tert-butyl (R)-(1-(5-(trifluoromethyl)pyrimidin-2-yl)pyrrolidin-3-yl)carbamate (86 mg, 0.26 mmol) synthesized in Reference Example 38-1.

¹H NMR (CDCl₃, 400 MHz): δ = 1.8 - 1.9 (m, 1H), 2.1 - 2.3 (m, 1H), 3.3 - 3.4 (m, 1H), 3.6 - 3.9 (m, 4H), 8.50 (s, 2H). The 2H content is not observable.

### Reference Example 39

Tert-butyl (R)-(1-(4-fluoro-3-(trifluoromethyl)phenyl)pyrrolidin-3-yl)carbamate

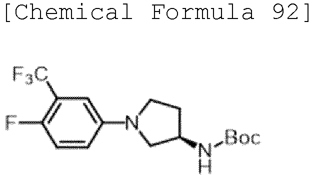

According to a technique similar to Reference Example 1-1, the title compound (pale yellow solid, 112 mg, 60%) was obtained using 4-bromo-1-fluoro-2-(trifluoromethyl)benzene (144 mg, 0.59 mmol) and tert-butyl (R)-pyrrolidin-3-ylcarbamate (100 mg, 0.54 mmol).

¹H NMR (CDCl₃, 400 MHz): δ = 1.45 (s, 9H), 1.9 - 2.1 (m, 1H), 2.2 - 2.4 (m, 1H), 3.15 (dd, 1H, J = 4, 9 Hz), 3.2 - 3.5 (m, 2H), 3.55 (dd, 1H, J = 6, 10 Hz), 4.37 (br s, 1H), 4.79 (d, 1H, J = 7 Hz), 6.5 - 6.6 (m, 2H), 7.04 (t, 1H, J = 10 Hz).

### Reference Example 40-1

(1R,2S,5R)-2-isopropyl-5-methylcyclohexyl (1S,2S)-2-((2-formylphenyl)carbamoyl)cyclopropane-1-carboxylate

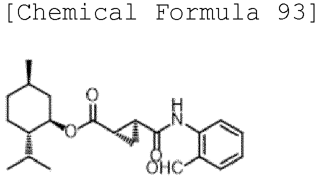

According to a technique similar to Reference Example 28-1, a crude form of the title compound was obtained using (1S,2S)-2-((((1R,2S,5R)-2-isopropyl-5-methylcyclohexyl)oxy)carbonyl)cyclopropane-1-carboxylic acid (1.17g, 4.35 mmol) and 2-aminobenzaldehyde (988 mg, 8.12 mmol).

### Reference Example 40-2

(1R,2S,5R)-2-isopropyl-5-methylcyclohexyl (1S,2S)-2-(quinazolin-2-yl)cyclopropane-1-carboxylate

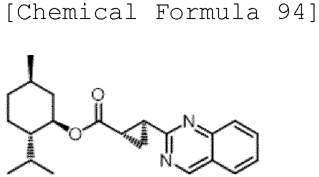

The crude form of (1R,2S,5R)-2-isopropyl-5-methylcyclohexyl (1S,2S)-2-((2-formylphenyl)carbamoyl)cyclopropane-1-carboxylate synthesized in Reference Example 40-1 and ammonium acetate were heated to reflux for 10 hours at 120°C in toluene. The reaction liquid was left to cool to room temperature, sodium hydrogen carbonate was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 10 to 50%), and the title compound (277 mg, 18%) was obtained.

¹H NMR (CDCl₃, 400 MHz): δ = 0.76 (d, 3H, J = 7 Hz), 0.80 (d, 3H, J = 7 Hz), 0.8 - 2.1 (m, 12H), 2.1 - 2.3 (m, 2H), 2.4 - 2.5 (m, 1H), 2.9 - 3.0 (m, 1H), 4.6 - 4.8 (m, 1H), 7.58 (ddd, 1H, J = 1, 7, 7 Hz), 7.8 - 8.0 (m, 3H), 9.27 (s, 1H).

### Reference Example 40-3

(1S,2S)-2-(quinazolin-2-yl)cyclopropane-1-carboxylic acid

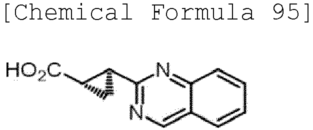

(1R,2S,5R)-2-isopropyl-5-methylcyclohexyl (1S,2S)-2-(quinazolin-2-yl)cyclopropane-1-carboxylate (277 mg, 0.79 mmol) synthesized in Reference Example 40-2 was dissolved in isopropanol (2.1 mL) and water (170 µL), sodium hydroxide (63 mg, 1.57 mmol) was added thereto, the mixture was stirred for 25 hours at 80°C, and then the solvent was distilled off under reduced pressure. 3N hydrochloric acid and water were added to a residue thus obtained, and the mixture was extracted with ethyl acetate. An organic layer thus separated was dried over anhydrous sodium sulfate, a solid precipitated using ethyl acetate and hexane was collected by filtration, and the title compound (pale yellow solid, 73 mg, 43%) was obtained.

¹H NMR (CDCl₃, 400 MHz): δ = 1.6 - 1.7 (m, 1H), 1.7 - 1.9 (m, 1H), 2.3 - 2.4 (m, 1H), 2.8 - 2.9 (m, 1H), 7.67 (ddd, 1H, J = 1, 7, 7 Hz), 7.91 (d, 1H, J = 7 Hz), 7.97 (ddd, 1H, J = 1, 7, 7 Hz), 8.03 (dd, 1H, J = 1, 7 Hz), 9.39 (s, 1H). The 1H content is not observable.

### Reference Example 41-1

Tert-butyl (R)-(1-(3-(methylsulfonyl)phenyl)pyrrolidin-3-yl)carbamate

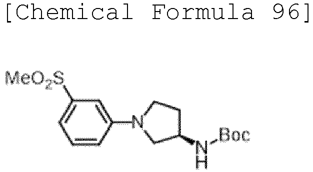

According to a technique similar to Reference Example 1-1, the title compound (pale yellow oil, 57 mg, 62%) was obtained using 1-bromo-3-(methylsulfonyl)benzene (63 mg, 0.27 mmol) and tert-butyl (R)-pyrrolidin-3-ylcarbamate (50 mg, 0.27 mmol).

¹H NMR (CDCl₃, 400 MHz): δ = 1.46 (s, 9H), 1.9 - 2.1 (m, 1H), 2.2 - 2.4 (m, 1H), 3.04 (s, 3H), 3.21 (dd, 1H, J = 4, 10 Hz), 3.3 - 3.5 (m, 2H), 3.63 (dd, 1H, J = 6, 10 Hz), 4.34 (br s, 1H), 4.78 (br s, 1H), 6.75 (dd, 1H, J = 2, 8 Hz), 7.02 (t, 1H, J = 2 Hz), 7.20 (d, 1H, J = 8 Hz), 7.38 (t, 1H, J = 8 Hz) .

### Reference Example 41-2

(R)-1-(3-(methylsulfonyl)phenyl)pyrrolidin-3-amine

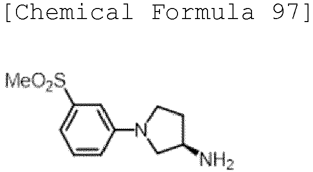

According to a technique similar to Reference Example 1-2, the title compound (pale yellow oil, 12 mg, 30%) was obtained using tert-butyl (R)-(1-(3-(methylsulfonyl)phenyl)pyrrolidin-3-yl)carbamate (57 mg, 0.17 mmol) synthesized in Reference Example 41-1.

¹H NMR (CDCl₃, 400 MHz): δ = 1.8 - 1.9 (m, 1H), 2.2 - 2.3 (m, 1H), 3.04 (s, 3H), 3.78 (dd, 1H, J = 6, 10 Hz), 3.3 - 3.6 (m, 3H), 3.77 (quin, 1H, J = 6 Hz), 6.74 (dd, 1H, J = 2, 8 Hz), 7.02 (t, 1H, J = 2 Hz), 7.17 (d, 1H, J = 8 Hz), 7.37 (t, 1H, J = 8 Hz). The 2H content is not observable.

### Reference Example 42-1

Tert-butyl (R)-(1-(3-cyanophenyl)pyrrolidin-3-yl)carbamate

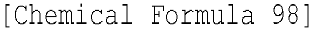

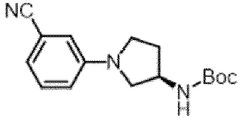

According to a technique similar to Reference Example 1-1, the title compound (pale yellow amorphous, 57 mg, 74%) was obtained using 3-bromobenzonitrile (54 mg, 0.30 mmol) and tert-butyl (R)-pyrrolidin-3-ylcarbamate (50 mg, 0.27 mmol).

¹H NMR (CDCl₃, 400 MHz): δ = 1.46 (s, 9H), 1.9 - 2.1 (m, 1H), 2.2 - 2.4 (m, 1H), 3.16 (dd, 1H, J = 4, 10 Hz), 3.3 - 3.5 (m, 2H), 3.57 (dd, 1H, J = 6, 10 Hz), 4.37 (br s, 1H), 4.76 (d, 1H, J = 6 Hz), 6.7 - 6.8 (m, 2H), 6.94 (d, 1H, J = 7 Hz), 7.2 - 7.3 (m, 1H).

### Reference Example 42-2

(R)-3-(3-aminopyrrolidin-1-yl)benzonitrile

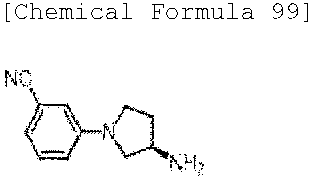

According to a technique similar to Reference Example 1-2, the title compound (pale yellow oil, 32 mg, 85%) was obtained using tert-butyl (R)-(1-(3-cyanophenyl)pyrrolidin-3-yl)carbamate (57 mg, 0.20 mmol) synthesized in Reference Example 42-1.

¹H NMR (CDCl₃, 400 MHz): δ = 1.8 - 1.9 (m, 1H), 2.2 - 2.3 (m, 1H), 3.11 (dd, 1H, J = 5, 9 Hz), 3.2 - 3.6 (m, 3H), 3.76 (quin, 1H, J = 6 Hz), 6.6 - 6.8 (m, 2H), 6.91 (d, 1H, J = 7 Hz), 7.2 - 7.3 (m, 1H). The 2H content is not observable.

### Reference Example 43-1

Ethyl trans-2-((2-amino-5-cyanophenyl)carbamoyl)cyclopropane-1-carboxylate

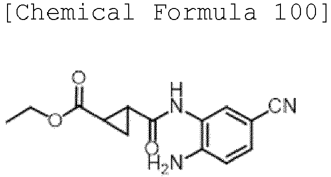

According to a technique similar to Reference Example 7-1, a crude form of the title compound was obtained using trans-2-(ethoxycarbonyl)cyclopropane-1-carboxylic acid (100 mg, 0.63 mmol) and 3,4-diaminobenzonitrile (101 mg, 0.76 mmol).

¹H NMR (CDCl₃, 400 MHz): δ = 1.27 (t, 3H, J = 7 Hz), 1.3 - 1.6 (m, 2H), 2.1 - 2.4 (m, 2H), 4.15 (q, 2H, J = 7 Hz), 4.75 (br s, 2H), 6.72 (d, 1H, J = 8 Hz), 6.72 (d, 1H, J = 8 Hz), 7.22 (dd, 1H, J = 2, 8 Hz), 7.57 (d, 1H, J = 2 Hz).

### Reference Example 43-2

Ethyl trans-2-(5-cyano-1H-benzo[d]imidazol-2-yl)cyclopropane-1-carboxylate

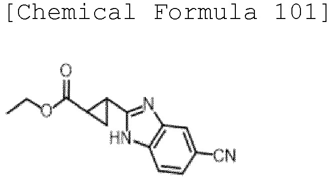

The crude form of ethyl trans-2-((2-amino-5-cyanophenyl)carbamoyl)cyclopropane-1-carboxylate synthesized in Reference Example 43-1 was heated for 5 hours at 85°C in acetic acid and then was left to cool to room temperature, and the solvent was distilled off under reduced pressure. An aqueous solution of sodium hydrogen carbonate was added thereto, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (hexane: ethyl acetate) (concentration gradient: 40 to 80%), and thus the title compound (light brown amorphous, 142 mg, 88%) was obtained.

¹H NMR (CDCl₃, 400 MHz): δ = 1.27 (t, 3H, J = 7 Hz), 1.7 - 1.9 (m, 2H), 2.4 - 2.6 (m, 1H), 2.7 - 2.8 (m, 1H), 4.15 (q, 2H, J = 7 Hz), 7.4 - 8.0 (m, 2H), 7.49 (d, 1H, J = 7 Hz), 11.58 (br s, 1H).

### Reference Example 43-3

Trans-2-(5-cyano-1H-benzo[d]imidazol-2-yl)cyclopropane-1-carboxylic acid

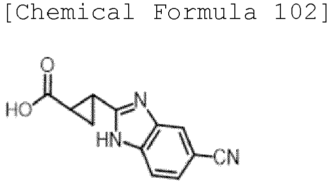

Ethyl trans-2-(5-cyano-1H-benzo[d]imidazol-2-yl)cyclopropane-1-carboxylate (142 mg, 0.56 mmol) synthesized in Reference Example 43-2 was dissolved in methanol (6 mL) and water (1 mL), lithium hydroxide monohydrate (47 mg, 1.11 mmol) was added thereto, the mixture was stirred at room temperature, and then the solvent was distilled off under reduced pressure. 3N hydrochloric acid was added to a residue thus obtained, and the solvent was distilled off under reduced pressure. A solid precipitated using ethyl acetate and hexane was collected by filtration, and the title compound (85 mg, 67%) was obtained.

¹H NMR (DMSO - d₆, 400 MHz): δ = 1.6 - 1.8 (m, 2H), 2.2 - 2.4 (m, 1H), 2.6 - 2.8 (m, 1H), 7.52 (dd, 1H, J = 1, 8 Hz), 7.61 (d, 1H, J = 8 Hz), 7.87 (s, 1H). The 2H content is not observable.

### Reference Example 44

Tert-butyl (R)-(1-(2-(trifluoromethyl)pyridin-4-yl)pyrrolidin-3-yl)carbamate

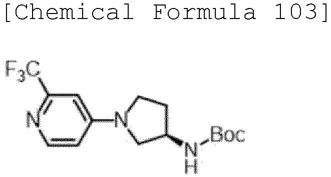

According to a technique similar to Reference Example 9, the title compound (white amorphous) was obtained using 4-chloro-2-(trifluoromethyl)pyridine (49 mg, 0.27 mmol) and tert-butyl (R)-pyrrolidin-3-ylcarbamate (50 mg, 0.27 mmol).

¹H NMR (CDCl₃, 400 MHz): δ = 1.46 (s, 9H), 1.9 - 2.1 (m, 1H), 2.2 - 2.4 (m, 1H), 3.24 (dd, 1H, J = 4, 10 Hz), 3.4 - 3.6 (m, 2H), 3.66 (dd, 1H, J = 6, 10 Hz), 4.38 (br s, 1H), 4.8 - 5.1 (m, 1H), 6.46 (dd, 1H, J = 2, 6 Hz), 6.72 (d, 1H, J = 2 Hz), 8.28 (d, 1H, J = 6 Hz).

### Reference Example 45-1

Tert-butyl (R)-(1-(6-(trifluoromethyl)pyridin-3-yl)piperidin-3-yl)carbamate

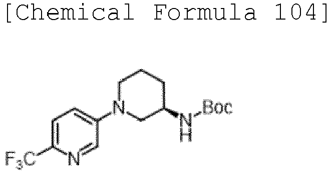

According to a technique similar to Reference Example 1-1, the title compound (light brown solid, 72 mg, 84%) was obtained using 5-bromo-2-(trifluoromethyl)pyridine (62 mg, 0.27 mmol) and tert-butyl (R)-piperidin-3-ylcarbamate (50 mg, 0.25 mmol).

¹H NMR (CDCl₃, 400 MHz): δ = 1.4 - 2.0 (m, 4H), 1.46 (s, 9H), 2.9 - 4.0 (m, 5H), 4.5 - 4.9 (m, 1H), 7.33 (s, 1H), 8.31 (s, 1H), 8.46 (d, 1H, J = 3 Hz).

### Reference Example 45-2

(R)-1-(6-(trifluoromethyl)pyridin-3-yl)piperidin-3-amine

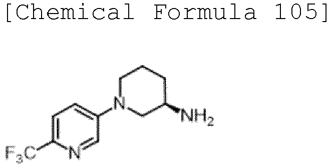

According to a technique similar to Reference Example 1-2, the title compound (light brown syrup, 50 mg, 97%) was obtained using tert-butyl (R)-(1-(6-(trifluoromethyl)pyridin-3-yl)piperidin-3-yl)carbamate (72 mg, 0.21 mmol) synthesized in Reference Example 45-1.

¹H NMR (CDCl₃, 400 MHz): δ = 1.2 - 1.4 (m, 1H), 1.6 - 1.8 (m, 1H), 1.8 - 2.1 (m, 2H), 2.68 (dd, 1H, J = 9, 12 Hz), 2.8 - 3.1 (m, 2H), 3.5 - 3.6 (m, 1H), 3.6 - 3.7 (m, 1H), 7.32 (s, 1H), 8.28 (d, 1H, J = 1 Hz), 8.45 (d, 1H, J = 3 Hz). The 2H content is not observable.

### Reference Example 46-1

2-Chloro-4-(trifluoromethyl) thiazole

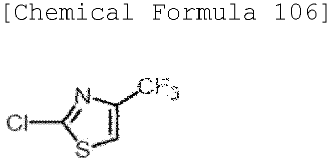

4-(Trifluoromethyl)thiazol-2-amine (100 mg, 0.60 mmol) and copper(II) chloride (96 mg, 0.71 mmol) were suspended in acetonitrile (8 mL), tert-butyl nitrite (92 mg, 0.89 mmol) was added thereto, and the mixture was stirred for one hour at room temperature. The reaction liquid was added to 1N hydrochloric acid that had been ice-cooled, and the reaction liquid was extracted with diethyl ether. An organic layer thus separated was dried over anhydrous sodium sulfate, insoluble materials were filtered, subsequently the solvent was distilled off at 30°C under reduced pressure (700 mbar), and thereby a crude form of the title compound was obtained.

¹H NMR (CDCl₃, 400 MHz): δ = 7.69 (s, 1H) .

### Reference Example 46-2

Tert-butyl (R)-(1-(4-(trifluoromethyl)thiazol-2-yl)pyrrolidin-3-yl)carbamate

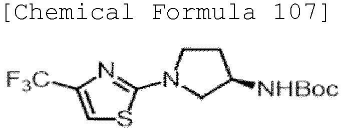

Tert-butyl (R)-pyrrolidin-3-ylcarbamate (111 mg, 0.60 mmol), the crude form of 2-chloro-4-(trifluoromethyl)thiazole synthesized in Reference Example 46-1, and potassium carbonate (99 mg, 0.72 mmol) were dissolved in acetonitrile (8 mL), and then the mixture was stirred for 17 hours at 60°C

To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (hexane: ethyl acetate) (concentration gradient: 10 to 60%), and the title compound (pale yellow solid, 147 mg, 73%) was obtained.

¹H NMR (CDCl₃, 400 MHz): δ = 1.45 (s, 9H), 1.9 - 2.1 (m, 1H), 2.2 - 2.4 (m, 1H), 3.35 (dd, 1H, J = 4, 10 Hz), 3.5 - 3.7 (m, 2H), 3.75 (dd, 1H, J = 6, 10 Hz), 4.38 (br s, 1H), 4.82 (br s, 1H), 6.92 (s, 1H).

### Reference Example 46-3

(R)-1-(4-(trifluoromethyl)thiazol-2-yl)pyrrolidin-3-amine

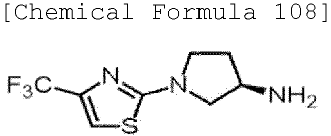

According to a technique similar to Reference Example 1-2, the title compound (pale yellow syrup, 94 mg, 91%) was obtained using tert-butyl (R)-(1-(4-(trifluoromethyl)thiazol-2-yl)pyrrolidin-3-yl)carbamate (147 mg, 0.44 mmol) synthesized in Reference Example 46-2.

¹H NMR (CDCl₃, 400 MHz): δ = 1.8 - 2.0 (m, 1H), 2.2 - 2.3 (m, 1H), 3.23 (dd, 1H, J = 4, 10 Hz), 3.4 - 3.6 (m, 1H), 3.6 - 3.7 (m, 2H), 3.7 - 3.9 (m, 1H), 6.90 (d, 1H, J = 1 Hz). The 2H content is not observable.

### Reference Example 47-1

Tert-butyl (R)-(1-(4-(trifluoromethyl)phenyl)piperidin-3-yl)carbamate

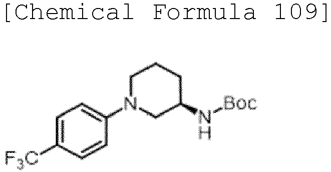

According to a technique similar to Reference Example 1-1, the title compound (pale yellow solid, 59 mg, 68%) was obtained using 1-bromo-4-(trifluoromethyl)benzene (62 mg, 0.27 mmol) and tert-butyl (R)-piperidin-3-ylcarbamate (50 mg, 0.25 mmol).

¹H NMR (CDCl₃, 400 MHz): δ = 1.4 - 1.9 (m, 13H), 2.8 - 3.9 (m, 5H), 4.4 - 4.9 (m, 1H), 6.94 (d, 2H, J = 9 Hz), 7.46 (d, 2H, J = 9 Hz).

### Reference Example 47-2

(R)-1-(4-(trifluoromethyl)phenyl)piperidin-3-amine

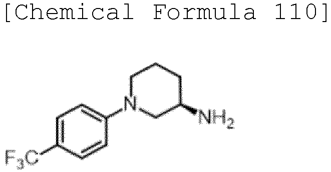

According to a technique similar to Reference Example 1-2, the title compound (light brown syrup, 42 mg, 100%) was obtained using tert-butyl (R)-(1-(4-(trifluoromethyl)phenyl)piperidin-3-yl)carbamate (59 mg, 0.17 mmol) synthesized in Reference Example 47-1.

¹H NMR (CDCl₃, 400 MHz): δ = 1.2 - 1.4 (m, 1H), 1.6 - 1.9 (m, 2H), 1.9 - 2.1 (m, 1H), 2.67 (dd, 1H, J = 9, 12 Hz), 2.8 - 3.0 (m, 2H), 3.54 (dt, 1H, J = 4, 12 Hz), 3.6 - 3.7 (m, 1H), 6.92 (d, 2H, J = 9 Hz), 7.45 (d, 2H, J = 9 Hz). The 2H content is not observable.

### Reference Example 48-1

Tert-butyl (R)-(1-(6-methoxy-5-(trifluoromethyl)pyridin-3-yl)pyrrolidin-3-yl)carbamate

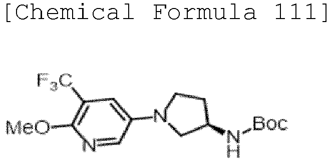

According to a technique similar to Reference Example 1-1, the title compound (pale yellow solid, 26 mg, 13%) was obtained using 5-bromo-2-methoxy-3-(trifluoromethyl)pyridine (151 mg, 0.59 mmol) and tert-butyl (R)-pyrrolidin-3-ylcarbamate (100 mg, 0.54 mmol).

¹H NMR (CDCl₃, 400 MHz): δ = 1.46 (s, 9H), 1.9 - 2.0 (m, 1H), 2.2 - 2.4 (m, 1H), 3.15 (dd, 1H, J = 4, 9 Hz), 3.29 (dt, 1H, J = 6, 9 Hz), 3.3 - 3.5 (m, 1H), 3.53 (dd, 1H, J = 6, 10 Hz), 3.96 (s, 3H), 4.38 (br s, 1H), 4.75 (d, 1H, J = 7 Hz), 7.12 (d, 1H, J = 3 Hz), 7.62 (d, 1H, J = 3 Hz) .

### Reference Example 48-2

(R)-1-(6-methoxy-5-(trifluoromethyl)pyridin-3-yl)pyrrolidin-3-amine

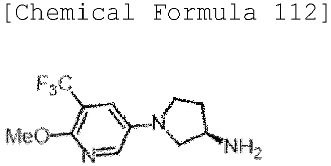

According to a technique similar to Reference Example 1-2, the title compound (pale yellow syrup, 18 mg, 100%) was obtained using tert-butyl (R)-(1-(6-methoxy-5-(trifluoromethyl)pyridin-3-yl)pyrrolidin-3-yl)carbamate (26 mg, 0.07 mmol) synthesized in Reference Example 48-1.

¹H NMR (CDCl₃, 400 MHz): δ = 1.7 - 1.9 (m, 1H), 2.2-2.3 (m, 1H), 3.01 (dd, 1H, J = 5, 9 Hz), 3.30 (dt, 1H, J = 6, 8 Hz), 3.4 - 3.5 (m, 2H), 3.76 (quin, 1H, J = 6 Hz), 3.96 (s, 3H), 7.11 (d, 1H, J = 3 Hz), 7.62 (d, 1H, J = 3 Hz). The 2H content is not observable.

### Reference Example 49-1

Tert-butyl (R)-(1-(4-(tert-butyl)thiazol-2-yl)pyrrolidin-3-yl)carbamate

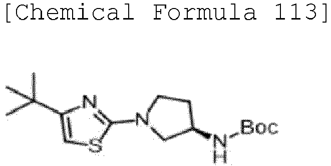

According to a technique similar to Reference Example 9, the title compound (colorless oil, 103 mg, 70%) was obtained using 2-bromo-4-(tert-butyl)thiazole (100 mg, 0.45 mmol) and tert-butyl (R)-pyrrolidin-3-ylcarbamate (127 mg, 0.68 mmol).

¹H NMR (CDCl₃, 400 MHz): δ = 1.27 (s, 9H), 1.45 (s, 9H), 1.9 - 2.0 (m, 1H), 2.2 - 2.4 (m, 1H), 3.31 (dd, 1H, J = 4, 10 Hz), 3.4 - 3.6 (m, 2H)), 3.71 (dd, 1H, J = 6, 11 Hz), 4.35 (br s, 1H), 4.74 (br s, 1H), 6.06 (s, 1H).

### Reference Example 49-2

(R)-1-(4-(tert-butyl)thiazol-2-yl)pyrrolidin-3-amine

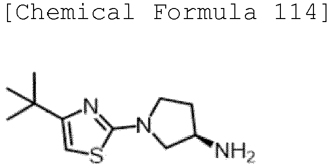

According to a technique similar to Reference Example 1-2, the title compound (colorless oil, 65 mg, 91%) was obtained using tert-butyl (R)-(1-(4-(tert-butyl)thiazol-2-yl)pyrrolidin-3-yl)carbamate (103 mg, 0.32 mmol) synthesized in Reference Example 49-1.

¹H NMR (CDCl₃, 400 MHz): δ = 1.27 (s, 9H), 1.7 - 1.9 (m, 1H), 2.1 - 2.3 (m, 1H), 3.17 (dd, 1H, J = 4, 10 Hz), 3.4 - 3.8 (m, 4H), 6.04 (s, 1H). The 2H content is not observable.

### Reference Example 50

Tert-butyl (R)-(1-(6-(trifluoromethyl)pyridazin-3-yl)pyrrolidin-3-yl)carbamate

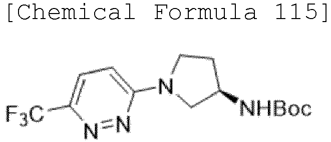

According to a technique similar to Reference Example 9, the title compound (white powder, 90 mg, 84%) was obtained using 3-chloro-6-(trifluoromethyl)pyridazine (60 mg, 0.322 mmol) and tert-butyl (R)-pyrrolidin-3-ylcarbamate (59 mg, 0.322 mmol).

¹H NMR (CDCl₃, 400 MHz): δ = 1.46 (s, 9H), 2.0 - 2.1 (m, 1H), 2.3 - 2.4 (m, 1H), 3.4 - 4.0 (m, 4H), 4.41 (br s, 1H), 4.71 (br s, 1H), 6.67 (d, 1H, J = 10 Hz), 7.48 (d, 1H, J = 10 Hz).

### Reference Example 51

Tert-butyl (R)-(1-(2-(trifluoromethyl)pyrimidin-5-yl)pyrrolidin-3-yl)carbamate

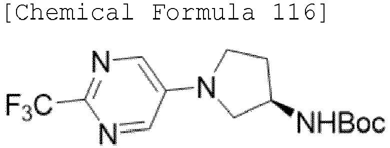

According to a technique similar to Reference Example 1-1, the title compound (yellow powder, 129 mg, 72%) was obtained using 5-bromo-2-(trifluoromethyl)pyrimidine (134 mg, 0.591 mmol) and tert-butyl (R)-pyrrolidin-3-ylcarbamate (100 mg, 0.537 mmol).

¹H NMR (CDCl₃, 400 MHz): δ = 1.46 (s, 9H), 2.0 - 2.1 (m, 1H), 2.3 - 2.5 (m, 1H), 3.28 (dd, 1H, J = 4, 10 Hz), 3.4 - 3.6 (m, 2H), 3.70 (dd, 1H, J = 6, 10 Hz), 4.42 (br s, 1H), 4.69 (br s, 1H), 8.10 (s, 2H).

### Reference Example 52

Tert-butyl (R)-(1-(6-fluorobenzo[d]thiazol-2-yl)pyrrolidin-3-yl)carbamate

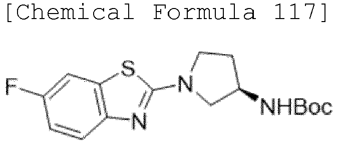

Tert-butyl (R)-pyrrolidin-3-ylcarbamate (50 mg, 0.268 mmol) and 2-chloro-6-fluorobenzothiazole (58 mg, 0.309 mmol) were dissolved in DMF (1 mL), subsequently cesium carbonate (101 mg, 0.309 mmol) was added thereto, and the mixture was stirred for 2 hours at 100°C

To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 25 to 40%), and the title compound (pale yellow powder, 85 mg, 94%) was obtained.

¹H NMR (CDCl₃, 400 MHz): δ = 1.46 (s, 9H), 2.0 - 2.1 (m, 1H), 2.3 - 2.4 (m, 1H), 3.44 (dd, 1H, J = 4, 10 Hz), 3.6 - 3.8 (m, 2H), 3.83 (dd, 1H, J = 6, 10 Hz), 4.41 (br s, 1H), 4.70 (br s, 1H), 7.02 (dd, 1H, J = 3, 9 Hz), 7.32 (dd, 1H, J = 3, 8 Hz), 7.50 (dd, 1H, J = 5, 9 Hz).

### Reference Example 53

Tert-butyl (R)-(1-(6-fluoroquinolin-2-yl)pyrrolidin-3-yl)carbamate

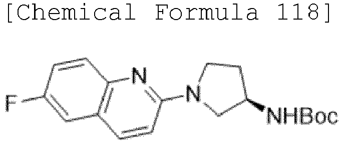

Tert-butyl (R)-pyrrolidin-3-ylcarbamate (50 mg, 0.268 mmol) and 2-chloro-6-fluoroquinoline (54 mg, 0.295 mmol) were dissolved in toluene (1.5 mL) and water (150 µL), subsequently potassium carbonate (44 mg, 0.322 mmol) were added thereto, and the mixture was heated to reflux overnight in a nitrogen atmosphere. To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 20 to 32%), and the title compound (white powder, 39 mg, 44%) was obtained.

¹H NMR (CDCl₃, 400 MHz): δ = 1.46 (s, 9H), 1.9 - 2.1 (m, 1H), 2.2 - 2.4 (m, 1H), 3.51 (dd, 1H, J = 4, 10 Hz), 3.6 - 3.8 (m, 2H), 3.87 (dd, 1H, J = 6, 10 Hz), 4.39 (br s, 1H), 4.73 (br s, 1H), 6.75 (d, 1H, J = 9 Hz), 7.2 - 7.4 (m, 2H), 7.67 (dd, 1H, J = 6, 9 Hz), 7.82 (d, 1H, J = 9 Hz).

### Reference Example 54-1

(1R,2S,5R)-2-isopropyl-5-methylcyclohexyl (1S,2S)-2-(1-cyclopropyl-1H-benzo[d]imidazol-2-yl)cyclopropane-1-carboxylate

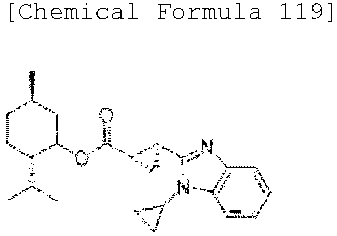

(1R,2S,5R)-2-isopropyl-5-methylcyclohexyl (1S,2S)-2-(1H-benzo[d]imidazol-2-yl)cyclopropane-1-carboxylate (408 mg, 1.1 mmol), cyclopropylboronic acid (184 mg, 2.1 mmol), and sodium carbonate (227 mg, 2.1 mmol) were dissolved in toluene (4 mL), subsequently copper(II) acetate monohydrate (214 mg, 1.1 mmol) and 2,2'-bipyridyl (167 mg, 1.1 mmol) suspended in toluene (7 mL) were added thereto, and the mixture was stirred overnight at 70°C

A saturated aqueous solution of ammonium chloride and water were added to the reaction liquid, and the mixture was extracted with ethyl acetate. An organic layer thus separated was washed with saturated brine and dried over anhydrous sodium sulfate, insoluble materials were filtered, and then the solvent was distilled off under reduced pressure. A residue thus obtained was purified by silica gel column chromatography (hexane: ethyl acetate) (concentration gradient: 10% to 25%), and the title compound (colorless oily material, 270 mg, 66%) was obtained.

¹H NMR (CDCl₃, 400 MHz): δ = 0.80 (d, 3H, J = 7 Hz), 0.9 - 1.5 (m, 15H), 1.6 - 2.1 (m, 6H), 2.32 (ddd, 1H, J = 4, 6, 9 Hz), 2.87 (ddd, 1H, J = 4, 6, 9 Hz), 3.30 (ddd, 1H, J = 4, 7, 11 Hz), 4.73 (td, 1H, J = 4, 11 Hz), 7.2 - 7.3 (m, 2H), 7.4 - 7.7 (m, 2H).

### Reference Example 54-2

(1S,2S)-2-(1-cyclopropyl-1H-benzo[d]imidazol-2-yl)cyclopropane-1-carboxylic acid

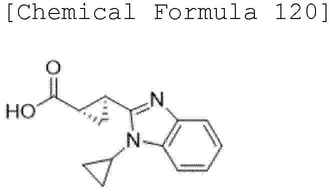

(1R,2S,5R)-2-isopropyl-5-methylcyclohexyl (1S,2S)-2-(1-cyclopropyl-1H-benzo[d]imidazol-2-yl)cyclopropane-1-carboxylate (270 mg, 0.709 mmol) synthesized in Reference Example 54-1 was suspended in isopropanol (2 mL) and water (150 µL), subsequently sodium hydroxide (57 mg, 1.42 mmol) was added thereto, and the mixture was stirred for 5 hours at 80°C

The solvent was distilled off under reduced pressure, subsequently a 0.2 Normal aqueous solution of sodium hydroxide (20 mL) was added to a residue thus obtained, and the mixture was washed twice with diethyl ether. 1 Normal hydrochloric acid (15 mL) was added to the aqueous layer to made the aqueous layer acidic, subsequently to a residue obtained by distilling off the solvent, toluene and water were added, and the mixture was suspended. A solid thus obtained was collected by filtration and dried under reduced pressure at room temperature, and the title compound (white powder, 48 mg, 28%) was obtained.

¹H NMR (DMSO - d₆, 400 MHz): δ = 1.1 - 1.4 (m, 4H), 1.71 (quin, 1H, J = 5 Hz), 1.8 - 1.9 (m, 1H), 2.3 - 2.5 (m, 1H), 2.93 (ddd, 1H, J = 4, 6, 9 Hz), 3.5 - 3.7 (m, 1H), 7.44 (quin, 2H, J = 8 Hz), 7.67 (d, 1H, J = 8 Hz), 7.77 (d, 1H, J = 8 Hz). The 1H content is not observable.

### Reference Example 55

Tert-butyl (R)-(1-(5-bromopyridin-3-yl)pyrrolidin-3-yl)carbamate

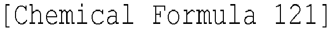

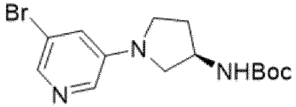

According to a technique similar to Reference Example 1-1, the title compound (white powder, 71 mg, 83%) was obtained using 3,5-dibromopyridine (178 mg, 0.751 mmol) and tert-butyl (R)-pyrrolidin-3-ylcarbamate (47 mg, 0.252 mmol) .

¹H NMR (CDCl₃, 400 MHz): δ = 1.46 (s, 9H), 1.9 - 2.1 (m, 1H), 2.2 - 2.4 (m, 1H), 3.17 (dd, 1H, J = 4, 10 Hz), 3.3 - 3.5 (m, 2H), 3.59 (dd, 1H, J = 6, 10 Hz), 4.38 (br s, 1H), 4.68 (br s, 1H), 6.95 (t, 1H, J = 2 Hz), 7.78 (d, 1H, J = 3 Hz), 8.80 (d, 1H, J = 2 Hz).

### Reference Example 56

2-(4-(Trifluoromethyl)phenyl)acetic acid-2,2-d₂

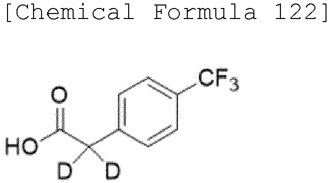

4-(Trifluoromethyl)phenylacetic acid (50 mg, 0.245 mmol) was dissolved in deuterated water (1 mL), subsequently a 40% solution of sodium deuteroxide (0.5 mL, 7.00 mmol) was added thereto, and the mixture was heated to reflux overnight in a nitrogen atmosphere. Deuterated water was added to the reaction liquid that had been left to cool to room temperature, the mixture was washed with diethyl ether, subsequently 3N hydrochloric acid was added to the aqueous layer to make the aqueous layer acidic, and the aqueous layer was extracted with diethyl ether. An organic layer thus separated was dried over anhydrous sodium sulfate, insoluble materials were filtered, and then the solvent was distilled off under reduced pressure. A residue thus obtained was suspended in chloroform, insoluble materials were filtered, subsequently the solvent was distilled off under reduced pressure, and the title compound (white powder, 30 mg, 59%) was obtained.

¹H NMR (DMSO - d₆, 400 MHz): δ = 7.43 (d, 2H, J = 8 Hz), 7.59 (d, 2H, J = 8 Hz). The 1H content is not observable.

### Reference Example 57

(R)-2-(4-bromophenyl)-N-(1-(6-(trifluoromethyl)pyridin-3-yl)pyrrolidin-3-yl)acetamide

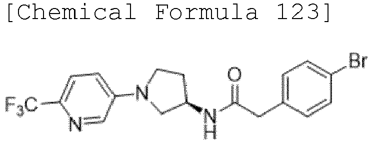

According to a technique similar to Reference Example 7-1, the title compound (pale yellow powder, 88 mg, 95%) was obtained using (R)-1-(6-(trifluoromethyl)pyridin-3-yl)pyrrolidin-3-amine (50 mg, 0.216 mmol) synthesized in Reference Example 32-2 and 4-bromophenylacetic acid (56 mg, 0.259 mmol).

¹H NMR (CDCl₃, 400 MHz): δ = 1.9 - 2.0 (m, 1H), 2.3-2.4 (m, 1H), 3.16 (dd, 1H, J = 4, 10 Hz), 3.3 - 3.5 (m, 2H), 3.53 (s, 2H), 3.68 (dd, 1H, J = 6, 10 Hz), 4.5 - 4.7 (m, 1H), 5.52 (br s, 1H), 6.80 (dd, 1H, J = 3, 9 Hz), 7.14 (d, 2H, J = 9 Hz), 7.4 - 7.5 (m, 3H), 7.98 (d, 1H, J = 3 Hz).

### Reference Example 58-1

Tert-butyl (1-(5-(trifluoromethyl)pyridin-3-yl)azetidin-3-yl)carbamate

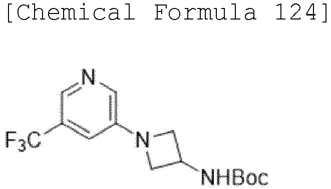

According to a technique similar to Reference Example 4, the title compound (pale yellow powder, 112 mg, 25%) was obtained using 3-bromo-5-(trifluoromethyl)pyridine (390 mg, 1.73 mmol) and tert-butyl azetidin-3-ylcarbamate hydrochloride (300 mg, 1.44 mmol).

¹H NMR (CDCl₃, 400 MHz): δ = 1.46 (s, 9H), 3.76 (dd, 2H, J = 6, 8 Hz), 4.32 (t, 2H, J = 7 Hz), 4.69 (br s, 1H), 4.99 (br s, 1H), 6.86 (s, 1H), 7.99 (d, 1H, J = 3 Hz), 8.27 (s, 1H).

### Reference Example 58-2

1-(5-(Trifluoromethyl)pyridin-3-yl)azetidin-3-amine

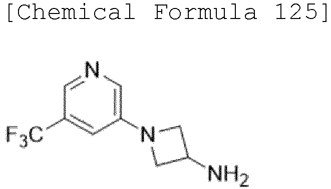

According to a technique similar to Reference Example 1-2, the title compound (white powder, 60 mg, 79%) was obtained using tert-butyl (1-(5-(trifluoromethyl)pyridin-3-yl)azetidin-3-yl)carbamate (112 mg, 0.353 mmol) synthesized in Reference Example 58-1.

¹H NMR (CDCl₃, 400 MHz): δ = 3.59 (dd, 2H, J = 6, 8 Hz), 4.0 - 4.1 (m, 1H), 4.27 (t, 2H, J = 7 Hz), 6.85 (t, 1H, J = 2 Hz), 7.99 (d, 1H, J = 3 Hz), 8.24 (s, 1H). The 2H content is not observable.

### Reference Example 59

Benzyl (R)-4-(4-(2-oxo-2-((1-(5-(trifluoromethyl)pyridin-3-yl)pyrrolidin-3-yl)amino)ethyl)phenyl)piperazine-1-carboxylate

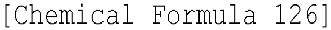

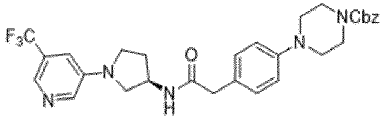

According to a technique similar to Reference Example 7-1, the title compound (yellow powder, 176 mg, 90%) was obtained using (R)-1-(5-(trifluoromethyl)pyridin-3-yl)pyrrolidin-3-amine (80 mg, 0.346 mmol) synthesized in Reference Example 1-2 and 2-(4-(4-((benzyloxy)carbonyl)piperazin-1-yl)phenyl)acetic acid (135 mg, 0.381 mmol).

¹H NMR (CDCl₃, 400 MHz): δ = 1.8 - 2.0 (m, 1H), 2.2-2.4 (m, 1H), 3.0 - 3.2 (m, 5H), 3.37 (t, 2H, J = 7 Hz), 3.51 (s, 2H), 3.6 - 3.7 (m, 5H), 4.5 - 4.7 (m, 1H), 5.16 (s, 2H), 5.53 (d, 1H, J = 7 Hz), 6.8 - 6.9 (m, 3H), 7.14 (d, 2H, J = 9 Hz), 7.3 - 7.4 (m, 5H), 8.07 (d, 1H, J = 3 Hz), 8.20 (s, 1H).

### Reference Example 60

Ethyl 2-(4-(1,4-dioxa-8-azaspiro[4.5]decan-8-yl)phenyl)acetate

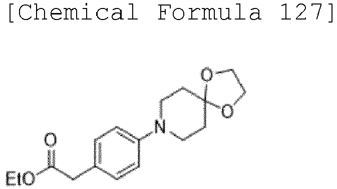

According to a technique similar to Reference Example 23-1, the title compound (yellow oily material, 16 mg, 4%) was obtained using ethyl 2-(4-bromophenyl)acetate (300 mg, 1.23 mmol) and 4-piperidone ethylene ketal (317 µL, 2.46 mmol) .

¹H NMR (CDCl₃, 400 MHz): δ = 1.25 (t, 3H, J = 7 Hz), 1.84 (t, 4H, J = 6 Hz), 3.31 (t, 4H, J = 6 Hz), 3.52 (s, 2H), 3.99 (s, 4H), 4.14 (q, 2H, J = 7 Hz), 6.90 (d, 2H, J = 9 Hz), 7.16 (d, 2H, J = 9 Hz).

### Reference Example 61-1

7-(5-(Trifluoromethyl)pyridin-3-yl)-3-oxa-1,7-diazaspiro[4.4]nonan-2-one

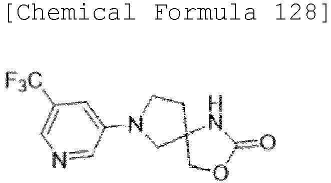

According to a technique similar to Reference Example 1-1, the title compound (white powder, 106 mg, 51%) was obtained using 3-bromo-5-(trifluoromethyl)pyridine (162 mg, 0.717 mmol) and benzyl N-(3-(hydroxymethyl)pyrrolidin-3-yl)carbamate (189 mg, 0.753 mmol).

¹H NMR (CDCl₃, 400 MHz): δ = 2.2 - 2.5 (m, 2H), 3.4-3.6 (m, 4H), 4.41 (d, 1H, J = 9 Hz), 4.45 (d, 1H, J = 9 Hz), 5.57 (s, 1H), 6.97 (t, 1H, J = 2 Hz), 8.14 (d, 1H, J = 3 Hz), 8.29 (s, 1H).

### Reference Example 61-2

(3-Amino-1-(5-(trifluoromethyl)pyridin-3-yl)pyrrolidin-3-yl)methanol

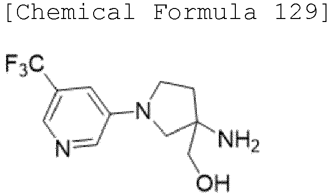

7-(5-(Trifluoromethyl)pyridin-3-yl)-3-oxa-1,7-diazaspiro[4.4]nonan-2-one (95 mg, 0.331 mmol) synthesized in Reference Example 61-1 was dissolved in ethanol (4 mL) and water (1 mL), lithium hydroxide monohydrate (278 mg, 6.61 mmol) was added thereto, and the mixture was heated to reflux overnight in a nitrogen atmosphere. To the reaction liquid that had been left to cool to room temperature, water was added, the mixture was stirred for a while, and then the mixture was extracted with ethyl acetate. An organic layer thus separated was washed with saturated brine and dried over anhydrous sodium sulfate, insoluble materials were filtered, subsequently the solvent was distilled off under reduced pressure, and the title compound (yellow powder, 86 mg, 100%) was obtained.

¹H NMR (CDCl₃, 400 MHz): δ = 1.8 - 2.0 (m, 1H), 2.1-2.2 (m, 1H), 3.19 (d, 1H, J = 10 Hz), 3.4 - 3.7 (m, 5H), 6.93 (t, 1H, J = 2 Hz), 8.11 (d, 1H, J = 3 Hz), 8.20 (s, 1H). The 3H content is not observable.

### Reference Example 62

2-(4-Isopropylphenyl)-N-(piperidin-4-yl)acetamide

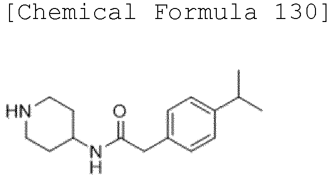

4-Amino-1-tert-butoxycarbonylpiperidine (1.00g, 4.54 mmol) and 2-(4-isopropylphenyl)acetic acid (0.971g, 5.45 mmol) were dissolved in DMF (13 mL), subsequently DIPEA (2.3 mL, 13.6 mmol) and HATU (2.42g, 6.36 mmol) were added thereto, and the mixture was stirred overnight at room temperature. Water was added to the reaction liquid, the mixture was stirred for a while, and then the mixture was extracted with ethyl acetate. An organic layer thus separated was washed with saturated brine and dried over anhydrous sodium sulfate, insoluble materials were filtered, subsequently the solvent was distilled off under reduced pressure, and a crude form of tert-butyl 4-(2-(4-isopropylphenyl)acetamido)piperidine-1-carboxylate (dark brown oily material, 2.45g) was obtained. According to a technique similar to Reference Example 1-2, the title compound (ocher-colored crystals, 0.911g, 77%) was obtained using the crude form of tert-butyl 4-(2-(4-isopropylphenyl)acetamido)piperidine-1-carboxylate (2.45g) thus obtained.

¹H NMR (CDCl₃, 400 MHz): δ = 1.1 - 1.3 (m, 9H), 1.8-1.9 (m, 2H), 2.65 (td, 2H, J = 3, 12 Hz), 2.8 - 3.0 (m, 3H), 3.52 (s, 2H), 3.8 - 4.0 (m, 1H), 5.24 (d, 1H, J = 6 Hz), 7.1 - 7.3 (m, 4H).

### Reference Example 63

(R)-2-(4-isopropylphenyl)-N-(pyrrolidin-3-yl)acetamide

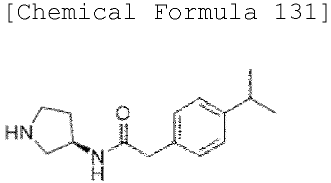

According to a technique similar to Reference Example 62, a crude form of tert-butyl (R)-3-(2-(4-isopropylphenyl)acetamido)pyrrolidine-1-carboxylate (4.25g) was synthesized using (3R)-(+)-1-(tert-butoxycarbonyl)-3-aminopyrrolidine (2.00g, 10.7 mmol) and 2-(4-isopropylphenyl)acetic acid (2.30g, 12.9 mmol), and then the title compound (pale peach-colored crystals, 2.22g, 84%) was obtained.

¹H NMR (CDCl₃, 400 MHz): δ = 1.25 (d, 6H, J = 7 Hz), 1.4 - 1.5 (m, 1H), 2.0 - 2.2 (m, 1H), 2.65 (dd, 1H, J = 4, 11 Hz), 2.8 - 3.0 (m, 3H), 3.11 (dd, 1H, J = 7, 11 Hz), 3.51 (s, 2H), 4.3 - 4.4 (m, 1H), 5.55 (br s, 1H), 7.16 (d, 2H, J = 8 Hz), 7.21 (d, 2H, J = 8 Hz). The 1H content is not observable.

### Reference Example 64-1

Tert-butyl (S)-(1-(5-(trifluoromethyl)pyridin-3-yl)pyrrolidin-3-yl) carbamate

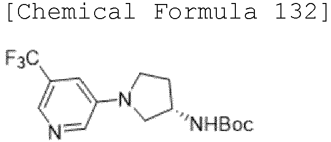

According to a technique similar to Reference Example 4, the title compound (pale yellow crystals, 185 mg, 20%) was obtained using tert-butyl (S)-pyrrolidin-3-ylcarbamate (500 mg, 2.68 mmol) and 5-bromo-2-(trifluoromethyl)pyridine (728 mg, 3.22 mmol).

¹H NMR (CDCl₃, 400 MHz): δ = 1.46 (s, 9H), 1.9 - 2.1 (m, 1H), 2.3 - 2.4 (m, 1H), 3.23 (dd, 1H, J = 4, 10 Hz), 3.3 - 3.6 (m, 2H), 3.65 (dd, 1H, J = 6, 10 Hz), 4.40 (br s, 1H), 4.71 (br s, 1H), 6.94 (s, 1H), 8.12 (d, 1H, J = 3 Hz), 8.21 (s, 1H).

### Reference Example 64-2

(S)-1-(5-(trifluoromethyl)pyridin-3-yl)pyrrolidin-3-amine

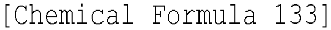

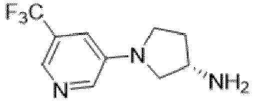

According to a technique similar to Reference Example 1-2, the title compound (pale yellow crystals, 100 mg, 77%) was obtained using tert-butyl (S)-(1-(5-(trifluoromethyl)pyridin-3-yl)pyrrolidin-3-yl)carbamate (184 mg, 0.555 mmol) synthesized in Reference Example 64-1.

¹H NMR (CDCl₃, 400 MHz): δ = 1.8 - 2.0 (m, 1H), 2.2-2.4 (m, 1H), 3.08 (dd, 1H, J = 5, 10 Hz), 3.3 - 3.6 (m, 3H), 3.81 (quin, 1H, J = 6 Hz)6.92 (s, 1H), 8.11 (d, 1H, J = 3 Hz), 8.17 (s, 1H). The 2H content is not observable.

### Reference Example 65

Tert-butyl 3-(2-(4-isopropylphenyl)acetamido)azetidine-1-carboxylate

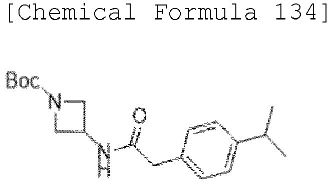

According to a technique similar to Reference Example 7-1, the title compound (pale yellow amorphous, 964 mg, 100%) was obtained using tert-butyl 3-aminoazetidine-1-carboxylate (500 mg, 2.90 mmol) and 2-(4-isopropylphenyl)acetic acid (624 mg, 3.50 mmol).

¹H NMR (CDCl₃, 400 MHz): δ = 1.26 (d, 6H, J = 7 Hz) , 2.8 - 3.0 (m, 1H), 3.54 (s, 2H), 3.5 - 3.7 (m, 2H), 4.2 - 4.4 (m, 2H), 4.5 - 4.7 (m, 1H), 6.6 - 6.8 (m, 1H), 7.15 (d, 2H, J = 8 Hz), 7.23 (d, 2H, J = 8 Hz).

### Reference Example 66

Tert-butyl (R)-(1-(5-(trifluoromethyl)pyridin-3-yl)piperidin-3-yl) carbamate

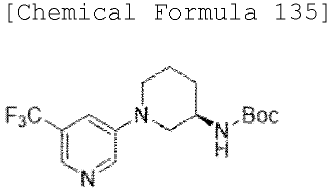

According to a technique similar to Reference Example 4, the title compound (yellow amorphous, 100 mg, 20%) was obtained using 3-bromo-5-(trifluoromethyl)pyridine (407 mg, 1.80 mmol) and (R)-3-(tert-butoxycarbonylamino)piperidine (300 mg, 1.50 mmol).

¹H NMR (CDCl₃, 400 MHz): δ = 1.46 (s, 9H), 1.6 - 1.8 (m, 1H), 1.8 - 2.0 (m, 2H), 2.9 - 3.2 (m, 2H), 3.2 - 3.4 (m, 1H), 3.5 - 3.6 (m, 1H), 3.8 - 3.9 (m, 1H), 4.7 - 4.8 (m, 1H), 7.33 (s, 1H), 8.32 (s, 1H), 8.46 (d, 1H, J = 2 Hz). The 1H content is not observable.

### Reference Example 67

Tert-butyl (1-(4-cyclopropylphenyl)-2-oxo-2-(((R)-1-(5-(trifluoromethyl)pyridin-3-yl)pyrrolidin-3-yl)amino)ethyl)carbamate

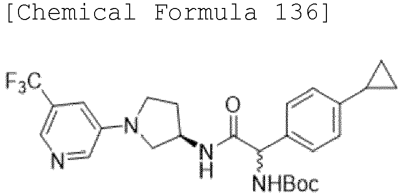

According to a technique similar to Reference Example 7-1, the title compounds (diastereomer A: Rf value in TLC (ethyl acetate: heptane = 1: 1) = 0.2, yellow amorphous, 15 mg, 5%; diastereomer B: Rf value in TLC (ethyl acetate: heptane = 1: 1) = 0.18, yellow amorphous, 15 mg, 5%, diastereomer mixture: 290 mg, 88%) were respectively obtained using (R)-1-(5-(trifluoromethyl)pyridin-3-yl)pyrrolidin-3-amine (150 mg, 0.65 mmol) and 2-((tert-butoxycarbonyl)amino)-2-(4-cyclopropylphenyl)acetic acid (189 mg, 0.65 mmol).

### Diastereomer A

¹H NMR (CDCl₃, 400 MHz): δ = 0.6 - 0.7 (m, 2H), 0.9-1.0 (m, 2H), 1.39 (s, 9H), 1.8 - 1.9 (m, 1H), 2.0 - 2.1 (m, 1H), 2.3 - 2.4 (m, 1H), 3.0 - 3.1 (m, 1H), 3.3 - 3.5 (m, 2H), 3.5 - 3.6 (m, 1H), 4.5 - 4.7 (m, 1H), 5.0 - 5.1 (m, 1H), 5.5 - 5.7 (m, 1H), 6.2 - 6.3 (m, 1H), 6.83 (s, 1H), 7.00 (d, 2H, J = 8 Hz), 7.19 (d, 2H, J = 8 Hz), 8.00 (d, 1H, J = 3 Hz), 8.14 (s, 1H).

### Diastereomer B

¹H NMR (CDCl₃, 400 MHz): δ = 0.6 - 0.8 (m, 2H), 0.9-1.0 (m, 2H), 1.40 (s, 9H)1.8 - 2.0 (m, 2H), 2.2 - 2.3 (m, 1H), 3.2 - 3.4 (m, 3H), 3.6 - 3.7 (m, 1H), 4.6 - 4.7 (m, 1H), 5.0 - 5.1 (m, 1H), 5.5 - 5.7 (m, 1H), 6.2 - 6.3 (m, 1H), 6.85 (s, 1H), 7.04 (d, 2H, J = 8 Hz), 7.22 (d, 2H, J = 8 Hz), 8.01 (d, 1H, J = 3 Hz), 8.12 (s, 1H).

### Reference Example 68

Tert-butyl (R)-(3-methyl-1-(5-(trifluoromethyl)pyridin-3-yl)pyrrolidin-3-yl)carbamate

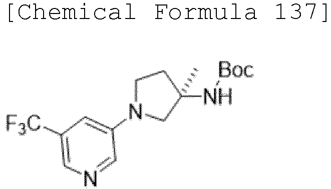

According to a technique similar to Reference Example 4, the title compound (yellow amorphous, 50 mg, 63%) was obtained using 3-bromo-5-(trifluoromethyl)pyridine (79 mg, 0.23 mmol) and tert-butyl (R)-(3-methylpyrrolidin-3-yl)carbamate (47 mg, 0.23 mmol).

¹H NMR (CDCl₃, 400 MHz): δ = 1.45 (s, 9H), 1.54 (s, 3H), 1.9 - 2.1 (m, 1H), 2.3 - 2.5 (m, 1H), 3.3 - 3.5 (m, 3H), 3.5 - 3.6 (m, 1H), 4.66 (br s, 1H), 6.92 (s, 1H), 8.10 (d, 1H, J = 3 Hz), 8.19 (s, 1H).

### Reference Example 69

Ethyl 2-(4-(3-oxa-8-azabicyclo[3.2.1]octan-8-yl)phenyl) acetate

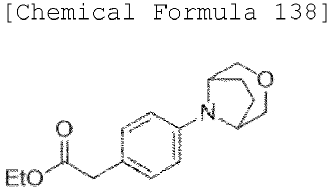

According to a technique similar to Reference Example 27, the title compound (pale yellow amorphous, 87 mg, 70%) was obtained using ethyl 4-bromophenylacetate (109 mg, 0.45 mmol) and 3-oxa-8-azabicyclo[3.2.1]octane hydrochloride (100 mg, 0.67 mmol).

¹H NMR (CDC13, 400 MHz): δ = 1.25 (t, 3H, J = 7 Hz), 1.9 - 2.1 (m, 4H), 3.51 (s, 2H), 3.4 - 3.6 (m, 2H), 3.91 (d, 2H, J = 10 Hz), 4.0 - 4.1 (m, 2H), 4.14 (q, 2H, J = 7 Hz), 6.74 (d, 2H, J = 8 Hz), 7.15 (d, 2H, J = 8 Hz).

### Reference Example 70

Ethyl 2-(4-((1S,4S)-2-oxa-5-azabicyclo[2.2.2]octan-5-yl)phenyl) acetate

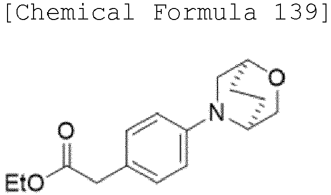

According to a technique similar to Reference Example 27, the title compound (pale yellow amorphous, 109 mg, 88%) was obtained using ethyl 4-bromophenylacetate (109 mg, 0.45 mmol) and (1S,4S)-2-oxa-5-azabicyclo[2.2.2]octane oxalate (106 mg, 0.67 mmol).

¹H NMR (CDCl₃, 400 MHz): δ = 1.25 (t, 3H, J = 7 Hz), 1.6 - 1.8 (m, 1H), 1.8 - 2.0 (m, 1H), 2.0 - 2.3 (m, 2H), 3.3 - 3.4 (m, 1H), 3.51 (s, 2H), 3.7 - 3.9 (m, 2H), 4.0-4.1 (m, 2H), 4.1 - 4.2 (m, 1H), 4.13 (q, 2H, J = 7 Hz), 6.59 (d, 2H, J = 8 Hz), 7.16 (d, 2H, J = 8 Hz).

### Reference Example 71

Tert-butyl 3-(trifluoromethyl)-1-(5-(trifluoromethyl)pyridin-3-yl)pyrrolidin-3-yl)carbamate

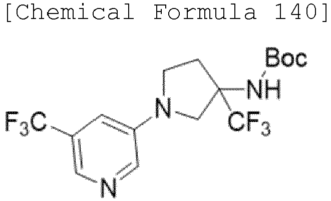

According to a technique similar to Reference Example 4, the title compound (yellow amorphous, 145 mg, 62%) was obtained using 3-bromo-5-(trifluoromethyl)pyridine (160 mg, 0.23 mmol) and tert-butyl 3-(trifluoromethyl)pyrrolidin-3-ylcarbamate (150 mg, 0.59 mmol) .

¹H NMR (CDCl₃, 400 MHz): δ = 1.45 (s, 9H), 2.5 - 2.8 (m, 2H), 3.5 - 3.7 (m, 2H), 3.8 - 3.9 (m, 1H), 3.9 - 4.0 (m, 1H), 4.85 (br s, 1H), 6.98 (s, 1H), 8.14 (d, 1H, J = 3 Hz), 8.26 (s, 1H).

### Reference Example 72

Tert-butyl (R)-(1-(6-(trifluoromethyl)pyridin-3-yl)piperidin-3-yl) carbamate

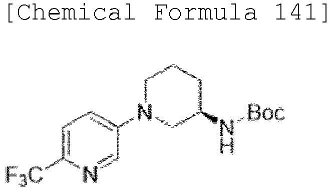

According to a technique similar to Reference Example 4, the title compound (yellow crystals, 400 mg, 77%) was obtained using 5-bromo-2-(trifluoromethyl)pyridine (407 mg, 1.80 mmol) and (R)-3-(tert-butoxycarbonylamino)piperidine (300 mg, 1.50 mmol).

¹H NMR (CDCl₃, 400 MHz): δ = 1.45 (s, 9H), 1.6 - 2.0 (m, 4H), 2.9 - 3.1 (m, 1H), 3.1 - 3.2 (m, 1H), 3.3 - 3.5 (m, 1H), 3.6 - 3.7 (m, 1H), 3.7 - 3.8 (m, 1H), 4.65 (br s, 1H), 7.17 (d, 1H, J = 7 Hz), 7.49 (d, 1H, J = 7 Hz), 8.33 (s, 1H).

### Reference Example 73-1

Tert-butyl (R)-3-(2-(4-(trifluoromethyl)phenyl)acetamido)pyrrolidine-1-carboxylate

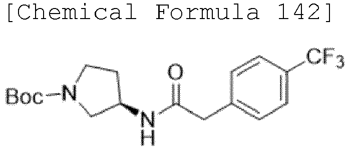

According to a technique similar to Reference Example 7-1, the title compound (white solid, 1.96g, 88%) was obtained using tert-butyl (R)-aminopyrrolidine-1-carbamate (1.11g, 6.0 mmol) and 2-(4-(trifluoromethyl)phenyl)acetic acid (1.35g, 6.6 mmol).

¹H NMR (CDCl₃, 400 MHz): δ = 1.45 (s, 9H), 1.6 - 1.9 (m, 1H), 2.0 - 2.2 (m, 1H), 3.0 - 3.5 (m, 3H), 3.5 - 3.7 (m, 1H), 3.60 (s, 2H), 4.3 - 4.5 (m, 1H), 5.53 (br s, 1H), 7.39 (d, 2H, J = 8 Hz), 7.61 (d, 2H, J = 8 Hz).

### Reference Example 73-2

(R)-N-(pyrrolidin-3-yl)-2-(4-(trifluoromethyl)phenyl)acetamide

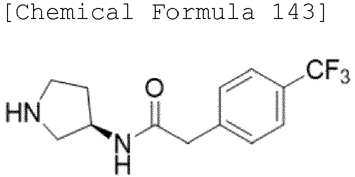

According to a technique similar to Reference Example 1-2, the title compound (white solid, 650 mg, 80%) was obtained using tert-butyl (R)-3-(2-(4-(trifluoromethyl)phenyl)acetamido)pyrrolidine-1-carboxylate (1.12g, 3.0 mmol) synthesized in Reference Example 73-1.

¹H NMR (CDCl₃, 400 MHz): δ = 1.4 - 1.6 (m, 1H), 2.0-2.2 (m, 1H), 2.71 (dd, 1H, J = 4, 11 Hz), 2.8 - 3.1 (m, 2H), 3.10 (dd, 1H, J = 7, 12 Hz), 3.57 (s, 2H), 4.3 - 4.4 (m, 1H), 5.69 (br s, 1H), 7.39 (d, 2H, J = 8 Hz) , 7.60 (d, 2H, J = 8 Hz). The 1H content is not observable.

### Reference Example 74-1

Tert-butyl (R)-3-(2-(4-cyclopropylphenyl)acetamido)pyrrolidine-1-carboxylate

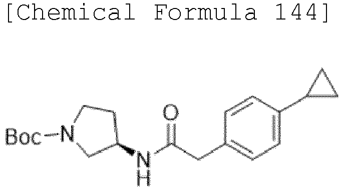

According to a technique similar to Reference Example 7-1, the title compound (yellow crystals, 2.0g, 70%) was obtained using tert-butyl 3-aminopyrrolidine-1-carboxylate (1.5 mL, 8.29 mmol) and 2-(4-cyclopropylphenyl)acetic acid (1.8g, 9.95 mmol) synthesized in Reference Example 33-2.

¹H NMR (CDCl₃, 400 MHz): δ = 0.6 - 0.7 (m, 2H), 0.9-1.0 (m, 2H), 1.43 (s, 9H), 1.5 - 1.7 (m, 1H), 1.8 - 1.9 (m, 1H), 2.0 - 2.1 (m, 1H), 2.9 - 3.1 (m, 1H), 3.2 - 3.5 (m, 2H), 3.51 (s, 2H), 3.5 - 3.6 (m, 1H), 4.3 - 4.5 (m, 1H), 5.3 - 5.5 (m, 1H), 7.04 (d, 2H, J = 8 Hz), 7.10 (d, 2H, J = 8 Hz).

### Reference Example 74-2

(R)-2-(4-cyclopropylphenyl)-N-(pyrrolidin-3-yl)acetamide

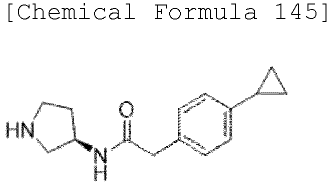

According to a technique similar to Reference Example 1-2, the title compound (pale yellow crystals, 1.1g, 77%) was obtained using tert-butyl (R)-3-(2-(4-cyclopropylphenyl)acetamido)pyrrolidine-1-carboxylate (2.0g, 5.81 mmol) synthesized in Reference Example 74-1.

¹H NMR (CDCl₃, 400 MHz): δ = 0.6 - 0.7 (m, 2H), 0.9-1.0 (m, 2H), 1.4 - 1.5 (m, 1H), 1.8 - 1.9 (m, 2H), 2.0-2.2 (m, 1H), 2.6 - 2.7 (m, 1H), 2.8 - 3.0 (m, 2H), 3.0-3.1 (m, 1H), 3.48 (s, 2H), 4.2 - 4.4 (br s, 1H), 5.62 (br s, 1H), 7.03 (d, 2H, J = 8 Hz), 7.11 (d, 2H, J = 8 Hz).

### Reference Example 75-1

Tert-butyl 3-(2-(4-cyclopropylphenyl) acetamido)azetidine-1-carbamate

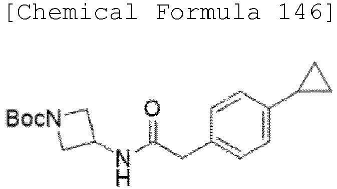

According to a technique similar to Reference Example 7-1, the title compound (white solid, 434 mg, 82%) was obtained using tert-butyl 3-aminoazetidine-1-carbamate (276 mg, 1.6 mmol) and 2-(4-cyclopropylphenyl)acetic acid (352 mg, 2.0 mmol) synthesized in Reference Example 33-2.

¹H NMR (CDCl₃, 400 MHz): δ = 0.6 - 0.8 (m, 2H), 0.9-1.1 (m, 2H), 1.41 (s, 9H), 1.8 - 2.0 (m, 1H), 3.53 (s, 2H), 3.58 (dd, 2H, J = 5, 10 Hz), 4.28 (t, 2H, J = 9 Hz), 4.5-4.7 (m, 1H), 5.71 (d, 1H, J = 7 Hz), 7.07 (d, 2H, J = 8 Hz), 7.12 (d, 2H, J = 8 Hz).

### Reference Example 75-2

N-(azetidin-3-yl)-2-(4-cyclopropylphenyl)acetamide

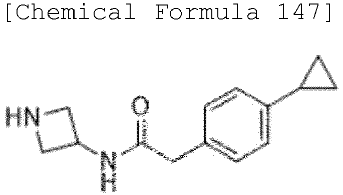

According to a technique similar to Reference Example 1-2, the title compound (white solid, 83 mg, 28%) was obtained using tert-butyl 3-(2-(4-cyclopropylphenyl)acetamido)azetidine-1-carbamate (431 mg, 1.3 mmol) synthesized in Reference Example 75-1.

¹H NMR (CDCl₃, 400 MHz): δ = 0.6 - 0.8 (m, 2H), 0.9-1.1 (m, 2H), 1.8 - 2.0 (m, 1H), 3.3 - 3.6 (m, 2H), 3.51 (s, 2H), 3.8 - 3.9 (m, 2H), 4.6 - 4.8 (m, 1H), 5.71 (d, 1H, J = 7 Hz), 7.06 (d, 2H, J = 8 Hz), 7.13 (d, 2H, J = 8 Hz). The 1H content is not observable.

### Reference Example 76

Ethyl 2-(3-bromophenoxy)-2-methylpropanoate

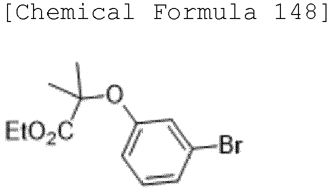

According to a technique similar to Reference Example 52, the title compound (white amorphous, 149 mg, 86%) was obtained using 3-bromophenol (173 mg, 1.0 mmol) and ethyl 2-bromo-2-methylpropanoate (195 mg, 1.0 mmol).

¹H NMR (CDCl₃, 400 MHz): δ = 1.26 (t, 3H, J = 7 Hz), 1.60 (s, 6H), 4.24 (q, 2H, J = 7 Hz), 6.7 - 6.8 (m, 1H), 7.0 - 7.2 (m, 3H).

### Reference Example 77

Tert-butyl (R)-(1-(3-(trifluoromethyl)phenyl)pyrrolidin-3-yl) carbamate

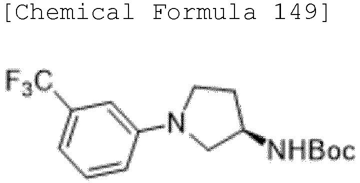

According to a technique similar to Reference Example 1-1, the title compound (yellow powder, 105 mg, 59%) was obtained using 3-bromobenzotrifluoride (89 µL, 0.644 mmol) and tert-butyl (R)-pyrrolidin-3-ylcarbamate (100 mg, 0.537 mmol).

¹H NMR (CDCl₃, 400 MHz): δ = 1.46 (s, 9H), 1.9 - 2.1 (m, 1H), 2.2 - 2.4 (m, 1H), 3.19 (dd, 1H, J = 4, 10 Hz), 3.3 - 3.5 (m, 2H), 3.60 (dd, 1H, J = 6, 10 Hz), 4.38 (br s, 1H), 4.69 (br s, 1H), 6.68 (dd, 1H, J = 2, 8 Hz), 6.73 (s, 1H), 6.93 (d, 1H, J = 8 Hz), 7.31 (t, 1H, J = 8 Hz).

### Example 1

(R)-2-(4-cyclopropylphenyl)-N-(1-(5-(trifluoromethyl)pyridin-3-yl)pyrrolidin-3-yl)acetamide

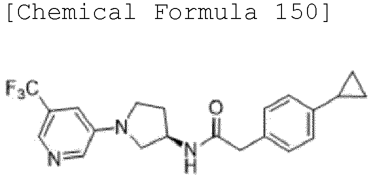

(R)-1-(5-(trifluoromethyl)pyridin-3-yl)pyrrolidin-3-amine (20 mg, 0.09 mmol) synthesized in Reference Example 1-2 and 2-(4-cyclopropylphenyl)acetic acid (30 mg, 0.09 mmol) synthesized in Reference Example 33-2 were dissolved in DMF (2.0 mL), subsequently DIPEA (29 µL, 0.17 mmol) and HATU (33 mg, 0.09 mmol) were added thereto, and the mixture was stirred overnight at room temperature. Water was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 0 to 100%), and the title compound (white powder, 18 mg, 53%) was obtained.

¹H NMR (CDCl₃, 400 MHz): δ = 0.6 - 0.7 (m, 2H), 0.9-1.0 (m, 2H), 1.8 - 2.0 (m, 2H), 2.2 - 2.4 (m, 1H), 3.11 (dd, 1H, J = 4, 10 Hz), 3.3 - 3.4 (m, 2H), 3.54 (s, 2H), 3.64 (dd, 1H, J = 6, 10 Hz), 4.6 - 4.7 (m, 1H), 5.60 (d, 1H, J = 6 Hz), 6.8 - 6.9 (m, 1H), 7.04 (d, 2H, J = 8 Hz), 7.12 (d, 2H, J = 8 Hz), 8.06 (d, 1H, J = 2 Hz), 8.19 (s, 1H) .

MS: 390.20 [M+H]⁺

### Example 2

(R)-2-(5-(trifluoromethyl)pyridin-2-yl)-N-(1-(5-(trifluoromethyl)pyridin-3-yl)pyrrolidin-3-yl)acetamide

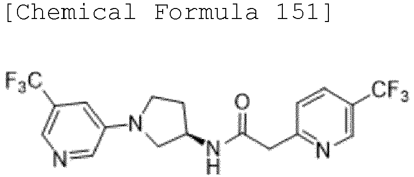

According to a technique similar to Example 1, the title compound (white crystals, 11 mg, 31%) was obtained using (R)-1-(5-(trifluoromethyl)pyridin-3-yl)pyrrolidin-3-amine (20 mg, 0.09 mmol) synthesized in Reference Example 1-2 and the crude form of 2-(5-(trifluoromethyl)pyridin-2-yl)acetic acid (26 mg) synthesized in Reference Example 31-2.

¹H NMR (CDCl₃, 400 MHz): δ = 2.0 - 2.1 (m, 2H), 2.3-2.4 (m, 1H), 3.25 (dd, 1H, J = 4, 10 Hz), 3.4 - 3.6 (m, 2H), 3.68 (dd, 1H, J = 6, 10 Hz), 3.81 (s, 2H), 4.6 - 4.7 (m, 1H), 6.9 - 7.0 (m, 1H), 7.4 - 7.5 (m, 2H), 8.12 (br s, 1H), 8.22 (br s, 1H), 8.77 (s, 1H).

MS: 419.15 [M+H]⁺

### Example 3

(R)-2-(4-isopropylphenyl)-N-(1-(2-(trifluoromethyl)pyrimidin-5-yl)pyrrolidin-3-yl)acetamide

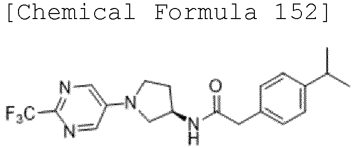

To tert-butyl (R)-(1-(2-(trifluoromethyl)pyrimidin-5-yl)pyrrolidin-3-yl)carbamate (41 mg, 0.12 mmol) synthesized in Reference Example 2, trifluoroacetic acid (2.0 mL) was added under ice cooling, and the mixture was stirred for 3 hours at room temperature. Under ice cooling, a saturated aqueous solution of sodium hydrogen carbonate and ethyl acetate were added to the reaction liquid, the mixture was stirred for a while, subsequently the organic layer was dried over anhydrous sodium sulfate, insoluble materials were filtered, subsequently the solvent was distilled off under reduced pressure, and thus the crude form of (R)-1-(2-(trifluoromethyl)pyrimidin-5-yl)pyrrolidin-3-amine (pale yellow oily material, 18 mg) was obtained. According to a technique similar to Example 1, the title compound (ivory-colored powder, 14 mg, 29%) was obtained using the crude form of (R)-1-(2-(trifluoromethyl)pyrimidin-5-yl)pyrrolidin-3-amine (18 mg) thus obtained and 2-(4-isopropylphenyl)acetic acid (26 mg, 0.15 mmol) .

¹H NMR (CDCl₃, 400 MHz): δ = 1.23 (d, 6H, J = 7 Hz), 1.9 - 2.0 (m, 1H), 2.3 - 2.4 (m, 1H), 2.8 - 2.9 (m, 1H), 3.17 (dd, 1H, J = 5, 10 Hz), 3.4 - 3.5 (m, 2H), 3.56 (s, 2H), 3.71 (dd, 1H, J = 6, 10 Hz), 4.6 - 4.7 (m, 1H), 5.50 (d, 1H, J = 6 Hz), 7.15 (d, 2H, J = 8 Hz), 7.21 (d, 2H, J = 8 Hz), 8.06 (s, 2H).

MS: 391.26 [M-H]⁻

### Example 4

(R)-2-(4-isopropylphenyl)-N-(1-(5-(trifluoromethyl)pyridin-3-yl)pyrrolidin-3-yl)acetamide

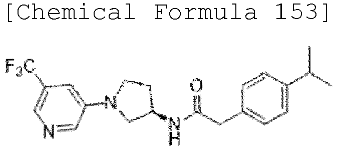

According to a technique similar to Example 3, a crude form of (R)-1-(5-(trifluoromethyl)pyridin-3-yl)pyrrolidin-3-amine (100 mg) was synthesized from tert-butyl (R)-1-(5-(trifluoromethyl)pyridin-3-yl)pyrrolidin-3-yl)carbamate (130 mg, 0.39 mmol) synthesized in Reference Example 1-1, and the title compound (white amorphous, 90 mg, 58%) was obtained using 2-(4-isopropylphenyl)acetic acid (85 mg, 0.48 mmol).

¹H NMR (CDC13, 400 MHz): δ = 1.22 (d, 6H, J = 7 Hz), 1.9 - 2.0 (m, 1H), 2.3 - 2.4 (m, 1H), 2.8 - 2.9 (m, 1H), 3.12 (dd, 1H, J = 4, 9 Hz), 3.3 - 3.4 (m, 2H), 3.55 (s, 2H), 3.6 - 3.7 (m, 1H), 4.6 - 4.7 (m, 1H), 5.7 - 5.8 (m, 1H), 6.87 (s, 1H), 7.1 - 7.3 (m, 4H), 8.04 (s, 1H), 8.17 (s, 1H).

MS: 391.19 [M-H]⁻

### Example 5

2-(4-cyclopropylphenyl)-N-((3S,4S)-4-hydroxy-1-(5-(trifluoromethyl)pyridin-3-yl)pyrrolidin-3-yl)acetamide

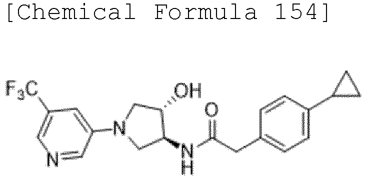

According to a technique similar to Example 3, (3S,4S)-4-amino-1-(5-(trifluoromethyl)pyridin-3-yl)pyrrolidin-3-ol (white crystals) was synthesized from tert-butyl ((3S,4S)-4-hydroxy-1-(5-(trifluoromethyl)pyridin-3-yl)pyrrolidin-3-yl)carbamate (106 mg, 0.31 mmol) synthesized in Reference Example 3, and the title compound (white crystals, 15 mg, 12%) was obtained using 2-(4-cyclopropylphenyl)acetic acid (54 mg, 0.31 mmol) synthesized in Reference Example 33-2.

¹H NMR (CDCl₃, 400 MHz): δ = 0.6 - 0.7 (m, 2H), 0.9-1.0 (m, 2H), 1.8 - 1.9 (m, 1H), 3.11 (dd, 1H, J = 5, 10 Hz), 3.27 (dd, 1H, J = 4, 10 Hz), 3.56 (s, 2H), 3.61 (dd, 1H, J = 6, 10 Hz), 3.76 (dd, 1H, J = 6, 10 Hz), 4.09 (br s, 1H), 4.3 - 4.4 (m, 2H), 5.97 (d, 1H, J = 6 Hz), 6.8 - 6.9 (m, 1H), 7.0 - 7.1 (m, 2H), 7.1 - 7.2 (m, 2H), 8.02 (d, 1H, J = 2 Hz), 8.17 (s, 1H).

MS: 406.14 [M+H]⁺

### Example 6

(R)-2-(4-cyclopropylphenyl)-N-(5-(5-(trifluoromethyl)pyridin-3-yl)-5-azaspiro[2.4]heptan-7-yl)acetamide

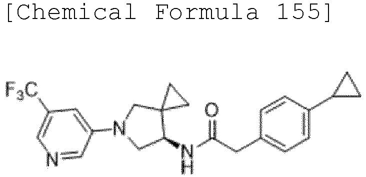

According to a technique similar to Example 3, (R)-5-(5-(trifluoromethyl)pyridin-3-yl)-5-azaspiro[2.4]heptan-7-amine (white crystals) was synthesized from tert-butyl (R)-(5-(5-(trifluoromethyl)pyridin-3-yl)-5-azaspiro[2.4]heptan-7-yl)carbamate (130 mg, 0.36 mmol) synthesized in Reference Example 4, and the title compound (white crystals, 30 mg, 20%) was obtained using 2-(4-cyclopropylphenyl)acetic acid (64 mg, 0.36 mmol) synthesized in Reference Example 33-2.

¹H NMR (CDCl₃, 400 MHz): δ = 0.5 - 0.6 (m, 1H), 0.6-0.7 (m, 4H), 0.8 - 0.9 (m, 1H), 0.9 - 1.0 (m, 2H), 1.8-1.9 (m, 1H), 3.09 (d, 1H, J = 9 Hz), 3.39 (dd, 1H, J = 2, 10 Hz), 3.45 (d, 1H, J = 10 Hz), 3.52 (d, 2H, J = 3 Hz), 3.74 (dd, 1H, J = 6, 10 Hz), 4.1 - 4.2 (m, 1H), 5.62 (d, 1H, J = 8 Hz), 6.8 - 6.9 (m, 1H), 7.0 - 7.1 (m, 2H), 7.1-7.2 (m, 2H), 8.04 (d, 1H, J = 3 Hz), 8.20 (s, 1H).

MS: 416.18 [M+H]⁺

### Example 7

(R)-2-(4-cyclopropylphenyl)-N-(5-(6-(trifluoromethyl)pyridin-3-yl)-5-azaspiro[2.4]heptan-7-yl)acetamide

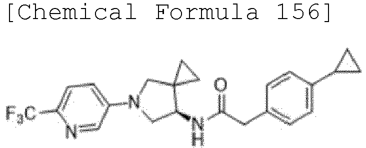

To tert-butyl (R)-(5-(6-(trifluoromethyl)pyridin-3-yl)-5-azaspiro[2.4]heptan-7-yl)carbamate (271 mg, 0.76 mmol) synthesized in Reference Example 5, a 2N hydrochloric acid-methanol solution (15 mL) was added under ice cooling, the mixture was stirred for 2 hours at room temperature, subsequently the solvent was distilled off under reduced pressure, and thereby (R)-5-(6-(trifluoromethyl)pyridin-3-yl)-5-azaspiro[2.4]heptan-7-amine (187 mg) was obtained. According to a technique similar to Example 1, the title compound (white crystals, 280 mg, 89%) was obtained using (R)-5-(6-(trifluoromethyl)pyridin-3-yl)-5-azaspiro[2.4]heptan-7-amine (187 mg) thus obtained and 2-(4-cyclopropylphenyl)acetic acid (154 mg, 0.87 mmol) synthesized in Reference Example 33-2.

¹H NMR (CDCl₃, 400 MHz): δ = 0.5 - 0.7 (m, 5H), 0.8-0.9 (m, 1H), 0.9 - 1.0 (m, 2H), 1.8 - 1.9 (m, 1H), 3.11 (d, 1H, J = 10 Hz), 3.40 (dd, 1H, J = 3, 10 Hz), 3.45 (d, 1H, J = 10 Hz), 3.53 (d, 2H, J = 3 Hz), 3.75 (dd, 1H, J = 6, 10 Hz), 4.1 - 4.2 (m, 1H), 5.56 (d, 1H, J = 7 Hz), 6.77 (dd, 1H, J = 3, 8 Hz), 7.0 - 7.1 (m, 2H), 7.1 - 7.2 (m, 2H), 7.48 (d, 1H, J = 9 Hz), 7.93 (d, 1H, J = 3 Hz).

MS: 415.19 [M+H]⁺

### Example 8

(R)-2-(4-cyclopropylphenyl)-N-(5-(4-(trifluoromethyl)thiazol-2-yl)-5-azaspiro[2.4]heptan-7-yl)acetamide

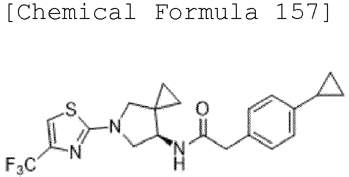

According to a technique similar to Example 7, (R)-5-(4-(trifluoromethyl)thiazol-2-yl)-5-azaspiro[2.4]heptan-7-amine (white crystals) was synthesized from tert-butyl (R)-(5-(4-(trifluoromethyl)thiazol-2-yl)-5-azaspiro[2.4]heptan-7-yl)carbamate (192 mg, 0.53 mmol) synthesized in Reference Example 6, and the title compound (white crystals, 177 mg, 79%) was obtained using 2-(4-cyclopropylphenyl)acetic acid (112 mg, 0.64 mmol) synthesized in Reference Example 33-2.

¹H NMR (CDCl₃, 400 MHz): δ = 0.5 - 0.6 (m, 1H), 0.6-0.7 (m, 4H), 0.7 - 0.8 (m, 1H), 0.9 - 1.0 (m, 2H), 1.8-1.9 (m, 1H), 3.32 (d, 1H, J = 10 Hz), 3.44 (dd, 1H, J = 2, 10 Hz), 3.52 (d, 2H, J = 2 Hz), 3.56 (d, 1H, J = 10 Hz), 3.83 (dd, 1H, J = 6, 10 Hz), 4.1 - 4.2 (m, 1H), 5.53 (d, 1H, J = 7 Hz), 6.93 (d, 1H, J = 1 Hz), 7.0 - 7.1 (m, 2H), 7.1 - 7.2 (m, 2H).

MS: 421.14 [M+H]⁺

### Example 9

2-(4-cyclopropylphenyl)-N-((3R,5S)-5-methyl-1-(5-(trifluoromethyl)pyridin-3-yl)pyrrolidin-3-yl)acetamide

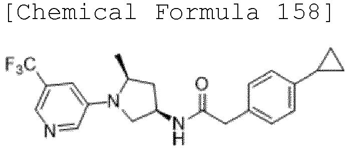

2-(4-cyclopropylphenyl)-N-((3R,5S)-5-methylpyrrolidin-3-yl)acetamide (150 mg, 0.58 mmol) synthesized in Reference Example 7-2, 3-bromo-5-(trifluoromethyl)pyridine (157 mg, 0.70 mmol), tris(dibenzylidene acetone)dipalladium(0) (53 mg, 0.06 mmol), XantPhos (67 mg, 0.12 mmol), and sodium tert-butoxide (112 mg, 1.16 mmol) were suspended in toluene (5.8 mL), and the suspension was stirred for 16 hours at 85°C under a nitrogen gas stream. To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 0 to 100%), and the title compound (white crystals, 45 mg, 19%) was obtained.

¹H NMR (CDCl₃, 400 MHz): δ = 0.6 - 0.7 (m, 2H), 0.9-1.0 (m, 2H), 1.22 (d, 3H, J = 6 Hz), 1.8 - 2.0 (m, 2H), 2.0 - 2.1 (m, 1H), 2.93 (dd, 1H, J = 7, 10 Hz), 3.54 (s, 2H), 3.80 (dd, 1H, J = 8, 10 Hz), 3.9 - 4.0 (m, 1H), 4.7 - 4.8 (m, 1H), 5.48 (d, 1H, J = 8 Hz), 6.88 (dd, 1H, J = 2, 2 Hz), 7.0 - 7.1 (m, 2H), 7.1 - 7.2 (m, 2H), 8.07 (d, 1H, J = 3 Hz), 8.17 (s, 1H).

MS: 404.16 [M+H]⁺

### Example 10

2-(4-cyclopropylphenyl)-N-(3-(5-(trifluoromethyl)pyridin-3-yl)-3-azabicyclo[3.1.0]hexan-1-yl)acetamide

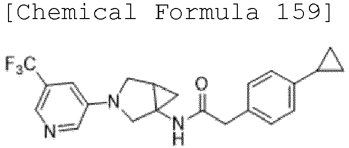

According to a technique similar to Example 7, 3-(5-(trifluoromethyl)pyridin-3-yl)-3-azabicyclo[3.1.0]hexan-1-amine (yellow oily material, 121 mg) was synthesized from tert-butyl (3-(5-(trifluoromethyl)pyridin-3-yl)-3-azabicyclo[3.1.0]hexan-1-yl)carbamate (103 mg, 0.30 mmol) synthesized in Reference Example 8, and the title compound (pale yellow amorphous, 73 mg, 61%) was obtained using 2-(4-cyclopropylphenyl)acetic acid (63 mg, 0.36 mmol) synthesized in Reference Example 33-2.

¹H NMR (CDCl₃, 400 MHz): δ = 0.6 - 0.7 (m, 2H), 0.8-0.9 (m, 1H), 0.9 - 1.1 (m, 3H), 1.8 - 2.0 (m, 2H), 3.3-3.4 (m, 1H), 3.5 - 3.6 (m, 4H), 3.7 - 3.8 (m, 1H), 5.89 (s, 1H), 6.90 (s, 1H), 7.07 (d, 2H, J = 8 Hz), 7.13 (d, 2H, J = 8 Hz), 8.06 (d, 1H, J = 2 Hz), 8.18 (s, 1H).

MS: 402.16 [M+H]⁺

### Example 11

(R)-2-(4-(trifluoromethyl)phenyl)-N-(1-(6-(trifluoromethyl)pyrazin-2-yl)pyrrolidin-3-yl)acetamide

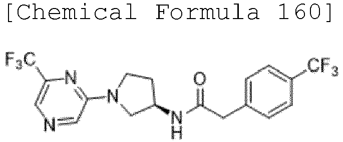

According to a technique similar to Example 7, (R)-1-(6-(trifluoromethyl)pyrazin-2-yl)pyrrolidin-3-amine was synthesized from tert-butyl (R)-(1-(6-(trifluoromethyl)pyrazin-2-yl)pyrrolidin-3-yl)carbamate (159 mg, 0.48 mmol) synthesized in Reference Example 9, and the title compound (white powder, 187 mg, 94%) was obtained using 2-(4-(trifluoromethyl)phenyl)acetic acid (117 mg, 0.57 mmol).

¹H NMR (CDCl₃, 400 MHz): δ = 1.9 - 2.0 (m, 1H), 2.3-2.4 (m, 1H), 3.38 (dd, 1H, J = 4, 11 Hz), 3.6 - 3.7 (m, 4H), 3.87 (dd, 1H, J = 6, 12 Hz), 4.6 - 4.7 (m, 1H), 5.53 (d, 1H, J = 6 Hz), 7.40 (d, 2H, J = 8 Hz), 7.61 (d, 2H, J = 8 Hz), 8.03 (s, 1H), 8.17 (s, 1H).

MS: 419.10 [M+H]⁺

### Example 12

(R)-N-(1-(6-cyano-5-(trifluoromethyl)pyridin-3-yl)pyrrolidin-3-yl)-2-(4-cyclopropylphenyl)acetamide

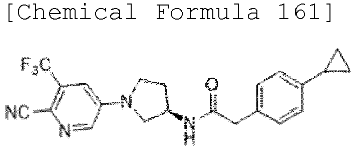

According to a technique similar to Example 3, (R)-1-(6-cyano-5-(trifluoromethyl)pyridin-3-yl)pyrrolidin-3-amine was synthesized from tert-butyl (R)-(1-(6-cyano-5-(trifluoromethyl)pyridin-3-yl)pyrrolidin-3-yl)carbamate (139 mg, 0.39 mmol) synthesized in Reference Example 10, and the title compound (white amorphous, 52 mg, 32%) was obtained using 2-(4-cyclopropylphenyl)acetic acid (82 mg, 0.47 mmol) synthesized in Reference Example 33-2.

¹H NMR (CDCl₃, 400 MHz): δ = 0.6 - 0.7 (m, 2H), 0.9-1.0 (m, 2H), 1.8 - 1.9 (m, 1H), 1.9 - 2.0 (m, 1H), 2.3-2.4 (m, 1H), 3.23 (dd, 1H, J = 8, 10 Hz), 3.4 - 3.6 (m, 4H), 3.7 - 3.8 (m, 1H), 4.5 - 4.7 (m, 1H), 5.80 (d, 1H, J = 6 Hz), 6.88 (d, 1H, J = 3 Hz), 7.02 (d, 2H, J = 8 Hz), 7.11 (d, 2H, J = 8 Hz), 8.00 (d, 1H, J = 2 Hz).

MS: 413.15 [M-H]⁻

### Example 13

Methyl 3-(2-(4-cyclopropylphenyl)acetamido)-1-(5-(trifluoromethyl)pyridin-3-yl) pyrrolidine-3-carboxylate

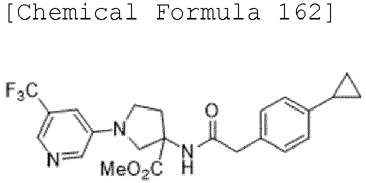

According to a technique similar to Example 9, the title compound (white amorphous, 53 mg, 38%) was obtained using methyl 3-(2-(4-cyclopropylphenyl)acetamido)pyrrolidine-3-carboxylate (94 mg, 0.31 mmol) synthesized in Reference Example 11-2 and 3-bromo-5-(trifluoromethyl)pyridine (84 mg, 0.37 mmol).

¹H NMR (CDCl₃, 400 MHz): δ = 0.6 - 0.7 (m, 2H), 0.9-1.0 (m, 2H), 1.8 - 1.9 (m, 1H), 2.3 - 2.4 (m, 1H), 2.5-2.6 (m, 1H), 3.4 - 3.5 (m, 2H), 3.55 (s, 2H), 3.5 - 3.6 (m, 1H), 3.75 (s, 3H), 3.98 (d, 1H, J = 10 Hz), 6.17 (s, 1H), 6.8 - 6.9 (m, 1H), 7.0 - 7.1 (m, 2H), 7.1 - 7.2 (m, 2H), 8.05 (d, 1H, J = 3 Hz), 8.20 (s, 1H).

MS: 448.14 [M+H]⁺

### Example 14

3-(2-(4-cyclopropylphenyl)acetamido)-1-(5-(trifluoromethyl)pyridin-3-yl)pyrrolidine-3-carboxylic acid

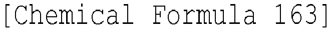

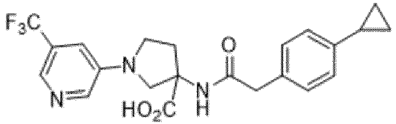

Methyl 3-(2-(4-cyclopropylphenyl)acetamido)-1-(5-(trifluoromethyl)pyridin-3-yl)pyrrolidine-3-carboxylate (50 mg, 0.11 mmol) synthesized in Example 13 was dissolved in THF (2.0 mL), a 2N aqueous solution of sodium hydroxide (2.0 mL) was added thereto, and the mixture was stirred for 3 hours at room temperature. a 1N aqueous solution of hydrochloric acid was added to the reaction liquid to neutralize the reaction liquid, and then the reaction liquid was extracted with a mixed liquid of chloroform and methanol. An organic layer thus separated was dried over anhydrous sodium sulfate, insoluble materials were filtered, and then the solvent was distilled off under reduced pressure. A residue thus obtained was purified by silica gel column chromatography (ethyl acetate: methanol) (concentration gradient: 0 to 90%), subsequently methanol and water were added to a powder thus obtained, and the mixture was stirred for a while. A precipitated powder was filtered and dried under reduced pressure for 3 hours at 100°C, and thereby the title compound (white powder, 38 mg, 79%) was obtained.

¹H NMR (CD₃OD, 400 MHz): δ = 0.5 - 0.6 (m, 2H), 0.8-0.9 (m, 2H), 1.8 - 1.9 (m, 1H), 2.4 - 2.6 (m, 2H), 3.4-3.6 (m, 4H), 3.6 - 3.7 (m, 1H), 3.9 - 4.0 (m, 1H), 4.60 (br s, 1H), 6.9 - 7.0 (m, 2H), 7.0 - 7.2 (m, 3H), 8.0 - 8.1 (m, 2H). The 1H content is not observable.

MS: 434.16 [M+H]⁺

### Example 15

(R)-2-(1H-indazol-6-yl)-N-(1-(5-(trifluoromethyl)pyridin-3-yl)pyrrolidin-3-yl)acetamide

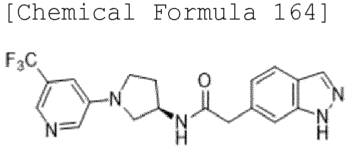

According to a technique similar to Example 1, the title compound (white powder, 82 mg, 74%) was obtained using (R)-1-(5-(trifluoromethyl)pyridin-3-yl)pyrrolidin-3-amine (66 mg, 0.28 mmol) synthesized in Reference Example 1-2 and 2-(1H-indazol-6-yl)acetic acid (50 mg, 0.28 mmol).

¹H NMR (CDCl₃, 400 MHz): δ = 1.8 - 2.0 (m, 1H), 2.3-2.4 (m, 1H), 3.13 (dd, 1H, J = 4, 10 Hz), 3.3 - 3.4 (m, 2H), 3.63 (dd, 1H, J = 6, 10 Hz), 3.70 (s, 2H), 4.6 - 4.7 (m, 1H), 5.62 (d, 1H, J = 7 Hz), 6.8 - 6.9 (m, 1H), 7.04 (dd, 1H, J = 1, 8 Hz), 7.39 (s, 1H), 7.72 (d, 1H, J = 8 Hz), 8.0 - 8.1 (m, 2H), 8.18 (s, 1H), 10.1 (br s, 1H).

MS: 388.15 [M-H]⁻

### Example 16

(R)-2-(4-cyclopropylphenyl)-N-(1-(4-(trifluoromethyl)thiazol-2-yl)pyrrolidin-3-yl) acetamide

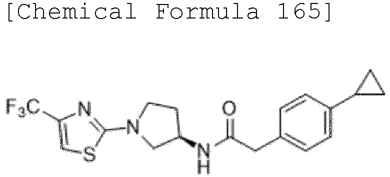

According to a technique similar to Example 1, the title compound (yellow-green powder, 30 mg, 55%) was obtained using (R)-1-(4-(trifluoromethyl)thiazol-2-yl)pyrrolidin-3-amine (33 mg, 0.14 mmol) synthesized in Reference Example 12-2 and 2-(4-cyclopropylphenyl)acetic acid (29 mg, 0.17 mmol) synthesized in Reference Example 33-2.

¹H NMR (CDCl₃, 400 MHz): δ = 0.6 - 0.7 (m, 2H), 0.9-1.0 (m, 2H), 1.8 - 2.0 (m, 2H), 2.2 - 2.4 (m, 1H), 3.23 (dd, 1H, J = 4, 10 Hz), 3.4 - 3.6 (m, 4H), 3.74 (dd, 1H, J = 6, 10 Hz), 4.5 - 4.7 (m, 1H), 5.47 (d, 1H, J = 6 Hz), 6.92 (s, 1H), 7.04 (d, 2H, J = 8 Hz), 7.10 (d, 2H, J = 2 Hz) .

MS: 394.14 [M-H]⁻

### Example 17

2-(4-Isobutylphenyl)-N-((R)-1-(5-(trifluoromethyl)pyridin-3-yl)pyrrolidin-3-yl)propanamide

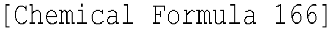

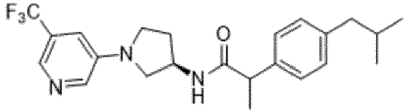

According to a technique similar to Example 1, the title compound (pale yellow amorphous, 50 mg, 79%) was obtained using (R)-1-(5-(trifluoromethyl)pyridin-3-yl)pyrrolidin-3-amine (35 mg, 0.15 mmol) synthesized in Reference Example 1-2 and 2-(4-isobutylphenyl)propanoic acid (37 mg, 0.18 mmol).

¹H NMR (CDCl₃, 400 MHz): δ = 0.8 - 0.9 (m, 6H), 1.51 (d, 1.5H, J = 8 Hz), 1.52 (d, 1.5H, J = 8 Hz), 1.8 - 2.0 (m, 2H), 2.2 - 2.3 (m, 1H), 2.4 - 2.5 (m, 2H), 3.03 (dd, 0.5H, J = 4, 10 Hz), 3.12 (dd, 0.5H, J = 4, 10 Hz), 3.2 - 3.4 (m, 2H), 3.5 - 3.7 (m, 2H), 4.5 - 4.7 (m, 1H), 5.67 (d, 0.5H, J = 7 Hz), 5.70 (d, 0.5H, J = 7 Hz), 6.8 - 6.9 (m, 1H), 7.0 - 7.1 (m, 2H), 7.1 - 7.2 (m, 2H), 8.01 (dd, 1H, J = 2, 2 Hz), 8.15 (s, 1H).

MS: 420.20 [M+H]⁺

### Example 18

(R)-2-(4-cyclopropylphenyl)-N-(1-(6-methyl-5-(trifluoromethyl)pyridin-3-yl)pyrrolidin-3-yl)acetamide

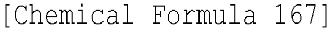

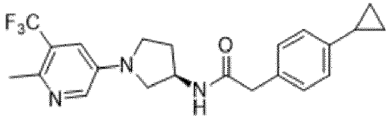

According to a technique similar to Example 7, (R)-1-(6-methyl-5-(trifluoromethyl)pyridin-3-yl)pyrrolidin-3-amine (white powder, 22 mg) was synthesized from tert-butyl (R)-(1-(6-methyl-5-(trifluoromethyl)pyridin-3-yl)pyrrolidin-3-yl)carbamate (22 mg, 0.06 mmol) synthesized in Reference Example 13, and the title compound (white amorphous, 5.9 mg, 23%) was obtained using 2-(4-cyclopropylphenyl)acetic acid (13 mg, 0.07 mmol) synthesized in Reference Example 33-2.

¹H NMR (CDCl₃, 400 MHz): δ = 0.6 - 0.7 (m, 2H), 0.9-1.0 (m, 2H), 1.8 - 1.9 (m, 2H), 2.2 - 2.4 (m, 1H), 2.57 (s, 3H), 3.08 (dd, 1H, J = 4, 10 Hz), 3.3 - 3.4 (m, 2H), 3.53 (s, 2H), 3.60 (dd, 1H, J = 6, 10 Hz), 4.5 - 4.7 (m, 1H), 5.50 (d, 1H, J = 8 Hz), 6.95 (d, 1H, J = 2 Hz), 7.04 (d, 2H, J = 8 Hz), 7.11 (d, 2H, J = 8 Hz), 7.93 (d, 1H, J = 2 Hz) .

MS: 404.16 [M+H]⁺

### Example 19

(R)-2-(4-cyclopropylphenyl)-N-(1-(5-(2-hydroxypropan-2-yl)pyridin-3-yl)pyrrolidin-3-yl)acetamide

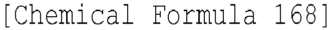

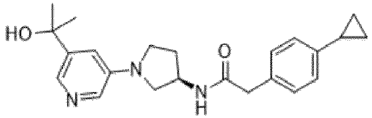

According to a technique similar to Example 1, the title compound (pale yellow amorphous, 81 mg, 86%) was obtained using (R)-2-(5-(3-aminopyrrolidin-1-yl)pyridin-3-yl)propan-2-ol (55 mg, 0.17 mmol) synthesized in Reference Example 14-2 and 2-(4-cyclopropylphenyl)acetic acid (36 mg, 0.20 mmol) synthesized in Reference Example 33-2.

¹H NMR (CDCl₃, 400 MHz): δ = 0.6 - 0.7 (m, 2H), 0.9 - 1.0 (m, 2H), 1.59 (s, 6H), 1.7 - 1.8 (m, 1H), 1.8 - 1.9 (m, 2H), 2.2 - 2.4 (m, 1H), 3.0 - 3.1 (m, 1H), 3.3 - 3.4 (m, 2H), 3.53 (s, 2H), 3.60 (dd, 1H, J = 4, 10 Hz), 4.6 - 4.7 (m, 1H), 5.61 (d, 1H, J = 7 Hz), 6.9 - 7.0 (m, 1H), 7.04 (d, 2H, J = 8 Hz), 7.12 (d, 2H, J = 8 Hz), 7.82 (br s, 1H), 8.04 (br s, 1H).

MS: 380.20 [M+H]⁺

### Example 20

(R)-2-(4-cyclopropylphenyl)-N-(1-(8-(trifluoromethyl)imidazo[1,2-a]pyridin-6-yl)pyrrolidin-3-yl)acetamide

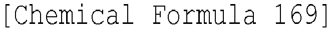

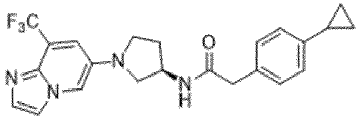

According to a technique similar to Example 7, (R)-1-(8-(trifluoromethyl)imidazo[1,2-a]pyridin-6-yl)pyrrolidin-3-amine (56 mg) was synthesized from tert-butyl (R)-(1-(8-(trifluoromethyl)imidazo[1,2-a]pyridin-6-yl)pyrrolidin-3-yl)-carbamate (90 mg, 0.24 mmol) synthesized in Reference Example 15, and the title compound (yellow amorphous, 58 mg, 56%) was obtained using 2-(4-cyclopropylphenyl)acetic acid (44 mg, 0.25 mmol) synthesized in Reference Example 33-2.

¹H NMR (CDCl₃, 400 MHz): δ = 0.6 - 0.7 (m, 2H), 0.9 - 1.0 (m, 2H), 1.8 - 2.0 (m, 2H), 2.3 - 2.4 (m, 1H), 3.0 - 3.1 (m, 1H), 3.2 - 3.3 (m, 1H), 3.3 - 3.4 (m, 1H), 3.5 - 3.6 (m, 1H), 3.56 (s, 2H), 4.6 - 4.7 (m, 1H), 6.70 (d, 1H, J = 6 Hz), 7.04 (d, 2H, J = 8 Hz), 7.08 (s, 1H), 7.15 (d, 2H, J = 8 Hz), 7.52 (s, 1H), 7.60 (s, 1H). The 1H content is not observable.

MS: 429.16 [M+H]⁺

### Example 21

3-(2-(4-cyclopropylphenyl)acetamido)-1-(5-(trifluoromethyl)pyridin-3-yl) pyrrolidine-3-carboxyami de

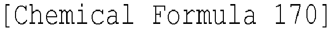

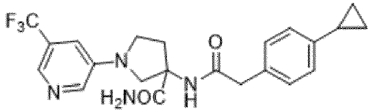

According to a technique similar to Example 1, the title compound (white powder, 11 mg, 73%) was obtained using 3-(2-(4-cyclopropylphenyl)acetamido)-1-(5-(trifluoromethyl)pyridin-3-yl)pyrrolidine-3-carboxylic acid (15 mg, 0.04 mmol) synthesized in Example 14 and ammonium acetate (5.3 mg, 0.07 mmol).

¹H NMR (DMSO - d₆, 400 MHz): δ = 0.5 - 0.6 (m, 2H), 0.8 - 0.9 (m, 2H), 1.8 - 1.9 (m, 1H), 2.3 - 2.4 (m, 2H), 3.2 - 3.5 (m, 4H), 3.5 - 3.6 (m, 1H), 3.82 (d, 1H, J = 10 Hz), 6.85 (d, 2H, J = 8 Hz), 6.9 - 7.1 (m, 3H), 7.11 (s, 1H), 7.29 (br s, 1H), 8.1 - 8.2 (m, 2H), 8.54 (s, 1H).

MS: 431.20 [M-H]⁻

### Example 22

(R)-2-(4-hydroxyphenyl)-N-(1-(5-(trifluoromethyl)pyridin-3-yl)pyrrolidin-3-yl)acetamide

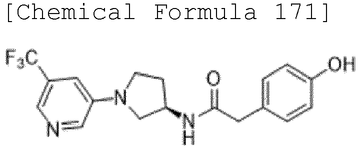

According to a technique similar to Example 1, the title compound (white amorphous, 292 mg, 92%) was obtained using (R)-1-(5-(trifluoromethyl)pyridin-3-yl)pyrrolidin-3-amine (200 mg, 0.86 mmol) synthesized in Example 1-2 and 2-(4-hydroxyphenyl)acetic acid (158 mg, 1.04 mmol).

¹H NMR (CDCl₃, 400 MHz): δ = 1.8 - 2.0 (m, 1H), 2.3 - 2.4 (m, 1H), 3.12 (dd, 1H, J = 5, 10 Hz), 3.3 - 3.4 (m, 2H), 3.51 (s, 2H), 3.65 (dd, 1H, J = 6, 10 Hz), 5.18 (s, 1H), 5.50 (d, 1H, J = 6 Hz), 6.8 - 6.9 (m, 2H), 6.9 - 7.0 (m, 1H), 7.1 - 7.2 (m, 2H), 8.07 (d, 2H, J = 3 Hz), 8.21 (s, 1H).

MS: 366.11 [M+H]⁺

### Example 23

(R)-2-(4-(1,1-dioxide thiomorpholino)phenyl)-N-(1-(5-(trifluoromethyl)pyridin-3-yl)pyrrolidin-3-yl)acetamide

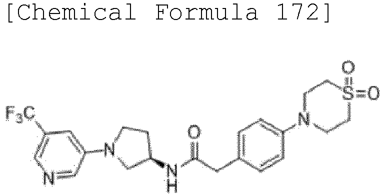

Ethyl 2-(4-(1,1-dioxide thiomorpholino)phenyl)acetate (30 mg, 0.10 mmol) synthesized in Reference Example 16 was dissolved in THF (2.0 mL), subsequently a 2N aqueous solution of sodium hydroxide (2.0 mL) was added thereto, the mixture was stirred for 20 minutes at 50°C, and the solvent was distilled off under reduced pressure. Water and ethyl acetate were added to a residue thus obtained, the mixture was stirred for a while, subsequently a 1N aqueous solution of hydrochloric acid was added to an aqueous layer thus separated to neutralize the aqueous layer, and the aqueous layer was extracted with a mixed liquid of chloroform and methanol. An organic layer thus separated was dried over anhydrous sodium sulfate, insoluble materials were filtered, subsequently the solvent was distilled off under reduced pressure, and thereby a crude form of 2-(4-(1,1-dioxide thiomorpholino)phenyl)acetic acid (pale yellow crystals) was obtained. According to a technique similar to Example 1, the title compound (white amorphous, 30 mg, 61%) was obtained using the crude form of 2-(4-(1,1-dioxide thiomorpholino)phenyl)acetic acid thus obtained and (R)-1-(5-(trifluoromethyl)pyridin-3-yl)pyrrolidin-3-amine (23 mg, 0.10 mmol) synthesized in Reference Example 1-2.

¹H NMR (CDCl₃, 400 MHz): δ = 1.9 - 2.0 (m, 1H), 2.3 - 2.4 (m, 1H), 3.0 - 3.1 (m, 4H), 3.16 (dd, 1H, J = 5, 10 Hz), 3.3 - 3.5 (m, 2H), 3.51 (s, 2H), 3.67 (dd, 1H, J = 6, 10 Hz), 3.8 - 3.9 (m, 4H), 4.6 - 4.7 (m, 1H), 5.53 (d, 1H, J = 7 Hz), 6.8 - 7.0 (m, 3H), 7.1 - 7.2 (m, 2H), 8.09 (d, 1H, J = 2 Hz), 8.21 (s, 1H).

MS: 481.18 [M-H]⁻

### Example 24

(R)-1-(4-chlorophenyl)-N-(1-(5-(trifluoromethyl)pyridin-3-yl)pyrrolidin-3-yl)cyclopropane-1-carboxyamide

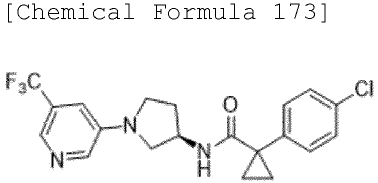

According to a technique similar to Example 1, the title compound (white amorphous, 70 mg, 79%) was obtained using (R)-1-(5-(trifluoromethyl)pyridin-3-yl)pyrrolidin-3-amine (50 mg, 0.22 mmol) synthesized in Reference Example 1-2 and 1-(4-chlorophenyl)cyclopropane-1-carboxylic acid (51 mg, 0.26 mmol).

¹H NMR (CDCl₃, 400 MHz): δ = 1.0 - 1.1 (m, 2H), 1.6 - 1.7 (m, 2H), 1.7 - 1.9 (m, 1H), 2.2 - 2.4 (m, 1H), 3.0 - 3.1 (m, 1H), 3.2 - 3.4 (m, 2H), 3.6 - 3.7 (m, 1H), 4.5 - 4.6 (m, 1H), 5.36 (d, 1H, J = 5 Hz), 6.89 (s, 1H), 7.2 - 7.4 (m, 4H), 8.05 (s, 1H), 8.19 (s, 1H).

MS: 410.07 [M+H]⁺

### Example 25

2-(4-Bromophenyl)-2-hydroxy-N-((R)-1-(5-(trifluoromethyl)pyridin-3-yl)pyrrolidin-3-yl)acetamide

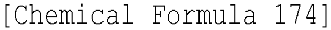

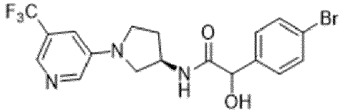

According to a technique similar to Example 1, the title compound (white powder, 68 mg, 71%) was obtained using (R)-1-(5-(trifluoromethyl)pyridin-3-yl)pyrrolidin-3-amine (50 mg, 0.22 mmol) synthesized in Reference Example 1-2 and 2-(4-bromophenyl)-2-hydroxyacetic acid (60 mg, 0.26 mmol) .

¹H NMR (CDCl₃, 400 MHz): δ = 2.0 - 2.1 (m, 1H), 2.3 - 2.4 (m, 1H), 3.1 - 3.2 (m, 1H), 3.3 - 3.5 (m, 2H), 3.6 - 3.7 (m, 1H), 3.92 (s, 0.5H), 4.15 (s, 0.5H), 4.6 - 4.7 (m, 1H), 5.06 (s, 1H), 6.8 - 7.0 (m, 2H), 7.31 (d, 2H, J = 8 Hz), 7.49 (d, 2H, J = 8 Hz), 7.94 (d, 1H, J = 8 Hz), 8.12 (d, 1H, J = 4 Hz).

MS: 444.00 [M+H]⁺

### Example 26

(R)-N-(1-(5-(trifluoromethyl)pyridin-3-yl)pyrrolidin-3-yl)-2-(4-(trimethylsilyl)phenyl)acetamide

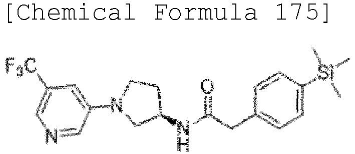

Ethyl 2-(4-(trimethylsilyl)phenyl)acetate (30 mg, 0.13 mmol) synthesized in Reference Example 17 was dissolved in methanol (2.0 mL), a 2N aqueous solution of sodium hydroxide (2.0 mL) was added thereto, the mixture was stirred for 4 hours at room temperature, and the solvent was distilled off under reduced pressure. Water was added thereto to obtain an aqueous solution, subsequently the aqueous solution was washed with ethyl acetate, a 1N aqueous solution of hydrochloric acid was added thereto to neutralize the aqueous solution, and the aqueous layer was extracted with a mixed liquid of chloroform and methanol. An organic layer thus separated was dried over anhydrous sodium sulfate, insoluble materials were filtered, subsequently the solvent was distilled off under reduced pressure, and thereby a crude form of 2-(4-(trimethylsilyl)phenyl)acetic acid was obtained. According to a technique similar to Example 1, the title compound (colorless oily material, 6.8 mg, 13%) was obtained using the crude form of 2-(4-(trimethylsilyl)phenyl)acetic acid thus obtained and (R)-1-(5-(trifluoromethyl)pyridin-3-yl)pyrrolidin-3-amine (29 mg, 0.13 mmol) synthesized in Reference Example 1-2.

¹H NMR (CDCl₃, 400 MHz): δ = 0.26 (s, 9H), 1.9 - 2.0 (m, 1H), 2.3 - 2.4 (m, 1H), 3.13 (dd, 1H, J = 4, 10 Hz), 3.3 - 3.4 (m, 2H), 3.58 (s, 2H), 3.66 (dd, 1H, J = 6, 10 Hz), 4.6 - 4.7 (m, 1H), 5.65 (d, 1H, J = 7 Hz), 6.89 (dd, 1H, J = 2, 2 Hz), 7.2 - 7.3 (m, 2H), 7.4 - 7.5 (m, 2H), 8.07 (d, 1H, J = 3 Hz), 8.19 (s, 1H).

MS: 422.16 [M+H]⁺

### Example 27

(R)-2-(4-(2-hydroxy-2-methylpropoxy)phenyl)-N-(1-(5-(trifluoromethyl)pyridin-3-yl)pyrrolidin-3-yl)acetamide

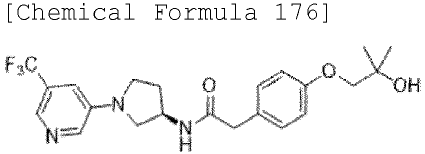

(R)-2-(4-hydroxyphenyl)-N-(1-(5-(trifluoromethyl)pyridin-3-yl)pyrrolidin-3-yl)acetamide (30 mg, 0.08 mmol) synthesized in Example 22 was dissolved in DMF (1.0 mL), subsequently 2,2-dimethyloxirane (22 µL, 0.25 mmol) and potassium carbonate (34 mg, 0.25 mmol) were added thereto, and the mixture was stirred for 16 hours at 110°C

To the reaction liquid that had been left to cool to room temperature, water was added, the mixture was stirred for a while, and then the mixture was extracted with a mixed liquid of chloroform and methanol. An organic layer thus separated was dried over anhydrous sodium sulfate, insoluble materials were filtered, and then the solvent was distilled off under reduced pressure. A residue thus obtained was purified by silica gel column chromatography (ethyl acetate: methanol) (concentration gradient: 0 to 40%), and the title compound (colorless oily material, 3.4 mg, 9.4%) was obtained.

¹H NMR (CDCl₃, 400 MHz): δ = 1.35 (s, 6H), 1.8 - 1.9 (m, 1H), 2.25 (s, 1H), 2.3 - 2.4 (m, 1H), 3.12 (dd, 1H, J = 5, 10 Hz), 3.3 - 3.4 (m, 2H), 3.53 (s, 2H), 3.65 (dd, 1H, J = 6, 10 Hz), 3.78 (s, 2H), 4.6 - 4.7 (m, 1H), 5.48 (d, 1H, J = 8 Hz), 6.8 - 6.9 (m, 3H), 7.1 - 7.2 (m, 2H), 8.07 (d, 1H, J = 2 Hz), 8.21 (s, 1H).

MS: 438.17 [M+H]⁺

### Example 28

2-(4-cyclopropylphenyl)-N-((3S,4S)-4-fluoro-1-(5-(trifluoromethyl)pyridin-3-yl)pyrrolidin-3-yl)acetamide

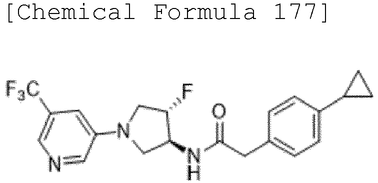

A 2N hydrochloric acid-methanol solution (12 mL) was added to tert-butyl (3S,4S)-3-(2-(4-cyclopropylphenyl)acetamido)-4-fluoropyrrolidine-1-carboxylate (369 mg, 1.02 mmol) synthesized in Reference Example 18 under ice cooling, the mixture was stirred for 2 hours at room temperature, subsequently the solvent was distilled off under reduced pressure, and thereby a crude form of (2-(4-cyclopropylphenyl)-N-((3S,4S)-4-fluoropyrrolidin-3-ylacetamide (230 mg) was obtained. The crude form of 2-(4-cyclopropylphenyl)-N-((3S,4S)-4-fluoropyrrolidin-3-ylacetamide (230 mg) thus obtained, 3-bromo-5-(trifluoromethyl)pyridine (230 mg, 1.02 mmol), tris(dibenzylidene acetone)dipalladium(0) (92 mg, 0.10 mmol), Xantphos (118 mg, 0.20 mmol), and potassium carbonate (281 mg, 2.04 mmol) were suspended in toluene (5.8 mL), and the suspension was stirred for 16 hours at 85°C under a nitrogen gas stream. The reaction liquid that had been left to cool to room temperature was filtered through Celite, and the solvent was distilled off under reduced pressure. A residue thus obtained was purified by silica gel column chromatography (heptane: ethyl acetate) (concentration gradient: 0 to 100%), and the title compound (white amorphous, 28 mg, 6.7%) was obtained.

¹H NMR (CDCl₃, 400 MHz): δ = 0.6 - 0.7 (m, 2H), 0.9 - 1.0 (m, 2H), 1.8 - 1.9 (m, 1H), 3.24 (d, 1H, J = 11 Hz), 3.4 - 3.6 (m, 4H), 3.7 - 3.8 (m, 1H), 4.6 - 4.7 (m, 1H), 5.1 - 5.3 (m, 1H), 5.6 - 5.7 (m, 1H), 6.88 (dd, 1H, J = 2, 2 Hz), 7.0 - 7.1 (m, 4H), 8.04 (d, 1H, J = 3 Hz), 8.21 (s, 1H) .

MS: 408.16 [M+H]⁺

### Example 29

(R)-2-(4-(dimethylamino)phenyl)-N-(1-(5-(trifluoromethyl)pyridin-3-yl)pyrrolidin-3-yl)acetamide

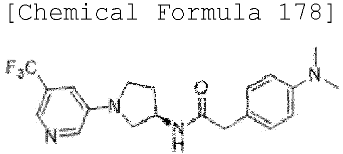

According to a technique similar to Example 1, the title compound (white amorphous, 40 mg, 51%) was obtained using (R)-1-(5-(trifluoromethyl)pyridin-3-yl)pyrrolidin-3-amine (46 mg, 0.20 mmol) synthesized in Reference Example 1-2 and 2-(4-(dimethylamino)phenyl)acetic acid (43 mg, 0.24 mmol) .

¹H NMR (CDCl₃, 400 MHz): δ = 1.8 - 1.9 (m, 1H), 2.3 - 2.4 (m, 1H), 2.94 (s, 6H), 3.09 (dd, 1H, J = 5, 10 Hz), 3.3 - 3.4 (m, 2H), 3.49 (s, 2H), 3.64 (dd, 1H, J = 6, 10 Hz), 4.6 - 4.7 (m, 1H), 5.63 (d, 1H, J = 7 Hz), 6.6 - 6.7 (m, 2H), 6.88 (dd, 1H, J = 2, 2 Hz), 7.0 - 7.1 (m, 2H), 8.06 (d, 1H, J = 3 Hz), 8.18 (s, 1H).

MS: 393.16 [M+H]⁺

### Example 30

(R)-2-(4-nitrophenyl)-N-(1-(5-(trifluoromethyl)pyridin-3-yl)pyrrolidin-3-yl)acetamide

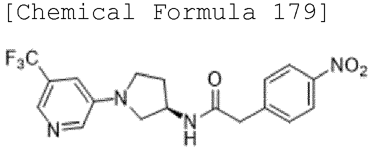

According to a technique similar to Example 1, the title compound (pale yellow amorphous, 219 mg, 86%) was obtained using (R)-1-(5-(trifluoromethyl)pyridin-3-yl)pyrrolidin-3-amine (150 mg, 0.65 mmol) synthesized in Reference Example 1-2 and 2-(4-nitrophenyl)acetic acid (141 mg, 0.78 mmol).

¹H NMR (CDCl₃, 400 MHz): δ = 2.0 - 2.1 (m, 1H), 2.3 - 2.4 (m, 1H), 3.20 (dd, 1H, J = 3, 10 Hz), 3.3 - 3.4 (m, 2H), 3.63 (dd, 1H, J = 6, 10 Hz), 3.67 (s, 2H), 4.6 - 4.7 (m, 1H), 6.59 (d, 1H, J = 7 Hz), 6.8 - 6.9 (m, 1H), 7.4 - 7.5 (m, 2H), 7.97 (d, 1H, J = 3 Hz), 8.11 (br s, 1H), 8.1 - 8.2 (m, 2H).

MS: 395.09 [M+H]⁺

### Example 31

(S)-2-(4-isobutylphenyl)-N-((R)-1-(5-(trifluoromethyl)pyridin-3-yl)pyrrolidin-3-yl)propanamide

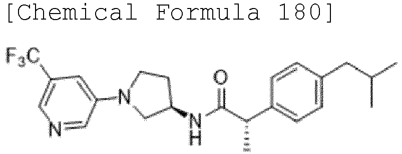

According to a technique similar to Example 1, the title compound (white crystals, 22 mg, 81%) was obtained using (R)-1-(5-(trifluoromethyl)pyridin-3-yl)pyrrolidin-3-amine (15 mg, 0.06 mmol) synthesized in Reference Example 1-2 and (S)-2-(4-isobutylphenyl)propanoic acid (16 mg, 0.08 mmol) .

¹H NMR (CDCl₃, 400 MHz): δ = 0.8 - 0.9 (m, 6H), 1.51 (d, 3H, J = 7 Hz), 1.8 - 1.9 (m, 2H), 2.2 - 2.3 (m, 1H), 2.45 (d, 2H, J = 7 Hz), 3.13 (dd, 1H, J = 4, 10 Hz), 3.2 - 3.4 (m, 2H), 3.5 - 3.6 (m, 1H), 3.63 (dd, 1H, J = 6, 10 Hz), 4.5 - 4.7 (m, 1H), 5.59 (d, 1H, J = 7 Hz), 6.86 (dd, 1H, J = 2, 2 Hz), 7.0 - 7.1 (m, 2H), 7.1 - 7.2 (m, 2H), 8.03 (d, 1H, J = 3 Hz), 8.16 (s, 1H).

MS: 418.25 [M-H]⁻

### Example 32

(R)-2-(3-fluoro-4-(trifluoromethyl)phenyl)-N-(1-(5-(trifluoromethyl)pyridin-3-yl)pyrrolidin-3-yl)acetamide

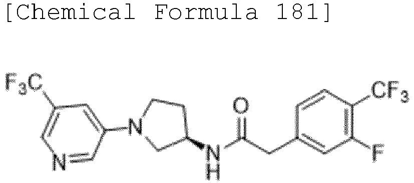

According to a technique similar to Example 1, the title compound (white amorphous, 25 mg, 66%) was obtained using (R)-1-(5-(trifluoromethyl)pyridin-3-yl)pyrrolidin-3-amine (20 mg, 0.09 mmol) synthesized in Reference Example 1-2 and 2-(3-fluoro-4-(trifluoromethyl)phenyl)acetic acid (23 mg, 0.10 mmol).

¹H NMR (CDCl₃, 400 MHz): δ = 2.0- 2.1 (m, 1H), 2.3 - 2.4 (m, 1H), 3.20 (dd, 1H, J = 4, 10 Hz), 3.3 - 3.5 (m, 2H), 3.60 (s, 2H), 3.65 (dd, 1H, J = 6, 10 Hz), 4.6 - 4.7 (m, 1H), 6.27 (d, 1H, J = 7 Hz), 6.86 (dd, 1H, J = 2, 2 Hz), 7.16 (s, 1H), 7.1 - 7.2 (m, 1H), 7.57 (t, 1H, J = 7 Hz), 8.02 (d, 1H, J = 3 Hz), 8.14 (s, 1H).

MS: 436.08 [M+H]⁺

### Example 33

(R)-2-(4-aminophenyl)-N-(1-(5-(trifluoromethyl)pyridin-3-yl)pyrrolidin-3-yl)acetamide

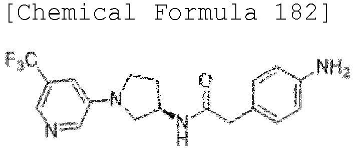

(R)-2-(4-nitrophenyl)-N-(1-(5-(trifluoromethyl)pyridin-3-yl)pyrrolidin-3-yl)acetamide (215 mg, 0.55 mmol) synthesized in Example 30 was dissolved in methanol (20 mL), palladium-carbon (20 mg) was added thereto, and the mixture was stirred for 6 hours at room temperature under a hydrogen gas stream. Insoluble materials were filtered, subsequently the solvent was distilled off under reduced pressure, a residue thus obtained was purified by silica gel column chromatography (ethyl acetate: methanol) (concentration gradient: 0 to 30%), and the title compound (white amorphous, 195 mg, 98%) was obtained.

¹H NMR (CDCl₃, 400 MHz): δ = 1.8 - 1.9(m, 1H), 2.3 - 2.4 (m, 1H), 3.10 (dd, 1H, J = 5, 10 Hz), 3.3 - 3.4 (m, 2H), 3.48 (s, 2H), 3.64 (dd, 1H, J = 6, 10 Hz), 3.69 (br s, 2H), 4.6 - 4.7 (m, 1H), 5.59 (d, 1H, J = 7 Hz), 6.6 - 6.7 (m, 2H), 6.8 - 6.9 (m, 1H), 7.0 - 7.1 (m, 2H), 8.06 (d, 1H, J = 3 Hz), 8.19 (s, 1H).

MS: 365.10 [M+H]⁺

### Example 34

(R)-1-(4-(trifluoromethyl)phenyl)-N-(1-(5-(trifluoromethyl)pyridin-3-yl)pyrrolidin-3-yl)cyclopropane-1-carboxyamide

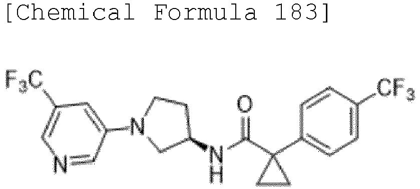

2-(4-(Trifluoromethyl)phenyl)acetonitrile (1.00g, 5.40 mmol) was dissolved in THF (8.0 mL), subsequently the solution was stirred for a while at -50°C, a THF solution of lithium bis(trimethylsilyl)amide (1.3M) (4.15 mL, 5.40 mmol) was added thereto, and then 1-bromo-2-chloroethane was added thereto. Subsequently, a THF solution of lithium bis(trimethylsilyl)amide (1.3M) (4.15 mL, 5.40 mmol) was added thereto again, the mixture was stirred for 30 minutes, and then the mixture was stirred for 2 hours at room temperature. Water was added to the reaction liquid, the mixture was extracted with ethyl acetate, an organic layer thus separated was dried over anhydrous sodium sulfate, insoluble materials were filtered, subsequently the solvent was distilled off under reduced pressure, and thereby a crude form of 1-4-(trifluoromethyl)phenyl)cyclopropane-1-carbonitrile (pale yellow powder, 219 mg) was obtained. The crude form of 1-4-(trifluoromethyl)phenyl)cyclopropane-1-carbonitrile (219 mg) was dissolved in THF (3.0 mL), subsequently a 4N aqueous solution of sodium hydroxide (3.0 mL) was added thereto, the mixture was stirred for 16 hours at 100°C, and the solvent was distilled off under reduced pressure. Water was added thereto to obtain an aqueous solution, subsequently the aqueous solution was washed with ethyl acetate, a 2N aqueous solution of hydrochloric acid was added thereto to neutralize the aqueous solution, and the aqueous layer was extracted with a mixed liquid of chloroform and methanol. An organic layer thus separated was dried over anhydrous sodium sulfate, insoluble materials were filtered, subsequently the solvent was distilled off under reduced pressure, and thereby a crude form of 1-(4-(trifluoromethyl)phenyl)cyclopropane-1-carboxylic acid (yellow crystals, 42 mg) was obtained. According to a technique similar to Example 1, the title compound (white amorphous, 43 mg, 1.8%) was obtained using the crude form of 1-(4-(trifluoromethyl)phenyl)cyclopropane-1-carboxylic acid (42 mg) and (R)-1-(5-(trifluoromethyl)pyridin-3-yl)pyrrolidin-3-amine (42 mg, 0.18 mmol) synthesized in Reference Example 1-2.

¹H NMR (CDCl₃, 400 MHz): δ = 1.0 - 1.2 (m, 2H), 1.6 - 1.7 (m, 2H), 1.7 - 1.8 (m, 1H), 2.3 - 2.4 (m, 1H), 3.05 (dd, 1H, J = 5, 10 Hz), 3.3 - 3.4 (m, 2H), 3.64 (dd, 1H, J = 6, 10 Hz), 4.5 - 4.6 (m, 1H), 5.26 (d, 1H, J = 6 Hz), 6.8 - 6.9 (m, 1H), 7.53 (d, 2H, J = 8 Hz), 7.64 (d, 2H, J = 8 Hz), 8.0 - 8.1 (m, 1H), 8.20 (s, 1H).

MS: 444.07 [M+H]⁺

### Example 35

(R)-2-(4-acetamidophenyl)-N-(1-(5-(trifluoromethyl)pyridin-3-yl)pyrrolidin-3-yl)acetamide

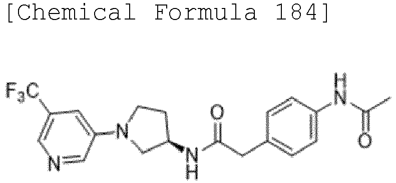

(R)-2-(4-aminophenyl)-N-(1-(5-(trifluoromethyl)pyridin-3-yl)pyrrolidin-3-yl)acetamide (30 mg, 0.08 mmol) synthesized in Example 33 was dissolved in chloroform (1.0 mL), subsequently acetyl chloride (5.9 µL, 0.08 mmol) and DIPEA (30 µL, 0.16 mmol) were added thereto, and the mixture was stirred for 60 hours at room temperature. Water was added to the reaction liquid, the mixture was stirred for a while, an organic layer thus separated was dried over anhydrous sodium sulfate, insoluble materials were filtered, and then the solvent was distilled off under reduced pressure. A residue thus obtained was purified by silica gel column chromatography (ethyl acetate: methanol) (concentration gradient: 0 to 40%), and the title compound (white amorphous, 6.0 mg, 18%) was obtained.

¹H NMR (CDCl₃, 400 MHz): δ = 1.9 - 2.0 (m, 1H), 2.18 (s, 3H), 2.3 - 2.4 (m, 1H), 3.12 (dd, 1H, J = 5, 10 Hz), 3.3 - 3.4 (m, 2H), 3.54 (s, 2H), 3.64 (dd, 1H, J = 6, 10 Hz), 4.6 - 4.7 (m, 1H), 5.66 (d, 1H, J = 7 Hz), 6.8 - 6.9 (m, 1H), 7.2 - 7.3 (m, 2H), 7.23 (br s, 1H), 7.4 - 7.5 (m, 2H), 8.05 (d, 1H, J = 2 Hz), 8.19 (s, 1H)

MS: 407.12 [M+H]⁺

### Example 36

N-(3-cyano-1-(5-(trifluoromethyl)pyridin-3-yl)pyrrolidin-3-yl)-2-(4-cyclopropylphenyl)acetamide

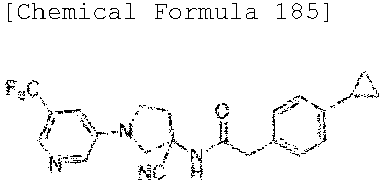

Trifluoroacetic acid (15 mL) was added to tert-butyl 3-cyano-3-(2-(4-cyclopropylphenyl)acetamido)pyrrolidine-1-carboxylate (454 mg, 1.23 mmol) synthesized in Reference Example 19 under ice cooling, the mixture was stirred for 4 hours at room temperature, subsequently a residue obtained by distilling off the solvent under reduced pressure was purified by silica gel column chromatography (ethyl acetate: methanol) (concentration gradient: 0 to 80%), and N-(3-cyanopyrrolidin-3-yl)-2-(4-cyclopropylphenyl)acetamide (brown oily material, 367 mg) was obtained. According to a technique similar to Example 9, the title compound (yellow powder, 30 mg, 5.8%) was obtained using N-(3-cyanopyrrolidin-3-yl)-2-(4-cyclopropylphenyl)acetamide (367 mg) thus obtained and 3-bromo-5-(trifluoromethyl)pyridine (278 mg, 1.23 mmol).

¹H NMR (CDCl₃, 400 MHz): δ = 0.6 - 0.7 (m, 2H), 0.9 - 1.0 (m, 2H), 1.8 - 1.9 (m, 1H), 2.1 - 2.2 (m, 1H), 2.4 - 2.5 (m, 1H), 3.49 (d, 1H, J = 10 Hz), 3.67 (d, 1H, J = 10 Hz), 3.6 - 3.8 (m, 4H), 6.9 - 7.0 (m, 1H), 7.0 - 7.1 (m, 2H), 7.1 - 7.2 (m, 2H), 8.00 (br s, 1H), 8.12 (d, 1H, J = 3 Hz), 8.20 (s, 1H).

MS: 415.13 [M+H]⁺

### Example 37

Methyl 3-(2-(1H-indol-6-yl)acetamido)-1-(5-(trifluoromethyl)pyridin-3-yl) pyrrolidine-3-carboxylat e

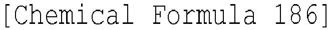

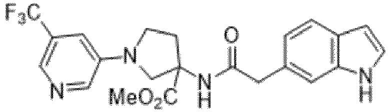

According to a technique similar to Example 7, methyl 3-amino-1-(5-(trifluoromethyl)pyridin-3-yl)pyrrolidine-3-carboxylate was synthesized from methyl 3-((tert-butoxycarbonyl)amino)-1-(5-(trifluoromethyl)pyridin-3-yl)pyrrolidine-3-carboxylate (34 mg, 0.09 mmol) synthesized in Reference Example 20-2, and the title compound (white powder, 26 mg, 67%) was obtained using 2-(1H-indol-6-yl)acetic acid (15 mg, 0.09 mmol).

¹H NMR (CDCl₃, 400 MHz): δ = 2.2 - 2.3 (m, 1H), 2.4 - 2.5 (m, 1H), 3.3 - 3.5 (m, 2H), 3.61 (d, 1H, J = 10 Hz), 3.71 (s, 2H), 3.76 (s, 3H), 4.00 (d, 1H, J = 10 Hz), 5.94 (s, 1H), 6.5 - 6.6 (m, 1H), 6.8 - 6.9 (m, 1H), 6.98 (dd, 1H, J = 2, 8 Hz), 7.2 - 7.3 (m, 2H), 7.62 (d, 1H, J = 8 Hz), 8.06 (d, 1H, J = 3 Hz), 8.16 (br s, 1H), 8.22 (s, 1H).

MS: 447.10 [M+H]⁺

### Example 38

Methyl 3-(2-(4-(trifluoromethyl)phenyl)acetamido)-1-(5-(trifluoromethyl)pyridin-3-yl)pyrrolidine-3-carboxylate

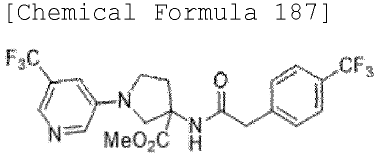

Methyl 3-(2-(4-(trifluoromethyl)phenyl)acetamido)pyrrolidine-3-carboxylate (294 mg, 0.89 mmol) synthesized in Reference Example 21-2, 3-bromo-5-(trifluoromethyl)pyridine (302 mg, 1.34 mmol), palladium acetate (20 mg, 0.09 mmol), XPhos (85 mg, 0.18 mmol), and cesium carbonate (628 mg, 1.78 mmol) were suspended in toluene (9.0 mL), and the suspension was stirred for 6 hours at 85°C under a nitrogen gas stream. The suspension was left to cool to room temperature, insoluble materials were filtered through Celite, and the solvent was distilled off under reduced pressure. A residue thus obtained was purified by silica gel column chromatography (heptane: ethyl acetate) (concentration gradient: 0 to 100%), and the title compound (white amorphous, 8.1 mg, 1.9%) was obtained.

¹H NMR (CDCl₃, 400 MHz): δ = 2.4 - 2.5 (m, 1H), 2.5 - 2.6 (m, 1H), 3.4 - 3.5 (m, 2H), 3.6 - 3.7 (m, 3H), 3.76 (s, 3H), 3.98 (d, 1H, J = 11 Hz), 6.24 (s, 1H), 6.9 - 7.0 (m, 1H), 7.40 (d, 2H, J = 8 Hz), 7.61 (d, 2H, J = 8 Hz), 8.06 (d, 1H, J = 3 Hz), 8.21 (s, 1H).

MS: 476.09 [M+H]⁺

### Example 39

(1S,2S)-2-(benzo[d]oxazol-2-yl)-N-((R)-1-(5-(trifluoromethyl)pyridin-3-yl)pyrrolidin-3-yl)cyclopropane-1-carboxyamide

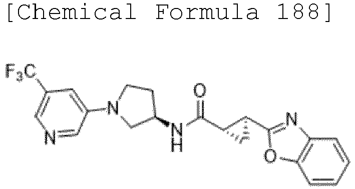

According to a technique similar to Example 26, (1S,2S)-2-(benzo[d]oxazol-2-yl)cyclopropane-1-carboxylic acid (white powder) was synthesized from methyl (1S,2S)-2-(benzo[d]oxazol-2-yl)cyclopropane-1-carboxylate (95 mg, 0.44 mmol) synthesized in Reference Example 25-2, and the title compound (white powder, 72 mg, 40%) was obtained using (R)-1-(5-(trifluoromethyl)pyridin-3-yl)pyrrolidin-3-amine (101 mg, 0.44 mmol) synthesized in Reference Example 1-2.

¹H NMR (CDCl₃, 400 MHz): δ = 1.6 - 1.7 (m, 1H), 1.8 - 1.9 (m, 1H), 2.0 - 2.1 (m, 1H), 2.1 - 2.2 (m, 1H), 2.3 - 2.4 (m, 1H), 2.7 - 2.8 (m, 1H), 3.2 - 3.3 (m, 1H), 3.4 - 3.5 (m, 1H), 3.5 - 3.6 (m, 1H), 3.6 - 3.7 (m, 1H), 4.7 - 4.8 (m, 1H), 6.18 (d, 1H, J = 6 Hz), 6.92 (s, 1H), 7.2 - 7.3 (m, 2H), 7.4 - 7.5 (m, 1H), 7.6 - 7.7 (m, 1H), 8.10 (d, 1H, J = 2 Hz), 8.20 (s, 1H).

MS: 417.11 [M+H]⁺

### Example 40

(R)-2-(4-morpholinophenyl)-N-(1-(4-(trifluoromethyl)thiazol-2-yl)pyrrolidin-3-yl) acetamide

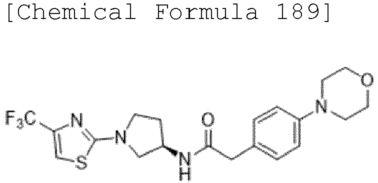

According to a technique similar to Example 1, the title compound (white powder, 60 mg, 46%) was obtained using (R)-1-(4-(trifluoromethyl)thiazol-2-yl)pyrrolidin-3-amine (70 mg, 0.30 mmol) synthesized in Reference Example 12-2 and 2-(4-morpholinophenyl)acetic acid (98 mg, 0.44 mmol) synthesized in Reference Example 23-2.

¹H NMR (CDCl₃, 400 MHz): δ = 1.8 - 1.9 (m, 1H), 2.3 - 2.4 (m, 1H), 3.1 - 3.2 (m, 4H), 3.23 (dd, 1H, J = 4, 10 Hz), 3.4 - 3.6 (m, 4H), 3.75 (dd, 1H, J = 6, 10 Hz), 3.8 - 3.9 (m, 4H), 4.5 - 4.7 (m, 1H), 5.48 (d, 1H, J = 7 Hz), 6.88 (d, 2H, J = 9 Hz), 6.92 (s, 1H), 7.12 (d, 2H, J = 8 Hz).

MS: 441.10 [M+H]⁺

### Example 41

Methyl 3-(2-(4-morpholinophenyl)acetamido)-1-(5-(trifluoromethyl)pyridin-3-yl) pyrrolidine-3-carboxylat e

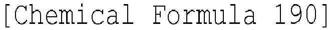

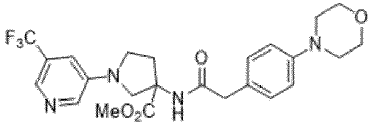

According to a technique similar to Example 7, methyl 3-amino-1-(5-(trifluoromethyl)pyridin-3-yl)pyrrolidine-3-carboxylate was synthesized from methyl 3-((tert-butoxycarbonyl)amino)-1-(5-(trifluoromethyl)pyridin-3-yl)pyrrolidine-3-carboxylate (39 mg, 0.10 mmol) synthesized in Reference Example 20-2, and the title compound (white powder, 21 mg, 43%) was obtained using 2-(4-morpholinophenyl)acetic acid (33 mg, 0.15 mmol) synthesized in Reference Example 23-2.

¹H NMR (CDCl₃, 400 MHz): δ = 2.3 - 2.4 (m, 1H), 2.5 - 2.6 (m, 1H), 3.1 - 3.2 (m, 4H), 3.4 - 3.5 (m, 2H), 3.53 (s, 2H)3.62 (d, 1H, J = 10 Hz), 3.76 (s, 3H), 3.8 - 3.9 (m, 4H), 4.00 (d, 1H, J = 10 Hz), 5.99 (s, 1H), 6.8 - 6.9 (m, 2H), 6.91 (dd, 1H, J = 2, 2 Hz), 7.1 - 7.2 (m, 2H), 8.07 (d, 1H, J = 3 Hz), 8.22 (s, 1H).

MS: 493.16 [M+H]⁺

### Example 42

(R)-2-(4-morpholinophenyl)-N-(1-(6-(trifluoromethyl)pyridin-3-yl)pyrrolidin-3-yl)acetamide

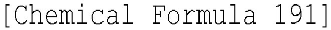

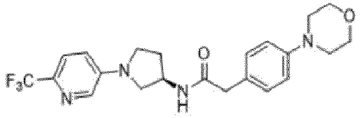

According to a technique similar to Example 1, the title compound (white powder, 59 mg, 45%) was obtained using (R)-1-(6-(trifluoromethyl)pyridin-3-yl)pyrrolidin-3-amine (70 mg, 0.30 mmol) synthesized in Reference Example 32-2 and 2-(4-morpholinophenyl)acetic acid (100 mg, 0.45 mmol) synthesized in Reference Example 23-2.

¹H NMR (CDCl₃, 400 MHz): δ = 1.8 - 1.9 (m, 1H), 2.3 - 2.4 (m, 1H), 3.1 - 3.2 (m, 5H), 3.3 - 3.4 (m, 2H), 3.51 (s, 2H), 3.66 (dd, 1H, J = 6, 10 Hz), 3.8 - 3.9 (m, 4H), 4.6 - 4.7 (m, 1H), 5.50 (d, 1H, J = 7 Hz), 6.79 (dd, 1H, J = 3, 9 Hz), 6.8 - 6.9 (m, 2H), 7.1 - 7.2 (m, 2H), 7.51 (d, 1H, J = 9 Hz), 7.97 (d, 1H, J = 9 Hz).

MS: 435.15 [M+H]⁺

### Example 43

2-(4-cyclopropylphenyl)-N-(3-ethyl-1-(5-(trifluoromethyl)pyridin-3-yl)pyrrolidin-3-yl)acetamide

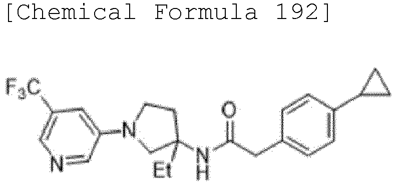

According to a technique similar to Example 38, 2-(4-cyclopropylphenyl)-N-(3-ethylpyrrolidin-3-yl)acetamide (yellow amorphous, 578 mg) was synthesized from tert-butyl 3-(2-(4-cyclopropylphenyl)acetamido)-3-ethylpyrrolidine-1-carboxylate (782 mg, 2.10 mmol) synthesized in Reference Example 24, and the title compound (orange-colored amorphous, 400 mg, 46%) was obtained using 3-bromo-5-(trifluoromethyl)pyridine (575 mg, 2.54 mmol).

¹H NMR (CDCl₃, 400 MHz): δ = 0.6 - 0.7 (m, 2H), 0.84 (t, 3H, J = 8 Hz), 0.9 - 1.0 (m, 2H), 1.8 - 1.9 (m, 2H), 1.9 - 2.0 (m, 1H), 2.0 - 2.1 (m, 1H), 2.2 - 2.3 (m, 1H), 3.2 - 3.4 (m, 3H), 3.49 (s, 2H), 3.65 (d, 1H, J = 10 Hz), 5.21 (s, 1H), 6.88 (dd, 1H, J = 2, 2 Hz), 6.9 - 7.0 (m, 2H), 7.0 - 7.1 (m, 2H), 8.05 (d, 1H, J = 3 Hz), 8.19 (s, 1H) .

MS: 418.17 [M+H]⁺

### Example 44

(1S,2S)-2-(6-fluorobenzo[d]oxazol-2-yl)-N-((R)-1-(5-(trifluoromethyl)pyridin-3-yl)pyrrolidin-3-yl)cyclopropane-1-carboxyamide

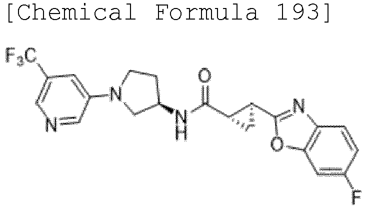

According to a technique similar to Example 26, (1S,2S)-2-(6-fluorobenzo[d]oxazol-2-yl)cyclopropane-1-carboxylic acid (white powder) was synthesized from methyl (1S,2S)-2-(6-fluorobenzo[d]oxazol-2-yl)cyclopropane-1-carboxylate (105 mg, 0.45 mmol) synthesized in Reference Example 22-4, and the title compound (white powder, 89 mg, 46%) was obtained using (R)-1-(5-(trifluoromethyl)pyridin-3-yl)pyrrolidin-3-amine (103 mg, 0.45 mmol) synthesized in Reference Example 1-2.

¹H NMR (CDCl₃, 400 MHz): δ = 1.6 - 1.7 (m, 1H), 1.7 - 1.8 (m, 1H), 2.0 - 2.1 (m, 1H), 2.1 - 2.2 (m, 1H), 2.3 - 2.4 (m, 1H), 2.7 - 2.8 (m, 1H), 3.27 (dd, 1H, J = 3, 10 Hz), 3.3 - 3.5 (m, 2H), 3.65 (dd, 1H, J = 6, 10 Hz), 4.7 - 4.8 (m, 1H), 6.71 (d, 1H, J = 7 Hz), 6.85 (dd, 1H, J = 2, 2 Hz), 7.0 - 7.1 (m, 1H), 7.16 (dd, 1H, J = 2, 8 Hz), 7.51 (dd, 1H, J = 5, 8 Hz), 8.04 (d, 1H, J = 3 Hz), 8.15 (d, 1H, J = 0.8 Hz).

MS: 435.10 [M+H]⁺

### Example 45

(1S,2S)-2-(5,6-difluorobenzo[d]oxazol-2-yl)-N-((R)-1-(5-(trifluoromethyl)pyridin-3-yl)pyrrolidin-3-yl)cyclopropane-1-carboxyamide

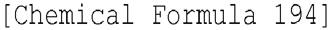

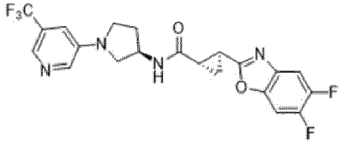

According to a technique similar to Example 26, (1S,2S)-2-(5,6-difluorobenzo[d]oxazol-2-yl)cyclopropane-1-carboxylic acid (white powder) was synthesized from methyl (1S,2S)-2-(5,6-difluorobenzo[d]oxazol-2-yl)cyclopropane-1-carboxylate (68 mg, 0.27 mmol) synthesized in Reference Example 26-2, and the title compound (white powder, 35 mg, 29%) was obtained using (R)-1-(5-(trifluoromethyl)pyridin-3-yl)pyrrolidin-3-amine (62 mg, 0.27 mmol) synthesized in Reference Example 1-2.

¹H NMR (CDCl₃, 400 MHz): δ = 1.6 - 1.7 (m, 1H), 1.7 - 1.8 (m, 1H), 2.0 - 2.1 (m, 1H), 2.1 - 2.2 (m, 1H), 2.3 - 2.4 (m, 1H), 2.7 - 2.8 (m, 1H), 3.29 (dd, 1H, J = 4, 10 Hz), 3.4 - 3.5 (m, 1H), 3.5 - 3.6 (m, 1H), 3.67 (dd, 1H, J = 6, 10 Hz), 4.7 - 4.8 (m, 1H), 6.25 (d, 1H, J = 7 Hz), 6.91 (dd, 1H, J = 2, 2 Hz), 7.30 (dd, 1H, J = 7, 9 Hz), 7.39 (dd, 1H, J = 8, 10 Hz), 8.10 (d, 1H, J = 3 Hz), 8.19 (d, 1H, J = 0.8 Hz).

MS: 453.09 [M+H]⁺

### Example 46

(R)-2-(4-(2-oxa-6-azaspiro[3.3]heptan-6-yl)phenyl)-N-(1-(5-(trifluoromethyl)pyridin-3-yl)pyrrolidin-3-yl)acetamide

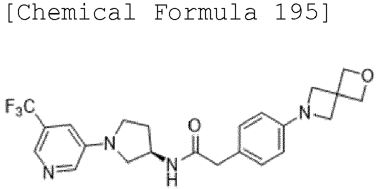

According to a technique similar to Example 26, 2-(4-(2-oxa-6-azaspiro[3.3]heptan-6-yl)phenyl)acetic acid (white powder, 1.02g) was synthesized from ethyl 2-(4-(2-oxa-6-azaspiro[3.3]heptan-6-yl)phenyl)acetate (1.26g, 4.82 mmol) synthesized in Reference Example 27, and the title compound (ivory-colored powder, 106 mg, 91%) was obtained using (R)-1-(5-(trifluoromethyl)pyridin-3-yl)pyrrolidin-3-amine (60 mg, 0.26 mmol) synthesized in Reference Example 1-2.

¹H NMR (CDCl₃, 400 MHz): δ = 1.8 - 1.9 (m, 1H), 2.3 - 2.4 (m, 1H), 3.09 (dd, 1H, J = 5, 10 Hz), 3.3 - 3.4 (m, 2H), 3.47 (s, 2H), 3.62 (dd, 1H, J = 6, 10 Hz), 4.00 (s, 4H), 4.6 - 4.7 (m, 1H), 4.83 (s, 4H), 5.73 (d, 1H, J = 7 Hz), 6.4 - 6.5 (m, 2H), 6.86 (dd, 1H, J = 3, 9 Hz), 7.0 - 7.1 (m, 2H), 8.04 (d, 1H, J = 3 Hz), 8.17 (d, 1H, J = 0.8 Hz).

MS: 447.19 [M+H]⁺

### Example 47

(1S,2S)-2-(benzo[d]oxazol-2-yl)-N-((R)-1-(4-(trifluoromethyl)thiazol-2-yl)pyrrolidin-3-yl) cyclopropane-1-carboxyamide

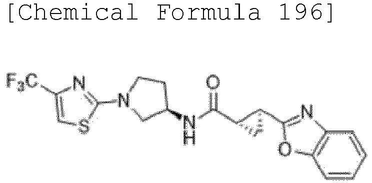

According to a technique similar to Example 26, (1S,2S)-2-(benzo[d]oxazol-2-yl)cyclopropane-1-carboxylic acid was synthesized from methyl (lS,2S)-2-(benzo[dJoxazol-2-yl)cyclopropane-1-carboxylate (110 mg, 0.51 mmol) synthesized in Reference Example 25-2, and the title compound (white powder, 90 mg, 42%) was obtained using (R)-1-(4-(trifluoromethyl)thiazol-2-yl)pyrrolidin-3-amine (120 mg, 0.51 mmol) synthesized in Reference Example 25-2.

¹H NMR (CDCl₃, 400 MHz): δ = 1.6 - 1.7 (m, 1H), 1.7 - 1.8 (m, 1H), 2.0 - 2.1 (m, 1H), 2.1 - 2.2 (m, 1H), 2.3 - 2.4 (m, 1H), 2.7 - 2.8 (m, 1H), 3.44 (dd, 1H, J = 4, 10 Hz), 3.5 - 3.7 (m, 2H), 3.80 (dd, 1H, J = 6, 10 Hz), 4.6 - 4.7 (m, 1H), 5.95 (d, 1H, J = 7 Hz), 6.95 (d, 1H, J = 0.8 Hz), 7.2 - 7.3 (m, 2H), 7.4 - 7.5 (m, 1H), 7.6 - 7.7 (m, 1H) .

MS: 423.07 [M+H]⁺

### Example 48

(1S,2S)-2-(6-fluorobenzo[d]oxazol-2-yl)-N-((R)-1-(6-(trifluoromethyl)pyridin-3-yl)pyrrolidin-3-yl)cyclopropane-1-carboxyamide

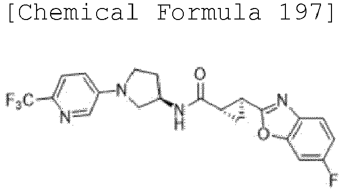

According to a technique similar to Example 26, (1S,2S)-2-(6-fluorobenzo[d]oxazol-2-yl)cyclopropane-1-carboxylic acid (ivory-colored powder) was synthesized from methyl (1S,2S)-2-(6-fluorobenzo[d]oxazol-2-yl)cyclopropane-1-carboxylate (121 mg, 0.51 mmol) synthesized in Reference Example 22-4, and the title compound (white powder, 118 mg, 53%) was obtained using (R)-1-(6-(trifluoromethyl)pyridin-3-yl)pyrrolidin-3-amine (119 mg, 0.51 mmol) synthesized in Reference Example 22-2.

¹H NMR (CDCl₃, 400 MHz): δ = 1.6 - 1.7 (m, 1H), 1.7 - 1.8 (m, 1H), 2.1 - 2.2 (m, 1H), 2.2 - 2.3 (m, 1H), 2.3 - 2.4 (m, 1H), 2.7 - 2.8 (m, 1H), 3.2 - 3.3 (m, 1H), 3.3 - 3.4 (m, 1H), 3.4 - 3.5 (m, 1H), 3.6 - 3.7 (m, 1H), 4.7 - 4.8 (m, 1H), 6.7 - 6.8 (m, 2H), 7.0 - 7.1 (m, 1H), 7.1 - 7.2 (m, 1H), 7.39 (d, 1H, J = 8 Hz), 7.50 (dd, 1H, J = 5, 8 Hz), 7.90 (s, 1H).

MS: 435.10 [M+H]⁺

### Example 49

(1S,2S)-2-(5-fluorobenzo[d]oxazol-2-yl)-N-((R)-1-(5-(trifluoromethyl)pyridin-3-yl)pyrrolidin-3-yl)cyclopropane-1-carboxyamide

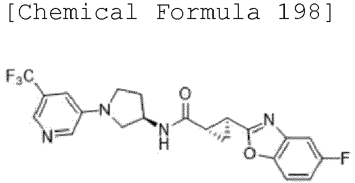

According to a technique similar to Example 26, (1S,2S)-2-(5-fluorobenzo[d]oxazol-2-yl)cyclopropane-1-carboxylic acid (ivory-colored powder) was synthesized from methyl (1S,2S)-2-(5-fluorobenzo[d]oxazol-2-yl)cyclopropane-1-carboxylate (47 mg, 0.20 mmol) synthesized in Reference Example 28-2, and the title compound (white powder, 51 mg, 59%) was obtained using (R)-1-(5-(trifluoromethyl)pyridin-3-yl)pyrrolidin-3-amine (66 mg, 0.29 mmol) synthesized in Reference Example 1-2.

¹H NMR (CDCl₃, 400 MHz): δ = 1.6 - 1.7 (m, 1H), 1.7 - 1.8 (m, 1H), 2.0 - 2.1 (m, 1H), 2.1 - 2.2 (m, 1H), 2.3 - 2.4 (m, 1H), 2.7 - 2.8 (m, 1H), 3.29 (dd, 1H, J = 4, 10 Hz), 3.4 - 3.5 (m, 1H), 3.5 - 3.6 (m, 1H), 3.69 (dd, 1H, J = 6, 10 Hz), 4.7 - 4.8 (m, 1H), 6.07 (d, 1H, J = 7 Hz), 6.9 - 7.0 (m, 1H), 7.0 - 7.1 (m, 1H), 7.29 (dd, 1H, J = 2, 8 Hz), 7.37 (dd, 1H, J = 4, 9 Hz), 8.12 (d, 1H, J = 3 Hz), 8.22 (s, 1H).

MS: 435.10 [M+H]⁺

### Example 50

(1R,2R)-2-(benzo[d]oxazol-2-yl)-N-((R)-1-(5-(trifluoromethyl)pyridin-3-yl)pyrrolidin-3-yl)cyclopropane-1-carboxyamide

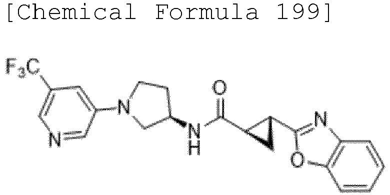

According to a technique similar to Example 26, trans-2-(benzo[d]oxazol-2-yl)cyclopropane-1-carboxylic acid (ivory-colored powder) was synthesized from ethyl trans-2-(benzo[d]oxazol-2-yl)cyclopropane-1-carboxylate (200 mg, 0.86 mmol) synthesized in Reference Example 29-3, and the title compound (white amorphous, 7.3 mg, 2.0%) was obtained using (R)-1-(5-(trifluoromethyl)pyridin-3-yl)pyrrolidin-3-amine (200 mg, 0.86 mmol) synthesized in Reference Example 1-2.

¹H NMR (CDCl₃, 400 MHz): δ = 1.6 - 1.7 (m, 1H), 1.7 - 1.8 (m, 1H), 2.0 - 2.1 (m, 1H), 2.1 - 2.2 (m, 1H), 2.3 - 2.4 (m, 1H), 2.7 - 2.8 (m, 1H), 3.27 (dd, 1H, J = 4, 10 Hz), 3.4 - 3.5 (m, 1H), 3.5 - 3.6 (m, 1H), 3.67 (dd, 1H, J = 6, 10 Hz), 4.7 - 4.8 (m, 1H), 6.07 (d, 1H, J = 8 Hz), 6.9 - 7.0 (m, 1H), 7.2 - 7.3 (m, 2H), 7.4 - 7.5 (m, 1H), 7.6 - 7.7 (m, 1H), 8.10 (d, 1H, J = 2 Hz), 8.20 (s, 1H).

MS: 417.09 [M+H]⁺

### Example 51

(1S,2S)-2-(benzo[d]oxazol-2-yl)-N-((R)-1-(5-(trifluoromethyl)pyridin-3-yl)pyrrolidin-3-yl)cyclopropane-1-carboxyamide

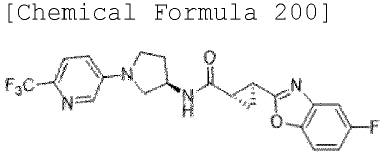

According to a technique similar to Example 26, (1S,2S)-2-(5-fluorobenzo[d]oxazol-2-yl)cyclopropane-1-carboxylic acid (ivory-colored powder) was synthesized from ethyl (1S,2S)-2-(5-fluorobenzo[d]oxazol-2-yl)cyclopropane-1-carboxylate (141 mg, 0.57 mmol) synthesized in Reference Example 30-3, and the title compound (white amorphous, 60 mg, 24%) was obtained using (R)-1-(6-(trifluoromethyl)pyridin-3-yl)pyrrolidin-3-amine (131 mg, 0.57 mmol) synthesized in Reference Example 32-2.

¹H NMR (CDCl₃, 400 MHz): δ = 1.6 - 1.7 (m, 1H), 1.7 - 1.8 (m, 1H), 2.0 - 2.1 (m, 1H), 2.1 - 2.2 (m, 1H), 2.3 - 2.4 (m, 1H), 2.7 - 2.8 (m, 1H), 3.30 (dd, 1H, J = 4, 10 Hz), 3.4 - 3.5 (m, 1H), 3.5 - 3.6 (m, 1H), 3.69 (dd, 1H, J = 6, 10 Hz), 4.7 - 4.8 (m, 1H), 6.20 (d, 1H, J = 7 Hz), 6.80 (dd, 1H, J = 2, 8 Hz), 7.0 - 7.1 (m, 1H), 7.2 - 7.3 (m, 1H), 7.36 (dd, 1H, J = 4,8 Hz), 7.46 (d, 1H, J = 8

Hz), 7.98 (d, 1H, J = 2 Hz).

MS: 435.10 [M+H]⁺

### Example 52

Trans-2-(1H-benzo[d]imidazol-2-yl)-N-((R)-1-(4-(trifluoromethyl)phenyl)pyrrolidin-3-yl)cyclopropane-1-carboxyamide

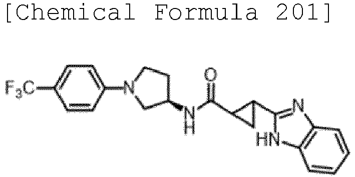

According to a technique similar to Example 3, a crude form of (R)-1-(4-(trifluoromethyl)phenyl)pyrrolidin-3-amine (317 mg) was synthesized from tert-butyl (R)-(1-(4-(trifluoromethyl)phenyl)pyrrolidin-3-yl)carbamate (273 mg, 0.83 mmol) synthesized in Reference Example 34, and diastereomer A of the title compound (Rf value in TLC (ethyl acetate) = 0.4, pale yellow amorphous, 16 mg, 19%) was obtained using trans-2-(1H-benzo[d]imidazol-2-yl)cyclopropane-1-carboxylic acid (40 mg, 0.20 mmol).

### Diastereomer A

¹H NMR (DMSO - d₆, 400 MHz): δ = 1.4 - 1.5 (m, 2H), 1.8 - 2.0 (m, 1H), 2.1 - 2.3 (m, 2H), 2.4 - 2.5 (m, 1H), 3.15 (dd, 1H, J = 4, 10 Hz), 3.3 - 3.5 (m, 2H), 3.55 (dd, 1H, J = 6, 10 Hz), 4.3 - 4.5 (m, 1H), 6.63 (d, 2H, J = 9 Hz), 7.0 - 7.2 (m, 2H), 7.3 - 7.5 (m, 2H), 7.46 (d, 2H, J = 9 Hz), 8.64 (d, 1H, J = 6 Hz), 12.38 (s, 1H).

MS: 415.16 [M+H]⁺

### Example 53

Trans-2-(1H-benzo[d]imidazol-2-yl)-N-((S)-1-(4-(trifluoromethyl)phenyl)pyrrolidin-3-yl)cyclopropane-1-carboxyamide

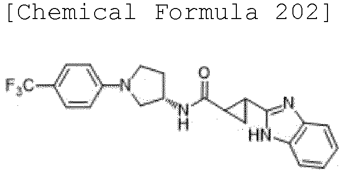

According to a technique similar to Example 3, a crude form of (S)-1-(4-(trifluoromethyl)phenyl)pyrrolidin-3-amine (170 mg) was synthesized from tert-butyl (S)-(1-(4-(trifluoromethyl)phenyl)pyrrolidin-3-yl)carbamate (236 mg, 0.72 mmol) synthesized in Reference Example 35, and diastereomer A of the title compound (Rf value in TLC (ethyl acetate) = 0.5, white powder, 14 mg, 17%) was obtained using trans-2-(1H-benzo[d]imidazol-2-yl)cyclopropane-1-carboxylic acid (40 mg, 0.20 mmol).

### Diastereomer A

¹H NMR (DMSO - d₆, 400 MHz): δ = 1.4 - 1.5 (m, 2H), 1.8 - 2.0 (m, 1H), 2.1 - 2.3 (m, 2H), 2.4 - 2.5 (m, 1H), 3.15 (dd, 1H, J = 4, 10 Hz), 3.3 - 3.6 (m, 3H), 4.3 - 4.5 (m, 1H), 6.63 (d, 2H, J = 9 Hz), 7.0 - 7.2 (m, 2H), 7.3 - 7.5 (m, 2H), 7.46 (d, 2H, J = 9 Hz), 8.64 (d, 1H, J = 7 Hz), 12.39 (s, 1H).

MS: 415.18 [M+H]⁺

### Example 54

(1S,2S)-2-(1H-benzo[d]imidazol-2-yl)-N-((R)-1-(6-(trifluoromethyl)pyridin-3-yl)pyrrolidin-3-yl)cyclopropane-1-carboxyamide

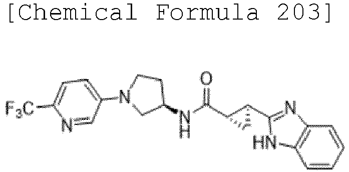

According to a technique similar to Example 3, a crude form of (R)-1-(6-(trifluoromethyl)pyridin-3-yl)pyrrolidin-3-amine (300 mg) was synthesized from tert-butyl (R)-(1-(6-(trifluoromethyl)pyridin-3-yl)pyrrolidin-3-yl)carbamate (429 mg, 1.30 mmol) synthesized in Reference Example 36, and the title compound (white powder, 32 mg, 48%) was obtained using (1S,2S)-2-(1H-benzo[d]imidazol-2-yl)cyclopropane-1-carboxylic acid (32 mg, 0.16 mmol).

¹H NMR (DMSO - d₆, 400 MHz): δ = 1.4 - 1.5 (m, 2H), 1.9 - 2.0 (m, 1H), 2.1 - 2.3 (m, 2H), 2.4 - 2.5 (m, 1H), 3.21 (dd, 1H, J = 4, 10 Hz), 3.3 - 3.5 (m, 2H), 3.60 (dd, 1H, J = 6, 10 Hz), 4.4 - 4.5 (m, 1H), 7.01 (d, 1H, J = 3, 9 Hz), 7.0 - 7.2 (m, 2H), 7.3 - 7.5 (m, 2H), 7.59 (d, 1H, J = 9 Hz), 8.04 (d, 1H, J = 3 Hz), 8.65 (d, 1H, J = 7 Hz), 12.41 (s, 1H).

MS: 416.18 [M+H]⁺

### Example 55

(1S,2S)-2-(1H-benzo[d]imidazol-2-yl)-N-((S)-1-(6-(trifluoromethyl)pyridin-3-yl)pyrrolidin-3-yl)cyclopropane-1-carboxyamide

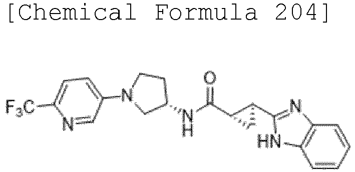

According to a technique similar to Example 3, a crude form of (S)-1-(6-(trifluoromethyl)pyridin-3-yl)pyrrolidin-3-amine (37 mg) was synthesized from tert-butyl (S)-(1-(6-(trifluoromethyl)pyridin-3-yl)pyrrolidin-3-yl)carbamate (59 mg, 0.18 mmol) synthesized in Reference Example 37, and the title compound (white powder, 48 mg, 73%) was obtained using (1S,2S)-2-(1H-benzo[d]imidazol-2-yl)cyclopropane-1-carboxylic acid (32 mg, 0.16 mmol).

¹H NMR (DMSO - d₆, 400 MHz): δ = 1.4 - 1.5 (m, 2H), 1.9 - 2.0 (m, 1H), 2.1 - 2.3 (m, 2H), 2.4 - 2.5 (m, 1H), 3.18 (dd, 1H, J = 4, 10 Hz), 3.3 - 3.6 (m, 2H), 3.60 (dd, 1H, J = 6, 10 Hz), 4.4 - 4.6 (m, 1H), 7.00 (d, 1H, J = 3, 9 Hz), 7.0 - 7.2 (m, 2H), 7.3 - 7.5 (m, 2H), 7.58 (d, 1H, J = 9 Hz), 8.02 (d, 1H, J = 3 Hz), 8.64 (d, 1H, J = 7 Hz), 12.34 (s, 1H).

MS: 416.18 [M+H]⁺

### Example 56

(1S,2S)-2-(1H-benzo[d]imidazol-2-yl)-N-((R)-1-(5-(trifluoromethyl)pyrimidin-2-yl)pyrrolidin-3-yl)cyclopropane-1-carboxyamide

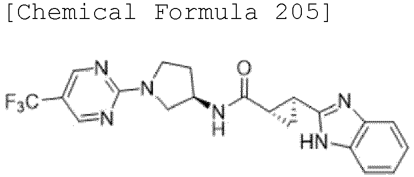

According to a technique similar to Example 1, the title compound (white powder, 28 mg, 68%) was obtained using (R)-1-(5-(trifluoromethyl)pyrimidin-2-yl)pyrrolidin-3-amine (45 mg, 0.20 mmol) synthesized in Reference Example 38-2 and (1S,2S)-2-(1H-benzo[d]imidazol-2-yl)cyclopropane-1-carboxylic acid (20 mg, 0.10 mmol).

¹H NMR (DMSO - d₆, 400 MHz): δ = 1.4 - 1.5 (m, 2H), 1.8 - 2.0 (m, 1H), 2.1 - 2.3 (m, 2H), 2.4 - 2.5 (m, 1H), 3.49 (dd, 1H, J = 4, 8 Hz), 3.6 - 3.7 (m, 2H), 3.75 (dd, 1H, J = 6, 12 Hz), 4.3 - 4.5 (m, 1H), 7.0 - 7.2 (m, 2H), 7.3 - 7.5 (m, 2H), 8.64 (d, 1H, J = 6 Hz), 8.71 (s, 2H), 12.39 (s, 1H).

MS: 417.20 [M+H]⁺

### Example 57

(1S,2S)-2-(1H-benzo[d]imidazol-2-yl)-N-((R)-1-(4-fluoro-3-(trifluoromethyl)phenyl)pyrrolidin-3-yl)cyclopropane-1-carboxyamide

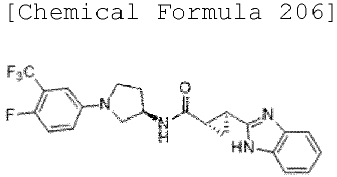

According to a technique similar to Example 3, a crude form of (R)-1-(4-fluoro-3-(trifluoromethyl)phenyl)pyrrolidin-3-amine (80 mg) was synthesized from tert-butyl (R)-(1-(4-fluoro-3-(trifluoromethyl)phenyl)pyrrolidin-3-yl)carbamate (112 mg, 0.32 mmol) synthesized in Reference Example 39, and the title compound (white powder, 35 mg, 77%) was obtained using (1S,2S)-2-(1H-benzo[d]imidazol-2-yl)cyclopropane-1-carboxylic acid (21 mg, 0.11 mmol).

¹H NMR (CDCl₃, 400 MHz): δ = 1.6 - 1.8 (m, 2H), 1.9 - 2.1 (m, 1H), 2.2 - 2.4 (m, 2H), 2.6 - 2.7 (m, 1H), 3.23 (dd, 1H, J = 4, 10 Hz), 3.30 (dt, 1H, J = 5, 9 Hz), 3.4 - 3.5 (m, 1H), 3.59 (dd, 1H, J = 6, 10 Hz), 4.6 - 4.7 (m, 1H), 6.51 (d, 1H, J = 7 Hz), 6.6 - 6.7 (m, 2H), 7.05 (t, 1H, J = 9 Hz), 7.2 - 7.3 (m, 2H), 7.4 - 7.5 (m, 2H). The 1H content is not observable.

MS: 433.18 [M+H]⁺

### Example 58

(1S,2S)-N-((R)-1-(4-fluoro-3-(trifluoromethyl)phenyl)pyrrolidin-3-yl)-2-(quinazolin-2-yl)cyclopropane-1-carboxyamide

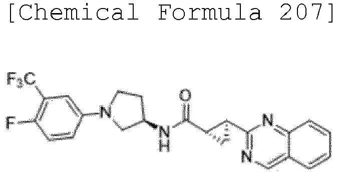

According to a technique similar to Example 3, a crude form of (R)-1-(4-fluoro-3-(trifluoromethyl)phenyl)pyrrolidin-3-amine (80 mg) was synthesized from tert-butyl (R)-(1-(4-fluoro-3-(trifluoromethyl)phenyl)pyrrolidin-3-yl)carbamate (112 mg, 0.32 mmol) synthesized in Reference Example 39, and the title compound (white powder, 23 mg, 56%) was obtained using (1S,2S)-2-(quinazolin-2-yl)cyclopropane-1-carboxylic acid (20 mg, 0.09 mmol) synthesized in Reference Example 40-3.

¹H NMR (CDCl₃, 400 MHz): δ = 1.69 (ddd, 1H, J = 4, 6, 8 Hz), 1.7 - 1.8 (m, 1H), 1.9 - 2.1 (m, 1H), 2.1 - 2.4 (m, 2H), 2.9 - 3.0 (m, 1H), 3.22 (dd, 1H, J = 4, 10 Hz), 3.31 (dt, 1H, J = 5, 9 Hz), 3.4 - 3.5 (m, 1H), 3.59 (dd, 1H, J = 6, 10 Hz), 4.6 - 4.8 (m, 1H), 5.88 (d, 1H, J = 7 Hz), 6.6 - 6.7 (m, 2H), 7.06 (t, 1H, J = 9 Hz), 7.5 - 7.6 (m, 1H), 7.8 - 8.0 (m, 3H), 9.25 (s, 1H).

MS: 445.19 [M+H]⁺

### Example 59

(1S,2S)-2-(1H-benzo[d]imidazol-2-yl)-N-((R)-1-(3-(methylsulfonyl)phenyl)pyrrolidin-3-yl)cyclopropane-1-carboxyamide

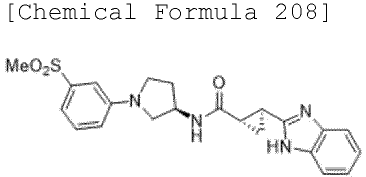

According to a technique similar to Example 1, the title compound (pale yellow powder, 7 mg, 65%) was obtained using (R)-1-(3-(methylsulfonyl)phenyl)pyrrolidin-3-amine (6 mg, 0.02 mmol) synthesized in Reference Example 41-2 and (1S,2S)-2-(1H-benzo[d]imidazol-2-yl)cyclopropane-1-carboxylic acid (5 mg, 0.02 mmol).

¹H NMR (CDCl₃, 400 MHz): δ = 1.5 - 1.8 (m, 2H), 2.0 - 2.1 (m, 1H), 2.2 - 2.4 (m, 2H), 2.6 - 2.7 (m, 1H), 3.04 (s, 3H), 3.27 (dd, 1H, J = 4, 10 Hz), 3.3 - 3.5 (m, 2H), 3.62 (dd, 1H, J = 6, 10 Hz), 4.5 - 4.7 (m, 1H), 6.53 (d, 1H, J = 7 Hz), 6.72 (dd, 1H, J = 2, 8 Hz), 7.00 (t, 1H, J = 2 Hz), 7.1 - 7.3 (m, 3H), 7.36 (t, 1H, J = 8 Hz), 7.4 - 7.6 (m, 2H). The 1H content is not observable.

MS: 425.19 [M+H]⁺

### Example 60

(1S,2S)-2-(1H-benzo[d]imidazol-2-yl)-N-((R)-1-(3-cyanophenyl)pyrrolidin-3-yl)cyclopropane-1-carboxyamide

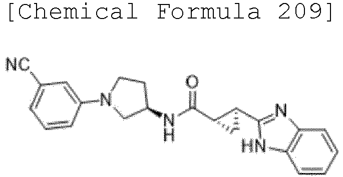

According to a technique similar to Example 1, the title compound (pale yellow powder, 9 mg, 96%) was obtained using (R)-3-(3-aminopyrrolidin-1-yl)benzonitrile (5 mg, 0.02 mmol) synthesized in Reference Example 42-2 and (1S,2S)-2-(1H-benzo[d]imidazol-2-yl)cyclopropane-1-carboxylic acid (5 mg, 0.02 mmol).

¹H NMR (CDCl₃, 400 MHz): δ = 1.6 - 1.8 (m, 2H), 1.9 - 2.1 (m, 1H), 2.2 - 2.4 (m, 2H), 2.6 - 2.8 (m, 1H), 3.2 - 3.5 (m, 2H), 3.22 (dd, 1H, J = 4, 10 Hz), 3.60 (dd, 1H, J = 6, 10 Hz), 4.5 - 4.7 (m, 1H), 6.61 (d, 1H, J = 7 Hz), 6.7 - 6.8 (m, 2H), 6.96 (d, 1H, J = 7 Hz), 7.1 - 7.3 (m, 3H), 7.4 - 7.6 (m, 2H). The 1H content is not observable.

MS: 372.21 [M+H]⁺

### Example 61

Trans-2-(5-cyano-1H-benzo[d]imidazol-2-yl)-N-((R)-1-(6-(trifluoromethyl)pyridin-3-yl)pyrrolidin-3-yl)cyclopropane-1-carboxyamide

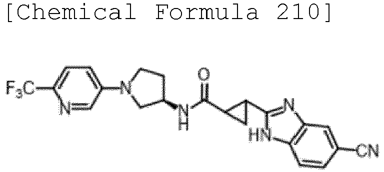

According to a technique similar to Example 1, diastereomer A of the title compound (upper spot in TLC (methanol/ethyl acetate = 1/9), white powder, 22 mg, 37%) and diastereomer B of the title compound (lower spot in TLC (methanol/ethyl acetate = 1/9), white powder, 19 mg, 32%) were obtained using (R)-1-(6-(trifluoromethyl)pyridin-3-yl)pyrrolidin-3-amine (31 mg, 0.14 mmol) synthesized in Reference Example 1-2 and trans-2-(5-cyano-1H-benzo[d]imidazol-2-yl)cyclopropane-l-carboxylic acid (31 mg, 0.14 mmol) synthesized in Reference Example 43-3.

### Diastereomer A

¹H NMR (CDCl₃, 400 MHz): δ = 1.6 - 1.8 (m, 2H), 2.0 - 2.1 (m, 1H), 2.3 - 2.5 (m, 2H), 2.6 - 2.7 (m, 1H), 3.31 (dd, 1H, J = 4, 10 Hz), 3.4 - 3.6 (m, 2H), 3.72 (dd, 1H, J = 6, 10 Hz), 4.6 - 4.8 (m, 1H), 6.16 (d, 1H, J = 7 Hz), 6.85 (dd, 1H, J = 3, 9 Hz), 7.4 - 7.6 (m, 3H), 7.8 - 7.9 (m, 1H), 8.03 (d, 1H, J = 3 Hz), 9.78 (br s, 1H).

MS: 439.28 [M-H]⁻

### Diastereomer B

¹H NMR (CDCl₃, 400 MHz): δ = 1.7 - 1.9 (m, 2H), 2.0 - 2.5 (m, 3H), 2.6 - 2.7 (m, 1H), 3.23 (dd, 1H, J = 4, 10 Hz), 3.3 - 3.6 (m, 2H), 3.58 (dd, 1H, J = 6, 10 Hz), 4.6 - 4.7 (m, 1H), 6.35 (d, 1H, J = 7 Hz), 6.77 (dd, 1H, J = 3, 9 Hz), 7.4 - 8.0 (m, 4H), 7.94 (d, 1H, J = 3 Hz), 10.05 (br s, 1H).

MS: 439.28 [M-H]⁻

### Example 62

(1S,2S)-2-(1H-benzo[d]imidazol-2-yl)-N-((R)-1-(2-(trifluoromethyl)pyridin-4-yl)pyrrolidin-3-yl)cyclopropane-1-carboxyamide

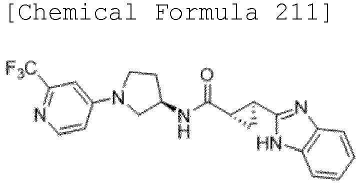

According to a technique similar to Example 3, a crude form of (R)-1-(2-(trifluoromethyl)pyridin-4-yl)pyrrolidin-3-amine (30 mg) was synthesized from tert-butyl (R)-(1-(2-(trifluoromethyl)pyridin-4-yl)pyrrolidin-3-yl)carbamate synthesized in Reference Example 44, and the title compound (white powder, 31 mg, 76%) was obtained using (1S,2S)-2-(1H-benzo[d]imidazol-2-yl)cyclopropane-1-carboxylic acid (20 mg, 0.10 mmol).

¹H NMR (CDCl₃, 400 MHz): δ = 1.6 - 1.8 (m, 2H), 2.0 - 2.2 (m, 1H), 2.2 - 2.5 (m, 2H), 2.5 - 2.7 (m, 1H), 3.30 (dd, 1H, J = 4, 10 Hz), 3.4 - 3.6 (m, 2H), 3.73 (dd, 1H, J = 6, 11 Hz), 4.6 - 4.7 (m, 1H), 6.19 (d, 1H, J = 7 Hz), 6.49 (dd, 1H, J = 2, 9 Hz), 6.74 (d, 1H, J = 2 Hz), 7.2 - 7.3 (m, 2H), 7.4 - 7.5 (m, 2H), 8.31 (d, 1H, J = 6 Hz). The 1H is not observable.

MS: 416.17 [M+H]⁺

### Example 63

Trans-2-(1H-benzo[d]imidazol-2-yl)-N-((R)-1-(6-(trifluoromethyl)pyridin-3-yl)piperidin-3-yl)cyclopropane-1-carboxyamide

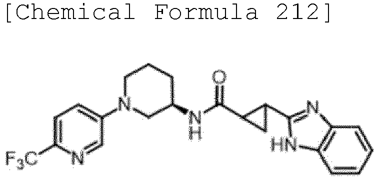

According to a technique similar to Example 1, diastereomer A of the title compound (upper spot in TLC (ethyl acetate), white powder, 14 mg, 16%) and diastereomer B of the title compound (lower spot in TLC (ethyl acetate), white powder, 5 mg, 6%) were obtained using (R)-1-(6-(trifluoromethyl)pyridin-3-yl)piperidin-3-amine (50 mg, 0.20 mmol) synthesized in Reference Example 45-2 and trans-2-(1H-benzo[d]imidazol-2-yl)cyclopropane-1-carboxylic acid (41 mg, 0.20 mmol).

### Diastereomer A

¹H NMR (CDCl₃, 400 MHz): δ = 1.6 - 2.0 (m, 6H), 2.2 - 2.4 (m, 1H), 2.5 - 2.7 (m, 1H), 3.08 (dd, 1H, J = 7, 12 Hz), 3.1 - 3.4 (m, 2H), 3.55 (dd, 1H, J = 3, 12 Hz), 4.1 - 4.3 (m, 1H), 6.14 (d, 1H, J = 8 Hz), 7.2 - 7.3 (m, 2H), 7.3 - 7.4 (m, 1H), 7.4 - 7.6 (m, 2H), 8.35 (s, 1H), 8.49 (d, 1H, J = 3 Hz). The 1H content is not observable.

MS: 430.17 [M+H]⁺

### Diastereomer B

¹H NMR (CDCl₃, 400 MHz): δ = 1.6 - 2.0 (m, 6H), 2.2 - 2.3 (m, 1H), 2.6 - 2.7 (m, 1H), 3.1 - 3.3 (m, 4H), 4.1 - 4.3 (m, 1H), 6.46 (d, 1H, J = 7 Hz), 7.1 - 7.3 (m, 3H), 7.4 - 7.6 (m, 2H), 8.23 (s, 1H), 8.28 (d, 1H, J = 3 Hz). The 1H content is not observable.

MS: 430.18 [M+H]⁺

### Example 64

(1S,2S)-2-(1H-benzo[d]imidazol-2-yl)-N-((R)-1-(4-(trifluoromethyl)thiazol-2-yl)pyrrolidin-3-yl)cyclopropane-1-carboxyamide

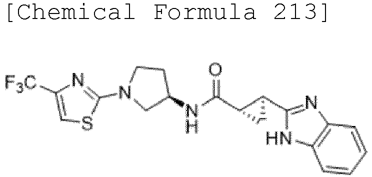

According to a technique similar to Example 1, the title compound (white powder, 20 mg, 47%) was obtained using (R)-1-(4-(trifluoromethyl)thiazol-2-yl)pyrrolidin-3-amine (24 mg, 0.10 mmol) synthesized in Reference Example 46-3 and (1S,2S)-2-(1H-benzo[d]imidazol-2-yl)cyclopropane-1-carboxylic acid (20 mg, 0.10 mmol).

¹H NMR (CDCl₃, 400 MHz): δ = 1.5 - 1.8 (m, 2H), 2.0 - 2.1 (m, 1H), 2.2 - 2.4 (m, 2H), 2.5 - 2.7 (m, 1H), 3.43 (dd, 1H, J = 4, 10 Hz), 3.5 - 3.7 (m, 2H), 3.80 (dd, 1H, J = 6, 11 Hz), 4.6 - 4.7 (m, 1H), 6.29 (d, 1H, J = 7 Hz), 6.94 (s, 1H), 7.2 - 7.3 (m, 2H), 7.4 - 7.6 (m, 2H). The 1H content is not observable.

MS: 422.13 [M+H]⁺

### Example 65

(1S,2S)-2-(1H-benzo[d]imidazol-2-yl)-N-((R)-1-(4-(trifluoromethyl)phenyl)piperidin-3-yl)cyclopropane-1-carboxyamide

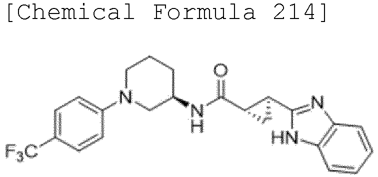

According to a technique similar to Example 1, the title compound (white powder, 30 mg, 70%) was obtained using (R)-1-(4-(trifluoromethyl)phenyl)piperidin-3-amine (22 mg, 0.10 mmol) synthesized in Reference Example 47-2 and (1S,2S)-2-(1H-benzo[d]imidazol-2-yl)cyclopropane-1-carboxylic acid (20 mg, 0.10 mmol).

¹H NMR (CDCl₃, 400 MHz): δ = 1.6 - 1.9 (m, 6H), 2.2 - 2.4 (m, 1H), 2.6 - 2.7 (m, 1H), 3.1 - 3.4 (m, 2H), 3.10 (dd, 1H, J = 7, 12 Hz), 3.57 (dd, 1H, J = 3, 12 Hz), 4.1 - 4.3 (m, 1H), 6.16 (d, 1H, J = 8 Hz), 6.97 (d, 2H, J = 9 Hz), 7.1 - 7.3 (m, 2H), 7.4 - 7.6 (m, 2H), 7.49 (d, 2H, J = 9 Hz), 9.60 (br s, 1H).

MS: 429.19 [M+H]⁺

### Example 66

Trans-2-(1H-indol-3-yl)-N-((R)-1-(6-(trifluoromethyl)pyridin-3-yl)pyrrolidin-3-yl)cyclopropane-1-carboxyamide

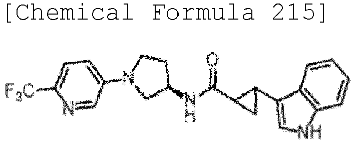

According to a technique similar to Example 3, diastereomer A of the title compound (Rf value in TLC (ethyl acetate) = 0.9, pale yellow powder, 6 mg, 19%) was obtained using (R)-1-(6-(trifluoromethyl)pyridin-3-yl)pyrrolidin-3-amine (17 mg, 0.07 mmol) synthesized in Reference Example 1-2 and trans-2-(1H-indol-3-yl)cyclopropane-1-carboxylic acid (15 mg, 0.07 mmol).

### Diastereomer A

¹H NMR (CDCl₃, 400 MHz): δ = 1.3 - 1.4 (m, 1H), 1.5 - 1.7 (m, 2H), 2.0 - 2.2 (m, 1H), 2.3 - 2.5 (m, 1H), 2.6 - 2.7 (m, 1H), 3.28 (dd, 1H, J = 4, 10 Hz), 3.4 - 3.6 (m, 2H), 3.72 (dd, 1H, J = 6, 10 Hz), 4.7 - 4.8 (m, 1H), 5.85 (d, 1H, J = 7 Hz), 6.84 (dd, 1H, J = 3, 9 Hz), 6.94 (dd, 1H, J = 1, 2 Hz), 7.14 (dt, 1H, J = 1, 7 Hz), 7.22 (dt, 1H, J = 1, 7 Hz), 7.36 (d, 1H, J = 8 Hz), 7.49 (d, 1H, J = 9 Hz), 7.65 (d, 1H, J = 8 Hz), 7.97 (br s, 1H), 8.02 (d, 1H, J = 3 Hz).

MS: 415.18 [M+H]⁺

### Example 67

(1S,2S)-2-(1H-benzo[d]imidazol-2-yl)-N-((R)-1-(6-methoxy-5-(trifluoromethyl)pyridin-3-yl)pyrrolidin-3-yl)cyclopropane-1-carboxyamide

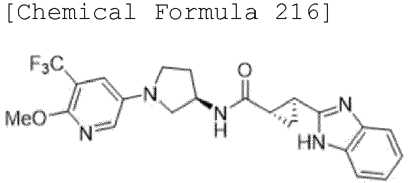

According to a technique similar to Example 1, the title compound (white powder, 21 mg, 65%) was obtained using (R)-1-(6-methoxy-5-(trifluoromethyl)pyridin-3-yl)pyrrolidin-3-amine (18 mg, 0.07 mmol) synthesized in Reference Example 48-2 and (1S,2S)-2-(1H-benzo[d]imidazol-2-yl)cyclopropane-1-carboxylic acid (14 mg, 0.07 mmol).

¹H NMR (CDCl₃, 400 MHz): δ = 1.6 - 1.8 (m, 2H), 1.9 - 2.1 (m, 1H), 2.2 - 2.4 (m, 2H), 2.6 - 2.7 (m, 1H), 3.2 - 3.5 (m, 3H), 3.57 (dd, 1H, J = 6, 10 Hz), 3.96 (s, 3H), 4.6 - 4.8 (m, 1H), 6.1 - 6.3 (m, 1H), 7.14 (d, 1H, J = 3 Hz), 7.2 - 7.3 (m, 2H), 7.3 - 7.7 (m, 2H), 7.65 (d, 1H, J = 3 Hz), 9.39 (br s, 1H).

MS: 444.17 [M-H]⁻

### Example 68

(1S,2S)-2-(1H-benzo[d]imidazol-2-yl)-N-((R)-1-(4-(tert-butyl)thiazol-2-yl)pyrrolidin-3-yl)cyclopropane-1-carboxyamide

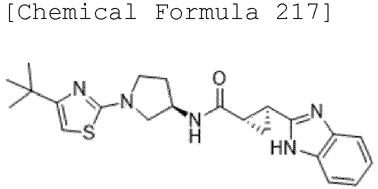

According to a technique similar to Example 1, the title compound (white powder, 23 mg, 57%) was obtained using (R)-1-(4-(tert-butyl)thiazol-2-yl)pyrrolidin-3-amine (22 mg, 0.10 mmol) synthesized in Reference Example 49-2 and (1S,2S)-2-(1H-benzo[d]imidazol-2-yl)cyclopropane-1-carboxylic acid (20 mg, 0.10 mmol).

¹H NMR (CDCl₃, 400 MHz): δ = 1.27 (s, 9H), 1.6 - 1.8 (m, 2H), 2.0 - 2.2 (m, 1H), 2.2 - 2.5 (m, 2H), 2.5 - 2.7 (m, 1H), 3.4 - 3.6 (m, 2H), 3.41 (dd, 1H, J = 4, 10 Hz), 3.77 (dd, 1H, J = 6, 11 Hz), 4.5 - 4.7 (m, 1H), 6.09 (s, 1H), 6.1 - 6.3 (m, 1H), 7.1 - 7.3 (m, 2H), 7.3 - 7.7 (m, 2H), 9.68 (br s, 1H).

MS: 410.21 [M+H]⁺

### Example 69

(1S,2S)-2-(1H-benzo[d]imidazol-2-yl)-N-((R)-1-(6-(trifluoromethyl)pyridazin-3-yl)pyrrolidin-3-yl)cyclopropane-1-carboxyamide

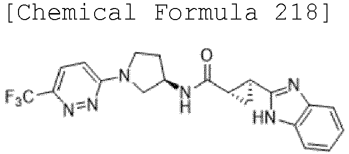

According to a technique similar to Example 3, a crude form of (R)-1-(6-(trifluoromethyl)pyridazin-3-yl)pyrrolidin-3-amine (91 mg) was synthesized from tert-butyl (R)-(1-(6-(trifluoromethyl)pyridazin-3-yl)pyrrolidin-3-yl)carbamate (90 mg, 0.271 mmol) synthesized in Reference Example 50, and the title compound (white powder, 67 mg, 60%) was obtained using (1S,2S)-2-(1H-benzo[d]imidazol-2-yl)cyclopropane-1-carboxylic acid (110 mg, 0.542 mmol).

¹H NMR (DMSO - d₆, 400 MHz): δ = 1.4 - 1.5 (m, 2H), 1.9 - 2.0 (m, 1H), 2.1 - 2.3 (m, 2H), 2.4 - 2.5 (m, 1H), 3.3 - 3.4 (m, 2H), 3.5 - 3.8 (m, 2H), 4.4 - 4.5 (m, 1H), 7.04 (d, 1H, J = 10 Hz), 7.10 (dd, 2H, J = 2, 6 Hz), 7.44 (br s, 2H), 7.77 (d, 1H, J = 10 Hz), 8.66 (d, 1H, J = 7 Hz), 12.4 (br s, 1H).

MS: 417.20 [M+H]⁺

### Example 70

(1S,2S)-2-(1H-benzo[d]imidazol-2-yl)-N-((R)-1-(2-(trifluoromethyl)pyrimidin-5-yl)pyrrolidin-3-yl)cyclopropane-1-carboxyamide

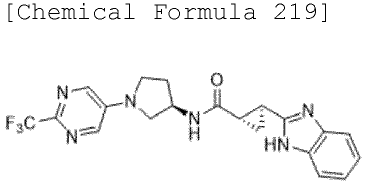

According to a technique similar to Example 3, a crude form of (R)-1-(2-(trifluoromethyl)pyrimidin-5-yl)pyrrolidin-3-amine (101 mg) was synthesized from tert-butyl (R)-(1-(2-(trifluoromethyl)pyrimidin-5-yl)pyrrolidin-3-yl)carbamate (129 mg, 0.388 mmol) synthesized in Reference Example 51, and the title compound (white powder, 145 mg, 90%) was obtained using (1S,2S)-2-(1H-benzo[d]imidazol-2-yl)cyclopropane-1-carboxylic acid (157 mg, 0.776 mmol).

¹H NMR (DMSO - d₆, 400 MHz): δ = 1.4 - 1.5 (m, 2H), 1.9 - 2.0 (m, 1H), 2.1 - 2.3 (m, 2H), 2.4 - 2.5 (m, 1H), 3.26 (dd, 1H, J = 4, 11 Hz), 3.3 - 3.5 (m, 2H), 3.64 (dd, 1H, J = 6, 11 Hz), 4.4 - 4.5 (m, 1H), 7.0 - 7.2 (m, 2H), 7.3 - 7.5 (m, 2H), 8.25 (s, 2H), 8.64 (d, 1H, J = 7 Hz), 12.4 (br s, 1H).

MS: 415.27 [M-H]⁻

### Example 71

(1S,2S)-2-(1H-benzo[d]imidazol-2-yl)-N-((R)-1-(5-(trifluoromethyl)pyridin-3-yl)pyrrolidin-3-yl)cyclopropane-1-carboxyamide

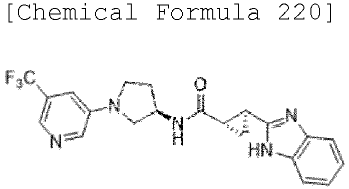

According to a technique similar to Example 3, a crude form of (R)-1-(5-(trifluoromethyl)pyridin-3-yl)pyrrolidin-3-amine (79 mg) was synthesized from tert-butyl (R)-(1-(5-(trifluoromethyl)pyridin-3-yl)pyrrolidin-3-yl)carbamate (102 mg, 0.308 mmol) synthesized in Reference Example 1-1, and the title compound (white powder, 73 mg, 57%) was obtained using (1S,2S)-2-(1H-benzo[d]imidazol-2-yl)cyclopropane-1-carboxylic acid (125 mg, 0.616 mmol).

¹H NMR (CDCl₃, 400 MHz): δ = 1.5 - 1.8 (m, 3H), 2.0 - 2.1 (m, 1H), 2.3 - 2.4 (m, 2H), 2.6 - 2.7 (m, 1H), 3.28 (dd, 1H, J = 4, 10 Hz), 3.3 - 3.6 (m, 2H), 3.67 (dd, 1H, J = 6, 10 Hz), 4.6 - 4.8 (m, 1H), 6.66 (d, 1H, J = 7 Hz), 6.92 (s, 1H), 7.1 - 7.3 (m, 2H), 7.49 (br s, 2H), 8.09 (d, 1H, J = 3 Hz), 8.20 (s, 1H).

MS: 416.18 [M+H]⁺

### Example 72

(1S,2S)-2-(1H-benzo[d]imidazol-2-yl)-N-((R)-1-(6-fluorobenzo[d]thiazol-2-yl)pyrrolidin-3-yl)cyclopropane-1-carboxyamide

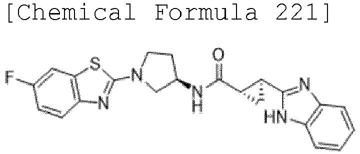

According to a technique similar to Example 3, a crude form of (R)-1-(6-fluorobenzo[d]thiazol-2-yl)pyrrolidin-3-amine (93 mg) was synthesized from tert-butyl (R)-(1-(6-fluorobenzo[d]thiazol-2-yl)pyrrolidin-3-yl)carbamate (85 mg, 0.253 mmol) synthesized in Reference Example 52, and the title compound (white powder, 50 mg, 47%) was obtained using (1S,2S)-2-(1H-benzo[d]imidazol-2-yl)cyclopropane-1-carboxylic acid (90 mg, 0.380 mmol).

¹H NMR (DMSO - d₆, 400 MHz): δ = 1.4 - 1.5 (m, 2H), 1.9 - 2.0 (m, 1H), 2.2 - 2.3 (m, 2H), 2.4 - 2.5 (m, 1H), 3.3 - 3.4 (m, 1H), 3.5 - 3.7 (m, 2H), 3.73 (dd, 1H, J = 6, 11 Hz), 4.4 - 4.5 (m, 1H), 7.0 - 7.2 (m, 3H), 7.3 - 7.5 (m, 3H), 7.71 (dd, 1H, J = 3, 9 Hz), 8.70 (d, 1H, J = 6 Hz), 12.4 (br s, 1H).

MS: 422.14 [M+H]⁺

### Example 73

(1S,2S)-2-(1-cyclopropyl-1H-benzo[d]imidazol-2-yl)-N-((R)-1-(6-(trifluoromethyl)pyridin-3-yl)pyrrolidin-3-yl)cyclopropane-1-carboxyamide

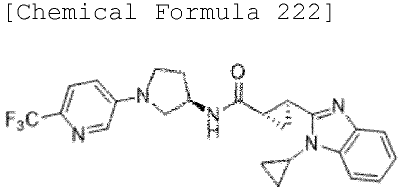

According to a technique similar to Example 1, the title compound (white powder, 15 mg, 35%) was obtained using (R)-1-(6-(trifluoromethyl)pyridin-3-yl)pyrrolidin-3-amine (21 mg, 0.090 mmol) synthesized in Reference Example 32-2 and (1S,2S)-2-(1-cyclopropyl-1H-benzo[d]imidazol-2-yl)cyclopropane-1-carboxylic acid (33 mg, 0.135 mmol) synthesized in Reference Example 54-2.

¹H NMR (CD₃OD, 400 MHz): δ = 1.0 - 1.2 (m, 2H), 1.2 - 1.4 (m, 2H), 1.6 - 1.7 (m, 2H), 2.0 - 2.3 (m, 2H), 2.3 - 2.5 (m, 1H), 2.8 - 3.0 (m, 1H), 3.3 - 3.6 (m, 4H), 3.71 (dd, 1H, J = 6, 11 Hz), 4.5 - 4.7 (m, 1H), 7.07 (dd, 1H, J = 3, 9 Hz), 7.1 - 7.3 (m, 2H), 7.50 (d, 1H, J = 7 Hz), 7.5 - 7.6 (m, 2H), 7.98 (d, 1H, J = 3 Hz). The 1H content is not observable.

MS: 456.20 [M+H]⁺

### Example 74

(1S,2S)-2-(1H-benzo[d]imidazol-2-yl)-N-((R)-1-(6-fluoroquinolin-2-yl)pyrrolidin-3-yl)cyclopropane-1-carboxyamide

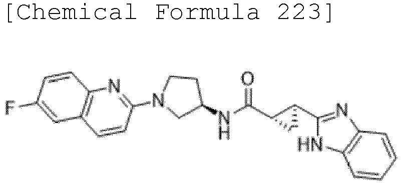

According to a technique similar to Example 3, a crude form of (R)-1-(6-fluoroquinolin-2-yl)pyrrolidin-3-amine (81 mg) was synthesized from tert-butyl (R)-(1-(6-fluoroquinolin-2-yl)pyrrolidin-3-yl)carbamate (39 mg, 0.118 mmol) synthesized in Reference Example 53, and the title compound (pale yellow powder, 10 mg, 21%) was obtained using (1S,2S)-2-(1H-benzo[d]imidazol-2-yl)cyclopropane-1-carboxylic acid (42 mg, 0.177 mmol).

¹H NMR (DMSO - d₆, 400 MHz): δ = 1.4 - 1.5 (m, 2H), 1.8 - 2.0 (m, 1H), 2.1 - 2.5 (m, 3H), 3.4 - 3.5 (m, 1H), 3.5 - 3.7 (m, 2H), 3.76 (dd, 1H, J = 6, 11 Hz), 4.3 - 4.5 (m, 1H), 6.95 (d, 1H, J = 9 Hz), 7.0 - 7.2 (m, 2H), 7.3 - 7.6 (m, 5H), 8.01 (d, 1H, J = 9 Hz), 8.64 (d, 1H, J = 7 Hz), 12.4 (br s, 1H).

MS: 416.18 [M+H]⁺

### Example 75

(1S,2S)-2-(1-cyclopropyl-1H-benzo[d]imidazol-2-yl)-N-((R)-1-(3-(trifluoromethyl)phenyl)pyrrolidin-3-yl)cyclopropane-1-carboxyamide

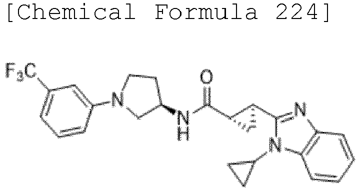

According to a technique similar to Example 3, a crude form of (R)-1-(3-(trifluoromethyl)phenyl)pyrrolidin-3-amine (99 mg) was synthesized from tert-butyl (R)-(1-(3-(trifluoromethyl)phenyl)pyrrolidin-3-yl)carbamate (105 mg, 0.319 mmol) synthesized in Reference Example 77, and the title compound (white powder, 27 mg, 28%) was obtained using (1S,2S)-2-(1-cyclopropyl-1H-benzo[d]imidazol-2-yl)cyclopropane-1-carboxylic acid (52 mg, 0.213 mmol) synthesized in Reference Example 54-2.

¹H NMR (DMSO - d₆, 400 MHz): δ = 0.9 - 1.3 (m, 4H), 1.4 - 1.6 (m, 2H), 1.9 - 2.0 (m, 1H), 2.1 - 2.3 (m, 2H), 2.6 - 2.8 (m, 1H), 3.1 - 3.2 (m, 1H), 3.3 - 3.5 (m, 3H), 3.56 (dd, 1H, J = 6, 10 Hz), 4.4 - 4.5 (m, 1H), 6.72 (s, 1H), 6.80 (dd, 1H, J = 2, 8 Hz), 6.89 (d, 1H, J = 8 Hz), 7.1 - 7.3 (m, 2H), 7.36 (t, 1H, J = 8 Hz), 7.47 (d, 1H, J = 7 Hz), 7.52 (d, 1H, J = 7 Hz), 8.62 (d, 1H, 7 Hz).

MS: 455.20 [M+H]⁺

### Example 76

(1S,2S)-2-(1H-benzo[d]imidazol-2-yl)-N-((R)-1-(5-bromopyridin-3-yl)pyrrolidin-3-yl)cyclopropane-1-carboxyamide

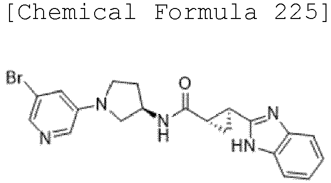

According to a technique similar to Example 3, a crude form of (R)-1-(5-bromopyridin-3-yl)pyrrolidin-3-amine (138 mg) was synthesized from tert-butyl (R)-(1-(5-bromopyridin-3-yl)pyrrolidin-3-yl)carbamate (71 mg, 0.209 mmol) synthesized in Reference Example 55, and the title compound (white powder, 56 mg, 62%) was obtained using (1S,2S)-2-(1H-benzo[d]imidazol-2-yl)cyclopropane-1-carboxylic acid (63 mg, 0.314 mmol).

¹H NMR (DMSO - d₆, 400 MHz): δ = 1.4 - 1.5 (m, 2H), 1.8 - 2.0 (m, 1H), 2.1 - 2.5 (m, 3H), 3.14 (dd, 1H, J = 4, 10 Hz), 3.3 - 3.5 (m, 2H), 3.53 (dd, 1H, J = 6, 10 Hz), 4.3 - 4.5 (m, 1H), 7.0 - 7.2 (m, 3H), 7.3 - 7.5 (m, 2H), 7.89 (d, 1H, J = 2 Hz), 7.91 (d, 1H, J = 2 Hz), 8.60 (d, 1H, J = 7 Hz), 12.4 (br s, 1H).

MS: 426.09 [M+H]⁺

### Example 77

(R)-2-(quinolin-6-yl)-N-(1-(5-(trifluoromethyl)pyridin-3-yl)pyrrolidin-3-yl)acetamide

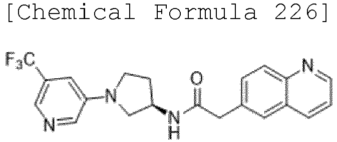

According to a technique similar to Example 1, the title compound (pale yellow amorphous, 88 mg, 100%) was obtained using (R)-1-(5-(trifluoromethyl)pyridin-3-yl)pyrrolidin-3-amine (50 mg, 0.216 mmol) synthesized in Reference Example 1-2 and 6-quinolineacetic acid (49 mg, 0.259 mmol).

¹H NMR (CDCl₃, 400 MHz): δ = 1.9 - 2.1 (m, 1H), 2.2 - 2.4 (m, 1H), 3.14 (dd, 1H, J = 4, 10 Hz), 3.2 - 3.4 (m, 2H), 3.57 (dd, 1H, J = 6, 10 Hz), 3.78 (s, 2H), 4.6 - 4.7 (m, 1H), 6.73 (s, 1H), 6.81 (d, 1H, J = 7 Hz), 7.38 (dd, 1H, J = 4, 8 Hz), 7.60 (dd, 1H, J = 2, 8 Hz), 7.69 (s, 1H), 7.85 (br s, 1H), 8.0 - 8.1 (m, 3H), 8.86 (d, 1H, J = 3 Hz).

MS: 401.19 [M+H]⁺

### Example 78

(R)-2-(1H-indol-3-yl)-N-(1-(5-(trifluoromethyl)pyridin-3-yl)pyrrolidin-3-yl)acetamide

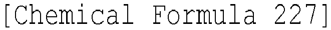

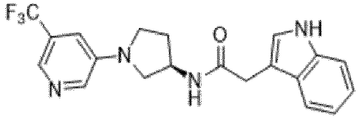

According to a technique similar to Example 1, the title compound (white amorphous, 87 mg, 100%) was obtained using (R)-1-(5-(trifluoromethyl)pyridin-3-yl)pyrrolidin-3-amine (50 mg, 0.216 mmol) synthesized in Reference Example 1-2 and 3-indoleacetic acid (45 mg, 0.259 mmol).

¹H NMR (CDCl₃, 400 MHz): δ = 1.7 - 1.9 (m, 1H), 2.2 - 2.4 (m, 1H), 3.07 (dd, 1H, J = 4, 10 Hz), 3.1 - 3.4 (m, 2H), 3.59 (dd, 1H, J = 6, 10 Hz), 3.76 (s, 2H), 4.5 - 4.7 (m, 1H), 5.79 (d, 1H, J = 6 Hz), 6.82 (s, 1H), 7.0 - 7.3 (m, 3H), 7.39 (d, 1H, J = 8 Hz), 7.49 (d, 1H, J = 7 Hz), 8.00 (d, 1H, J = 3 Hz), 8.18 (s, 2H).

MS: 389.19 [M+H]⁺

### Example 79

(R)-2-(quinolin-7-yl)-N-(1-(5-(trifluoromethyl)pyridin-3-yl)pyrrolidin-3-yl)acetamide hydrochloride

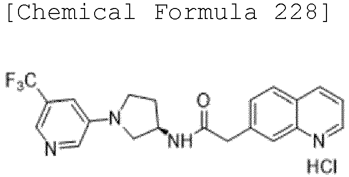

According to a technique similar to Example 1, (R)-2-(quinolin-7-yl)-N-(1-(5-(trifluoromethyl)pyridin-3-yl)pyrrolidin-3-yl)acetamide (colorless oily material, 56 mg) was obtained using (R)-1-(5-(trifluoromethyl)pyridin-3-yl)pyrrolidin-3-amine (99 mg, 0.427 mmol) synthesized in Reference Example 1-2 and 7-quinolineacetic acid (80 mg, 0.427 mmol). (R)-2-(quinolin-7-yl)-N-(1-(5-(trifluoromethyl)pyridin-3-yl)pyrrolidin-3-yl)acetamide (56 mg) thus obtained was dissolved in ethyl acetate (2 mL), a 2M solution (2 mL) of hydrogen chloride in ethyl acetate was added thereto, the mixture was stirred for a while, subsequently the solvent was distilled off under reduced pressure, and the title compound (yellow powder, 52 mg, 28%) was obtained.

¹H NMR (DMSO - d₆, 400 MHz): δ = 1.9 - 2.1 (m, 1H), 2.1 - 2.3 (m, 1H), 3.2 - 3.9 (m, 6H), 4.4 - 4.5 (m, 1H), 7.2 - 7.3 (m, 1H), 7.84 (d, 1H, J = 8 Hz), 7.9 - 8.1 (m, 1H), 8.1 - 8.3 (m, 4H), 8.7 - 8.8 (m, 1H), 9.0 - 9.1 (m, 1H), 9.24 (d, 1H, J = 4 Hz). The 1H content is not observable.

MS: 401.16 [M+H]⁺

### Example 80

(R)-2-(4-cyclopropylphenyl)-N-(1-(4-(trifluoromethyl)oxazol-2-yl)pyrrolidin-3-yl)acetamide

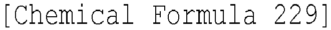

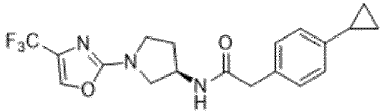

According to a technique similar to Example 27, the title compound (white powder, 61 mg, 92%) was obtained using 2-(4-cyclopropylphenyl)acetic acid (43 mg, 0.176 mmol) synthesized in Reference Example 33-2 and 2-bromo-4-(trifluoromethyl)-1,3-oxazole (38 mg, 0.176 mmol).

¹H NMR (CDCl₃, 400 MHz): δ = 0.6 - 0.8 (m, 2H), 0.9 - 1.0 (m, 2H), 1.8 - 2.0 (m, 2H), 2.2 - 2.3 (m, 1H), 3.28 (dd, 1H, J = 5, 11 Hz), 3.4 - 3.6 (m, 4H), 3.77 (dd, 1H, J = 6, 11 Hz), 4.5 - 4.6 (m, 1H), 5.44 (d, 1H, J = 7 Hz), 7.0 - 7.2 (m, 4H), 7.49 (dd, 1H, J = 1, 3 Hz).

MS: 402.12 [M+Na]⁺

### Example 81

(R)-2-(1-methyl-5-(trifluoromethyl)-1H-pyrazol-3-yl)-N-(1-(5-(trifluoromethyl)pyridin-3-yl)pyrrolidin-3-yl)acetamide

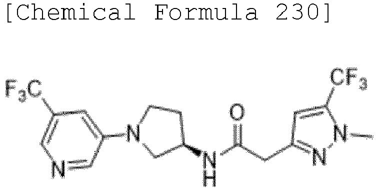

According to a technique similar to Example 1, the title compound (white powder, 62 mg, 98%) was obtained using (R)-1-(5-(trifluoromethyl)pyridin-3-yl)pyrrolidin-3-amine (35 mg, 0.151 mmol) synthesized in Reference Example 1-2 and 2-(1-methyl-5-(trifluoromethyl)-1H-pyrazol-3-yl)acetic acid (33 mg, 0.159 mmol).

¹H NMR (CDCl₃, 400 MHz): δ = 2.0 - 2.1 (m, 1H), 2.3 - 2.5 (m, 1H), 3.23 (dd, 1H, J = 4, 10 Hz), 3.3 - 3.6 (m, 4H), 3.68 (dd, 1H, J = 6, 10 Hz), 3.91 (s, 3H), 4.6 - 4.7 (m, 1H), 6.54 (s, 1H), 6.68 (d, 1H, J = 6 Hz), 6.94 (t, 1H, J = 2 Hz), 8.12 (d, 1H, J = 3 Hz), 8.21 (d, 1H, J = 1 Hz).

MS: 444.09 [M+Na]⁺

### Example 82

(R)-2-(6-(trifluoromethyl)pyridin-3-yl)-N-(1-(5-(trifluoromethyl)pyridin-3-yl)pyrrolidin-3-yl)acetamide

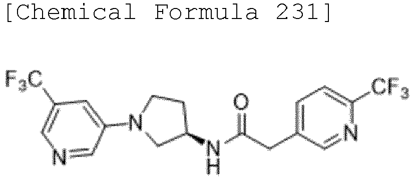

According to a technique similar to Example 1, the title compound (white powder, 27 mg, 74%) was obtained using (R)-1-(5-(trifluoromethyl)pyridin-3-yl)pyrrolidin-3-amine (20 mg, 0.0865 mmol) synthesized in Reference Example 1-2 and 2-(6-(trifluoromethyl)pyridin-3-yl)acetic acid (20 mg, 0.0952 mmol).

¹H NMR (CDCl₃, 400 MHz): δ = 2.0 - 2.1 (m, 1H), 2.3 - 2.5 (m, 1H), 3.24 (dd, 1H, J = 4, 10 Hz), 3.3 - 3.6 (m, 2H), 3.62 (s, 2H), 3.67 (dd, 1H, J = 6, 10 Hz), 4.6 - 4.7 (m, 1H), 6.02 (d, 1H, J = 7 Hz), 6.92 (s, 1H), 7.68 (d, 1H, J = 8 Hz), 7.90 (dd, 1H, J = 1, 8 Hz), 8.08 (d, 1H, 3 Hz), 8.20 (s, 1H), 8.62 (d, 1H, J = 2 Hz).

MS: 419.09 [M+H]⁺

### Example 83

(R)-2-(4-(trifluoromethyl)phenyl)-N-(1-(5-(trifluoromethyl)pyridin-3-yl)pyrrolidin-3-yl)acetamide-2,2-d₂

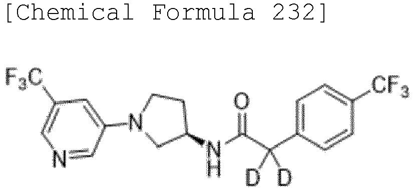

According to a technique similar to Example 1, the title compound (white crystals, 34 mg, 57%) was obtained using (R)-1-(5-(trifluoromethyl)pyridin-3-yl)pyrrolidin-3-amine (34 mg, 0.145 mmol) synthesized in Reference Example 1-2 and 2-(4-(trifluoromethyl)phenyl)acetic acid-2,2-d₂ (30 mg, 0.145 mmol) synthesized in Reference Example 56.

¹H NMR (CDCl₃, 400 MHz): δ = 1.9 - 2.1 (m, 1H), 2.3 - 2.5 (m, 1H), 3.19 (dd, 1H, J = 5, 10 Hz), 3.3 - 3.5 (m, 2H), 3.67 (dd, 1H, J = 6, 10 Hz), 4.6 - 4.7 (m, 1H), 5.62 (d, 1H, J = 6 Hz), 6.9 - 7.0 (m, 1H), 7.40 (d, 2H, J = 8 Hz), 7.61 (d, 2H, J = 8 Hz), 8.10 (d, 1H, J = 3 Hz), 8.21 (s, 1H).

MS: 420.10 [M+H]⁺

### Example 84

(R)-2-(4-(3,3-difluoropyrrolidin-1-yl)phenyl)-N-(1-(6-(trifluoromethyl)pyridin-3-yl)pyrrolidin-3-yl)acetamide

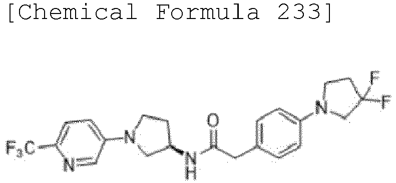

According to a technique similar to Example 9, the title compound (pale yellow powder, 22 mg, 24%) was obtained using (R)-2-(4-bromophenyl)-N-(1-(6-(trifluoromethyl)pyridin-3-yl)pyrrolidin-3-yl)acetamide (88 mg, 0.205 mmol) synthesized in Reference Example 57 and 3,3-difluoropyrrolidine hydrochloride (35 mg, 0.247 mmol).

¹H NMR (CDCl₃, 400 MHz): δ = 1.8 - 2.0 (m, 1H), 2.2 - 2.6 (m, 3H), 3.11 (dd, 1H, J = 5, 10 Hz), 3.38 (t, 2H, J = 7 Hz), 3.4 - 3.6 (m, 4H), 3.6 - 3.7 (m, 3H), 4.5 - 4.7 (m, 1H), 5.49 (d, 1H, J = 7 Hz), 6.4 - 6.6 (m, 2H), 6.79 (dd, 1H, J = 3, 8 Hz), 7.0 - 7.2 (m, 2H), 7.47 (d, 1H, J = 9 Hz), 7.97 (d, 1H, J = 3 Hz).

MS: 477.12 [M+Na]⁺

### Example 85

(R)-2-(1H-indol-6-yl)-N-(1-(6-(trifluoromethyl)pyridin-3-yl)pyrrolidin-3-yl)acetamide

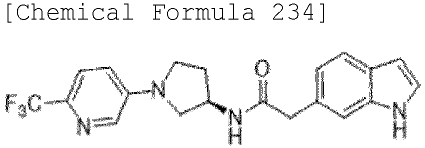

According to a technique similar to Example 1, the title compound (pale yellow amorphous, 35 mg, 84%) was obtained using (R)-1-(6-(trifluoromethyl)pyridin-3-yl)pyrrolidin-3-amine (25 mg, 0.108 mmol) synthesized in Reference Example 32-2 and 2-(1H-indol-6-yl)acetic acid (23 mg, 0.130 mmol).

¹H NMR (CDCl₃, 400 MHz): δ = 1.8 - 1.9 (m, 1H), 2.2 - 2.4 (m, 1H), 3.10 (dd, 1H, J = 5, 10 Hz), 3.2 - 3.4 (m, 2H), 3.65 (dd, 1H, J = 6, 10 Hz), 3.70 (s, 2H), 4.5 - 4.7 (m, 1H), 5.45 (d, 1H, J = 7 Hz), 6.5 - 6.6 (m, 1H), 6.75 (dd, 1H, J = 3, 8 Hz), 6.96 (dd, 1H, J = 1, 8 Hz), 7.2 - 7.3 (m, 2H), 7.45 (d, 1H, J = 9 Hz), 7.62 (d, 1H, J = 8 Hz), 7.94 (d, 1H, J = 3 Hz), 8.17 (br s, 1H).

MS: 389.12 [M+H]⁺

### Example 86

2-(4-((2R,6S)-2,6-dimethylmorpholino)phenyl)-N-((R)-1-(5-(trifluoromethyl)pyridin-3-yl)pyrrolidin-3-yl)acetamide

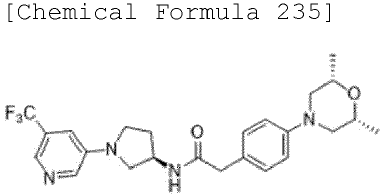

According to a technique similar to Example 9, the title compound (white powder, 12 mg, 22%) was obtained using (R)-2-(4-bromophenyl)-N-(1-(5-(trifluoromethyl)pyridin-3-yl)pyrrolidin-3-yl)acetamide (50 mg, 0.117 mmol) synthesized in Example 171 and cis-2,6-dimethylmorpholine (16 mg, 0.140 mmol).

¹H NMR (CDCl₃, 400 MHz): δ = 1.26 (d, 6H, J = 6 Hz), 1.8 - 2.0 (m, 1H), 2.2 - 2.5 (m, 3H), 3.11 (dd, 1H, J = 5, 10 Hz), 3.37 (t, 2H, J = 7 Hz), 3.4 - 3.5 (m, 2H), 3.51 (s, 2H), 3.65 (dd, 1H, J = 6, 10 Hz), 3.7 - 3.9 (m, 2H), 4.5 - 4.7 (m, 1H), 5.52 (d, 1H, J = 7 Hz), 6.8 - 7.0 (m, 3H), 7.12 (d, 2H, J = 9 Hz), 8.07 (d, 1H, J = 3 Hz), 8.20 (s, 1H).

MS: 463.18 [M+H]⁺

### Example 87

(R)-2-(4-(pyrrolidin-1-yl)phenyl)-N-(1-(5-(trifluoromethyl)pyridin-3-yl)pyrrolidin-3-yl)acetamide

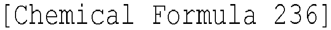

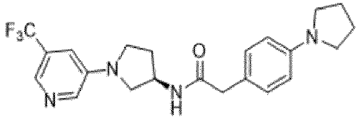

According to a technique similar to Example 9, the title compound (white crystals, 8 mg, 16%) was obtained using (R)-2-(4-bromophenyl)-N-(1-(5-(trifluoromethyl)pyridin-3-yl)pyrrolidin-3-yl)acetamide (50 mg, 0.117 mmol) synthesized in Example 171 and pyrrolidine (12 µL, 0.140 mmol) .

¹H NMR (CDCl₃, 400 MHz): δ = 1.8 - 2.1 (m, 5H), 2.2 - 2.4 (m, 1H), 3.08 (dd, 1H, J = 5, 10 Hz), 3.2 - 3.3 (m, 4H), 3.35 (t, 2H, J = 7 Hz), 3.49 (s, 2H), 3.64 (dd, 1H, J = 6, 10 Hz), 4.5 - 4.7 (m, 1H), 5.55 (d, 1H, J = 7 Hz), 6.52 (d, 2H, J = 9 Hz), 6.8 - 6.9 (m, 1H), 7.05 (d, 2H, J = 9 Hz), 8.06 (d, 1H, J = 3 Hz), 8.19 (s, 1H).

MS: 419.17 [M+H]⁺

### Example 88

(R)-2-(4-((2-methoxyethyl)(methyl)amino)phenyl)-N-(1-(5-(trifluoromethyl)pyridin-3-yl)pyrrolidin-3-yl)acetamide

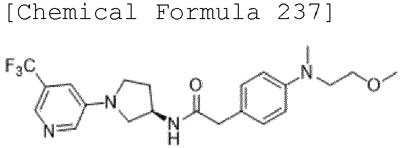

According to a technique similar to Example 9, the title compound (white amorphous, 2.5 mg, 5%) was obtained using (R)-2-(4-bromophenyl)-N-(1-(5-(trifluoromethyl)pyridin-3-yl)pyrrolidin-3-yl)acetamide (50 mg, 0.117 mmol) synthesized in Example 171 and N-(2-methoxyethyl)methylamine (15 µL, 0.140 mmol).

¹H NMR (CDCl₃, 400 MHz): δ = 1.8 - 2.0 (m, 1H), 2.2 - 2.4 (m, 1H), 2.97 (s, 3H), 3.10 (dd, 1H, J = 5, 10 Hz), 3.3 - 3.4 (m, 5H), 3.4 - 3.6 (m, 6H), 3.65 (dd, 1H, J = 6, 10 Hz), 4.5 - 4.7 (m, 1H), 5.54 (d, 1H, J = 7 Hz), 6.69 (d, 2H, J = 9 Hz), 6.8 - 7.0 (m, 1H), 7.06 (d, 2H, J = 9 Hz), 8.07 (d, 1H, J = 2 Hz), 8.20 (s, 1H).

MS: 437.17 [M+H]⁺

### Example 89

2-(4-(Trifluoromethyl)phenyl)-N-(1-(5-(trifluoromethyl)pyridin-3-yl)azetidin-3-yl)acetamide

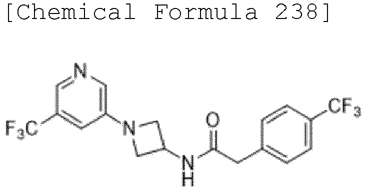

According to a technique similar to Example 1, the title compound (white crystals, 22 mg, 80%) was obtained using 1-(5-(trifluoromethyl)pyridin-3-yl)azetidin-3-amine (15 mg, 0.06691 mmol) synthesized in Reference Example 58-2 and 4-(trifluoromethyl)phenylacetic acid (17 mg, 0.0829 mmol) .

¹H NMR (DMSO - d₆, 400 MHz): δ = 3.56 (s, 2H), 3.77 (dd, 2H, J = 5, 8 Hz), 4.26 (t, 2H, J = 8 Hz), 4.5 - 4.7 (m, 1H), 7.1 - 7.2 (m, 1H), 7.49 (d, 2H, J = 8 Hz), 7.67 (d, 2H, J = 8 Hz), 8.11 (t, 1H, J = 2 Hz), 8.23 (s, 1H), 8.89 (d, 1H, J = 7 Hz).

MS: 404.06 [M+H]⁺

### Example 90

2-(1H-indol-6-yl)-N-(1-(5-(trifluoromethyl)pyridin-3-yl)azetidin-3-yl)acetamide

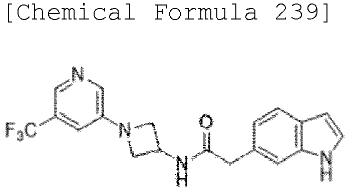

According to a technique similar to Example 1, the title compound (white crystals, 20 mg, 75%) was obtained using 1-(5-(trifluoromethyl)pyridin-3-yl)azetidin-3-amine (15 mg, 0.0691 mmol) synthesized in Reference Example 58-2 and 2-(1H-indol-6-yl)acetic acid (15 mg, 0.0829 mmol).

¹H NMR (DMSO - d₆, 400 MHz): δ = 3.48 (s, 2H), 3.76 (dd, 2H, J = 6, 8 Hz), 4.25 (t, 2H, J = 8 Hz), 4.5 - 4.7 (m, 1H), 6.37 (t, 1H, J = 2 Hz), 6.90 (dd, 1H, J = 1, 8 Hz), 7.13 (t, 1H, J = 2 Hz), 7.2 - 7.3 (m, 2H), 7.44 (t, 1H, J = 8 Hz), 8.10 (d, 1H, J = 3 Hz), 8.23 (s, 1H), 8.72 (d, 1H, J = 7 Hz), 11.0 (s, 1H).

MS: 375.09 [M+H]⁺

### Example 91

(R)-2-(1-methyl-1H-indol-6-yl)-N-(1-(5-(trifluoromethyl)pyridin-3-yl)pyrrolidin-3-yl)acetamide

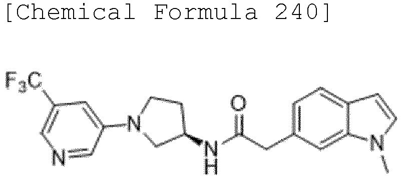

According to a technique similar to Example 1, the title compound (white crystals, 14 mg, 63%) was obtained using (R)-1-(5-(trifluoromethyl)pyridin-3-yl)pyrrolidin-3-amine (12 mg, 0.0529 mmol) synthesized in Reference Example 1-2 and 2-(1-methyl-1H-indol-6-yl)acetic acid (10 mg, 0.0529 mmol).

¹H NMR (CDCl₃, 400 MHz): δ = 1.8 - 1.9 (m, 1H), 2.2 - 2.4 (m, 1H), 3.08 (dd, 1H, J = 5, 10 Hz), 3.2 - 3.4 (m, 2H), 3.64 (dd, 1H, J = 6, 10 Hz), 3.73 (s, 2H), 3.77 (s, 3H), 4.5 - 4.7 (m, 1H), 5.57 (d, 1H, J = 7 Hz), 6.48 (d, 1H, J = 2 Hz), 6.8 - 6.9 (m, 1H), 6.95 (dd, 1H, J = 1, 8 Hz), 7.07 (d, 1H, J = 3 Hz), 7.19 (s, 1H), 7.60 (d, 1H, J = 8 Hz), 8.05 (d, 1H, J = 3 Hz), 8.19 (s, 1H).

MS: 403.11 [M+H]⁺

### Example 92

(R)-2-(1-methyl-1H-indol-6-yl)-N-(1-(6-(trifluoromethyl)pyridin-3-yl)pyrrolidin-3-yl)acetamide

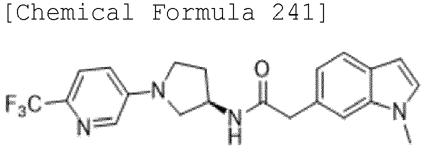

According to a technique similar to Example 1, the title compound (white crystals, 9 mg, 41%) was obtained using (R)-1-(6-(trifluoromethyl)pyridin-3-yl)pyrrolidin-3-amine (12 mg, 0.0529 mmol) synthesized in Reference Example 32-2 and 2-(1-methyl-1H-indol-6-yl)acetic acid (10 mg, 0.0529 mmol).

¹H NMR (CDCl₃, 400 MHz): δ = 1.8 - 1.9 (m, 1H), 2.2 - 2.4 (m, 1H), 3.09 (dd, 1H, J = 5, 10 Hz), 3.2 - 3.4 (m, 2H), 3.65 (dd, 1H, J = 6, 10 Hz), 3.73 (s, 2H), 3.76 (s, 3H), 4.5 - 4.7 (m, 1H), 5.56 (d, 1H, J = 7 Hz), 6.48 (d, 1H, J = 3 Hz), 6.75 (dd, 1H, J = 3, 9 Hz), 6.94 (dd, 1H, J = 1, 8 Hz), 7.07 (d, 1H, J = 3 Hz), 7.19 (s, 1H), 7.45 (d, 1H, J = 9 Hz), 7.59 (d, 1H, J = 8 Hz), 7.93 (d, 1H, J = 3 Hz).

MS: 403.11 [M+H]⁺

### Example 93

(R)-2-(4-(4-methylpiperazin-1-yl)phenyl)-N-(1-(5-(trifluoromethyl)pyridin-3-yl)pyrrolidin-3-yl)acetamide

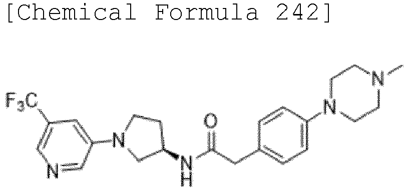

Ethyl 2-(4-(4-methylpiperazin-1-yl)phenyl)acetate (95 mg, 0.362 mmol) was dissolved in methanol (2 mL) and water (2 mL), lithium hydroxide monohydrate (46 mg, 1.09 mmol) was added thereto, and the mixture was stirred overnight at room temperature. Water was added to the reaction liquid, the mixture was washed with ethyl acetate, 6M hydrochloric acid (190 µL) was added to an aqueous layer, and the aqueous layer was washed again with ethyl acetate. The aqueous layer was distilled off under reduced pressure, and a crude form of 2-(4-(4-methylpiperidin-1-yl)phenyl)acetic acid (colorless oily material, 192 mg) was obtained. The crude form of 2-(4-(4-methylpiperidin-1-yl)phenyl)acetic acid (96 mg) thus obtained and (R)-1-(5-(trifluoromethyl)pyridin-3-yl)pyrrolidin-3-amine (38 mg, 0.163 mmol) synthesized in Reference Example 1-2 were dissolved in methanol (1.5 mL), subsequently DMT-MM (90 mg, 0.326 mmol) and DIPEA (166 µL, 0.978 mmol) were added thereto, and the mixture was stirred overnight at room temperature. Water was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by NH silica gel column chromatography (heptane: ethyl acetate) (concentration gradient: 50 to 100%), and the title compound (white powder, 51 mg, 69%) was obtained.

¹H NMR (CDCl₃, 400 MHz): δ = 1.8 - 2.0 (m, 1H), 2.2 - 2.4 (m, 4H), 2.58 (t, 4H, J = 5 Hz), 3.11 (dd, 1H, J = 5, 10 Hz), 3.21 (t, 4H, J = 5 Hz), 3.37 (t, 2H, J = 7 Hz), 3.51 (s, 2H), 3.65 (dd, 1H, J = 6, 10 Hz), 4.5 - 4.7 (m, 1H), 5.52 (d, 1H, J = 7 Hz), 6.8 - 7.0 (m, 3H), 7.1 - 7.2 (m, 2H), 8.07 (d, 1H, J = 3 Hz), 8.20 (s, 1H).

MS: 448.17 [M+H]⁺

### Example 94

(R)-2-(4-(piperazin-1-yl)phenyl)-N-(1-(5-(trifluoromethyl)pyridin-3-yl)pyrrolidin-3-yl)acetamide

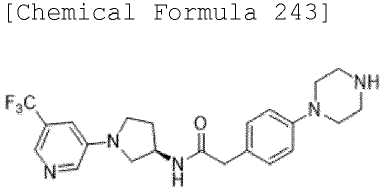

According to a technique similar to Example 33, the title compound (white powder, 122 mg, 91%) was obtained using benzyl (R)-4-(4-(2-oxo-2-((1-(5-(trifluoromethyl)pyridin-3-yl)pyrrolidin-3-yl)amino)ethyl)phenyl)piperazine-1-carboxylate (176 mg, 0.310 mmol) synthesized in Reference Example 59.

¹H NMR (CDCl₃, 400 MHz): δ = 1.8 - 2.0 (m, 1H), 2.2 - 2.4 (m, 1H), 3.0 - 3.2 (m, 9H), 3.37 (t, 2H, J = 7 Hz), 3.51 (s, 2H), 3.65 (dd, 1H, J = 6, 10 Hz), 4.5 - 4.7 (m, 1H), 5.51 (d, 1H, J = 7 Hz), 6.8 - 7.0 (m, 3H), 7.1 - 7.2 (m, 2H), 8.08 (d, 1H, J = 3 Hz), 8.20 (s, 1H). The 1H content is not observable.

MS: 434.15 [M+Na]⁺

### Example 95

(R)-2-(4-(4-acetylpiperazin-1-yl)phenyl)-N-(1-(5-(trifluoromethyl)pyridin-3-yl)pyrrolidin-3-yl)acetamide

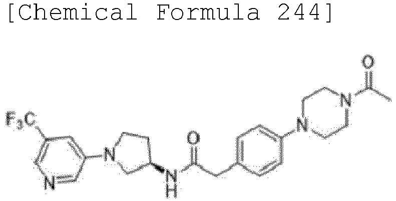

(R)-2-(4-(piperazin-1-yl)phenyl)-N-(1-(5-(trifluoromethyl)pyridin-3-yl)pyrrolidin-3-yl)acetamide (45 mg, 0.104 mmol) synthesized in Example 94 and triethylamine (36 µL, 0.104 mmol) were dissolved in chloroform (2 mL), subsequently acetic anhydride (12 µL, 0.125 mmol) was added thereto under ice cooling, and the mixture was stirred at room temperature. After 18 hours, the solvent was

distilled off under reduced pressure, a residue thus obtained was purified by NH silica gel column chromatography (heptane: ethyl acetate) (concentration gradient: 90 to 100%), and the title compound (white powder, 43 mg, 86%) was obtained.

¹H NMR (CDCl₃, 400 MHz): δ = 1.8 - 2.0 (m, 1H), 2.15 (s, 3H), 2.2 - 2.4 (m, 1H), 3.0 - 3.2 (m, 5H), 3.38 (t, 2H, J = 7 Hz), 3.51 (s, 2H), 3.6 - 3.7 (m, 3H), 3.77 (t, 2H, J = 5 Hz), 4.5 - 4.7 (m, 1H), 5.52 (d, 1H, J = 7 Hz), 6.8 - 7.0 (m, 3H), 7.1 - 7.2 (m, 2H), 8.08 (d, 1H, J = 3 Hz), 8.20 (s, 1H).

MS: 498.14 [M+Na]⁺

### Example 96

(R)-2-(4-(2-oxopiperidin-1-yl)phenyl)-N-(1-(5-(trifluoromethyl)pyridin-3-yl)pyrrolidin-3-yl)acetamide

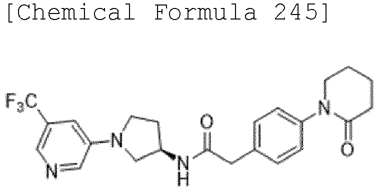

According to a technique similar to Example 1, the title compound (white powder, 45 mg, 47%) was obtained using (R)-1-(5-(trifluoromethyl)pyridin-3-yl)pyrrolidin-3-amine (50 mg, 0.215 mmol) synthesized in Reference Example 1-2 and 2-(4-(2-oxopiperidin-1-yl)phenyl)acetic acid (55 mg, 0.236 mmol).

¹H NMR (CDCl₃, 400 MHz): δ = 1.8 - 2.1 (m, 5H), 2.2 - 2.4 (m, 1H), 2.53 (t, 2H, J = 6 Hz), 3.18 (dd, 1H, J = 5, 10 Hz), 3.3 - 3.7 (m, 7H), 4.5 - 4.7 (m, 1H), 5.88 (d, 1H, J = 7 Hz), 6.8 - 7.0 (m, 1H), 7.2 - 7.3 (m, 4H), 8.08 (d, 1H, J = 3 Hz), 8.20 (s, 1H).

MS: 469.11 [M+H]⁺

### Example 97

(R)-2-(6-chlorobenzo[d]oxazol-2-yl)-N-(1-(5-(trifluoromethyl)pyridin-3-yl)pyrrolidin-3-yl)acetamide

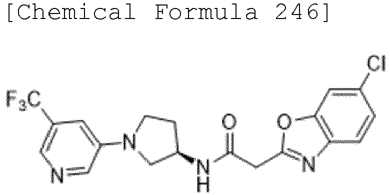

According to a technique similar to Example 1, the title compound (yellow powder, 57 mg, 71%) was obtained using (R)-1-(5-(trifluoromethyl)pyridin-3-yl)pyrrolidin-3-amine (44 mg, 0.189 mmol) synthesized in Reference Example 1-2 and 2-(6-chloro-1,3-benzoxazol-2-yl)acetic acid (40 mg, 0.189 mmol).

¹H NMR (CDCl₃, 400 MHz): δ = 2.1 - 2.2 (m, 1H), 2.3 - 2.5 (m, 1H), 3.33 (dd, 1H, J = 4, 10 Hz), 3.4 - 3.6 (m, 2H), 3.73 (dd, 1H, J = 6, 10 Hz), 3.69 (s, 2H), 4.6 - 4.8 (m, 1H), 6.9 - 7.0 (m, 1H), 7.34 (dd, 1H, J = 2, 9 Hz), 7.45 (d, 1H, J = 9 Hz), 7.65 (d, 1H, J = 2 Hz), 7.99 (d, 1H, J = 6 Hz), 8.13 (d, 1H, J = 3 Hz), 8.22 (s, 1H).

MS: 425.06 [M+H]⁺

### Example 98

(R)-2-(benzo[d]oxazol-2-yl)-N-(1-(5-(trifluoromethyl)pyridin-3-yl)pyrrolidin-3-yl)acetamide

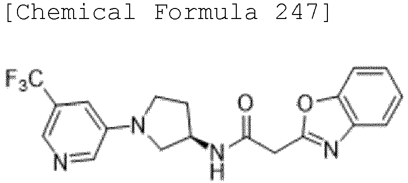

According to a technique similar to Example 1, the title compound (yellow powder, 51 mg, 76%) was obtained using (R)-1-(5-(trifluoromethyl)pyridin-3-yl)pyrrolidin-3-amine (40 mg, 0.173 mmol) synthesized in Reference Example 1-2 and sodium 2-(benzo[d]oxazol-2-yl)acetate monohydrate (41 mg, 0.190 mmol).

¹H NMR (CDCl₃, 400 MHz): δ = 2.1 - 2.2 (m, 1H), 2.3 - 2.5 (m, 1H), 3.34 (dd, 1H, J = 4, 10 Hz), 3.4 - 3.6 (m, 2H), 3.73 (dd, 1H, J = 6, 10 Hz), 3.97 (s, 2H), 4.6 - 4.8 (m, 1H), 6.9 - 7.0 (m, 1H), 7.3 - 7.4 (m, 2H), 7.5 - 7.6 (m, 1H), 7.6 - 7.7 (m, 1H), 8.13 (d, 1H, J = 3 Hz), 8.21 (s, 2H).

MS: 391.10 [M+H]⁺

### Example 99

Trans-2-(3,5-dichlorophenyl)-N-((R)-1-(5-(trifluoromethyl)pyridin-3-yl)pyrrolidin-3-yl)cyclopropane-1-carboxyamide

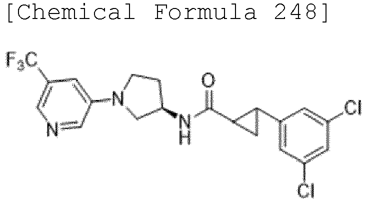

According to a technique similar to Example 1, diastereomer A (upper spot in TLC (ethyl acetate), white powder, 25 mg, 42%) and diastereomer B (lower spot in TLC (ethyl acetate), white powder, 19 mg, 32%) of the title compound were obtained using (R)-1-(5-(trifluoromethyl)pyridin-3-yl)pyrrolidin-3-amine (30 mg, 0.130 mmol) synthesized in Reference Example 1-2 and trans-2-(3,5-dichlorophenyl)cyclopropane-1-carboxylic acid (33 mg, 0.143 mmol).

### Diastereomer A

¹H NMR (CDCl₃, 400 MHz): δ = 1.2 - 1.3 (m, 1H), 1.5 - 1.7 (m, 2H), 2.0 - 2.2 (m, 1H), 2.3 - 2.6 (m, 2H), 3.28 (dd, 1H, J = 4, 10 Hz), 3.4 - 3.6 (m, 2H), 3.69 (dd, 1H, J = 6, 10 Hz), 4.6 - 4.8 (m, 1H), 5.87 (d, 1H, J = 7 Hz), 6.9 - 7.0 (m, 3H), 7.19 (t, 1H, J = 2 Hz), 8.13 (d, 1H, J = 3 Hz), 8.22 (s, 1H).

MS: 444.02 [M+H]⁺

### Diastereomer B

¹H NMR (CDCl₃, 400 MHz): δ = 1.2 - 1.3 (m, 1H), 1.5 - 1.7 (m, 2H), 2.0 - 2.2 (m, 1H), 2.3 - 2.5 (m, 2H), 3.27 (dd, 1H, J = 4, 10 Hz), 3.4 - 3.6 (m, 2H), 3.68 (dd, 1H, J = 6, 10 Hz), 4.6 - 4.8 (m, 1H), 5.88 (d, 1H, J = 7 Hz), 6.9 - 7.0 (m, 3H), 7.18 (t, 1H, J = 2 Hz), 8.12 (d, 1H, J = 3 Hz), 8.21 (s, 1H).

MS: 444.01 [M+H]⁺

### Example 100

Trans-2-(4-bromophenyl)-N-((R)-1-(5-(trifluoromethyl)pyridin-3-yl)pyrrolidin-3-yl)cyclopropane-1-carboxyamide

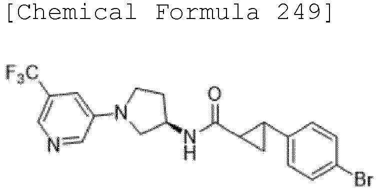

According to a technique similar to Example 1, diastereomer A (upper spot in TLC (ethyl acetate), white powder, 26 mg, 43%) and diastereomer B (lower spot in TLC (ethyl acetate), white powder, 23 mg, 39%) of the title compound were obtained using (R)-1-(5-(trifluoromethyl)pyridin-3-yl)pyrrolidin-3-amine (30 mg, 0.130 mmol) synthesized in Reference Example 1-2 and trans-2-(4-bromophenyl)cyclopropane-1-carboxylic acid (34 mg, 0.143 mmol).

### Diastereomer A

¹H NMR (CDCl₃, 400 MHz): δ = 1.2 - 1.3 (m, 1H), 1.5 - 1.7 (m, 2H), 2.0 - 2.2 (m, 1H), 2.3 - 2.6 (m, 2H), 3.27 (dd, 1H, J = 5, 10 Hz), 3.4 - 3.6 (m, 2H), 3.69 (dd, 1H, J = 6, 10 Hz), 4.6 - 4.8 (m, 1H), 5.80 (d, 1H, J = 7 Hz), 6.9 - 7.0 (m, 3H), 7.3 - 7.5 (m, 2H), 8.13 (d, 1H, J = 3 Hz), 8.22 (s, 1H).

MS: 454.00 [M+H]⁺

### Diastereomer B

¹H NMR (CDCl₃, 400 MHz): δ = 1.2 - 1.3 (m, 1H), 1.5 - 1.7 (m, 2H), 2.0 - 2.2 (m, 1H), 2.3 - 2.6 (m, 2H), 3.26 (dd, 1H, J = 4, 10 Hz), 3.4 - 3.6 (m, 2H), 3.68 (dd, 1H, J = 6, 10 Hz), 4.6 - 4.8 (m, 1H), 5.84 (d, 1H, J = 7 Hz), 6.9 - 7.0 (m, 3H), 7.3 - 7.4 (m, 2H), 8.12 (d, 1H, J = 3 Hz), 8.21 (s, 1H).

MS: 454.01 [M+H]⁺

### Example 101

(R)-2-(4-(1,4-dioxa-8-azaspiro[4.5]decan-8-yl)phenyl)-N-(1-(5-(trifluoromethyl)pyridin-3-yl)pyrrolidin-3-yl)acetamide

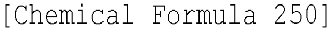

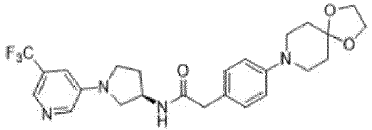

Ethyl 2-(4-(1,4-dioxa-8-azaspiro[4.5]decan-8-yl)phenyl)acetate (16 mg, 0.0524 mmol) synthesized in Reference Example 60 was dissolved in methanol (1 mL) and water (1 mL), lithium hydroxide monohydrate (7 mg, 0.176 mmol) was added thereto, and the mixture was stirred overnight at room temperature. Water was added to the reaction liquid, the mixture was washed with ethyl acetate, 2M hydrochloric acid (88 µL) was added to an aqueous layer to neutralize the aqueous layer, and the aqueous layer was extracted with a mixed liquid of chloroform and methanol. An organic layer thus separated was dried over anhydrous sodium sulfate, insoluble materials were filtered, subsequently the solvent was distilled off under reduced pressure, and a crude form of 2-(4-(1,4-dioxa-8-azaspiro[4.5]decan-8-yl)phenyl)acetic acid (white powder, 28 mg) was obtained. According to a technique similar to Example 1, the title compound (white powder, 18 mg, 70%) was obtained using the crude form of 2-(4-(1,4-dioxa-8-azaspiro[4.5]decan-8-yl)phenyl)acetic acid (28 mg) thus obtained and (R)-1-(5-(trifluoromethyl)pyridin-3-yl)pyrrolidin-3-amine (28 mg, 0.0524 mmol) synthesized in Reference Example 1-2.

¹H NMR (CDCl₃, 400 MHz): δ = 1.8 - 2.0 (m, 5H), 2.2 - 2.4 (m, 1H), 3.11 (dd, 1H, J = 5, 10 Hz), 3.3 - 3.4 (m, 6H), 3.50 (s, 2H), 3.65 (dd, 1H, J = 6, 10 Hz), 4.00 (s, 4H), 4.5 - 4.7 (m, 1H), 5.50 (d, 1H, J = 7 Hz), 6.8 - 7.0 (m, 3H), 7.0 - 7.2 (m, 2H), 8.08 (d, 1H, J = 3 Hz), 8.20 (s, 1H).

MS: 491.18 [M+H]⁺

### Example 102

(R)-2-(4-(4-oxopiperidin-1-yl)phenyl)-N-(1-(5-(trifluoromethyl)pyridin-3-yl)pyrrolidin-3-yl)acetamide

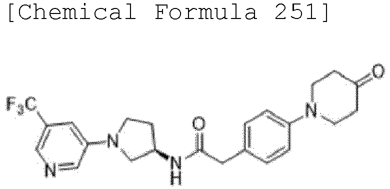

(R)-2-(4-(1,4-dioxa-8-azaspiro[4.5]decan-8-yl)phenyl)-N-(1-(5-(trifluoromethyl)pyridin-3-yl)pyrrolidin-3-yl)acetamide (17 mg, 0.032 mmol) synthesized in Example 101 was dissolved in ethanol (1.0 mL), 2N hydrochloric acid (1.0 mL) was added thereto, and the mixture was heated to reflux overnight in a nitrogen atmosphere. To the reaction liquid that had been left to cool to room temperature, a saturated aqueous solution of sodium hydrogen carbonate was added to neutralize the reaction liquid, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (ethyl acetate: methanol) (concentration gradient: 0 to 5%), and the title compound (white powder, 2 mg, 14%) was obtained.

¹H NMR (CDCl₃, 400 MHz): δ = 1.8 - 2.0 (m, 1H), 2.3 - 2.4 (m, 1H), 2.56 (t, 4H, 6 Hz), 3.14 (dd, 1H, J = 5, 10 Hz), 3.3 - 3.5 (m, 2H), 3.52 (s, 2H), 3.60 (t, 4H, J = 6 Hz), 3.66 (dd, 1H, J = 6, 10 Hz), 4.5 - 4.7 (m, 1H), 5.52 (d, 1H, J = 6 Hz), 6.8 - 7.0 (m, 3H), 7.16 (d, 2H, J = 9 Hz), 8.09 (d, 1H, J = 3 Hz), 8.21 (s, 1H).

MS: 469.13 [M+Na]⁺

### Example 103

2-(4-cyclopropylphenyl)-N-(3-(hydroxymethyl)-1-(5-(trifluoromethyl)pyridin-3-yl)pyrrolidin-3-yl)acetamide

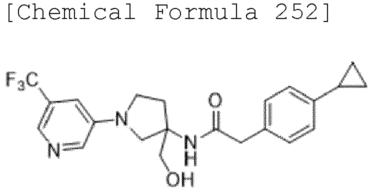

(3-Amino-1-(5-(trifluoromethyl)pyridin-3-yl)pyrrolidin-3-yl)methanol (51 mg, 0.195 mmol) synthesized in Reference Example 61-2 and 2-(4-cyclopropylphenyl)acetic acid (34 mg, 0.195 mmol) synthesized in Reference Example 33-2 were dissolved in ethanol (3 mL), subsequently DMT-MM (81 mg, 0.293 mmol) was added thereto, and the mixture was stirred overnight at room temperature. Water was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (ethyl acetate: methanol) (concentration gradient: 0 to 5%), and the title compound (white powder, 50 mg, 61%) was obtained.

¹H NMR (CDCl₃, 400 MHz): δ = 0.6 - 0.7 (m, 2H), 0.9 - 1.0 (m, 2H), 1.8 - 1.9 (m, 1H), 2.1 - 2.3 (m, 2H), 3.2 - 3.5 (m, 3H), 3.54 (s, 2H), 3.59 (d, 1H, J = 11 Hz), 3·82 (d, 2H, J = 6 Hz), 4.03 (t, 1H, J = 6 Hz), 5.62 (s, 1H), 6.8 - 7.1 (m, 5H), 8.06 (d, 1H, J = 3 Hz), 8.22 (s, 1H).

MS: 420.14 [M+H]⁺

### Example 104

(3-(2-(4-cyclopropylphenyl)acetamido)-1-(5-(trifluoromethyl)pyridin-3-yl)pyrrolidin-3-yl)methyl acetate

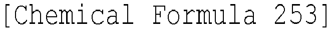

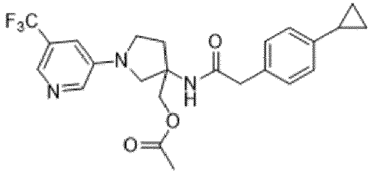

According to a technique similar to Example 95, the title compound (colorless oily material, 10 mg, 91%) was obtained using 2-(4-cyclopropylphenyl)-N-(3-(hydroxymethyl)-1-(5-(trifluoromethyl)pyridin-3-yl)pyrrolidin-3-yl)acetamide (10 mg, 0.0238 mmol) synthesized in Example 103.

¹H NMR (CDCl₃, 400 MHz): δ = 0.6 - 0.7 (m, 2H), 0.9 - 1.0 (m, 2H), 1.8 - 1.9 (m, 1H), 2.02 (s, 3H), 2.1 - 2.4 (m, 2H), 3.3 - 3.5 (m, 2H), 3.51 (s, 2H), 3.55 (d, 1H, J = 11 Hz), 3.63 (d, 1H, J = 11 Hz), 4.35 (dd, 2H, J = 12, 16 Hz), 5.49 (s, 1H), 6.8 - 7.2 (m, 5H), 8.08 (d, 1H, J = 2 Hz), 8.22 (s, 1H).

MS: 484.14 [M+Na]⁺

### Example 105

2-(4-cyclopropylphenyl)-N-(3-(methoxymethyl)-1-(5-(trifluoromethyl)pyridin-3-yl)pyrrolidin-3-yl)acetamide

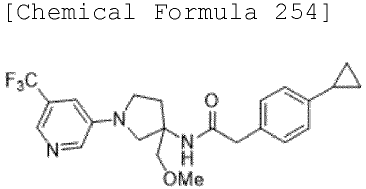

2-(4-cyclopropylphenyl)-N-(3-(hydroxymethyl)-1-(5-(trifluoromethyl)pyridin-3-yl)pyrrolidin-3-yl)acetamide (37 mg, 0.0882 mmol) synthesized in Example 103 was dissolved in THF (1 mL), subsequently 60% sodium hydride (4 mg, 0.0926 mmol) and iodomethane (6 µL, 0.0926 mmol) were added thereto under ice cooling, and the mixture was stirred overnight at room temperature. A saturated aqueous solution of sodium hydrogen carbonate was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 60 to 80%), and the title compound (white powder, 16 mg, 42%) was obtained.

¹H NMR (CDCl₃, 400 MHz): δ = 0.6 - 0.7 (m, 2H), 0.9 - 1.0 (m, 2H), 1.8 - 1.9 (m, 1H), 2.1 - 2.5 (m, 2H), 3.33 (s, 3H), 3.39 (t, 2H, J = 7 Hz), 3.4 - 3.7 (m, 6H), 5.58 (s, 1H), 6.8 - 7.0 (m, 1H), 7.03 (d, 2H, J = 8 Hz), 7.11 (d, 2H, J = 8 Hz), 8.08 (d, 1H, J = 3 Hz), 8.20 (s, 1H).

MS: 434.15 [M+Na]⁺

### Example 106

Trans-2-(4-cyclopropylphenyl)-N-((R)-1-(5-(trifluoromethyl)pyridin-3-yl)pyrrolidin-3-yl)cyclopropane-1-carboxyamide

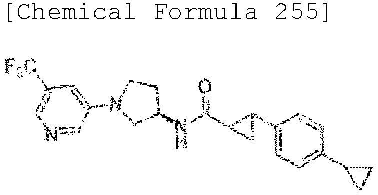

According to a technique similar to Example 1, diastereomer A (upper spot in TLC (ethyl acetate), white powder, 37 mg, 33%) and diastereomer B (lower spot in TLC (ethyl acetate), white powder, 35 mg, 31%) of the title compound were obtained using (R)-1-(5-(trifluoromethyl)pyridin-3-yl)pyrrolidin-3-amine (62 mg, 0.218 mmol) synthesized in Reference Example 1-2 and trans-2-(4-cyclopropylphenyl)cyclopropane-1-carboxylic acid (59 mg, 0.294 mmol).

### Diastereomer A

¹H NMR (CDCl₃, 400 MHz): δ = 0.6 - 0.7 (m, 2H), 0.9 - 1.0 (m, 2H), 1.2 - 1.3 (m, 1H), 1.5 - 1.7 (m, 2H), 1.8 - 1.9 (m, 1H), 2.0 - 2.1 (m, 1H), 2.3 - 2.5 (m, 2H), 3.26 (dd, 1H, J = 4, 10 Hz), 3.3 - 3.6 (m, 2H), 3.68 (dd, 1H, J = 6, 10 Hz), 4.6 - 4.8 (m, 1H), 5.78 (d, 1H, J = 7 Hz), 6.9 - 7.0 (m, 5H), 8.13 (d, 1H, J = 3 Hz), 8.21 (s, 1H).

MS: 416.14 [M+H]⁺

Diastereomer B

¹H NMR (CDCl₃, 400 MHz): δ = 0.6 - 0.7 (m, 2H), 0.9 - 1.0 (m, 2H), 1.2 - 1.3 (m, 1H), 1.5 - 1.7 (m, 2H), 1.8 - 1.9 (m, 1H), 2.0 - 2.1 (m, 1H), 2.3 - 2.5 (m, 2H), 3.24 (dd, 1H, J = 4, 10 Hz), 3.3 - 3.6 (m, 2H), 3.68 (dd, 1H, J = 6, 10 Hz), 4.6 - 4.8 (m, 1H), 5.78 (d, 1H, J = 7 Hz), 6.9 - 7.0 (m, 5H), 8.12 (d, 1H, J = 2 Hz), 8.21 (s, 1H).

MS: 416.14 [M+H]⁺

### Example 107

Trans-2-(3,4-difluorophenyl)-N-((R)-1-(5-(trifluoromethyl)pyridin-3-yl)pyrrolidin-3-yl)cyclopropane-1-carboxyamide

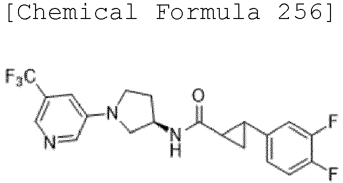

According to a technique similar to Example 1, diastereomer A (upper spot in TLC (ethyl acetate), white powder, 42 mg, 38%) and diastereomer B (lower spot in TLC (ethyl acetate), white powder, 36 mg, 33%) of the title compound were obtained using (R)-1-(5-(trifluoromethyl)pyridin-3-yl)pyrrolidin-3-amine (62 mg, 0.218 mmol) synthesized in Reference Example 1-2 and trans-2-(3,4-difluorophenyl)cyclopropane-1-carboxylic acid (58 mg, 0.294 mmol).

### Diastereomer A

¹H NMR (CDCl₃, 400 MHz): δ = 1.1 - 1.3 (m, 1H), 1.5 - 1.7 (m, 2H), 2.0 - 2.2 (m, 1H), 2.3 - 2.6 (m, 2H), 3.27 (dd, 1H, J = 4, 10 Hz), 3.4 - 3.6 (m, 2H), 3.69 (dd, 1H, J = 6, 10 Hz), 4.6 - 4.8 (m, 1H), 5.90 (d, 1H, J = 7 Hz), 6.8 - 7.1 (m, 4H), 8.12 (d, 1H, J = 3 Hz), 8.21 (s, 1H).

MS: 412.10 [M+H]⁺

### Diastereomer B

¹H NMR (CDCl₃, 400 MHz): δ = 1.1 - 1.3 (m, 1H), 1.5 - 1.7 (m, 2H), 2.0 - 2.2 (m, 1H), 2.3 - 2.6 (m, 2H), 3.27 (dd, 1H, J = 4, 10 Hz), 3.4 - 3.6 (m, 2H), 3.68 (dd, 1H, J = 6, 10 Hz), 4.6 - 4.8 (m, 1H), 5.87 (d, 1H, J = 7 Hz), 6.7 - 7.1 (m, 4H), 8.12 (d, 1H, J = 2 Hz), 8.21 (s, 1H).

MS: 412.10 [M+H]⁺

### Example 108

Trans-2-(4-chlorophenyl)-N-((R)-1-(5-(trifluoromethyl)pyridin-3-yl)pyrrolidin-3-yl)cyclopropane-1-carboxyamide

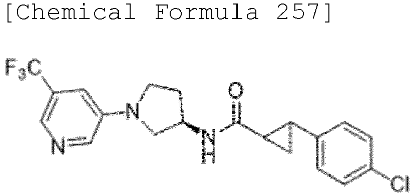

According to a technique similar to Example 1, diastereomer A (upper spot in TLC (ethyl acetate), white powder, 39 mg, 35%) and diastereomer B (lower spot in TLC (ethyl acetate), white powder, 33 mg, 30%) of the title compound were obtained using (R)-1-(5-(trifluoromethyl)pyridin-3-yl)pyrrolidin-3-amine (62 mg, 0.218 mmol) synthesized in Reference Example 1-2 and trans-2-(4-chlorophenyl)cyclopropane-1-carboxylic acid (58 mg, 0.294 mmol).

### Diastereomer A

¹H NMR (CDCl₃, 400 MHz): δ = 1.2 - 1.3 (m, 1H), 1.5 - 1.7 (m, 2H), 2.0 - 2.1 (m, 1H), 2.3 - 2.6 (m, 2H), 3.27 (dd, 1H, J = 4, 10 Hz), 3.3 - 3.6 (m, 2H), 3.68 (dd, 1H, J = 6, 10 Hz), 4.6 - 4.8 (m, 1H), 5.90 (d, 1H, J = 7 Hz), 6.9 - 7.1 (m, 3H), 7.2 - 7.3 (m, 2H), 8.12 (d, 1H, J = 3 Hz), 8.21 (s, 1H).

MS: 410.07 [M+H]⁺

### Diastereomer B

¹H NMR (CDCl₃, 400 MHz): δ = 1.2 - 1.3 (m, 1H), 1.5 - 1.7 (m, 2H), 2.0 - 2.2 (m, 1H), 2.3 - 2.6 (m, 2H), 3.26 (dd, 1H, J = 4, 10 Hz), 3.3 - 3.6 (m, 2H), 3.68 (dd, 1H, J = 6, 10 Hz), 4.6 - 4.8 (m, 1H), 5.87 (d, 1H, J = 7 Hz), 6.9 - 7.1 (m, 3H), 7.2 - 7.3 (m, 2H), 8.11 (d, 1H, J = 3 Hz), 8.21 (s, 1H).

MS: 410.07 [M+H]⁺

### Example 109

2-(4-Isopropylphenyl)-N-(1-(5-(trifluoromethyl)pyridin-3-yl)piperidin-4-yl)acetamide

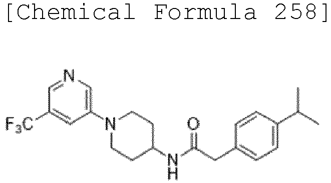

According to a technique similar to Example 9, the title compound (pale yellow crystals, 62 mg, 39%) was obtained using 2-(4-isopropylphenyl)-N-(piperidin-4-yl)acetamide (100 mg, 0.384 mmol) synthesized in Reference Example 62 and 3-bromo-5-(trifluoromethyl)pyridine (104 mg, 0.461 mmol).

¹H NMR (CDCl₃, 400 MHz): δ = 1.1 - 1.5 (m, 8H), 2.02 (d, 2H, J = 11 Hz), 2.8 - 3.1 (m, 3H), 3.5 - 3.7 (m, 4H), 3.9 - 4.1 (m, 1H), 5.32 (d, 1H, J = 6 Hz), 7.1 - 7.3 (m, 5H), 8.29 (s, 1H), 8.41 (s, 1H).

MS: 404.25 [M-H]⁻

### Example 110

(R)-2-(4-isopropylphenyl)-N-(1-(6-methoxy-5-(trifluoromethyl)pyridin-3-yl)pyrrolidin-3-yl)acetamide

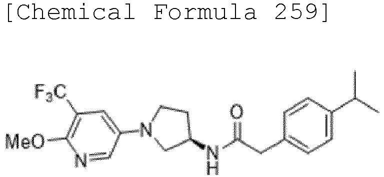

According to a technique similar to Example 9, the title compound (pale yellow crystals, 292 mg, 57%) was obtained using (R)-2-(4-isopropylphenyl)-N-(pyrrolidin-3-yl)acetamide (296 mg, 1.20 mmol) synthesized in Reference Example 63 and 5-bromo-2-methoxy-3-(trifluoromethyl)pyridine (369 mg, 1.44 mmol).

¹H NMR (CDCl₃, 400 MHz): δ = 1.23 (d, 6H, J = 7 Hz), 1.8 - 1.9 (m, 1H), 2.2 - 2.4 (m, 1H), 2.8 - 3.0 (m, 1H), 3.06 (dd, 1H, J = 5, 10 Hz), 3.2 - 3.4 (m, 2H), 3.5 - 3.6 (m, 3H), 3.95 (s, 3H), 4.5 - 4.7 (m, 1H), 5.53 (d, 1H, J = 7 Hz), 7.09 (d, 1H, J = 3 Hz), 7.16 (d, 2H, J = 8 Hz), 7.20 (d, 2H, J = 8 Hz), 7.59 (d, 1H, J = 3 Hz).

MS: 420.26 [M-H]⁻

### Example 111

(R)-2-(4-isopropylphenyl)-N-(1-(5-(trifluoromethyl)pyrimidin-2-yl)pyrrolidin-3-yl)acetamide

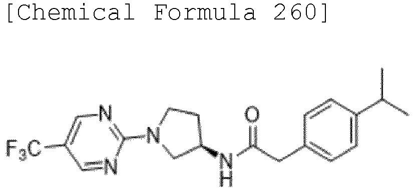

According to a technique similar to Example 27, the title compound (white crystals, 125 mg, 82%) was obtained using (R)-2-(4-isopropylphenyl)-N-(pyrrolidin-3-yl)acetamide (95 mg, 0.386 mmol) synthesized in Reference Example 63 and 2-chloro-5-(trifluoromethyl)pyrimidine (77 mg, 0.424 mmol).

¹H NMR (CDCl₃, 400 MHz): δ = 1.23 (d, 6H, J = 7 Hz), 1.8 - 2.0 (m, 1H), 2.2 - 2.4 (m, 1H), 2.8 - 3.0 (m, 1H), 3.36 (dd, 1H, J = 5, 12 Hz), 3.55 (s, 2H), 3.63 (t, 2H, J = 7 Hz), 3.88 (dd, 1H, J = 6, 12 Hz), 4.5 - 4.7 (m, 1H), 5.50 (d, 1H, J = 6 Hz), 7.15 (d, 2H, J = 8 Hz), 7.19 (d, 2H, J = 8 Hz), 8.48 (s, 2H).

### MS: 391.23 [M-H]⁻

### Example 112

(R)-2-(4-isopropylphenyl)-N-(1-(4-(trifluoromethyl)thiazol-2-yl)pyrrolidin-3-yl)acetamide

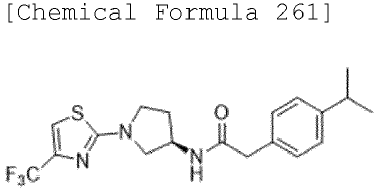

According to a technique similar to Example 9, the title compound (pale yellow crystals, 77 mg, 47%) was obtained using (R)-2-(4-isopropylphenyl)-N-(pyrrolidin-3-yl)acetamide (100 mg, 0.406 mmol) synthesized in Reference Example 63 and 2-bromo-4-(trifluoromethyl)thiazole (113 mg, 0.487 mmol).

¹H NMR (CDCl₃, 400 MHz): δ = 1.24 (d, 6H, J = 7 Hz), 1.8 - 2.0 (m, 1H), 2.2 - 2.4 (m, 1H), 2.8 - 3.0 (m, 1H), 3.24 (dd, 1H, J = 5, 11 Hz), 3.4 - 3.6 (m, 4H), 3.75 (dd, 1H, J = 6, 11 Hz), 4.5 - 4.7 (m, 1H), 5.52 (d, 1H, J = 6 Hz), 6.92 (s, 1H), 7.14 (d, 2H, J = 8 Hz), 7.21 (d, 2H, J = 8 Hz).

MS: 396.19 [M-H]⁻

### Example 113

(R)-2-(4-isopropylphenyl)-N-(1-(6-(trifluoromethyl)pyridin-3-yl)pyrrolidin-3-yl)acetamide

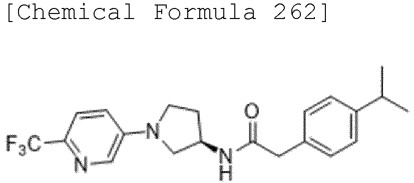

According to a technique similar to Example 9, the title compound (white crystals, 93 mg, 58%) was obtained using (R)-2-(4-isopropylphenyl)-N-(pyrrolidin-3-yl)acetamide (100 mg, 0.406 mmol) synthesized in Reference Example 63 and 5-bromo-2-(trifluoromethyl)pyridine (110 mg, 0.487 mmol).

¹H NMR (CDCl₃, 400 MHz): δ = 1.23 (d, 6H, J = 7 Hz), 1.8 - 2.0 (m, 1H), 2.2 - 2.4 (m, 1H), 2.8 - 3.0 (m, 1H), 3.13 (dd, 1H, J = 5, 10 Hz), 3.38 (t, 2H, J = 7 Hz), 3.55 (s, 2H), 3.66 (dd, 1H, J = 6, 10 Hz), 4.5 - 4.7 (m, 1H), 5.57 (d, 1H, J = 6 Hz), 6.78 (dd, 1H, J = 2, 9 Hz), 7.16 (d, 2H, J = 8 Hz), 7.20 (d, 2H, J = 8 Hz), 7.46 (d, 1H, J = 9 Hz), 7.95 (d, 1H, J = 2 Hz).

MS: 390.23 [M-H]⁻

### Example 114

(S)-2-(4-isopropylphenyl)-N-(1-(5-(trifluoromethyl)pyridin-3-yl)pyrrolidin-3-yl)acetamide

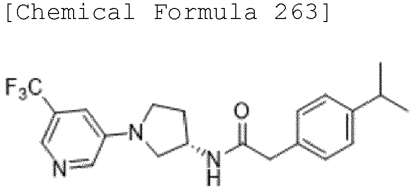

According to a technique similar to Example 1, the title compound (white crystals, 144 mg, 86%) was obtained using (S)-1-(5-(trifluoromethyl)pyridin-3-yl)pyrrolidin-3-amine (99 mg, 0.428 mmol) synthesized in Reference Example 64-2 and 2-(4-isopropylphenyl)acetic acid (92 mg, 0.514 mmol) .

¹H NMR (CDCl₃, 400 MHz): δ = 1.23 (d, 6H, J = 7 Hz), 1.8 - 2.0 (m, 1H), 2.2 - 2.4 (m, 1H), 2.8 - 3.0 (m, 1H), 3.12 (dd, 1H, J = 5, 10 Hz), 3.36 (t, 2H, J = 7 Hz), 3.55 (s, 2H), 3.65 (dd, 1H, J = 6, 10 Hz), 4.5 - 4.7 (m, 1H), 5.61 (d, 1H, J = 6 Hz), 6.89 (s, 1H), 7.16 (d, 2H, J = 8 Hz), 7.20 (d, 2H, J = 8 Hz), 8.06 (d, 1H, J = 3 Hz), 8.19 (s, 1H).

MS: 390.19 [M-H]⁻

### Example 115

(R)-2-(4-(trifluoromethoxy)phenyl)-N-(1-(5-(trifluoromethyl)pyridin-3-yl)pyrrolidin-3-yl)acetamide

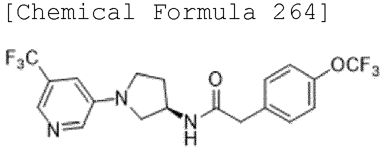

According to a technique similar to Example 1, the title compound (white crystals, 9 mg, 46%) was obtained using (R)-1-(5-(trifluoromethyl)pyridin-3-yl)pyrrolidin-3-amine (10 mg, 0.0432 mmol) synthesized in Reference Example 1-2 and 2-(4-(trifluoromethoxy)phenyl)acetic acid (11 mg, 0.0519 mmol).

¹H NMR (CDCl₃, 400 MHz): δ = 1.9 - 2.1 (m, 1H), 2.3 - 2.4 (m, 1H), 3.18 (dd, 1H, J = 4, 10 Hz), 3.3 - 3.5 (m, 2H), 3.57 (s, 2H), 3.67 (dd, 1H, J = 7, 10 Hz), 4.6 - 4.7 (m, 1H), 5.65 (d, 1H, J = 5 Hz), 6.91 (s, 1H), 7.1 - 7.4 (m, 4H), 8.09 (s, 1H), 8.21 (s, 1H).

MS: 432.14 [M-H]⁻

### Example 116

(R)-2-(4-(2,2,2-trifluoroethoxy)phenyl)-N-(1-(5-(trifluoromethyl)pyridin-3-yl)pyrrolidin-3-yl)acetamide

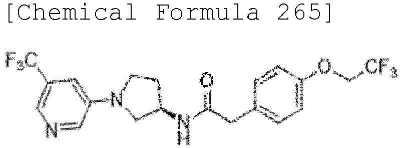

According to a technique similar to Example 1, the title compound (white crystals, 21 mg, 99%) was obtained using (R)-1-(5-(trifluoromethyl)pyridin-3-yl)pyrrolidin-3-amine (10 mg, 0.0432 mmol) synthesized in Reference Example 1-2 and 2-(4-(2,2,2-trifluoroethoxy)phenyl)acetic acid (12 mg, 0.0519 mmol).

¹H NMR (CDCl₃, 400 MHz): δ = 1.8 - 2.0 (m, 1H), 2.2 - 2.4 (m, 1H), 3.14 (dd, 1H, J = 5, 10 Hz), 3.3 - 3.5 (m, 2H), 3.54 (s, 2H), 3.65 (dd, 1H, J = 6, 10 Hz), 4.34 (q, 2H, J = 8 Hz), 4.5 - 4.7 (m, 1H), 5.59 (d, 1H, J = 6 Hz), 6.8 - 7.0 (m, 3H), 7.21 (d, 2H, J = 9 Hz), 8.07 (d, 1H, J = 2 Hz), 8.20 (s, 1H).

MS: 446.14 [M-H]⁻

### Example 117

2-(4-Isopropylphenyl)-N-(1-(5-(trifluoromethyl)pyridin-3-yl)azetidin-3-yl)acetamide

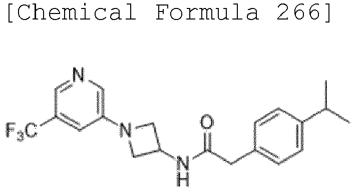

According to a technique similar to Example 36, a crude form of N-(azetidin-3-yl)-2-(4-isopropylphenyl)acetamide (600 mg) was synthesized from tert-butyl 3-(2-(4-isopropylphenyl)acetamido)azetidine-1-carboxylate (964 mg, 2.90 mmol) synthesized in Reference Example 65, and the title compound (white crystals, 57 mg, 43%) was obtained using a portion of the crude form thus obtained (100 mg) and 3-bromo-5-(trifluoromethyl)pyridine (118 mg, 0.52 mmol).

¹H NMR (DMSO - d₆, 400 MHz) : δ = 1.18 (d, 6H, J = 7 Hz), 2.7 - 2.9 (m, 1H), 3.38 (s, 2H), 3.7 - 3.8 (m, 2H), 4.2 - 4.3 (m, 2H), 4.5 - 4.7 (m, 1H), 7.1 - 7.2 (m, 5H), 8.10 (d, 1H, J = 2 Hz), 8.23 (s, 1H), 8.7 - 8.8 (m, 1H).

MS: 378.21 [M+H]⁺

### Example 118

2-(4-Isopropylphenyl)-N-(1-(6-(trifluoromethyl)pyridin-3-yl)azetidin-3-yl)acetamide

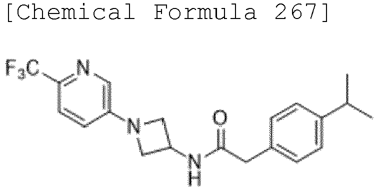

According to a technique similar to Example 36, the title compound (white crystals, 55 mg, 34%) was obtained using the crude form of N-(azetidin-3-yl)-2-(4-isopropylphenyl)acetamide (100 mg) synthesized from tert-butyl 3-(2-(4-isopropylphenyl)acetamido)azetidine-1-carboxylate in Example 117, and 5-bromo-2-(trifluoromethyl)pyridine (118 mg, 0.52 mmol).

¹H NMR (DMSO - d₆, 400 MHz) : δ = 1.17 (d, 6H, J = 7 Hz), 2.7 - 2.9 (m, 1H), 3.38 (s, 2H), 3.7 - 3.9 (m, 2H), 4.2 - 4.3 (m, 2H), 4.5 - 4.7 (m, 1H), 6.95 (dd, 1H, J = 3, 8 Hz), 7.17 (s, 4H), 7.61 (d, 1H, J = 8 Hz), 7.93 (d, 1H, J = 3 Hz), 8.7 - 8.8 (m, 1H).

MS: 378.21 [M+H]⁺

### Example 119

(R)-2-(3,5-dichlorophenyl)-N-(1-(5-(trifluoromethyl)pyridin-3-yl)pyrrolidin-3-yl)acetamide

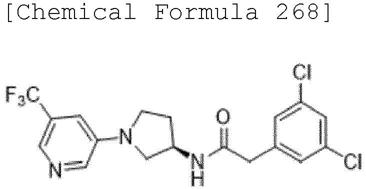

According to a technique similar to Example 1, the title compound (white crystals, 83 mg, 76%) was obtained using (R)-1-(5-(trifluoromethyl)pyridin-3-yl)pyrrolidin-3-amine (60 mg, 0.26 mmol) synthesized in Reference Example 1-2 and 2-(3,5-dichlorophenyl)acetic acid (64 mg, 0.31 mmol) .

¹H NMR (DMSO - d₆, 400 MHz): δ = 1.8 - 2.0 (m, 1H), 2.1 - 2.3 (m, 1H), 3.1 - 3.7 (m, 4H), 3.46 (s, 2H), 4.3 - 4.5 (m, 1H), 7.11 (s, 1H), 7.30 (d, 2H, J = 2 Hz), 7.47 (t, 1H, J = 2 Hz), 8.14 (s, 1H), 8.20 (d, 1H, J = 2 Hz), 8.4 - 8.5 (m, 1H).

MS: 416.13 [M-H]⁻

### Example 120

(R)-2-(3-chloro-5-fluorophenyl)-N-(1-(5-(trifluoromethyl)pyridin-3-yl)pyrrolidin-3-yl)acetamide

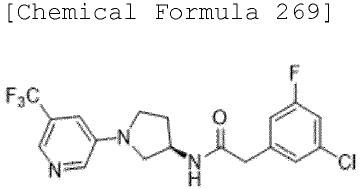

According to a technique similar to Example 1, the title compound (white crystals, 70 mg, 67%) was obtained using (R)-1-(5-(trifluoromethyl)pyridin-3-yl)pyrrolidin-3-amine (60 mg, 0.26 mmol) synthesized in Reference Example 1-2 and 2-(3-chloro-5-fluorophenyl)acetic acid (58 mg, 0.31 mmol) .

¹H NMR (DMSO - d₆, 400 MHz): δ = 1.8 - 2.0 (m, 1H), 2.1 - 2.3 (m, 1H), 3.1 - 3.2 (m, 1H), 3.2 - 3.6 (m, 3H), 3.45 (s, 2H), 4.3 - 4.5 (m, 1H), 7.0 - 7.1 (m, 2H), 7.17 (s, 1H), 7.28 (dt, 1H, J = 3, 8 Hz), 8.13 (s, 1H), 8.19 (d, 1H, J = 3 Hz), 8.4 - 8.5 (m, 1H).

MS: 400.16 [M-H]⁻

### Example 121

(R)-2-(4-cyclopropylphenyl)-N-(1-(6-(trifluoromethyl)pyridin-3-yl)pyrrolidin-3-yl)acetamide

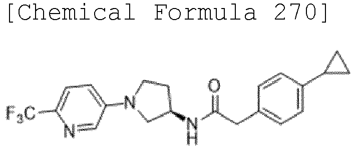

According to a technique similar to Example 1, the title compound (white crystals, 89 mg, 100%) was obtained using (R)-1-(6-(trifluoromethyl)pyridin-3-yl)pyrrolidin-3-amine (60 mg, 0.18 mmol) synthesized in Reference Example 32-2 and 2-(4-cyclopropylphenyl)acetic acid (51 mg, 0.29 mmol) synthesized in Reference Example 33-2.

¹H NMR (CDCl₃, 400 MHz): δ = 0.6 - 0.7 (m, 2H), 0.9 - 1.0 (m, 2H), 1.8 - 2.0 (m, 2H), 2.2 - 2.4 (m, 1H), 3.0 - 3.2 (m, 1H), 3.3 - 3.4 (m, 2H), 3.53 (s, 2H), 3.6 - 3.7 (m, 1H), 4.5 - 4.7 (m, 1H), 5.5 - 5.7 (m, 1H), 6.77 (dd, 1H, J = 3, 8 Hz), 7.03 (d, 2H, J = 8 Hz), 7.11 (d, 2H, J = 8 Hz), 7.46 (d, 1H, J = 8 Hz), 7.94 (d, 1H, J = 3 Hz).

MS: 388.22 [M-H]⁻

### Example 122

(R)-2-(4-isopropylphenyl)-N-(1-(5-(trifluoromethyl)thiophen-2-yl)pyrrolidin-3-yl)acetamide

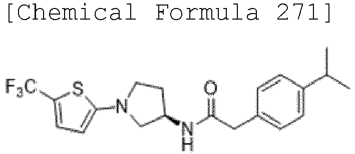

According to a technique similar to Example 9, the title compound (white crystals, 49 mg, 30%) was obtained using (R)-2-(4-isopropylphenyl)-N-(pyrrolidin-3-yl)acetamide (100 mg, 0.41 mmol) synthesized in Reference Example 63 and 2-bromo-5-(trifluoromethyl)thiophene (114 mg, 0.49 mmol).

¹H NMR (CDCl₃, 400 MHz): δ = 1.23 (d, 6H, J = 7 Hz), 1.8 - 2.0 (m, 1H), 2.2 - 2.4 (m, 1H), 2.8 - 3.0 (m, 1H), 3.0 - 3.1 (m, 1H), 3.2 - 3.4 (m, 2H), 3.54 (s, 2H), 3.5 - 3.6 (m, 1H), 4.5 - 4.7 (m, 1H), 5.4 - 5.6 (m, 1H), 5.63 (d, 1H, J = 4 Hz), 7.0 - 7.2 (m, 1H), 7.14 (d, 2H, J = 8 Hz), 7.20 (d, 2H, J = 8 Hz).

MS: 395.21 [M-H]⁻

### Example 123

(R)-2-(4-(trifluoromethyl)phenyl)-N-(1-(5-(trifluoromethyl)pyridin-3-yl)pyrrolidin-3-yl)acetamide

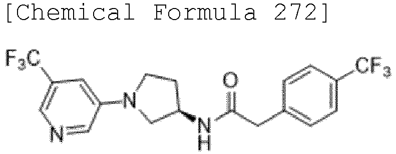

According to a technique similar to Example 1, the title compound (white crystals, 85 mg, 92%) was obtained using (R)-1-(5-(trifluoromethyl)pyridin-3-yl)pyrrolidin-3-amine (50 mg, 0.22 mmol) synthesized in Reference Example 1-2 and 2-(4-(trifluoromethyl)phenyl)acetic acid (53 mg, 0.26 mmol).

¹H NMR (CDCl₃, 400 MHz): δ = 1.9 - 2.1 (m, 1H), 2.2 - 2.4 (m, 1H), 3.1 - 3.3 (m, 1H), 3.3 - 3.4 (m, 2H), 3.5 - 3.7 (m, 1H), 3.63 (s, 2H), 4.6 - 4.7 (m, 1H), 6.4 - 6.6 (m, 1H), 6.81 (s, 1H), 7.40 (d, 2H, J = 8 Hz), 7.59 (d, 2H, J = 8 Hz), 7.95 (d, 1H, J = 2 Hz), 8.09 (s, 1H).

MS: 416.19 [M-H]⁻

### Example 124

(R)-2-(1H-indol-6-yl)-N-(1-(5-(trifluoromethyl)pyridin-3-yl)pyrrolidin-3-yl)acetamide

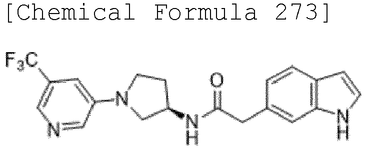

According to a technique similar to Example 1, the title compound (white crystals, 89 mg, 100%) was obtained using (R)-1-(5-(trifluoromethyl)pyridin-3-yl)pyrrolidin-3-amine (50 mg, 0.22 mmol) synthesized in Reference Example 1-2 and 2-(1H-indol-6-yl)acetic acid (53 mg, 0.26 mmol).

¹H NMR (CDCl₃, 400 MHz): δ = 0.6 - 0.7 (m, 2H), 0.9 - 1.0 (m, 2H), 1.7 - 2.0 (m, 2H), 2.2 - 2.4 (m, 1H), 2.9 - 3.1 (m, 1H), 3.1 - 3.4 (m, 2H), 3.4 - 3.6 (m, 1H), 3.52 (s, 2H), 4.5 - 4.7 (m, 1H), 4.67 (q, 2H, J = 9 Hz), 5.5 - 5.6 (m, 1H), 6.75 (d, 1H, J = 9 Hz), 6.92 (dd.1H, J = 3, 9 Hz), 7.03 (d, 2H, J = 8 Hz), 7.11 (d, 2H, J = 8 Hz), 7.39 (d, 1H, J = 3 Hz).

MS: 387.22 [M-H]⁻

### Example 125

(R)-2-(4-cyclopropylphenyl)-N-(1-(6-(2,2,2-trifluoroethoxy)pyridin-3-yl)pyrrolidin-3-yl)acetamide

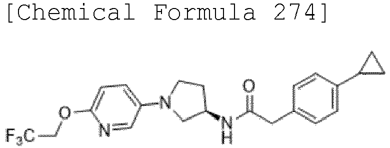

According to a technique similar to Example 9, the title compound (yellow amorphous, 40 mg, 45%) was obtained using (R)-2-(4-cyclopropylphenyl)-N-(pyrrolidin-3-yl)acetamide (50 mg, 0.20 mmol) synthesized in Reference Example 74-2 and 5-bromo-2-(2,2,2-trifluoroethoxy)pyridine (61 mg, 0.24 mmol).

¹H NMR (CDCl₃, 400 MHz): δ = δ = 0.6 - 0.7 (m, 2H), 0.9 - 1.0 (m, 2H), 1.7 - 2.0 (m, 2H), 2.2 - 2.4 (m, 1H), 2.9 - 3.1 (m, 1H), 3.1 - 3.4 (m, 2H), 3.4 - 3.6 (m, 1H), 3.52 (s, 2H), 4.5 - 4.7 (m, 1H), 4.67 (q, 2H, J = 9 Hz), 5.5 - 5.6 (m, 1H), 6.75 (d, 1H, J = 9 Hz), 6.92 (dd, 1H, J = 3, 9 Hz), 7.03 (d, 2H, J = 8 Hz), 7.11 (d, 2H, J = 8 Hz), 7.39 (d, 1H, J = 3 Hz).

MS: 420.16 [M+H]⁺

### Example 126

(R)-2-(4-cyclopropylphenyl)-N-(1-(5-(2,2,2-trifluoroethoxy)pyridin-3-yl)pyrrolidin-3-yl)acetamide

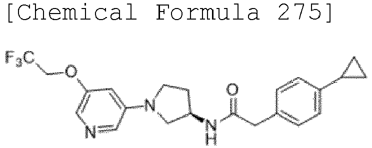

According to a technique similar to Example 9, the title compound (yellow crystals, 36 mg, 40%) was obtained using (R)-2-(4-cyclopropylphenyl)-N-(pyrrolidin-3-yl)acetamide (50 mg, 0.20 mmol) synthesized in Reference Example 74-2 and 3-bromo-5-(2,2,2-trifluoroethoxy)pyridine (61 mg, 0.24 mmol).

¹H NMR (CDCl₃, 400 MHz): δ = 0.6 - 0.7 (m, 2H), 0.9 - 1.0 (m, 2H), 1.8 - 2.0 (m, 2H), 2.2 - 2.4 (m, 1H), 3.0 - 3.1 (m, 1H), 3.31 (t, 2H, J = 7 Hz), 3.53 (s, 2H), 3.5 - 3.7 (m, 1H), 4.37 (q, 2H, J = 8 Hz), 4.5 - 4.7 (m, 1H), 5.5 - 5.7 (m, 1H), 6.33 (t, 1H, J = 2 Hz), 7.03 (d, 2H, J = 8 Hz), 7.11 (d, 2H, J = 8 Hz), 7.6 - 7.7 (m, 2H).

MS: 420.16 [M+H]⁺

### Example 127

(R)-2-(4-cyclopropylphenyl)-N-(1-(4-(2,2,2-trifluoroethoxy)pyridin-2-yl)pyrrolidin-3-yl)acetamide

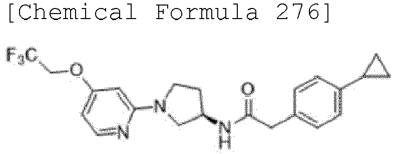

According to a technique similar to Example 27, the title compound (yellow amorphous, 7 mg, 6%) was obtained using (R)-2-(4-cyclopropylphenyl)-N-(pyrrolidin-3-yl)acetamide (50 mg, 0.20 mmol) synthesized in Reference Example 74-2 and 2-chloro-4-(2,2,2-trifluoroethoxy)pyridine (51 mg, 0.24 mmol).

¹H NMR (CDCl₃, 400 MHz): δ = 0.6 - 0.7 (m, 2H), 0.9 - 1.0 (m, 2H), 1.8 - 1.9 (m, 2H), 2.2 - 2.3 (m, 1H), 3.1 - 3.3 (m, 1H), 3.4 - 3.5 (m, 2H), 3.52 (s, 2H), 3.6 - 3.8 (m, 1H), 4.35 (q, 2H, J = 8 Hz), 4.5 - 4.6 (m, 1H), 5.4 - 5.6 (m, 1H), 5.79 (d, 1H, J = 2 Hz), 6.20 (dd, 1H, J = 2, 6 Hz), 7.03 (d, 2H, J = 8 Hz), 7.10 (d, 2H, J = 8 Hz), 8.02 (d, 1H, J = 6 Hz).

MS: 420.16 [M+H]⁺

### Example 128

(R)-2-(4-cyclopropylphenyl)-N-(1-(2-methoxy-5-(trifluoromethyl)pyridin-3-yl)pyrrolidin-3-yl)acetamide

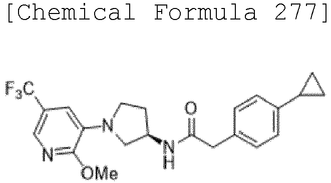

According to a technique similar to Example 9, the title compound (white crystals, 46 mg, 27%) was obtained using (R)-2-(4-cyclopropylphenyl)-N-(pyrrolidin-3-yl)acetamide (100 mg, 0.40 mmol) synthesized in Reference Example 74-2 and 3-bromo-2-methoxy-5-(trifluoromethyl)pyridine (123 mg, 0.48 mmol).

¹H NMR (CDCl₃, 400 MHz): δ = 0.6 - 0.7 (m, 2H), 0.9 - 1.0 (m, 2H), 1.7 - 2.0 (m, 2H), 2.2 - 2.3 (m, 1H), 3.2 - 3.3 (m, 2H), 3.4 - 3.5 (m, 1H), 3.52 (s, 2H), 3.5 - 3.6 (m, 1H), 3.96 (s, 3H), 4.4 - 4.6 (m, 1H), 5.5 - 5.6 (m, 1H), 6.81 (d, 1H, J = 2 Hz), 7.03 (d, 2H, J = 8 Hz), 7.11 (d, 2H, J = 8 Hz), 7.86 (d, 1H, J = 2 Hz).

MS: 420.16 [M+H]⁺

### Example 129

(R)-2-(4-cyclopropylphenyl)-N-(1-(5-(trifluoromethyl)pyridin-3-yl)piperidin-3-yl)acetamide

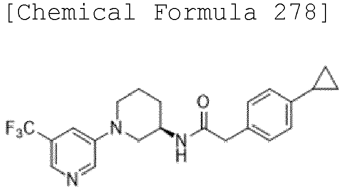

According to a technique similar to Example 3, a crude form of (R)-1-(5-(trifluoromethyl)pyridin-3-yl)piperidin-3-amine (70 mg) was synthesized from tert-butyl (R)-(1-(5-(trifluoromethyl)pyridin-3-yl)piperidin-3-yl)carbamate (100 mg, 0.29 mmol) synthesized in Reference Example 66, and the title compound (white crystals, 80 mg, 58%) was obtained using the crude form of (R)-1-(5-(trifluoromethyl)pyridin-3-yl)piperidin-3-amine thus obtained (70 mg) and 2-(4-cyclopropylphenyl)acetic acid (78 mg, 0.44 mmol) synthesized in Reference Example 33-2.

¹H NMR (DMSO - d₆, 400 MHz): δ = 0.5 - 0.7 (m, 2H), 0.8 - 1.0 (m, 2H), 1.4 - 1.6 (m, 2H), 1.7 - 2.0 (m, 3H), 2.7 - 2.9 (m, 1H), 2.9 - 3.1 (m, 1H), 3.2 - 3.5 (m, 2H), 3.6 - 3.8 (m, 3H), 6.97 (d, 2H, J = 8 Hz), 7.12 (d, 2H, J = 8 Hz), 7.52 (s, 1H), 8.0 - 8.2 (m, 1H), 8.24 (s, 1H), 8.54 (d, 1H, J = 3 Hz).

MS: 404.17 [M+H]⁺

### Example 130

(R)-2-(4-cyclopropylphenyl)-N-(1-(6-(trifluoromethyl)pyridin-3-yl)piperidin-3-yl)acetamide

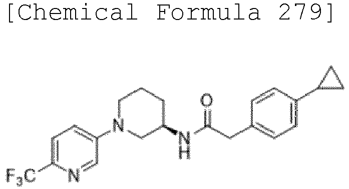

According to a technique similar to Example 3, a crude form of (R)-1-(6-(trifluoromethyl)pyridin-3-yl)piperidin-3-amine (280 mg) was synthesized from tert-butyl (R)-(1-(6-(trifluoromethyl)pyridin-3-yl)piperidin-3-yl)carbamate (400 mg, 1.16 mmol) synthesized in Reference Example 72, and the title compound (pale yellow crystals, 65 mg, 54%) was obtained using a portion of the crude form of (R)-1-(6-(trifluoromethyl)pyridin-3-yl)piperidin-3-amine (70 mg) thus obtained and 2-(4-cyclopropylphenyl)acetic acid (78 mg, 0.44 mmol) synthesized in Reference Example 33-2.

¹H NMR (DMSO - d₆, 400 MHz): δ = 0.5 - 0.7 (m, 2H), 0.8 - 1.0 (m, 2H), 1.4 - 1.6 (m, 2H), 1.7 - 1.9 (m, 3H), 2.8 - 3.0 (m, 1H), 3.0 - 3.1 (m, 1H), 3.2 - 3.5 (m, 2H), 3.6 - 3.8 (m, 3H), 6.97 (d, 2H, J = 8 Hz), 7.11 (d, 2H, J = 8 Hz), 7.37 (dd, 1H, J = 3, 7 Hz), 7.58 (d, 1H, J = 8 Hz), 8.0 - 8.2 (m, 1H), 8.37 (d, 1H, J = 3 Hz).

MS: 404.17 [M+H]⁺

### Example 131

(R)-2-(4-(2-hydroxypropan-2-yl)phenyl)-N-(1-(5-(trifluoromethyl)pyridin-3-yl)pyrrolidin-3-yl)acetamide

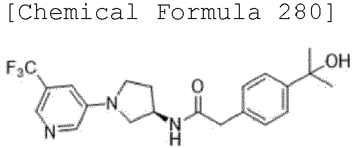

(R)-1-(5-(trifluoromethyl)pyridin-3-yl)pyrrolidin-3-amine (39 mg, 0.20 mmol) synthesized in Reference Example 76-2, 2-(4-(2-hydroxypropan-2-yl)phenyl)acetic acid (27 mg, 0.24 mmol), and DMT-MM (46 mg, 0.19 mmol) were dissolved in isopropanol (0.80 mL), and then the solution was stirred overnight at room temperature. Water was added to the reaction liquid, the mixture was stirred for a while, and then the mixture was extracted with ethyl acetate. An organic layer thus separated was dried over anhydrous

sodium sulfate, insoluble materials were filtered, and then the solvent was distilled off under reduced pressure. A residue thus obtained was purified by silica gel column chromatography (chloroform: methanol) (concentration gradient: 0 to 40%), and the title compound (white amorphous, 57 mg, 99%) was obtained.

¹H NMR (CDCl₃, 400 MHz): δ = 1.58 (s, 3H), 1.59 (s, 3H), 1.7 - 1.9 (br s, 1H), 1.9 - 2.0 (m, 1H), 2.3 - 2.4 (m, 1H), 3.1 - 3.2 (m, 2H), 3.3 - 3.5 (m, 2H), 3.58 (s, 2H), 3.6 - 3.7 (m, 1H), 4.6 - 4.7 (m, 1H), 5.5 - 5.6 (m, 1H), 6.92 (s, 1H), 7.26 (d, 1H, J = 8 Hz), 7.47 (d, 2H, J = 8 Hz), 8.0 - 8.1 (m, 1H), 8.21 (s, 1H).

MS: 430.14 [M+Na]⁺

### Example 132

(R)-2-(4-(3-methylpyrazin-2-yl)phenyl)-N-(1-(5-(trifluoromethyl)pyridin-3-yl)pyrrolidin-3-yl)acetamide

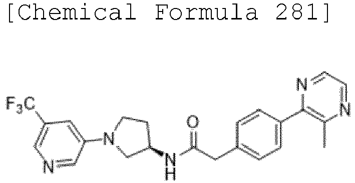

According to a technique similar to Example 1, the title compound (white crystals, 14 mg, 62%) was obtained using (R)-1-(5-(trifluoromethyl)pyridin-3-yl)pyrrolidin-3-amine (10 mg, 0.048 mmol) synthesized in Reference Example 1-2 and 2-(4-(3-methylpyrazin-2-yl)phenyl)acetic acid (10 mg, 0.044 mmol).

¹H NMR (CDCl₃, 400 MHz): δ = 1.9 - 2.1 (m, 1H), 2.3 - 2.4 (m, 1H), 2.63 (s, 3H), 3.1 - 3.2 (m, 1H), 3.3 - 3.5 (m, 2H), 3.6 - 3.7 (m, 1H), 3.66 (s, 2H), 4.6 - 4.7 (m, 1H), 5.7 - 5.9 (m, 1H), 6.90 (s, 1H), 7.39 (d, 2H, J = 8H), 7.59 (d, 2H, J = 8 Hz), 8.07 (d, 1H, J = 3 Hz), 8.19 (s, 1H), 8.46 (d, 1H, J = 2 Hz), 8.48 (d, 1H, J = 2 Hz).

MS: 442.15 [M+H]⁺

### Example 133

(R)-2-(4-(4-methyloxazol-5-yl)phenyl)-N-(1-(5-(trifluoromethyl)pyridin-3-yl)pyrrolidin-3-yl)acetamide

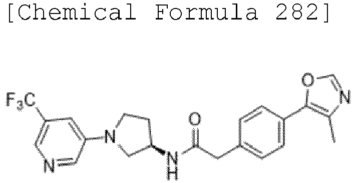

According to a technique similar to Example 1, the title compound (white crystals, 8.6 mg, 100%) was obtained using (R)-1-(5-(trifluoromethyl)pyridin-3-yl)pyrrolidin-3-amine (6 mg, 0.024 mmol) synthesized in Reference Example 1-2 and 2-(4-(4-methyloxazol-5-yl)phenyl)acetic acid (4 mg, 0.020 mmol).

¹H NMR (CDCl₃, 400 MHz): δ = 1.9 - 2.0 (m, 1H), 2.3 - 2.4 (m, 1H), 2.44 (s, 3H), 3.1 - 3.2 (m, 1H), 3.3 - 3.5 (m, 2H), 3.61 (s, 2H), 3.6 - 3.7 (m, 1H), 4.6 - 4.7 (m, 1H), 5.4 - 5.6 (m, 1H), 6.91 (s, 1H), 7.34 (d, 2H, J = 8 Hz)7.59 (d, 2H, J = 8 Hz), 7.83 (s, 1H), 8.08 (s, 1H), 8.20 (s, 1H).

MS: 429.17 [M-H]⁻

### Example 134

(R)-3-(3-(2-(4-cyclopropylphenyl)acetamido)pyrrolidin-1-yl)-5-(trifluoromethyl)pyridine 1-oxide

(3R)-3-(2-(4-cyclopropylphenyl)acetamido)-1-(5-(trifluoromethyl)pyridin-3-yl)pyrrolidine 1-oxide

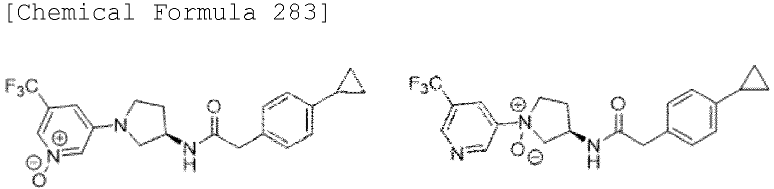

(R)-2-(4-cyclopropylphenyl)-N-(1-(5-(trifluoromethyl)pyridin-3-yl)pyrrolidin-3-yl)acetamide (85 mg, 0.22 mmol) synthesized in Example 1 was dissolved in dichloromethane (3.0 mL), m-CPBA (purity 65%, 58 mg, 0.22 mmol) was added thereto, and the mixture was stirred overnight at room temperature. The reaction liquid was washed with a saturated aqueous solution of sodium hydrogen carbonate, an organic layer thus separated was dried over anhydrous sodium sulfate, insoluble materials were filtered, and then the solvent was distilled off under reduced pressure. A residue thus obtained was purified by silica gel column chromatography (chloroform: methanol) (concentration gradient: 0 to 20%), and (R)-3-(3-(2-(4-cyclopropylphenyl)acetamido)pyrrolidin-1-yl)-5-(trifluoromethyl)pyridine 1-oxide (Rf value in TLC (dichloromethane: methanol = 10: 1) = 0.4, pale yellow amorphous, 12 mg, 13%) and (3R)-3-(2-(4-cyclopropylphenyl)acetamido)-1-(5-(trifluoromethyl)pyridin-3-yl)pyrrolidine 1-oxide (Rf value in TLC (dichloromethane: methanol = 10: 1) = 0.2, white amorphous, 42 mg, 47%) were obtained.

(R)-3-(3-(2-(4-cyclopropylphenyl)acetamido)pyrrolidin-1-yl)-5-(trifluoromethyl)pyridine 1-oxide

¹H NMR (CDCl₃, 400 MHz): δ = 0.6 - 0.7 (m, 2H), 0.9 - 1.0 (m, 2H), 1.8 - 2.0 (m, 2H), 2.2 - 2.4 (m, 1H), 3.0 - 3.1 (m, 1H), 3.31 (t, 2H, J = 7 Hz), 3.54 (s, 2H), 3.5 - 3.7 (m, 1H), 4.5 - 4.7 (m, 1H), 5.7 - 5.9 (m, 1H), 6.54 (s, 1H), 7.04 (d, 2H, J = 8 Hz), 7.12 (d, 2H, J = 8 Hz), 7.63 (s, 1H), 7.81 (s, 1H).

MS: 406.14 [M+H]⁺

(3R)-3-(2-(4-cyclopropylphenyl)acetamido)-1-(5-(trifluoromethyl)pyridin-3-yl)pyrrolidine 1-oxide

¹H NMR (CDCl₃, 400 MHz): δ = 0.6 - 0.7 (m, 2H), 0.9 -

1.0 (m, 2H), 1.8 - 1.9 (m, 1H), 2.4 - 2.6 (m, 1H), 2.7 - 2.9 (m, 1H), 3.50 (s, 2H), 3.5 - 3.7 (m, 1H), 3.8 - 4.0 (m, 2H), 4.0 - 4.1 (m, 1H), 5.1 - 5.3 (m, 1H), 7.03 (d, 2H, J = 8 Hz), 7.18 (d, 2H, J = 8 Hz), 7.7 - 7.8 (m, 1H), 8.82 (s, 1H), 8.95 (s, 1H), 9.15 (s, 1H).

MS: 406.14 [M+H]⁺

### Example 135

(R)-2-(4-cyclopropylphenyl)-2-methyl-N-(1-(5-(trifluoromethyl)pyridin-3-yl)pyrrolidin-3-yl)propanamide

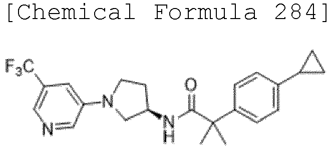

According to a technique similar to Example 1, the title compound (white amorphous, 45 mg, 94%) was obtained using (R)-1-(5-(trifluoromethyl)pyridin-3-yl)pyrrolidin-3-amine (25 mg, 0.11 mmol) synthesized in Reference Example 1-2 and 2-(4-cyclopropylphenyl)-2-methylpropanoic acid (23 mg, 0.11 mmol) .

¹H NMR (CDCl₃, 400 MHz): δ = 0.6 - 0.7 (m, 2H), 0.9 - 1.0 (m, 2H), 1.54 (s, 3H), 1.55 (s, 3H), 1.7 - 1.9 (m, 2H), 2.2 - 2.4 (m, 1H), 3.0 - 3.1 (m, 1H), 3.2 - 3.4 (m, 2H), 3.6 - 3.7 (m, 1H), 4.5 - 4.6 (m, 1H), 5.2 - 5.3 (m, 1H), 6.88 (s, 1H), 7.02 (d, 2H, J = 8 Hz), 7.21 (d, 2H, J = 8 Hz), 8.05 (s, 1H), 8.19 (s, 1H).

MS: 416.21 [M-H]⁻

### Example 136

(R)-2-(4-cyclopropylphenyl)-N-(1-(3-fluoro-5-(trifluoromethyl)phenyl)pyrrolidin-3-yl)acetamide

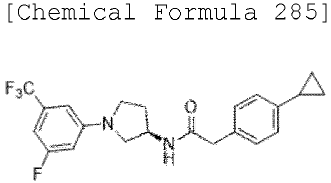

According to a technique similar to Example 9, the title compound (pale yellow amorphous, 34 mg, 49%) was obtained using (R)-2-(4-cyclopropylphenyl)-N-(pyrrolidin-3-yl)acetamide (42 mg, 0.17 mmol) synthesized in Reference Example 74-2 and 1-fluoro-3-iodo-5-(trifluoromethyl)benzene (50 mg, 0.17 mmol).

¹H NMR (CDCl₃, 400 MHz): δ = 0.6 - 0.7 (m, 2H), 0.9 - 1.0 (m, 2H), 1.8 - 2.0 (m, 2H), 2.2 - 2.4 (m, 1H), 3.0 - 3.1 (m, 1H), 3.2 - 3.4 (m, 2H), 3.53 (s, 2H), 3.5 - 3.7 (m, 1H), 4.5 - 4.7 (m, 1H), 5.4 - 5.6 (m, 1H), 6.2 - 6.4 (m, 1H), 6.47 (s, 1H), 6.5 - 6.7 (m, 1H), 7.04 (d, 2H, J = 8 Hz), 7.11 (d, 2H, J = 8 Hz).

MS: 407.15 [M+H]⁺

### Example 137

2-Amino-2-(4-cyclopropylphenyl)-N-((R)-1-(5-(trifluoromethyl)pyridin-3-yl)pyrrolidin-3-yl)acetamide

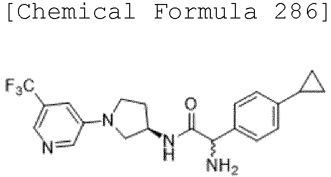

To diastereomer A of tert-butyl (1-(4-cyclopropylphenyl)-2-oxo-2-(((R)-1-(5-(trifluoromethyl)pyridin-3-yl)pyrrolidin-3-yl)amino)ethyl)carbamate (15 mg, 0.030 mmol) synthesized in Reference Example 67, trifluoroacetic acid (0.50 mL) was added under ice cooling, and the mixture was stirred for 1 hour at room temperature. The solvent of the reaction liquid was distilled off under reduced pressure, a residue thus obtained was purified by silica gel column chromatography (chloroform: methanol) (concentration gradient: 0 to 20%), and diastereomer A of 2-amino-2-(4-cyclopropylphenyl)-N-((R)-1-(5-(trifluoromethyl)pyridin-3-yl)pyrrolidin-3-yl)acetamide (pale yellow amorphous, 3.8 mg, 9%) was obtained. According to a technique similar to that described above, diastereomer B of 2-amino-2-(4-cyclopropylphenyl)-N-((R)-1-(5-(trifluoromethyl)pyridin-3-yl)pyrrolidin-3-yl)acetamide (pale yellow amorphous, 3.4 mg, 8%) was obtained using diastereomer B of tert-butyl (1-(4-cyclopropylphenyl)-2-oxo-2-(((R)-1-(5-(trifluoromethyl)pyridin-3-yl)pyrrolidin-3-yl)amino)ethyl)carbamate (15 mg, 0.030 mmol) synthesized in Reference Example 67.

### Diastereomer A

¹H NMR (CDCl₃, 400 MHz): δ = 0.6 - 0.8 (m, 2H), 0.9 - 1.0 (m, 2H), 1.2 - 1.3 (br s, 2H), 1.8 - 2.0 (m, 1H), 2.0 - 2.1 (m, 1H), 2.3 - 2.5 (m, 1H), 3.1 - 3.3 (m, 1H), 3.3 - 3.6 (m, 2H), 3.6 - 3.7 (m, 1H), 4.49 (s, 1H), 4.5 - 4.7 (m, 1H), 6.94 (s, 1H)7.01 (d, 2H, J = 8 Hz), 7.21 (d, 2H, J = 8 Hz), 7.4 - 7.5 (m, 1H), 8.11 (d, 1H, J = 3 Hz), 8.22 (s, 1H).

MS: 405.14 [M+H]⁺

### Diastereomer B

¹H NMR (CDCl₃, 400 MHz): δ = 0.6 - 0.7 (m, 2H), 0.9 - 1.0 (m, 2H), 1.2 - 1.3 (m, 2H), 1.8 - 1.9 (m, 1H), 2.0 - 2.1 (m, 1H), 2.3 - 2.4 (m, 1H), 3.2 - 3.3 (m, 1H), 3.3 - 3.6 (m, 2H), 3.6 - 3.7 (m, 1H), 4.50 (s, 1H), 4.6 - 4.7 (m, 1H), 6.95 (s, 1H), 7.05 (d, 2H, J = 8 Hz), 7.25 (d, 2H, J = 8 Hz), 7.4 - 7.5 (m, 1H), 8.13 (d, 1H, J = 3 Hz), 8.22 (s, 1H).

MS: 405.14 [M+H]⁺

### Example 138

2-(4-cyclopropylphenyl)-2-(dimethylamino)-N-((R)-1-(5-(trifluoromethyl)pyridin-3-yl)pyrrolidin-3-yl)acetamide hydrochloride

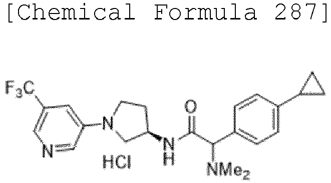

According to a technique similar to Example 137, a crude form of 2-amino-2-(4-cyclopropylphenyl)-N-((R)-1-(5-(trifluoromethyl)pyridin-3-yl)pyrrolidin-3-yl)acetamide (200 mg) was obtained using tert-butyl (1-(4-cyclopropylphenyl)-2-oxo-2-(((R)-1-(5-(trifluoromethyl)pyridin-3-yl)amino)ethyl)carbamate (290 mg, 0.57 mmol) synthesized in Reference Example 67. A portion of the crude form of 2-amino-2-(4-cyclopropylphenyl)-N-((R)-1-(5-(trifluoromethyl)pyridin-3-yl)pyrrolidin-3-yl)acetamide (50 mg) thus obtained was dissolved in dichloromethane (1.0 mL), paraformaldehyde (11 mg, 0.36 mmol), acetic acid (14 µL, 0.24 mmol), and triacetoxyborohydride (127 mg, 0.6 mmol) were added thereto, and the mixture was stirred overnight at room temperature. The reaction liquid was diluted with ethyl acetate, an organic layer was washed with a saturated aqueous solution of sodium hydrogen carbonate, water, and saturated brine, subsequently the separated organic layer was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 10 to 70%), and 2-(4-cyclopropylphenyl)-2-(dimethylamino)-N-((R)-1-(5-(trifluoromethyl)pyridin-3-yl)pyrrolidin-3-yl)acetamide was obtained. 2-(4-cyclopropylphenyl)-2-(dimethylamino)-N-((R)-1-(5-(trifluoromethyl)pyridin-3-yl)pyrrolidin-3-yl)acetamide thus obtained was dissolved in methanol (0.5 mL), subsequently a 1N aqueous solution of hydrochloric acid (0.5 mL) was added thereto, and the solvent was distilled off under reduced pressure. Distilled water (0.1 mL) was added to a residue thus obtained, the residue was freeze-dried, and the title compound (yellow crystals, 10 mg, 19%) was obtained.

¹H NMR (DMSO - d₆, 400 MHz): δ = 0.6 - 0.8 (m, 2H), 0.9 - 1.1 (m, 2H), 1.7 - 2.3 (m, 3H), 2.50 (s, 3H), 2.87 (s, 3H), 3.0 - 3.8 (m, 4H), 4.3 - 4.5 (m, 1H), 4.8 - 5.0 (m, 1H), 7.12 (d, 1H, J = 8 Hz), 7.18 (d, 1H, J = 8 Hz), 7.2 - 7.4 (m, 1H), 7.4 - 7.5 (m, 2H), 8.1 - 8.3 (m, 2H), 9.3 - 9.6 (m, 1H), 10.3 - 10.5 (m, 1H).

MS: 433.18 [M+H]⁺

### Example 139

(R)-2-(1-methyl-1H-indol-5-yl)-N-(1-(5-(trifluoromethyl)pyridin-3-yl)pyrrolidin-3-yl)acetamide

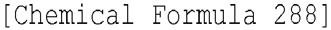

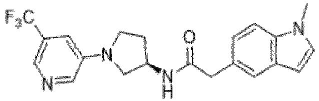

According to a technique similar to Example 1, the title compound (white crystals, 6 mg, 26%) was obtained using (R)-1-(5-(trifluoromethyl)pyridin-3-yl)pyrrolidin-3-amine (13 mg, 0.058 mmol) synthesized in Reference Example 1-2 and 2-(1-methyl-1H-indol-5-yl)acetic acid (10 mg, 0.053 mmol) .

¹H NMR (CDCl₃, 400 MHz): δ = 1.7 - 1.9 (m, 1H), 2.2 - 2.4 (m, 1H), 3.0 - 3.1 (m, 1H), 3.2 - 3.4 (m, 2H), 3.6 - 3.7 (m, 1H), 3, 70 (s, 2H), 3.80 (s, 3H), 4.5 - 4.7 (m, 1H), 5.5 - 5.6 (m, 1H), 6.44 (d, 1H, J = 6 Hz), 6.86 (s, 1H), 7.0 - 7.1 (m, 2H), 7.29 (d, 1H, J - 9 Hz), 7.46 (d, 1H, J = 1 Hz), 8.03 (d, 1H, J = 3 Hz), 8.17 (s, 1H).

MS: 403.13 [M+H]⁺

### Example 140

(R)-2-(2,4-bis(trifluoromethyl)phenyl)-N-(1-(5-(trifluoromethyl)pyridin-3-yl)pyrrolidin-3-yl)acetamide

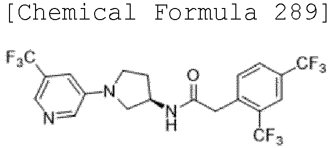

According to a technique similar to Example 1, the title compound (white crystals, 56 mg, 89%) was obtained using (R)-1-(5-(trifluoromethyl)pyridin-3-yl)pyrrolidin-3-amine (30 mg, 0.13 mmol) synthesized in Reference Example 1-2 and 2-(2,4-bis(trifluoromethyl)phenyl)acetic acid (44 mg, 0.16 mmol).

¹H NMR (CDCl₃, 400 MHz): δ = 2.0 - 2.2 (m, 1H), 2.2 - 2.4 (m, 1H), 3.1 - 3.3 (m, 1H), 3.3 - 3.5 (m, 2H), 3.6 - 3.7 (m, 1H), 3.79 (s, 2H), 4.6 - 4.8 (m, 1H), 6.5 - 6.6 (m, 1H), 6.83 (s, 1H), 7.68 (d, 1H, J = 8 Hz), 7.82 (d, 1H, J = 8 Hz), 7.91 (s, 1H), 8.00 (d, 1H, J = 2 Hz), 8.12 (s, 1H).

MS: 484.11 [M-H]⁻

### Example 141

(R)-2-(2-fluoro-4-(trifluoromethyl)phenyl)-N-(1-(5-(trifluoromethyl)pyridin-3-yl)pyrrolidin-3-yl)acetamide

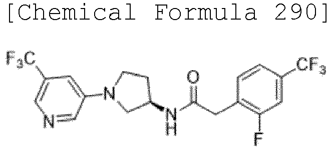

According to a technique similar to Example 1, the title compound (white crystals, 59 mg, 100%) was obtained using (R)-1-(5-(trifluoromethyl)pyridin-3-yl)pyrrolidin-3-amine (30 mg, 0.13 mmol) synthesized in Reference Example 1-2 and 2-(2-fluoro-4-(trifluoromethyl)phenyl)acetic acid (36 mg, 0.16 mmol).

¹H NMR (CDCl₃, 400 MHz): δ = 2.0 - 2.1 (m, 1H), 2.2 - 2.4 (m, 1H), 3.1 - 3.3 (m, 1H), 3.3 - 3.5 (m, 2H), 3.6 - 3.7 (m, 1H), 3.64 (s, 2H), 4.6 - 4.8 (m, 1H), 6.6 - 6.8 (m, 1H), 6.82 (s, 1H), 7.33 (d, 1H, J = 10 Hz), 7.41 (d, 1H, J = 8 Hz), 7.49 (t, 1H, J = 8 Hz), 7.99 (d, 1H, J = 8 Hz), 8.12 (s, 1H).

MS: 434.12 [M-H]⁻

### Example 142

(R)-2-(1-methyl-1H-indazol-5-yl)-N-(1-(5-(trifluoromethyl)pyridin-3-yl)pyrrolidin-3-yl)acetamide

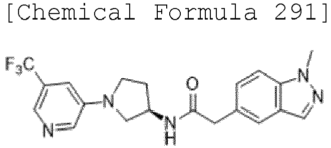

According to a technique similar to Example 1, the title compound (white crystals, 36 mg, 56%) was obtained using (R)-1-(5-(trifluoromethyl)pyridin-3-yl)pyrrolidin-3-amine (30 mg, 0.13 mmol) synthesized in Reference Example 1-2 and 2-(1-methyl-1H-indazol-5-yl)acetic acid (32 mg, 0.16 mmol).

¹H NMR (CDCl₃, 400 MHz): δ = 1.8 - 2.0 (m, 1H), 2.2 - 2.4 (m, 1H), 3.0 - 3.2 (m, 1H), 3.35 (t, 2H, J = 7 Hz), 3.6 - 3.7 (m, 1H), 3.69 (s, 2H), 4.08 (s, 3H), 4.6 - 4.7 (m, 1H), 5.5 - 5.6 (m, 1H), 6.87 (s, 1H), 7.2 - 7.3 (m, 1H), 7.38 (d, 1H, J = 9 Hz), 7.59 (s, 1H), 7.94 (s, 1H), 8.04 (d, 1H, J = 2 Hz), 8.18 (s, 1H).

MS: 404.10 [M+H]⁺

### Example 143

(R)-2-(1-methyl-1H-indazol-5-yl)-N-(1-(4-(trifluoromethyl)thiazol-2-yl)pyrrolidin-3-yl)acetamide

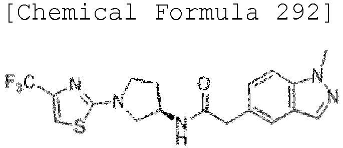

According to a technique similar to Example 1, the title compound (white crystals, 35 mg, 66%) was obtained using (R)-1-(4-(trifluoromethyl)thiazol-2-yl)pyrrolidin-3-amine (31 mg, 0.13 mmol) synthesized in Reference Example 12-2 and 2-(1-methyl-1H-indazol-5-yl)acetic acid (32 mg, 0.16 mmol).

¹H NMR (CDCl₃, 400 MHz): δ = 1.8 - 2.0 (m, 1H), 2.2 - 2.4 (m, 1H), 3.1 - 3.3 (m, 1H), 3.4 - 3.6 (m, 2H), 3.68 (s, 2H), 3.7 - 3.8 (m, 1H), 4.09 (s, 3H), 4.5 - 4.7 (m, 1H), 5.4 - 5.6 (m, 1H), 6.91 (s, 1H), 7.2 - 7.3 (m, 1H), 7.38 (d, 1H, J = 8 Hz), 7.58 (s, 1H), 7.95 (s, 1H).

MS: 410.06 [M+H]⁺

### Example 144

(R)-2-(1-methyl-1H-indazol-5-yl)-N-(1-(6-(trifluoromethyl)pyridin-3-yl)pyrrolidin-3-yl)acetamide

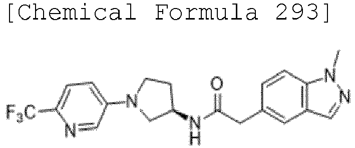

According to a technique similar to Example 1, the title compound (white crystals, 35 mg, 67%) was obtained using (R)-1-(6-(trifluoromethyl)pyridin-3-yl)pyrrolidin-3-amine (30 mg, 0.13 mmol) synthesized in Reference Example 32-2 and 2-(1-methyl-1H-indazol-5-yl)acetic acid (32 mg, 0.16 mmol).

¹H NMR (CDCl₃, 400 MHz): δ = 1.8 - 2.0 (m, 1H), 2.2 - 2.4 (m, 1H), 3.0 - 3.2 (m, 1H), 3.36 (t, 2H, J = 7 Hz), 3.6 - 3.7 (m, 1H), 3.69 (s, 2H), 4.08 (s, 3H), 4.5 - 4.7 (m, 1H), 5.4 - 5.6 (m, 1H), 6.76 (dd, 1H, J = 3, 9 Hz), 7.2 - 7.3 (m, 1H), 7.37 (d, 1H, J = 9 Hz), 7.45 (d, 1H, J = 8 Hz), 7.59 (s, 1H), 7.9 - 8.0 (m, 2H).

MS: 404.10 [M+H]⁺

### Example 145

(R)-2-(4-(2,2-dimethylmorpholino)phenyl)-N-(1-(5-(trifluoromethyl)pyridin-3-yl)pyrrolidin-3-yl)acetamide

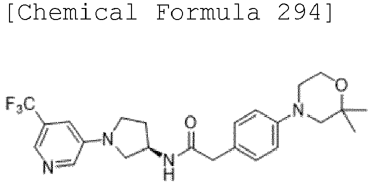

According to a technique similar to Example 9, the title compound (yellow amorphous, 2.1 mg, 5%) was obtained using (R)-2-(4-bromophenyl)-N-(1-(5-(trifluoromethyl)pyridin-3-yl)pyrrolidin-3-yl)acetamide (40 mg, 0.093 mmol) synthesized in Example 171 and 2,2-dimethylmorpholine (21 mg, 0.19 mmol).

¹H NMR (CDCl₃, 400 MHz): δ = 1.32 (s, 6H), 1.8 - 2.0 (m, 1H), 2.3 - 2.4 (m, 1H), 2.94 (s, 2H), 3.0 - 3.2 (m, 3H), 3.3 - 3.4 (m, 2H), 3.51 (s, 2H), 3.6 - 3.7 (m, 1H)3.8 - 3.9 (m, 2H), 4.5 - 4.7 (m, 1H), 5.4 - 5.6 (m, 1H), 6.85 (d, 2H, J = 8 Hz), 6.94 (s, 1H), 7.12 (d, 2H, J = 8 Hz), 8.0 - 8.4 (m, 2H).

MS: 463.15 [M+H]⁺

### Example 146

(R)-2-(4-(1H-pyrazol-1-yl)phenyl)-N-(1-(5-(trifluoromethyl)pyridin-3-yl)pyrrolidin-3-yl)acetamide

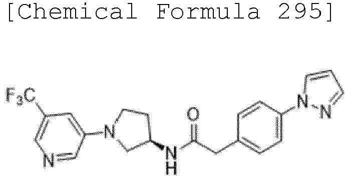

(R)-2-(4-bromophenyl)-N-(1-(5-(trifluoromethyl)pyridin-3-yl)pyrrolidin-3-yl)acetamide (40 mg, 0.093 mmol) synthesized in Example 171, pyrazole (13 mg, 0.19 mmol), copper(I) iodide (2 mg, 9.3 µmmol), N,N'-dimethylethylenediamine (2 µL, 18.6 µmmol), and cesium carbonate (91 mg, 0.30 mmol) were suspended in DMF (1.0 mL), and the suspension was stirred for 2 hours at 200°C (microwaved). Ethyl acetate was added to the reaction liquid that had been left to cool to room temperature, and then the organic layer was washed with water and saturated brine. An organic layer thus separated was dried over anhydrous sodium sulfate, insoluble materials were filtered, and then the solvent was distilled off under reduced pressure. A residue thus obtained was purified by silica gel column chromatography (hexane: ethyl acetate) (concentration gradient: 0 to 100%), and the title compound (white crystals, 22 mg, 57%) was obtained.

¹H NMR (CDCl₃, 400 MHz): δ = 1.9 - 2.0 (m, 1H), 2.3 - 2.4 (m, 1H), 3.1 - 3.2 (m, 1H), 3.3 - 3.5 (m, 2H), 3.62 (s, 2H), 3.6 - 3.7 (m, 1H), 4.6 - 4.7 (m, 1H), 5.5 - 5.6 (m, 1H), 6.4 - 6.5 (m, 1H), 6.9 - 7.0 (m, 1H), 7.35 (d, 2H, J = 9 Hz), 7.69 (d, 2H, J = 9 Hz), 7.73 (d, 1H, J = 1 Hz), 7.92 (d, 1H, J = 1 Hz), 8.0 - 8.1 (m, 1H), 8.20 (s, 1H).

MS: 416.11 [M+H]⁺

### Example 147

(R)-N-(3-methyl-1-(5-(trifluoromethyl)pyridin-3-yl)pyrrolidin-3-yl)-2-(4-morpholinophenyl)acetamide

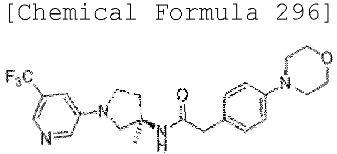

According to a technique similar to Example 3, a crude form of (R)-3-methyl-1-(5-(trifluoromethyl)pyridin-3-yl)pyrrolidin-3-amine (50 mg) was synthesized from tert-butyl (R)-(3-methyl-1-(5-(trifluoromethyl)pyridin-3-yl)pyrrolidin-3-yl)carbamate (47 mg, 0.23 mmol) synthesized in Reference Example 68, and the title compound (white crystals, 22 mg, 61%) was obtained using a portion of the crude form of (R)-3-methyl-1-(5-(trifluoromethyl)pyridin-3-yl)pyrrolidin-3-amine (20 mg) thus obtained and 2-(4-morpholinophenyl)acetic acid (30 mg, 0.12 mmol) synthesized in Reference Example 23-2.

¹H NMR (CDCl₃, 400 MHz): δ = 1.53 (s, 3H), 1.9 - 2.1 (m, 1H), 2.2 - 2.4 (m, 1H), 3.13 (t, 4H, J = 4 Hz), 3.2 - 3.4 (m, 3H), 3.46 (s, 2H), 3.6 - 3.7 (m, 1H), 3.86 (t, 4H, J = 4 Hz), 5.37 (br s, 1H), 6.84 (d, 2H, J = 8 Hz), 6.89 (s, 1H), 7.09 (d, 2H, J = 8 Hz), 8.06 (s, 1H), 8.19 (s, 1H).

MS: 449.15 [M+H]⁺

### Example 148

(R)-2-(4-cyclopropylphenyl)-N-(3-methyl-1-(5-(trifluoromethyl)pyridin-3-yl)pyrrolidin-3-yl)acetamide

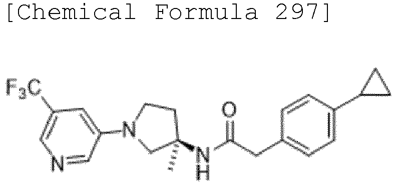

According to a technique similar to Example 3, a crude form of (R)-3-methyl-1-(5-(trifluoromethyl)pyridin-3-yl)pyrrolidin-3-amine (20 mg) was synthesized from tert-butyl (R)-(3-methyl-1-(5-(trifluoromethyl)pyridin-3-yl)pyrrolidin-3-yl)carbamate (47 mg, 0.23 mmol) synthesized in Reference Example 68, and the title compound (white amorphous, 12 mg, 100%) was obtained using a portion of the crude form of (R)-3-methyl-1-(5-(trifluoromethyl)pyridin-3-yl)pyrrolidin-3-amine (7 mg) thus obtained and 2-(4-cyclopropylphenyl)acetic acid (8 mg, 0.044 mmol) synthesized in Reference Example 33-2.

¹H NMR (CDCl₃, 400 MHz): δ = 0.6 - 0.7 (m, 2H), 0.9 - 1.0 (m, 2H), 1.53 (s, 3H), 1.8 - 1.9 (m, 1H), 1.9 - 2.1 (m, 1H), 2.2 - 2.4 (m, 1H), 3.2 - 3.4 (m, 3H), 3.48 (s, 2H), 3.62 (d, 1H, J = 10 Hz), 5.36 (br s, 1H), 6.88 (s, 1H), 7.00 (d, 2H, J = 8 Hz), 7.07 (d, 2H, J = 8 Hz), 8.05 (d, 1H, J = 2 Hz), 8.19 (s, 1H).

MS: 404.12 [M+H]⁺

### Example 149

2-(4-(3-Oxa-8-azabicyclo[3.2.1]octan-8-yl)phenyl)-N-((R)-1-(5-(trifluoromethyl)pyridin-3-yl)pyrrolidin-3-yl)acetamide

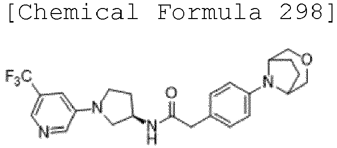

Ethyl 2-(4-(3-oxa-8-azabicyclo[3.2.1]octan-8-yl)phenyl)acetate (87 mg, 0.32 mmol) synthesized in Reference Example 69 was dissolved in ethanol (1.6 mL), an 8N aqueous solution of sodium hydroxide (0.20 mL, 1.6 mmol) was added thereto, and the mixture was stirred for 2 hours at room temperature. A 1N aqueous solution of hydrochloric acid (1.6 mL, 1.6 mmol) was added to the reaction liquid in an ice bath, and then the mixture was distilled off under reduced pressure. A mixed liquid (5 mL) of chloroform: methanol = 10: 1 was added to a residue thus obtained, insoluble materials were filtered, subsequently the solvent was distilled off under reduced pressure, and a crude form of 2-(4-(3-oxa-8-azabicyclo[3.2.1]octan-8-yl)phenyl)acetic acid (white crystals) was obtained. Subsequently, according to a technique similar to Example 1, the title compound (pale yellow crystals, 50 mg, 71%) was obtained using (R)-1-(5-(trifluoromethyl)pyridin-3-yl)pyrrolidin-3-amine (35 mg, 0.15 mmol) synthesized in Reference Example 1-2 and a half of the crude form of 2-(4-(3-oxa-8-azabicyclo[3.2.1]octan-8-yl)phenyl)acetic acid synthesized as described above.

¹H NMR (CDCl₃, 400 MHz): δ = 1.8 - 2.2 (m, 5H), 2.2 - 2.4 (m, 1H), 3.0 - 3.2 (m, 1H), 3.37 (t, 2H, J = 7 Hz), 3.4 - 3.6 (m, 2H), 3.49 (s, 2H), 3.6 - 3.7 (m, 1H), 3.8 - 3.9 (m, 2H), 4.0 - 4.1 (m, 2H), 4.5 - 4.7 (m, 1H), 5.5 - 5.6 (m, 1H), 6.76 (d, 2H, J = 9 Hz), 6.90 (s, 1H), 7.10 (d, 2H, J = 9 Hz)8.08 (d, 1H, J = 3 Hz), 8.20 (s, 1H).

MS: 461.17 [M+H]⁺

### Example 150

2-(4-(3-Oxa-8-azabicyclo[3.2.1]octan-8-yl)phenyl)-N-((R)-1-(6-(trifluoromethyl)pyridin-3-yl)pyrrolidin-3-yl)acetamide

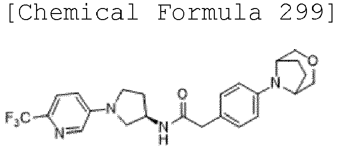

According to a technique similar to Example 149, a crude form of 2-(4-(3-oxa-8-azabicyclo[3.2.1]octan-8-yl)phenyl)acetic acid (white crystals) was obtained from ethyl 2-(4-(3-oxa-8-azabicyclo[3.2.1]octan-8-yl)phenyl)acetate (87 mg, 0.32 mmol) synthesized in Reference Example 69. The title compound (pale yellow crystals, 51 mg, 73%) was obtained using (R)-1-(6-(trifluoromethyl)pyridin-3-yl)pyrrolidin-3-amine (35 mg, 0.15 mmol) synthesized in Reference Example 32-2 and a half of the crude form of 2-(4-(3-oxa-8-azabicyclo[3.2.1]octan-8-yl)phenyl)acetic acid synthesized as described above.

¹H NMR (CDCl₃, 400 MHz): δ = 1.8 - 2.2 (m, 5H), 2.2 - 2.4 (m, 1H), 3.0 - 3.2 (m, 1H), 3.3 - 3.4 (m, 2H), 3.4 - 3.6 (m, 2H), 3.49 (s, 2H), 3.6 - 3.8 (m, 1H), 3.8 - 3.9 (m, 2H), 4.0 - 4.1 (m, 2H), 4.5 - 4.7 (m, 1H), 5.5 - 5.6 (m, 1H), 6.7 - 6.8 (m, 3H), 7.08 (d, 2H, J = 8 Hz), 7.46 (d, 1H, J = 8 Hz), 7.96 (d, 1H, J = 3 Hz).

MS: 461.17 [M+H]⁺

### Example 151

(R)-2-(benzo[d]oxazol-5-yl)-N-(1-(6-(trifluoromethyl)pyridin-3-yl)pyrrolidin-3-yl)acetamide

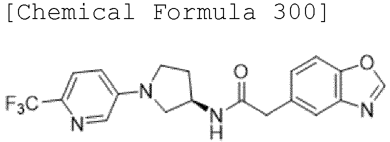

According to a technique similar to Example 1, the title compound (white crystals, 26 mg, 52%) was obtained using (R)-1-(6-(trifluoromethyl)pyridin-3-yl)pyrrolidin-3-amine (30 mg, 0.13 mmol) synthesized in Reference Example 32-2 and 2-(benzo[d]oxazol-5-yl)acetic acid (35 mg, 0.16 mmol) .

¹H NMR (CDCl₃, 400 MHz): δ = 1.8 - 2.0 (m, 1H), 2.2 - 2.4 (m, 1H), 3.1 - 3.3 (m, 1H), 3.3 - 3.5 (m, 2H), 3.6 - 3.7 (m, 1H), 3.70 (s, 2H), 3.6 - 3.7 (m, 1H), 5.5 - 5.7 (m, 1H), 6.76 (dd, 1H, J = 3, 9 Hz)7.30 (dd, 1H, J = 1, 8 Hz), 7.45 (d, 1H, J = 9 Hz), 7.56 (d, 1H, J = 8 Hz), 7.67 (d, 1H, J = 1 Hz), 7.94 (d, 1H, J = 3 Hz), 8.11 (s, 1H).

MS: 391.10 [M+H]⁺

### Example 152

(R)-2-(benzo[d]oxazol-5-yl)-N-(1-(5-(trifluoromethyl)pyridin-3-yl)pyrrolidin-3-yl)acetamide

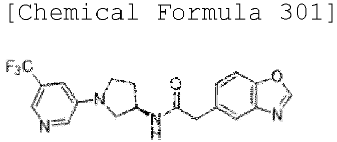

According to a technique similar to Example 1, the title compound (pale yellow crystals, 33 mg, 65%) was obtained using (R)-1-(5-(trifluoromethyl)pyridin-3-yl)pyrrolidin-3-amine (30 mg, 0.13 mmol) synthesized in Reference Example 1-2 and 2-(benzo[d]oxazol-5-yl)acetic acid (35 mg, 0.16 mmol).

¹H NMR (CDCl₃, 400 MHz): δ = 1.8 - 2.0 (m, 1H), 2.2 - 2.4 (m, 1H), 3.0 - 3.2 (m, 1H), 3.3 - 3.5 (m, 2H), 3.6 - 3.7 (m, 1H), 3.71 (s, 2H), 3.6 - 3.7 (m, 1H), 5.6 - 5.8 (m, 1H)6.88 (s, 1H), 7.31 (dd, 1H, J = 2, 8 Hz), 7.57 (d, 1H, J = 8 Hz), 7.68 (d, 1H, J = 2 Hz), 8.0 - 8.3 (m, 2H), 8.12 (s, 1H).

MS: 391.10 [M+H]⁺

### Example 153

(R)-2-(2-methylbenzo[d]oxazol-5-yl)-N-(1-(5-(trifluoromethyl)pyridin-3-yl)pyrrolidin-3-yl)acetamide

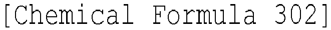

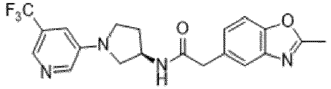

According to a technique similar to Example 1, the title compound (pale yellow crystals, 48 mg, 89%) was obtained using (R)-1-(5-(trifluoromethyl)pyridin-3-yl)pyrrolidin-3-amine (30 mg, 0.13 mmol) synthesized in Reference Example 1-2 and 2-(2-methylbenzo[d]oxazol-5-yl)acetic acid (35 mg, 0.16 mmol) synthesized as described above.

¹H NMR (CDCl₃, 400 MHz): δ = 1.8 - 2.0 (m, 1H), 2.2 - 2.4 (m, 1H), 2.65 (s, 3H), 3.0 - 3.2 (m, 1H), 3.36 (t, 2H, J = 7 Hz), 3.6 - 3.7 (m, 1H), 3.89 (s, 2H), 4.6 - 4.7 (m, 1H), 5.5 - 5.7 (m, 1H), 6.88 (s, 1H), 7.19 (dd, 1H, J = 2, 8 Hz), 7.44 (d, 1H, J = 8 Hz), 7.52 (d, 1H, J = 2 Hz), 8.05 (d, 1H, J = 2 Hz), 8.19 (s, 1H).

MS: 405.12 [M+H]⁺

### Example 154

(R)-2-(2-methylbenzo[d]oxazol-5-yl)-N-(1-(6-(trifluoromethyl)pyridin-3-yl)pyrrolidin-3-yl)acetamide

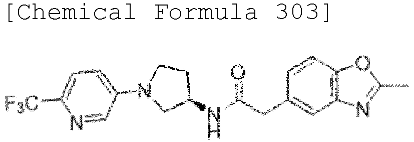

According to a technique similar to Example 1, the title compound (white crystals, 53 mg, 100%) was obtained using (R)-1-(6-(trifluoromethyl)pyridin-3-yl)pyrrolidin-3-amine (30 mg, 0.13 mmol) synthesized in Reference Example 32-2 and 2-(2-methylbenzo[d]oxazol-5-yl)acetic acid (31 mg, 0.16 mmol).

¹H NMR (CDCl₃, 400 MHz): δ = 1.8 - 2.0 (m, 1H), 2.2 - 2.4 (m, 1H), 2.64 (s, 3H), 3.0 - 3.2 (m, 1H), 3.37 (t, 2H, J = 7 Hz), 3.6 - 3.7 (m, 1H), 3.68 (s, 2H), 4.6 - 4.7 (m, 1H), 5.6 - 5.8 (m, 1H), 6.75 (dd, 1H, J = 3, 9 Hz), 7.19 (dd, 1H, J = 2, 8 Hz), 7.43 (d, 1H, J = 8 Hz), 7.45 (d, 1H, J = 9 Hz), 7.51 (d, 1H, J = 2 Hz), 7.91 (d, 1H, J = 3 Hz).

MS: 405.11 [M+H]⁺

### Example 155

2-(4-((1S,4S)-2-oxa-5-azabicyclo[2.2.2]octan-5-yl)phenyl)-N-((R)-1-(5-(trifluoromethyl)pyridin-3-yl)pyrrolidin-3-yl)acetamide

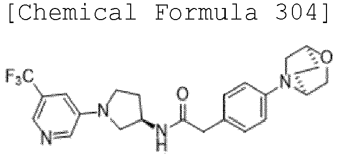

Ethyl 2-(4-((1S,4S)-2-oxa-5-azabicyclo[2.2.2]octan-5-yl)phenyl)acetate (112 mg, 0.41 mmol) synthesized in Reference Example 70 was dissolved in ethanol (2.0 mL), an 8N aqueous solution of sodium hydroxide (0.30 mL, 2.4 mmol) was added thereto, and the mixture was stirred for 2 hours at room temperature. A 1N aqueous solution of hydrochloric acid (2.4 mL, 2.4 mmol) was added to the reaction liquid in an ice bath, and then the mixture was distilled off under reduced pressure. A mixed liquid (5 mL) of chloroform: methanol = 10: 1 was added to a residue thus obtained, insoluble materials were filtered, subsequently the solvent was distilled off under reduced pressure, and a crude form of 2-(4-((1S,4S)-2-oxa-5-azabicyclo[2.2.2]octan-5-yl)phenyl)acetic acid (white crystals, 84 mg) was obtained. Subsequently, according to a technique similar to Example 131, the title compound (pale yellow crystals, 40 mg, 47%) was obtained using (R)-1-(5-(trifluoromethyl)pyridin-3-yl)pyrrolidin-3-amine (42 mg, 0.18 mmol) synthesized in Reference Example 1-2 and a portion of the crude form of 2-(4-((1S,4S)-2-oxa-5-azabicyclo[2.2.2]octan-5-yl)phenyl)acetic acid (41 mg) synthesized as described above.

¹H NMR (CDCl₃, 400 MHz): δ = 1.6 - 1.8 (m, 1H), 1.8 - 2.0 (m, 2H), 2.0 - 2.3 (m, 2H), 2.3 - 2.4 (m, 1H), 3.0 - 3.2 (m, 1H), 3.2 - 3.4 (m, 3H), 3.49 (s, 2H), 3.6 - 3.7 (m, 1H), 3.7 - 3.9 (m, 2H), 4.0 - 4.1 (m, 2H), 4.1 - 4.2 (m, 1H), 4.5 - 4.7 (m, 1H), 5.5 - 5.6 (m, 1H), 6.60 (d, 2H, J = 8 Hz), 6.91 (s, 1H), 7.09 (d, 2H, J = 8 Hz), 8.08 (s, 1H), 8.20 (s, 1H).

MS: 461.17 [M+H]⁺

### Example 156

2-(4-((1S,4S)-2-oxa-5-azabicyclo[2.2.2]octan-5-yl)phenyl)-N-((R)-1-(6-(trifluoromethyl)pyridin-3-yl)pyrrolidin-3-yl)acetamide

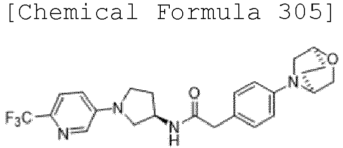

According to a technique similar to Example 155, a crude form of 2-(4-((1S,4S)-2-oxa-5-azabicyclo[2.2.2]octan-5-yl)phenyl)acetic acid (white crystals, 84 mg) was obtained from the crude form of ethyl 2-(4-((1S,4S)-2-oxa-5-azabicyclo[2.2.2]octan-5-yl)phenyl)acetate (112 mg, 0.41 mmol) synthesized in Reference Example 70. Subsequently, the title compound (white crystals, 16 mg, 18%) was obtained using (R)-1-(6-(trifluoromethyl)pyridin-3-yl)pyrrolidin-3-amine (42 mg, 0.18 mmol) synthesized in Reference Example 32-2 and a portion of the crude form of 2-(4-((1S,4S)-2-oxa-5-azabicyclo[2.2.2]octan-5-yl)phenyl)acetic acid (41 mg) synthesized as described above.

¹H NMR (CDCl₃, 400 MHz): δ = 1.6 - 1.8 (m, 1H), 1.8 - 2.0 (m, 2H), 2.0 - 2.3 (m, 2H), 2.3 - 2.4 (m, 1H), 3.0 - 3.2 (m, 1H), 3.3 - 3.5 (m, 3H), 3.49 (s, 2H), 3.6 - 3.7 (m, 1H), 3.7 - 3.9 (m, 2H), 4.0 - 4.1 (m, 2H), 4.1 - 4.2 (m, 1H), 4.5 - 4.7 (m, 1H), 5.5 - 5.6 (m, 1H), 6.59 (d, 2H, J = 8 Hz), 6.78 (dd, 1H, J = 3, 9 Hz), 7.08 (d, 2H, J = 8 Hz), 7.47 (d, 1H, J = 9 Hz), 7.99 (s, 1H).

MS: 461.17 [M+H]⁺

### Example 157

(R)-2-(benzo[d]thiazol-6-yl)-N-(1-(5-(trifluoromethyl)pyridin-3-yl)pyrrolidin-3-yl)acetamide

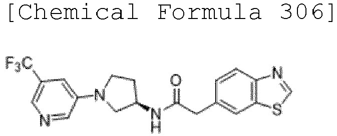

According to a technique similar to Example 1, the title compound (pale yellow amorphous, 33 mg, 61%) was obtained using (R)-1-(5-(trifluoromethyl)pyridin-3-yl)pyrrolidin-3-amine (30 mg, 0.13 mmol) synthesized in Reference Example 1-2 and 2-(benzo[d]thiazol-6-yl)acetic acid (31 mg, 0.16 mmol).

¹H NMR (CDCl₃, 400 MHz): δ = 1.9 - 2.0 (m, 1H), 2.3 - 2.4 (m, 1H), 3.1 - 3.2 (m, 1H), 3.3 - 3.5 (m, 2H), 3.6 - 3.7 (m, 1H), 3.74 (s, 2H), 4.6 - 4.7 (m, 1H), 5.6 - 5.8 (m, 1H), 6.90 (s, 1H), 7.41 (dd, 1H, J = 2, 8 Hz), 7.89 (d, 1H, J = 2 Hz), 8.0 - 8.1 (m, 1H), 8.11 (d, 1H, J = 8 Hz), 8.19 (s, 1H), 9.00 (s, 1H).

MS: 407.08 [M+H]⁺

### Example 158

(R)-2-(benzo[d]thiazol-6-yl)-N-(1-(6-(trifluoromethyl)pyridin-3-yl)pyrrolidin-3-yl)acetamide

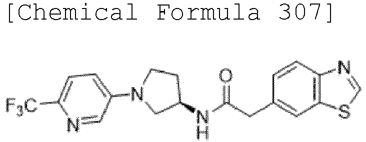

According to a technique similar to Example 1, the title compound (white crystals, 20 mg, 36%) was obtained using (R)-1-(6-(trifluoromethyl)pyridin-3-yl)pyrrolidin-3-amine (30 mg, 0.13 mmol) synthesized in Reference Example 32-2 and 2-(benzo[d]thiazol-6-yl)acetic acid (31 mg, 0.16 mmol) .

¹H NMR (CDCl₃, 400 MHz): δ = 1.9 - 2.0 (m, 1H), 2.3 - 2.4 (m, 1H), 3.1 - 3.2 (m, 1H), 3.3 - 3.5 (m, 2H), 3.6 - 3.7 (m, 1H), 3.73 (s, 2H), 4.6 - 4.7 (m, 1H), 5.7 - 5.8 (m, 1H), 6.76 (dd, 1H, J = 2, 8 Hz), 7.40 (d, 1H, J = 2 Hz), 7.45 (d, 1H, J = 8 Hz), 7.88 (s, 1H), 7.91 (d, 1H, J = 3 Hz), 8.10 (d, 1H, J = 8 Hz), 9.00 (s, 1H).

MS: 429.05 [M+Na]⁺

### Example 159

(R)-2-(1H-indazol-5-yl)-N-(1-(5-(trifluoromethyl)pyridin-3-yl)pyrrolidin-3-yl)acetamide

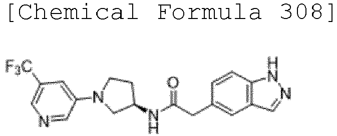

According to a technique similar to Example 1, the title compound (white crystals, 59 mg, 57%) was obtained using (R)-1-(5-(trifluoromethyl)pyridin-3-yl)pyrrolidin-3-amine (60 mg, 0.26 mmol) synthesized in Reference Example 1-2 and 2-(1H-indazol-5-yl)acetic acid (56 mg, 0.32 mmol).

¹H NMR (DMSO - d₆, 400 MHz): δ = 1.8 - 2.0 (m, 1H), 2.1 - 2.3 (m, 1H), 3.1 - 3.3 (m, 1H), 3.3 - 3.7 (m, 4H), 3.49 (s, 2H), 4.39 (br s, 1H), 7.11 (s, 1H), 7.25 (d, 1H, J = 8 Hz), 7.45 (d, 1H, J = 8 Hz), 7.59 (s, 1H), 8.00 (s, 1H), 8.15 (s, 1H), 8.21 (s, 1H), 8.3 - 8.5 (m, 1H).

MS: 390.12 [M+H]⁺

### Example 160

(R)-2-(1H-indazol-5-yl)-N-(1-(6-(trifluoromethyl)pyridin-3-yl)pyrrolidin-3-yl)acetamide

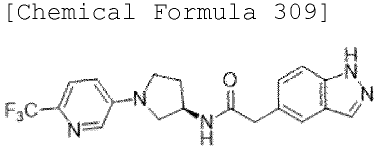

According to a technique similar to Example 1, the title compound (white crystals, 55 mg, 53%) was obtained using (R)-1-(6-(trifluoromethyl)pyridin-3-yl)pyrrolidin-3-amine (60 mg, 0.26 mmol) synthesized in Reference Example 32-2 and 2-(1H-indazol-5-yl)acetic acid (56 mg, 0.32 mmol).

¹H NMR (DMSO - d₆, 400 MHz): δ = 1.8 - 2.0 (m, 1H), 2.1 - 2.3 (m, 1H), 3.1 - 3.5 (m, 3H), 3.49 (s, 2H), 3.5 - 3.7 (m, 1H), 4.3 - 4.5 (m, 1H), 7.01 (dd, 1H, J = 3, 8 Hz), 7.24 (d, 1H, J = 8 Hz), 7.45 (d, 1H, J = 8 Hz), 7.5 - 7.7 (m, 2H), 7.99 (s, 1H), 8.04 (d, 2H, J = 3 Hz), 8.3 - 8.5 (m, 1H).

MS: 390.12 [M+H]⁺

### Example 161

2-(4-cyclopropylphenyl)-N-(3-(trifluoromethyl)-1-(5-(trifluoromethyl)pyridin-3-yl)pyrrolidin-3-yl)acetamide

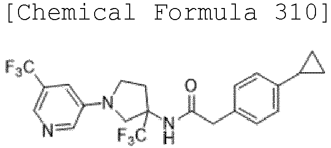

According to a technique similar to Example 3, a crude form of 3-(trifluoromethyl)-1-(5-(trifluoromethyl)pyridin-3-yl)pyrrolidin-3-amine (58 mg) was synthesized from tert-butyl (3-(trifluoromethyl)-1-(5-(trifluoromethyl)pyridin-3-yl)pyrrolidin-3-yl)carbamate (145 mg, 0.36 mmol) synthesized in Reference Example 71, and the title compound (white amorphous, 11 mg, 25%) was obtained using a portion of the crude form of 3-(trifluoromethyl)-1-(5-(trifluoromethyl)pyridin-3-yl)pyrrolidin-3-amine (29 mg) thus obtained and 2-(4-cyclopropylphenyl)acetic acid (26 mg, 0.15 mmol) synthesized in Reference Example 33-2.

¹H NMR (CDCl₃, 400 MHz): δ = 0.6 - 0.7 (m, 2H), 0.9 - 1.0 (m, 2H), 1.8 - 1.9 (m, 1H), 2.5 - 2.6 (m, 2H), 3.4 - 3.6 (m, 2H), 3.56 (s, 2H), 3.86 (d, 1H, J = 11 Hz), 3.96 (d, 1H, J = 11 Hz), 5.45 (br s, 1H), 6.94 (s, 1H), 7.40 (d, 2H, J = 8 Hz), 7.90 (d, 2H, J = 8 Hz), 8.0 - 8.2 (m, 1H), 8.2 - 8.3 (m, 1H).

MS: 458.13 [M+H]⁺

### Example 162

N-(3-(trifluoromethyl)-1-(5-(trifluoromethyl)pyridin-3-yl)pyrrolidin-3-yl)-2-(4-(trifluoromethyl)phenyl)acetamide

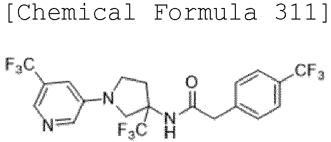

According to a technique similar to Example 3, a crude form of 3-(trifluoromethyl)-1-(5-(trifluoromethyl)pyridin-3-yl)pyrrolidin-3-amine (58 mg) was synthesized from tert-butyl (3-(trifluoromethyl)-1-(5-(trifluoromethyl)pyridin-3-yl)pyrrolidin-3-yl)carbamate (145 mg, 0.36 mmol) synthesized in Reference Example 71, and the title compound (white amorphous, 25 mg, 51%) was obtained using a portion of the crude form of 3-(trifluoromethyl)-1-(5-(trifluoromethyl)pyridin-3-yl)pyrrolidin-3-amine (29 mg) thus obtained and 2-(4-(trifluoromethyl)phenyl)acetic acid (30 mg, 0.15 mmol).

¹H NMR (CDCl₃, 400 MHz): δ = 2.5 - 2.7 (m, 2H), 3.4 - 3.6 (t, 2H, J = 7 Hz), 3.66 (s, 2H), 3.91 (d, 1H, J = 11 Hz), 3.95 (d, 1H, J = 11 Hz), 5.52 (br s, 1H), 6.96 (s, 1H), 7.37 (d, 2H, J = 8 Hz), 7.62 (d, 2H, J = 8 Hz), 8.1 - 8.2 (m, 1H), 8.2 - 8.3 (m, 1H).

MS: 486.09 [M+H]⁺

### Example 163

(R)-2-(1H-benzo[d][1,2,3]triazol-5-yl)-N-(1-(5-(trifluoromethyl)pyridin-3-yl)pyrrolidin-3-yl)acetamide

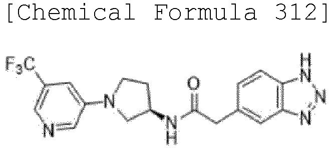

According to a technique similar to Example 1, the title compound (white crystals, 30 mg, 57%) was obtained using (R)-1-(5-(trifluoromethyl)pyridin-3-yl)pyrrolidin-3-amine (30 mg, 0.13 mmol) synthesized in Reference Example 1-2 and 2-(1H-benzo[d][1,2,3]triazol-5-yl)acetic acid (28 mg, 0.16 mmol).

¹H NMR (CD₃OD, 400 MHz): δ = 2.0 - 2.1 (m, 1H), 2.2 - 2.4 (m, 1H), 3.2 - 3.3 (m, 1H), 3.3 - 3.5 (m, 2H), 3.6 - 3.7 (m, 1H), 3.66 (s, 2H), 4.4 - 4.6 (m, 1H), 7.11 (s, 1H), 7.38 (d, 1H, J = 9 Hz), 7.7 - 7.8 (m, 2H), 8.04 (s, 1H), 8.07 (d, 1H, J = 3 Hz). The 2H content is not observable.

MS: 391.11 [M+H]⁺

### Example 164

(R)-2-(1H-benzo[d][1,2,3]triazol-5-yl)-N-(1-(6-(trifluoromethyl)pyridin-3-yl)pyrrolidin-3-yl)acetamide

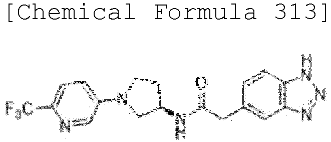

According to a technique similar to Example 1, the title compound (white crystals, 20 mg, 37%) was obtained using (R)-1-(6-(trifluoromethyl)pyridin-3-yl)pyrrolidin-3-amine (30 mg, 0.13 mmol) synthesized in Reference Example 32-2 and 2-(1H-benzo[d][1,2,3]triazol-5-yl)acetic acid (28 mg, 0.16 mmol).

¹H NMR (CD₃OD, 400 MHz): δ = 2.0 - 2.1 (m, 1H), 2.2 - 2.4 (m, 1H), 3.2 - 3.3 (m, 1H), 3.4 - 3.6 (m, 2H), 3.6 - 3.7 (m, 1H), 3.66 (s, 2H), 4.4 - 4.6 (m, 1H), 7.01 (dd, 1H, J = 3,9 Hz), 7.38 (d, 1H, J = 8 Hz), 7.52 (d, 1H, J = 9 Hz), 7.7 - 7.8 (m, 2H), 7.93 (d, 1H, J = 3 Hz). The 2H content is not observable.

MS: 391.11 [M+H]⁺

### Example 165

2-(4-(2-Oxa-5-azabicyclo[2.2.1]heptan-5-yl)phenyl)-N-((R)-1-(5-(trifluoromethyl)pyridin-3-yl)pyrrolidin-3-yl)acetamide

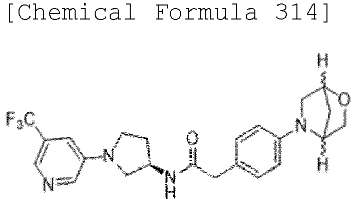

According to a technique similar to Example 38, a crude form of ethyl 2-(4-(2-oxa-5-azabicyclo[2.2.1]heptan-5-yl)phenyl)acetate (106 mg) was synthesized using ethyl 4-bromophenylacetate (109 mg, 0.45 mmol) and 2-oxa-5-azabicyclo[2.2.1]heptane hydrochloride (91 mg, 0.67 mmol), and subsequently, according to a technique similar to Example 155, a crude form of 2-(4-(2-oxa-5-azabicyclo[2.2.1]heptan-5-yl)phenyl)acetic acid (white crystals, 88 mg) was obtained from the crude form of ethyl 2-(4-(2-oxa-5-azabicyclo[2.2.1]heptan-5-yl)phenyl)acetate (106 mg) obtained as described above. Subsequently, diastereomer A of the title compound (pale yellow amorphous, 41 mg, 47%) was obtained using (R)-1-(5-(trifluoromethyl)pyridin-3-yl)pyrrolidin-3-amine (44 mg, 0.19 mmol) synthesized in Reference Example 1-2 and a portion of the crude form of 2-(4-(2-oxa-5-azabicyclo[2.2.1]heptan-5-yl)phenyl)acetic acid (44 mg) synthesized as described above.

¹H NMR (CDCl₃, 400 MHz): δ = 1.8 - 2.1 (m, 3H), 2.2 - 2.4 (m, 1H), 3.0 - 3.2 (m, 2H), 3.36 (t, 2H, J = 7 Hz), 3.49 (s, 2H), 3.54 (d, 1H, J = 9 Hz), 3.6 - 3.7 (m, 1H), 3.8 - 3.9 (m, 2H), 4.38 (s, 1H), 4.5 - 4.7 (m, 1H), 4.65 (s, 1H), 5.5 - 5.7 (m, 1H), 6.54 (d, 2H, J = 8 Hz), 6.90 (s, 1H), 7.07 (d, 2H, J = 8 Hz), 8.07 (s, 1H), 8.19 (s, 1H).

MS: 447.17 [M+H]⁺

### Example 166

2-(4-(2-Oxa-5-azabicyclo[2.2.1]heptan-5-yl)phenyl)-N-((R)-1-(6-(trifluoromethyl)pyridin-3-yl)pyrrolidin-3-yl)acetamide

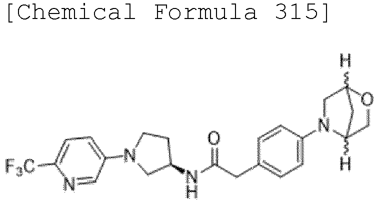

According to a technique similar to Example 165, diastereomer A of the title compound (pale yellow crystals, 41 mg, 47%) was obtained using (R)-1-(6-(trifluoromethyl)pyridin-3-yl)pyrrolidin-3-amine (44 mg, 0.19 mmol) synthesized in Reference Example 32-2 and a portion of the crude form of 2-(4-(2-oxa-5-azabicyclo[2.2.1]heptan-5-yl)phenyl)acetic acid (44 mg) synthesized in Example 165.

¹H NMR (CDCl₃, 400 MHz): δ = 1.8 - 2.1 (m, 3H), 2.2 - 2.4 (m, 1H), 3.1 - 3.2 (m, 2H), 3.38 (t, 2H, J = 7 Hz), 3.49 (s, 2H), 3.55 (d, 1H, J = 9 Hz), 3.6 - 3.7 (m, 1H), 3.8 - 3.9 (m, 2H), 4.38 (s, 1H), 4.5 - 4.7 (m, 1H), 4.65 (s, 1H), 5.5 - 5.7 (m, 1H), 6.54 (d, 2H, J = 8 Hz), 6.7 - 6.9 (m, 1H), 7.07 (d, 2H, J = 8 Hz), 7.47 (d, 1H, J = 8 Hz), 7.9 - 8.0 (m, 1H).

MS: 447.15 [M+H]⁺

### Example 167

(R)-2-(1-methyl-1H-benzo[d][1,2,3]triazol-5-yl)-N-(1-(5-(trifluoromethyl)pyridin-3-yl)pyrrolidin-3-yl)acetamide

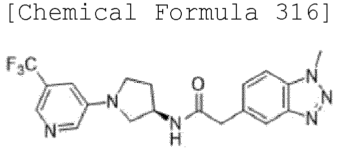

According to a technique similar to Example 1, the title compound (yellow amorphous, 39 mg, 64%) was obtained using (R)-1-(5-(trifluoromethyl)pyridin-3-yl)pyrrolidin-3-amine (35 mg, 0.15 mmol) synthesized in Reference Example 1-2 and 2-(1-methyl-1H-benzo[d][1,2,3]triazol-5-yl)acetic acid (30 mg, 0.16 mmol).

¹H NMR (CDCl₃, 400 MHz): δ = 1.9 - 2.1 (m, 1H), 2.2 - 2.4 (m, 1H), 3.1 - 3.2 (m, 1H), 3.2 - 3.4 (m, 2H), 3.5 - 3.7 (m, 1H), 3.37 (s, 2H), 4.27 (s, 3H), 4.6 - 4.7 (m, 1H), 6.48 (br s, 1H), 6.78 (s, 1H), 7.47 (d, 1H, J = 9 Hz), 7.50 (d, 1H, J = 9 Hz), 7.88 (s, 1H), 7.96 (d, 1H, J = 3 Hz), 8.10 (s, 1H).

MS: 405.12 [M+H]⁺

### Example 168

(R)-2-(1-methyl-1H-benzo[d][1,2,3]triazol-5-yl)-N-(1-(6-(trifluoromethyl)pyridin-3-yl)pyrrolidin-3-yl)acetamide

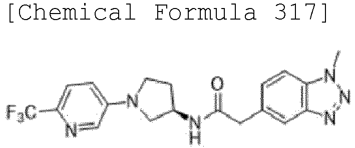

According to a technique similar to Example 1, the title compound (white crystals, 8 mg, 13%) was obtained using (R)-1-(6-(trifluoromethyl)pyridin-3-yl)pyrrolidin-3-amine (35 mg, 0.15 mmol) synthesized in Reference Example 32-2 and 2-(1-methyl-1H-benzo[d][1,2,3]triazol-5-yl)acetic acid (30 mg, 0.16 mmol).

¹H NMR (CD₃OD, 400 MHz): δ = 2.0 - 2.2 (m, 1H), 2.2 - 2.4 (m, 1H), 3.2 - 3.4 (m, 1H), 3.4 - 3.6 (m, 2H), 3.6 - 3.7 (m, 1H), 3.70 (s, 2H), 4.32 (s, 3H), 4.4 - 4.6 (m, 1H), 7.04 (dd, 1H, J = 3, 9 Hz), 7.52 (dd, 1H, J = 1, 8 Hz), 7.56 (d, 1H, J = 8 Hz), 7.69 (d, 1H, J = 8 Hz), 7.88 (s, 1H), 7.96 (d, 1H, J = 3 Hz). The 1H content is not observable.

MS: 405.12 [M+H]⁺

### Example 169

2-(4-cyclopropylphenyl)-N-(1-(4-(trifluoromethyl)phenyl)azetidin-3-yl)acetamide

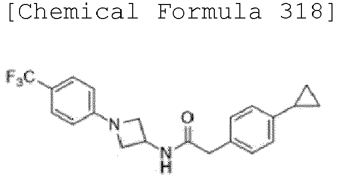

According to a technique similar to Example 9, the title compound (pale yellow solid, 33 mg, 50%) was obtained using N-(azetidin-3-yl)-2-(4-cyclopropylphenyl)acetamide (40 mg, 0.17 mmol) synthesized in Reference Example 75-2 and 1-bromo-4-(trifluoromethyl)benzene (29 µL, 0.21 mmol).

¹H NMR (CDCl₃, 400 MHz): δ = 0.6 - 0.8 (m, 2H), 0.9 - 1.1 (m, 2H), 1.8 - 2.0 (m, 1H), 3.5 - 3.7 (m, 4H), 4.23 (t, 2H, J = 8 Hz), 4.7 - 4.9 (m, 1H), 5.78 (d, 1H, J = 7 Hz), 6.39 (d, 2H, J = 8 Hz), 7.06 (d, 2H, J = 8 Hz), 7.13 (d, 2H, J = 8 Hz), 7.42 (d, 2H, J = 8 Hz).

MS: 397.12 [M+Na]⁺

### Example 170

2-(4-cyclopropylphenyl)-N-(1-(4-fluoro-3-(trifluoromethyl)phenyl)azetidin-3-yl)acetamide

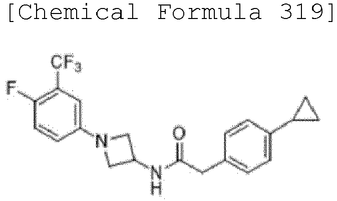

According to a technique similar to Example 9, the title compound (pale yellow solid, 27 mg, 38%) was obtained using N-(azetidin-3-yl)-2-(4-cyclopropylphenyl)acetamide (40 mg, 0.17 mmol) synthesized in Reference Example 75-2 and 4-bromo-1-fluoro-2-(trifluoromethyl)benzene (29 µL, 0.21 mmol).

¹H NMR (CDCl₃, 400 MHz): δ = 0.6 - 0.8 (m, 2H), 0.9 - 1.1 (m, 2H), 1.8 - 2.0 (m, 1H), 3.4 - 3.7 (m, 4H), 4.16 (t, 2H, J = 8 Hz), 4.7 - 4.9 (m, 1H), 5.78 (d, 1H, J = 6 Hz), 6.3 - 6.6 (m, 2H), 6.9 - 7.2 (m, 5H).

MS: 415.11 [M+Na]⁺

### Example 171

(R)-2-(4-bromophenyl)-N-(1-(5-(trifluoromethyl)pyridin-3-yl)pyrrolidin-3-yl)acetamide

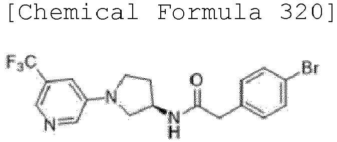

According to a technique similar to Example 1, the title compound (white solid, 100 mg, 100%) was obtained using (R)-1-(5-(trifluoromethyl)pyridin-3-yl)pyrrolidin-3-amine (52 mg, 0.22 mmol) synthesized in Reference Example 1-2 and 2-(4-bromophenyl)acetic acid (60 mg, 0.28 mmol).

¹H NMR (CDCl₃, 400 MHz): δ = 1.9 - 2.1 (m, 1H), 2.2 - 2.4 (m, 1H), 3.14 (dd, 1H, J = 4, 10 Hz), 3.36 (t, 2H, J = 7 Hz), 3.52 (s, 2H), 3.62 (dd, 1H, J = 6, 10 Hz), 4.6 - 4.7 (m, 1H), 6.22 (d, 1H, J = 7 Hz), 6.8 - 6.9 (m, 1H), 7.14 (d, 2H, J = 8 Hz), 7.46 (d, 2H, J = 8 Hz), 7.98 (d, 1H, J = 3 Hz), 8.13 (s, 1H).

MS: 428.02 [M+H]⁺

### Example 172

(R)-2-(4-cyclopropylphenyl)-N-(1-(6-fluorobenzo[d]thiazol-2-yl)pyrrolidin-3-yl)acetamide

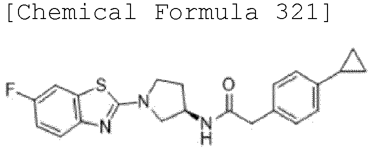

(R)-2-(4-cyclopropylphenyl)-N-(pyrrolidin-3-yl)acetamide (49 mg, 0.20 mmol) synthesized in Reference Example 74-2 was dissolved in DMF (1.0 mL), subsequently 2-chloro-6-fluorobenzo[d]thiazole (45 mg, 0.24 mmol) and cesium carbonate (78 mg, 0.24 mmol) were added thereto, and the mixture was stirred for 2 hours at 100°C

Saturated sodium hydrogen carbonate was added to the reaction liquid that had been left to cool to room temperature, and then the mixture was extracted with chloroform. An organic layer thus separated was dried over anhydrous sodium sulfate, insoluble materials were filtered, and then the solvent was distilled off under reduced pressure. A residue thus obtained was purified by silica gel column chromatography (heptane: ethyl acetate) (concentration gradient: 50 to 100%), and the title compound (white solid, 60 mg, 71%) was obtained.

¹H NMR (CDCl₃, 400 MHz): δ = 0.6 - 0.7 (m, 2H), 0.9-1.0 (m, 2H), 1.8 - 2.0 (m, 2H), 2.2 - 2.4 (m, 1H), 3.31 (dd, 1H, J = 5, 11 Hz), 3.5 - 3.7 (m, 2H), 3.53 (s, 2H), 3.82 (dd, 1H, J = 7, 11 Hz), 4.5 - 4.7 (m, 1H), 5.48 (d, 1H, J = 7 Hz), 6.9 - 7.1 (m, 3H), 7.11 (d, 2H, J = 8 Hz), 7.31 (dd, 1H, J = 3, 8 Hz), 7.48 (dd, 1H, J = 5, 9 Hz).

MS: 396.13 [M+H]⁺

### Example 173

(R)-N-(1-(5-bromothiazol-2-yl)pyrrolidin-3-yl)-2-(4-cyclopropylphenyl)acetamide

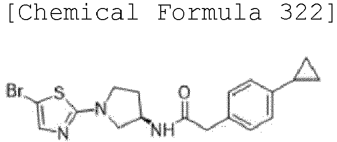

According to a technique similar to Example 172, the title compound (pale yellow solid, 26 mg, 32%) was obtained using (R)-2-(4-isopropylphenyl)-N-(pyrrolidin-3-yl)acetamide (49 mg, 0.20 mmol) synthesized in Reference Example 74-2 and 2,5-dibromothiazole (58 mg, 0.24 mmol).

¹H NMR (CDCl₃, 400 MHz): δ = 0.6 - 0.8 (m, 2H), 0.9-1.0 (m, 2H), 1.8 - 2.0 (m, 2H), 2.2 - 2.4 (m, 1H), 3.16 (dd, 1H, J = 5, 11 Hz), 3.3 - 3.5 (m, 2H), 3.52 (s, 2H), 3.67 (dd, 1H, J = 6, 10 Hz), 4.5 - 4.7 (m, 1H), 5.46 (d, 1H, J = 7 Hz), 7.0 - 7.1 (m, 3H), 7.11 (d, 2H, J = 8 Hz).

MS: 428.02 [M+Na]⁺

### Example 174

(R)-2-(4-cyclopropylphenyl)-N-(1-(4-fluoro-3-(trifluoromethyl)phenyl)pyrrolidin-3-yl) acetamide

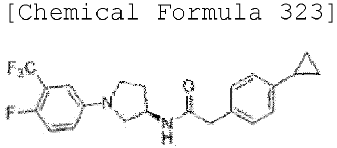

According to a technique similar to Example 9, the title compound (white solid, 102 mg, 84%) was obtained using (R)-2-(4-cyclopropylphenyl)-N-(pyrrolidin-3-yl)acetamide (73 mg, 0.30 mmol) synthesized in Reference Example 74-2 and 4-bromo-1-fluoro-2-(trifluoromethyl)benzene (51 µL, 0.36 mmol).

¹H NMR (CDCl₃, 400 MHz): δ = 0.6 - 0.8 (m, 2H), 0.9-1.1 (m, 2H), 1.8 - 2.0 (m, 2H), 2.2 - 2.4 (m, 1H), 3.04 (dd, 1H, J = 4, 9 Hz), 3.2 - 3.4 (m, 2H), 3.4 - 3.6 (m, 1H), 3.52 (s, 2H), 4.5 - 4.7 (m, 1H), 5.50 (d, 1H, J = 7 Hz), 6.5 - 6.7 (m, 2H), 6.9 - 7.2 (m, 5H).

MS: 407.16 [M+H]⁺

### Example 175

(R)-2-(4-cyclopropylphenyl)-N-(1-(3-methoxyphenyl)pyrrolidin-3-yl)acetamide

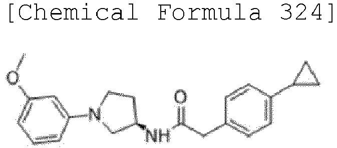

According to a technique similar to Example 9, the title compound (white solid, 203 mg, 58%) was obtained using (R)-2-(4-cyclopropylphenyl)-N-(pyrrolidin-3-yl)acetamide (244 mg, 1.0 mmol) synthesized in Reference Example 74-2 and 1-bromo-3-methoxybenzene (152 µL, 1.2 mmol) .

¹H NMR (CDCl₃, 400 MHz): δ = 0.6 - 0.7 (m, 2H), 0.9-1.0 (m, 2H), 1.7 - 1.9 (m, 2H), 2.1 - 2.3 (m, 1H), 3.05 (dd, 1H, J = 4, 10 Hz), 3.2 - 3.4 (m, 2H), 3.5 - 3.6 (m, 3H), 3.51 (s, 3H), 4.5 - 4.7 (m, 1H), 5.53 (d, 1H, J = 7 Hz), 6.07 (t, 1H, J = 2 Hz), 6.15 (dd, 1H, J = 1, 8 Hz), 6.28 (dd, 1H, J = 2, 8 Hz), 7.03 (d, 2H, J = 8 Hz), 7.0-7.2 (m, 3H).

MS: 351.19 [M+H]⁺

### Example 176

(R)-2-(4-(3,3-difluoropyrrolidin-1-yl)phenyl)-N-(1-(5-(trifluoromethyl)pyridin-3-yl)pyrrolidin-3-yl)acetamide

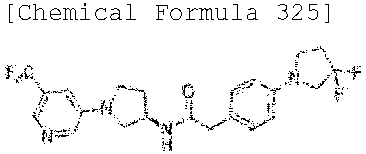

According to a technique similar to Example 9, the title compound (white solid, 11 mg, 24%) was obtained using (R)-2-(4-bromophenyl)-N-(1-(5-(trifluoromethyl)pyridin-3-yl)pyrrolidin-3-yl)acetamide (43 mg, 0.10 mmol) synthesized in Example 171 and 3,3-difluoropyrrolidine hydrochloride (17 mg, 0.12 mmol).

¹H NMR (CDCl₃, 400 MHz): δ = 1.8 - 2.0 (m, 1H), 2.2-2.4 (m, 1H), 2.4 - 2.6 (m, 2H), 3.10 (dd, 1H, J = 5, 10 Hz), 3.36 (t, 2H, J = 7 Hz), 3.4 - 3.6 (m, 4H), 3.6 - 3.8 (m, 3H), 4.5 - 4.7 (m, 1H), 5.52 (d, 1H, J = 7 Hz), 6.52 (d, 2H, J = 9 Hz), 6.90 (s, 1H), 7.11 (d, 2H, J = 8 Hz), 8.07 (s, 1H), 8.20 (s, 1H).

MS: 455.15 [M+H]⁺

### Example 177

Ethyl (R)-2-(3-(3-(2-(4-cyclopropylphenyl)acetamido)pyrrolidin-1-yl)phenoxy)-2-methylpropanoate

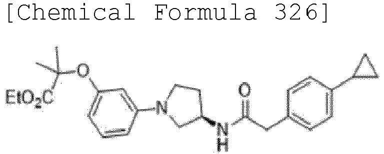

According to a technique similar to Example 9, the title compound (white solid, 43 mg, 24%) was obtained using (R)-2-(4-cyclopropylphenyl)-N-(pyrrolidin-3-yl)acetamide (98 mg, 0.40 mmol) synthesized in Reference Example 74-2 and ethyl 2-(3-bromophenoxy)-2-methylpropanoate (138 mg, 0.48 mmol) synthesized in Reference Example 76.

¹H NMR (CDCl₃, 400 MHz): δ = 0.6 - 0.8 (m, 2H), 0.9-1.0 (m, 2H), 1.25 (t, 3H, J = 7 Hz), 1.59 (s, 6H), 1.7-2.0 (m, 2H), 2.1 - 2.3 (m, 1H), 3.01 (dd, 1H, J = 4, 10 Hz), 3.1 - 3.4 (m, 2H), 3.4 - 3.6 (m, 3H), 4.22 (dd, 2H, J = 7, 15 Hz), 4.5 - 4.7 (m, 1H), 5.55 (d, 1H, J = 7 Hz), 6.0 - 6.1 (m, 1H), 6.1 - 6.2 (m, 2H), 7.0 - 7.1 (m, 3H), 7.11 (d, 2H, J = 8 Hz).

MS: 451.22 [M+H]⁺

### Example 178

(R)-2-(4-cyclopropylphenyl)-N-(1-(3-hydroxyphenyl)pyrrolidin-3-yl) acetamide

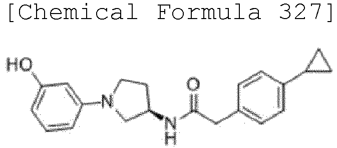

Pyridine hydrochloride (500 mg) was added to (R)-2-(4-cyclopropylphenyl)-N-(1-(3-methoxyphenyl)pyrrolidin-3-yl)acetamide (35 mg, 0.10 mmol) synthesized in Example 175, and the mixture was heated for 3 hours at 150°C

Saturated sodium hydrogen carbonate was added to the reaction liquid that had been left to cool to room temperature, and then the mixture was extracted with ethyl acetate. An organic layer thus separated was washed with saturated brine and dried over anhydrous sodium sulfate, insoluble materials were filtered, and then the solvent was distilled off under reduced pressure. A residue thus obtained was purified by NH silica gel column chromatography (heptane: ethyl acetate) (concentration gradient: 50 to 100%), and the title compound (white solid, 26 mg, 77%) was obtained.

¹H NMR (CDCl₃, 400 MHz): δ = 1.5 - 2.0 (m, 5H), 2.1-2.3 (m, 1H), 3.04 (dd, 1H, J = 4, 10 Hz), 3.1 - 3.4 (m, 2H), 3.4 - 3.6 (m, 3H), 4.5 - 4.7 (m, 1H), 4.8 - 5.0 (m, 1H), 5.54 (d, 1H, J = 7 Hz), 6.0 - 6.4 (m, 4H), 7.0 - 7.2 (m, 3H), 7.29 (d, 2H, J = 8 Hz).

MS: 337.17 [M+H]⁺

### Example 179

(R)-2-(4-cyclopropylphenyl)-N-(1-(6-(trifluoromethyl)pyridin-2-yl)pyrrolidin-3-yl)acetamide

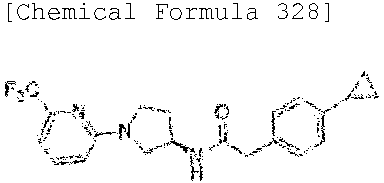

According to a technique similar to Example 172, the title compound (pale yellow solid, 21 mg, 26%) was obtained using (R)-2-(4-cyclopropylphenyl)-N-(pyrrolidin-3-yl)acetamide (49 mg, 0.20 mmol) synthesized in Reference Example 74-2 and 2-chloro-6-(trifluoromethyl)pyridine (44 mg, 0.24 mmol).

¹H NMR (CDCl₃, 400 MHz): δ = 0.6 - 0.7 (m, 2H), 0.9-1.0 (m, 2H), 1.8 - 2.0 (m, 2H), 2.2 - 2.4 (m, 1H), 3.24 (dd, 1H, J = 5, 11 Hz), 3.4 - 3.6 (m, 4H), 3.76 (dd, 1H, J = 6, 11 Hz), 4.5 - 4.7 (m, 1H), 5.60 (d, 1H, J = 7 Hz), 6.45 (d, 1H, J = 8 Hz), 6.90 (d, 1H, J = 7 Hz), 7.02 (d, 2H, J = 8 Hz), 7.10 (d, 2H, J = 8 Hz), 7.53 (t, 1H, J = 8 Hz).

MS: 412.13 [M+Na]⁺

### Example 180

(R)-2-(4-cyclopropylphenyl)-N-(1-(4,6-dimethoxypyrimidin-2-yl)pyrrolidin-3-yl) acetamide

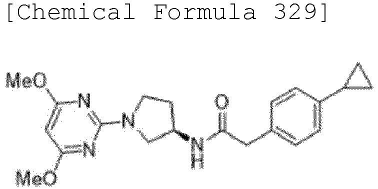

According to a technique similar to Example 172, the title compound (white solid, 56 mg, 73%) was obtained using (R)-2-(4-cyclopropylphenyl)-N-(pyrrolidin-3-yl)acetamide (49 mg, 0.20 mmol) synthesized in Reference Example 74-2 and 2-chloro-4,6-dimethoxypyridine (44 mg, 0.24 mmol).

¹H NMR (CDCl₃, 400 MHz): δ = 0.6 - 0.8 (m, 2H), 0.9-1.1 (m, 2H), 1.7 - 2.0 (m, 2H), 2.1 - 2.3 (m, 1H), 3.2-3.4 (m, 1H), 3.5 - 3.7 (m, 4H), 3.7 - 3.9 (m, 1H), 3.84 (s, 6H), 4.4 - 4.6 (m, 1H), 5.37 (s, 1H), 5.51 (d, 1H, J = 5 Hz), 7.03 (d, 2H, J = 8 Hz), 7.11 (d, 2H, J = 7 Hz).

MS: 405.16 [M+Na]⁺

### Example 181

(R)-2-(4-morpholinophenyl)-N-(1-(5-(trifluoromethyl)pyridin-3-yl)pyrrolidin-3-yl)acetamide

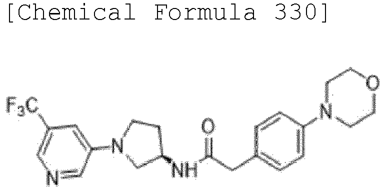

According to a technique similar to Example 9, the title compound (white solid, 12 mg, 13%) was obtained using (R)-2-(4-bromophenyl)-N-(1-(5-(trifluoromethyl)pyridin-3-yl)pyrrolidin-3-yl)acetamide (86 mg, 0.20 mmol) synthesized in Example 171 and morpholine (35 µL, 0.40 mmol).

¹H NMR (CDCl₃, 400 MHz): δ = 1.8 - 2.0 (m, 1H), 2.2-2.4 (m, 1H), 3.0 - 3.2 (m, 5H), 3.37 (t, 2H, J = 7 Hz).3.51 (s, 2H), 3.65 (dd, 1H, J = 7, 10 Hz), 3.8 - 3.9 (m, 4H), 4.5 - 4.7 (m, 1H), 5.4 - 5.6 (m, 1H), 6.8 - 7.0 (m, 3H), 7.14 (d, 2H, J = 8 Hz), 8.07 (d, 1H, J = 2 Hz), 8.20 (s, 1H).

MS: 435.17 [M+H]⁺

### Example 182

(R)-2-(4-cyclopropylphenyl)-N-(1-(4-(trifluoromethyl)pyridin-2-yl)pyrrolidin-3-yl)acetamide

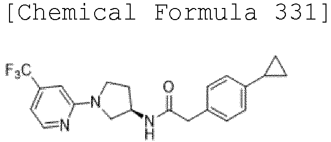

According to a technique similar to Example 172, the title compound (white solid, 40 mg, 51%) was obtained using (R)-2-(4-cyclopropylphenyl)-N-(pyrrolidin-3-yl)acetamide (49 mg, 0.20 mmol) synthesized in Reference Example 74-2 and 2-chloro-4-(trifluoromethyl)pyridine (44 mg, 0.24 mmol) .

¹H NMR (CDCl₃, 400 MHz): δ = 0.6 - 0.7 (m, 2H), 0.9-1.0 (m, 2H), 1.8 - 2.0 (m, 2H), 2.2 - 2.4 (m, 1H), 3.23 (dd, 1H, J = 5, 11 Hz), 3.4 - 3.6 (m, 4H), 3.76 (dd, 1H, J = 6, 10 Hz), 4.5 - 4.7 (m, 1H), 5.49 (d, 1H, J = 6 Hz), 6.48 (s, 1H), 6.73 (d, 1H, J = 5 Hz), 7.03 (d, 2H, J = 8 Hz), 7.11 (d, 2H, J = 8 Hz), 8.25 (d, 1H, J = 5 Hz).

MS: 390.14 [M+H]⁺

### Example 183

(R)-2-(4-(trifluoromethyl)phenyl)-N-(1-(6-(trifluoromethyl)pyridin-3-yl)pyrrolidin-3-yl)acetamide

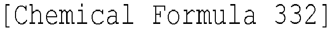

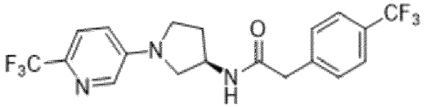

According to a technique similar to Example 9, the title compound (pale yellow solid, 24 mg, 29%) was obtained using (R)-N-(pyrrolidin-3-yl)-2-(4-(trifluoromethyl)phenyl)acetamide (55 mg, 0.20 mmol) synthesized in Reference Example 73-2 and 5-bromo-2-(trifluoromethyl)pyridine (54 mg, 0.24 mmol).

¹H NMR (CDCl₃, 400 MHz): δ = 1.9 - 2.1 (m, 1H), 2.3-2.5 (m, 1H), 3.19 (dd, 1H, J = 4, 11 Hz), 3.3 - 3.5 (m, 2H), 3.62 (s, 2H), 3.67 (dd, 1H, J = 6, 8 Hz), 4.6 - 4.7 (m, 1H), 5.70 (d, 1H, J = 6 Hz), 6.79 (dd, 1H, J = 3, 9 Hz), 7.40 (d, 2H, J = 8 Hz), 7.47 (d, 1H, J = 9 Hz), 7.61 (d, 2H, J = 8 Hz), 7.95 (d, 1H, J = 2 Hz).

MS: 418.11 [M+H]⁺

### Example 184

(R)-N-(1-(4-fluoro-3-(trifluoromethyl)phenyl)pyrrolidin-3-yl)-2-(4-(trifluoromethyl)phenyl)acetamide

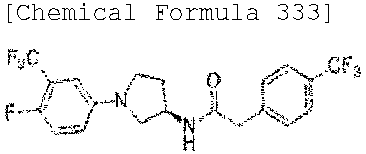

According to a technique similar to Example 9, the title compound (pale yellow solid, 22 mg, 25%) was obtained using (R)-N-(pyrrolidin-3-yl)-2-(4-(trifluoromethyl)phenyl)acetamide (55 mg, 0.20 mmol) synthesized in Reference Example 73-2 and 4-bromo-1-fluoro-2-(trifluoromethyl)benzene (58 mg, 0.24 mmol).

¹H NMR (CDCl₃, 400 MHz): δ = 1.8 - 2.0 (m, 1H), 2.2-2.4 (m, 1H), 3.11 (dd, 1H, J = 4, 10 Hz), 3.2 - 3.5 (m, 2H), 3.57 (dd, 1H, J = 6, 10 Hz), 3.61 (s, 2H), 4.5 - 4.7 (m, 1H), 5.56 (d, 1H, J = 6 Hz), 6.5 - 6.7 (m, 2H), 7.05 (t, 1H, J = 9 Hz), 7.39 (d, 2H, J = 8 Hz), 7.61 (d, 2H, J = 8 Hz).

MS: 435.11 [M+H]⁺

### Example 185

2-(4-(Trifluoromethyl)phenyl)-N-((R)-1-(6-(trifluoromethyl)pyridin-3-yl)pyrrolidin-3-yl)propanamide

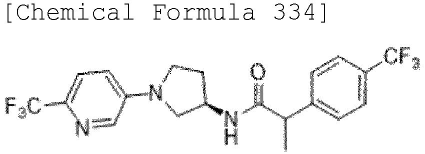

Tert-butyl (R)-3-(2-(4-(trifluoromethyl)phenyl)acetamido)pyrrolidine-1-carbamate (149 mg, 0.40 mmol) synthesized in Reference Example 73-1 was dissolved in DMF (2 mL), sodium hydride (60% oil, 24 mg, 0.60 mmol) and methyl iodide (37 µL, 0.60 mmol) were added thereto, and the mixture was stirred for one hour at room temperature. Saturated sodium hydrogen carbonate was added to a residue thus obtained under ice cooling, and then the mixture was extracted with chloroform. An organic layer thus separated was dried over anhydrous sodium sulfate, insoluble materials were filtered, subsequently the solvent was distilled off under reduced pressure, and thereby a crude form of tert-butyl (3R)-3-(2-(4-trifluoromethyl)phenyl)propanamido)pyrrolidine-1-carbamate was obtained. Subsequently, according to a technique similar to Example 36, the title compound (white solid, 25 mg, 15% in three stages) was obtained using the crude form of tert-butyl (3R)3-(2-(4-trifluoromethyl)phenyl)propanamido)pyrrolidine-1-carbamate thus obtained and 5-bromo-2-(trifluoromethyl)pyridine (92 mg, 0.41 mmol).

¹H NMR (CDCl₃, 400 MHz): δ = 1.53 (d, 3H, J = 7 Hz), 1.9 - 2.1 (m, 1H), 2.3 - 2.5 (m, 1H), 3.07 (dd, 1H, J = 4, 10 Hz), 3.3 - 3.5 (m, 2H), 3.5 - 3.7 (m, 2H), 4.5 - 4.7 (m, 1H), 5.69 (d, 1H, J = 6 Hz), 6.75 (dd, 1H, J = 3, 7 Hz), 7.3 - 7.5 (m, 3H), 7.57 (d, 2H, J = 8 Hz), 7.89 (d, 1H, J = 3 Hz) .

MS: 454.09 [M+Na]⁺

### Example 186

(1S,2S)-2-(1H-benzo[d]imidazol-2-yl)-N-((R)-1-(5-(trifluoromethyl)pyridin-3-yl)piperidin-3-yl)cyclopropane-1-carboxyamide

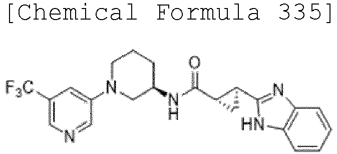

According to a technique similar to Example 3, a crude form of (R)-1-(5-(trifluoromethyl)pyridin-3-yl)piperidin-3-amine (166 mg) was synthesized from tert-butyl (R)-(1-(5-(trifluoromethyl)pyridin-3-yl)piperidin-3-yl)carbamate (76 mg, 0.219 mmol) synthesized in Reference Example 66, and the title compound (white powder, 70 mg, 74%) was obtained using (1S,2S)-2-(1H-benzo[d]imidazol-2-yl)cyclopropane-1-carboxylic acid (66 mg, 0.329 mmol).

¹H NMR (DMSO - d₆, 400 MHz): δ = 1.4 - 1.6 (m, 4H), 1.7 - 1.9 (m, 2H), 2.2 - 2.5 (m, 2H), 2.88 (dd, 1H, J = 9, 12 Hz), 2.9 - 3.1 (m, 1H), 3.6 - 3.9 (m, 3H), 7.0 - 7.2 (m, 2H), 7.3 - 7.6 (m, 3H), 8.24 (s, 1H), 8.37 (d, 1H, J = 7 Hz), 8.56 (d, 1H, J = 3 Hz), 12.4 (br s, 1H).

MS: 430.18 [M+H]⁺

### Example 187

(R)-2-(1H-indol-6-yl)-N-(1-(4-(trifluoromethyl)thiazol-2-yl)pyrrolidin-3-yl) acetamid e

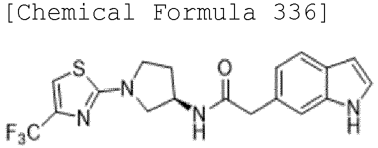

According to a technique similar to Example 1, the title compound (pale yellow powder, 36 mg, 58%) was obtained using (R)-1-(4-(trifluoromethyl)thiazol-2-yl)pyrrolidin-3-amine (38 mg, 0.160 mmol) synthesized in Reference Example 12-2 and 2-(1H-indol-6-yl)acetic acid (34 mg, 0.192 mmol).

¹H NMR (CDCl₃, 400 MHz): δ = 1.7 - 1.9 (m, 1H), 2.2-2.4 (m, 1H), 3.19 (dd, 1H, J = 5, 11 Hz), 3.3 - 3.6 (m, 2H), 3.69 (s, 2H), 3.72 (dd, 1H, J = 6, 11 Hz), 4.5 - 4.7 (m, 1H), 5.59 (d, 1H, J = 6 Hz), 6.5 - 6.6 (m, 1H), 6.89 (d, 1H, J = 1 Hz), 6.95 (dd, 1H, J = 1, 8 Hz)7.2 - 7.3 (m, 2H), 7.61 (d, 1H, J = 8 Hz), 8.25 (br s, 1H).

MS: 417.07 [M+Na]⁺

### Example 188

### Pharmacological test 1

T-type calcium blocking action

### (Testing method)

### (1) Construction of cells steadily expressing human Cav3.2 channels

A sequence obtained by adding HindIII site and a kozak sequence (GCCACC) to the 5'-side of human Cav3.2 channel ORF (Open Reading Frame) gene and adding a KpnI site to the 3'-side of the gene, was incorporated into pcDNA3.1(+) (Thermo Fisher Scientific, #V790-20), and the plasmid was introduced into HEK293 cells (ATCC No. CRL-1573) according to the protocol of FuGENE (registered trademark) HD Transfection Reagent (Promega, #E2311).

### (2) Method for measuring intracellular calcium concentration

The cells steadily expressing human Cav3.2 channels produced by testing method (1) were seeded on a 96-well plate (Dulbecco's modified Eagle medium (DMEM) medium containing 10% fetal bovine serum (FBS) was used as a medium). The cells were seeded and cultured for 48 hours under conditions of 37°C and 5% CO₂. Thereafter, the cells were washed with buffer A in order to remove the medium. Each well was treated with 80 pL of a fluorescent dye solution produced by mixing 22 µg of a fluorescent calcium indicator, Fluo-4 AM (Dojindo Laboratories., #F311), 20 pL of DMSO, 1 pL of 10% Pluronic F-127 (Thermo Fisher Scientific, #P6866), and 4 mL of buffer A. The cells were allowed to stand for 45 minutes at room temperature under light-shielded conditions, and then washed with buffer B in order to completely replace the solution. 70 µL of DMSO having the same concentration as that of a test compound as a negative control and 70 pL of a mixture containing 1 µM of a known blocker RQ-00311651 and the test compound as a positive control were each added to each well in a form of being contained in the buffer B. The test compound was dissolved in 100% DMSO and added at a final concentration of 0.1%. Then, the cells were further allowed to stand at room temperature under light-shielded conditions for 15 minutes and placed in a microplate reader EnVision (PerkinElmer). Background fluorescence was measured in each well, and then a 5 pg/mL grammicidin solution was added to buffer B at 10 pL/well. After 90 seconds, baseline fluorescence was measured. Thereafter, 83.8 mM KC1 was added to buffer B at 20 pL/well, and an increase in fluorescence caused by calcium influx generated by the stimulus was measured for 10 seconds. A blocking percentage was calculated from a maximum fluorescence increase value from a baseline, a logarithmic value of a compound concentration and blockery activity were plotted, and thereby an IC₅₀ value was calculated. In the measurement, fluorescence at 510 nm that was emitted when the cells were irradiated with excitation light at 485 nm was observed.

### (Buffer composition)

Buffer A: 140 mM NaCl, 5 mM KCl, 1 mM MgCl₂, 0.5 mM CaCl₂, 10 mM Glucose, 10 mM HEPES, pH 7.3
Buffer B: 137.9 mM Choline-Cl, 4.1 mM KCl, 1 mM MgCl₂, 0.5 mM CaCl₂, 10 mM Glucose, 10 mM HEPES, pH 7.3

### (Test results)

The test results are presented in Tables 1 to 3. As a result, it has been found that all the compounds in Examples tested this time had a blocking action on Cav3.2 channels.

In addition, a Cav3.1 channel blocking action in the compound of Example 144 was examined in a similar manner except that the Cav3.2 channel ORF gene was replaced with a Cav3.1 channel ORF gene and that the concentration of CaCl₂ in each of buffer A and buffer B was changed to 1.0 mM. As a result, an IC₅₀ value thereof was 0.47 pM.

**[Table 1]**

| Test compound | IC₅₀ (µm) | Test compound | IC₅₀ (µm) | Test compound | IC₅₀ (µm) |
|---|---|---|---|---|---|
| Example 1 | 0.013 | Example 65 | 8.9 | Example 126 | 0.83 |
| Example 2 | 1.3 | Example 66 | 0.069 | Example 127 | 1.1 |
| Example 3 | 0.85 | Example 67 | 0.029 | Example 128 | >10 |
| Example 4 | 0.014 | Example 68 | 0.075 | Example 129 | 0.55 |
| Example 5 | 0.29 | Example 69 | 1.2 | Example 130 | >10 |
| Example 6 | 0.13 | Example 70 | 0.21 | Example 131 | 0.20 |
| Example 7 | 0.097 | Example 71 | 0.0079 | Example 132 | 0.19 |
| Example 8 | 0.20 | Example 72 | 0.21 | Example 133 | 0.055 |
| Example 9 | 0.48 | Example 73 | 1.0 | Example 134A | 0.93 |
| Example 10 | 0.41 | Example 74 | 0.13 | Example 134B | 1.0 |
| Example 11 | 0.18 | Example 75 | 0.31 | Example 135 | 0.23 |
| Example 12 | 0.28 | Example 76 | 0.067 | Example 136 | 1.5 |
| Example 13 | 0.018 | Example 77 | 0.20 | Example 137A | 0.10 |
| Example 14 | 5.2 | Example 78 | 0.27 | Example 137B | 0.82 |
| Example 15 | 0.37 | Example 79 | 0.15 | Example 138 | 0.69 |
| Example 16 | 0.32 | Example 80 | 0.72 | Example 139 | 0.0054 |
| Example 17 | 0.38 | Example 81 | 0.55 | Example 140 | 1.1 |
| Example 18 | 0.090 | Example 82 | 0.75 | Example 141 | 0.18 |
| Example 19 | 3.5 | Example 83 | 0.082 | Example 142 | 0.0094 |
| Example 20 | >10 | Example 84 | 0.18 | Example 143 | 0.12 |

**[Table 2]**

| | | | | | |
|---|---|---|---|---|---|
| Example 21 | 1.2 | Example 85 | 0.11 | Example 144 | 0.022 |
| Example 22 | 1.6 | Example 86 | 0.37 | Example 145 | 0.13 |
| Example 23 | 0.15 | Example 87 | 0.0054 | Example 146 | 0.023 |
| Example 24 | 0.72 | Example 88 | 0.019 | Example 147 | 0.17 |
| Example 25 | 2.0 | Example 89 | 0.43 | Example 148 | 0.20 |
| Example 26 | 0.15 | Example 90 | 0.13 | Example 149 | 0.069 |
| Example 27 | 0.70 | Example 91 | 0.032 | Example 150 | 0.40 |
| Example 28 | 0.20 | Example 92 | 0.072 | Example 151 | 2.7 |
| Example 29 | 0.040 | Example 93 | 0.34 | Example 152 | 1.3 |
| Example 30 | 0.65 | Example 94 | 0.91 | Example 153 | 0.57 |
| Example 31 | 0.21 | Example 95 | 0.23 | Example 154 | 1.0 |
| Example 32 | 0.15 | Example 96 | 0.41 | Example 155 | 0.075 |
| Example 33 | 1.0 | Example 97 | 0.25 | Example 156 | 1.3 |
| Example 34 | 0.24 | Example 98 | 1.1 | Example 157 | 0.28 |
| Example 35 | 0.63 | Example 99A | 0.090 | Example 158 | 1.1 |
| Example 36 | 0.98 | Example 99B | 0.48 | Example 159 | 0.51 |
| Example 37 | 0.027 | Example 100A | 0.0093 | Example 160 | 6.4 |
| Example 38 | 0.035 | Example 100B | 0.46 | Example 161 | 0.26 |
| Example 39 | 0.022 | Example 101 | 0.10 | Example 162 | 0.56 |
| Example 40 | 0.30 | Example 102 | 0.15 | Example 163 | 1.4 |

**[Table 3]**

| | | | | | |
|---|---|---|---|---|---|
| Example 41 | 0.025 | Example 103 | 0.14 | Example 164 | 2.9 |
| Example 42 | 0.25 | Example 104 | 0.46 | Example 165 | 0.030 |
| Example 43 | 0.19 | Example 105 | 0.24 | Example 166 | 0.22 |
| Example 44 | 0.014 | Example 106A | 0.011 | Example 167 | 0.16 |
| Example 45 | 0.011 | Example 106B | 0.4 | Example 168 | 0.17 |
| Example 46 | 0.26 | Example 107A | 0.0047 | Example 169 | >10 |
| Example 47 | 0.35 | Example 107B | 0.56 | Example 170 | 0.88 |
| Example 48 | 0.24 | Example 108A | 0.0057 | Example 171 | 0.11 |
| Example 49 | 0.0071 | Example 108B | 0.2 | Example 172 | 0.55 |
| Example 50 | 0.68 | Example 109 | 0.24 | Example 173 | 1.1 |
| Example 51 | 0.12 | Example 110 | 0.29 | Example 174 | 0.20 |
| Example 52 | 0.11 | Example 111 | 9.9 | Example 175 | 0.49 |
| Example 53 | 1.0 | Example 112 | 0.11 | Example 176 | 0.0073 |
| Example 54 | 0.087 | Example 113 | 0.10 | Example 177 | 0.30 |
| Example 55 | 0.47 | Example 114 | 1.2 | Example 178 | 0.59 |
| Example 56 | 0.29 | Example 115 | 0.091 | Example 179 | 0.24 |
| Example 57 | 0.020 | Example 116 | 0.040 | Example 180 | 2.1 |
| Example 58 | 4.4 | Example 117 | 0.071 | Example 181 | 0.025 |
| Example 59 | 1.8 | Example 118 | 2.1 | Example 182 | 0.72 |
| Example 60 | 0.47 | Example 119 | 2.3 | Example 183 | 0.20 |
| Example 61A | 1.1 | Example 120 | 0.82 | Example 184 | 3.0 |
| Example 61B | 2.2 | Example 121 | 0.12 | Example 185 | 1.4 |
| Example 62 | 0.41 | Example 122 | 1.5 | Example 186 | 0.19 |
| Example 63A | 0.19 | Example 123 | 0.044 | Example 187 | 0.51 |
| Example 63B | >10 | Example▪124 | 0.062 | | |
| Example 64 | 0.037 | Example 125 | >10 | | |

### Example 189

### Pharmacological test 2

### Histamine-induced pruritus test (intradermal administration)

Using 7-week-old male C57BL/6J mice (Jackson Laboratory), an action on pruritus was examined using a change in scratching behavior time as an index.

The experiment was performed with 3 to 6 mice per group. Histamine was dissolved in Posphate Bufferd Saline (PBS) to prepare a histamine solution. The compound of Example 144 (hereinafter referred to as "compound A") was suspended in a 1% aqueous solution of methyl cellulose to prepare an administration solution.

Mice were left in a test chamber for five minutes or more in order to be acclimatized to the environment. Thereafter, mice were administered ID with a mixed solution of compound A 3 µg + histamine 100 µg/site, a mixed solution of compound A 30 µg + histamine 100 µg/site, or a mixed solution of compound A 300 µg + histamine 100 µg/site. As a control group, mice were administered ID with a mixed solution of vehicle and histamine solution. For 30 minutes after administration, scratching behavior time with hind paw at administration site was accumulated every five minutes.

The scratching behavior time data was analyzed by one-way ANOVA followed by Dunnett's test or two-way ANOVA followeded by Bonferroni post-hoc test. A significance level was 5% (compared to the control group). GraphPad Prism 7.04 (GraphPad Software, Inc.) was used for statistical calculation, and the data was indicated as mean values and standard errors.

### (Test results)

Results are illustrated in Figs. 1A and 1B. Scratching behavior was not caused by administration of PBS alone, and mice caused scratching behavior by administration of the histamine solution. Intradermal administration of compound A at the same time as the histamine solution reduced the scratching behavior time depending on the dose of compound A. Therefore, this indicates that compound A suppresses histamine-induced scratching behavior in dose dependent manner.

### Example 190

### Pharmacological test 3

### Histamine-induced pruritus test (oral administration)

### (Testing method)

The experiment was performed with 3 to 8 mice per group. Mice were fasted for 90 minutes or more. Thereafter, mice were forcibly gastrically administered with compound A (1, 3, 10, or 30 mg/kg) as drug or vehicle as a control group using a disposable syringe and a mouse sonde. After one hour, mice were administered ID with 100 µg/site of histamine solution at their neck to cause pruritus. For 30 minutes after histamine administration, scratching behavior time with hind paw at administration site was accumulated every five minutes.

Data analysis was performed by a similar means to the above.

### (Test results)

Results are illustrated in Figs. 2A and 2B. The scratching behavior time was reduced depending on the dose of compound A similarly to pharmacological test 2. Therefore, this indicates that compound A suppresses histamine-induced scratching behavior in dose dependent manner. In addition, gastrically administered compound A suppressed the scratching behavior caused by intradermal administration. Therefore, this indicates that oral administration is possible.

The above results indicate that compound A has a pruritus suppressing action. In addition, various routes of administration are available.

### Example 191

### Pharmacological test 4

### Chloroquine-induced pruritus test

Chloroquine, which is an antimalarial drug, is known to cause itch resistant to an antihistamine agent as a side effect.

### (Testing method)

Groups of 8 male ICR mice weighing 23 ± 3 g were used. A day before testing, hair was removed at the administration sites by clippers. On the testing day, the mice were individually placed in an observation cage to acclimate for 30 minutes, followed by administration of test compounds or chloroquine. 60 minutes before chloroquine administration, mice were p.o. administered with a medium or compound (10 or 30 mg/kg). Chloroquine at 5 mg/kg was administered SC into the rostral of the back with a 27 gauge needle. The scratching behaviors were recorded for 30 minutes by visual observation immediately after chloroquine administration. The number of scratching behaviors toward the administration site was counted. That is, scratching behavior toward other sites such as ears and face was excluded. All values represented mean ± SEM for number of scratches in individual groups.

### (Test results)

Results are illustrated in Fig. 3. The number of scratching behaviors due to chloroquine administration tended to decrease depending on the dose of compound A. This indicates that compound A suppresses non-histaminergic pruritus in dose dependent manner.

### Example 192

### Pharmacological test 5

### Substance P-induced pruritus test

### (Testing method)

Groups of 8 male ICR mice weighing 23 ± 3 g were used. A day before testing, hair was removed at the administration sites by clippers. On the testing day, the mice were individually placed in an observation cage (chamber) to acclimate for 30 minutes, followed by administration of test compounds or substance P. 60 minutes before substance P administration, mice were p.o. administered with a medium or compound (10 or 30 mg/kg). Substance P (250 nmol/site) was administered ID in a volume of 50 µL/site into the rostral of the back with an insulin syringe. The scratching behaviors were recorded for 30 minutes by visual observation immediately after substance P administration. The number of scratching behaviors toward the administration site was counted. That is, scratching behavior toward other sites such as ears and face was excluded. All values represented mean ± SEM for number of scratches in individual groups.

### (Test results)

Results are illustrated in Fig. 4. The number of scratching behaviors due to substance P administration tended to be significantly reduced by administration of compound A. It was suggested that compound A strongly suppressed pruritus by suppressing histaminergic pruritus and non-histaminergic pruritus.

### Industrial Applicability

The compound of the present invention has a blocking action on Cav3.2 channels and can be used as a medicament for treating or preventing pruritus.

## Claims

1. A medicament for treating or preventing pruritus, the medicament comprising a compound represented by the following General Formula (I), a tautomer of the compound, a stereoisomer of the compound, a pharmaceutically acceptable salt thereof, or a solvate thereof as an active ingredient: 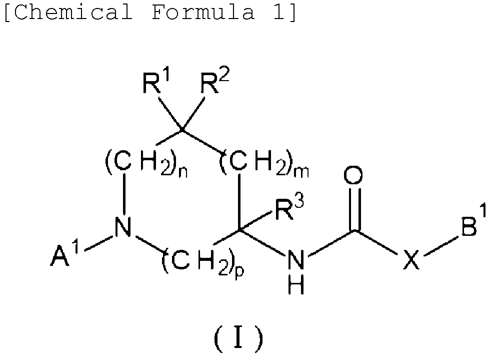
wherein A¹ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one or two nitrogen atoms and carbon atoms, while herein, the heteroaryl ring or the heterocondensed ring may have a substituent and is bonded to a nitrogen atom of the adjacent cyclic amine by means of a carbon atom constituting these rings;
B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one or two nitrogen atoms and carbon atoms, while herein, the heteroaryl ring or the heterocondensed ring may have a substituent and is bonded to adjacent X by means of a carbon atom constituting these rings;
R¹ and R², which may be identical or different, each represent a hydrogen atom, a halogen atom, a hydroxy group, a C₁₋₈ alkyl group, or a C₁₋₈ alkyl group substituted with one to three halogen atoms; or
R¹, R², and the carbon atom to which R¹ and R² are bonded may be joined together and form a 3- to 5-membered cycloalkyl group;
R³ represents a hydrogen atom, a halogen atom, a carboxyl group, a cyano group, a carbamoyl group, a C₁₋₈ alkyl group, a C₁₋₈ alkoxycarbonyl group, a C₁₋₈ alkoxy-substituted C₁₋₈ alkyl group, a C₁₋₈ alkyl group substituted with a hydroxy group, a C₁₋₈ alkyl group substituted with one to three halogen atoms, or a C₁₋₈ alkyl group substituted with an acyloxy group; or
R² and R³ may be joined together and form methylene or ethylene;
X represents: 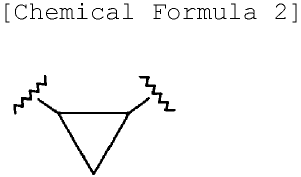
or 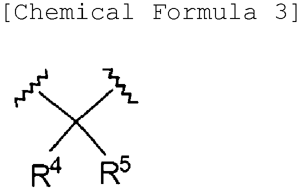
here, the wavy line represents a bonding position;
R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group; or
R⁴, R⁵, and the carbon atom to which R⁴ and R⁵ are bonded may be joined together and form a 3- to 5-membered cycloalkyl group;
n and m, which may be identical or different, each represent 0 or 1; and
p represents 1 or 2;
further, the substituent that may be carried by the phenyl of A¹, the heteroaryl ring of A¹, and the heterocondensed ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group.

2. The medicament according to claim 1, wherein
X represents:

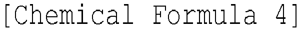

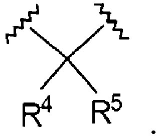

3. The medicament according to claim 1 or 2, wherein A¹ represents a phenyl which may have a substituent.

4. The medicament according to claim 1 or 2, wherein A¹ represents a phenyl which may have a substituent, or a 4-membered to 6-membered heteroaryl ring composed of one to three identical or different heteroatoms selected from an oxygen atom, a sulfur atom, and a nitrogen atom and carbon atoms as ring-constituting atoms which may have a substituent.

5. The medicament according to claim 4, wherein the heteroaryl ring of A¹ is thiophene, oxazole, thiazole, pyridine, pyrazine, pyrimidine, or pyridazine.

6. The medicament according to claim 4, wherein the heteroaryl ring of A¹ is pyridine.

7. The medicament according to claim 1 or 2, wherein A¹ represents a heterocondensed ring composed of a 4-membered to 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.

8. The medicament according to claim 7, wherein the heterocondensed ring of A¹ is quinoline or benzo[d]thiazole.

9. The medicament according to any one of claims 1 to 8, wherein B¹ represents a phenyl which may have a substituent.

10. The medicament according to any one of claims 1 to 8, wherein B¹ represents a phenyl which may have a substituent, or a 5-membered or 6-membered heteroaryl ring composed of one to three identical or different heteroatoms selected from an oxygen atom, a sulfur atom, and a nitrogen atom and carbon atoms as ring-constituting atoms which may have a substituent.

11. The medicament according to claim 10, wherein the heteroaryl ring of B¹ is pyridine.

12. The medicament according to any one of claims 9 to 11, wherein when B¹ represents a phenyl which may have a substituent or a 6-membered heteroaryl ring which may have a substituent, the substituent is substituted at a 4-position (para-position).

13. The medicament according to any one of claims 1 to 8, wherein B¹ represents a heterocondensed ring composed of a 5-membered or 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.

14. The medicament according to claim 13, wherein the heterocondensed ring of B¹ represents indole, benzimidazole, indazole, benzoxazole, benzothiazole, benzo[d][1,2,3] triazole, or quinoline.

15. The medicament according to claim 13, wherein the heterocondensed ring of B¹ represents indazole.

16. The medicament according to any one of claims 1 to 15, wherein n represents 1 and m represents 0.

17. The medicament according to any one of claims 1 to 16, wherein p represents 1.

18. The medicament according to any one of claims 1 to 17, wherein R¹ and R² each represent a hydrogen atom.

19. The medicament according to any one of claims 1 to 18, wherein R³ represents a hydrogen atom.

20. A medicament for treating or preventing pruritus, the medicament comprising a compound represented by the following General Formula (II), a tautomer of the compound, a stereoisomer of the compound, a pharmaceutically acceptable salt thereof, or a solvate thereof as an active ingredient: 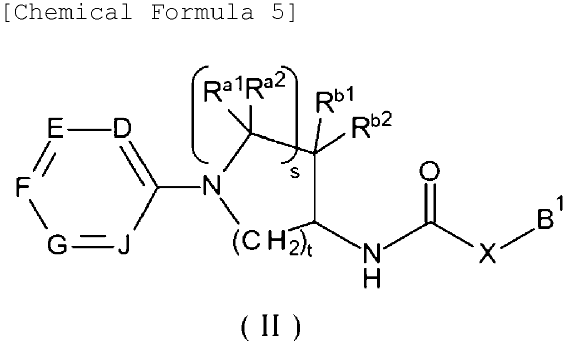
wherein D, E, F, G, and J are such that any two of them each represent N while the others represent CRs which may be identical or different, or any one of them represents N while the others represent CRs which may be identical or different, or all of them are CRs which may be identical or different;
here, R represents a hydrogen atom, a C₁₋₈ alkyl group, a C₁₋₈ alkoxy group, a C₁₋₈ alkyl group substituted with one to three halogen atoms, a C₁₋₈ alkoxy group which may be substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, or a C₁₋₈ alkoxy group substituted with a C₁₋₈ alkoxycarbonyl group;
R^{a1}, R^{a2}, R^{b1}, and R^{b2}, which may be identical or different, each represent a hydrogen atom, a halogen atom, a hydroxy group, a C₁₋₈ alkyl group, or a C₁₋₈ alkyl group substituted with one to three halogen atoms; or
R^{a1}, R^{a2}, and the carbon atom to which R^{a1} and R^{a2} are bonded may be joined together and form a 3- to 5-membered cycloalkyl group, or R^{b1}, R^{b2}, and the carbon atom to which R^{b1} and R^{b2} are bonded may be joined together and form a 3-to 5-membered cycloalkyl group;
s represents 0, 1, or 2;
t represents 1 or 2;
B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to adjacent X by mean of a carbon atom constituting these rings;
X represents: 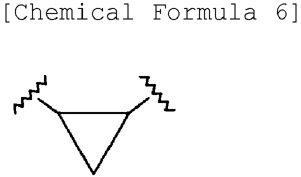
or 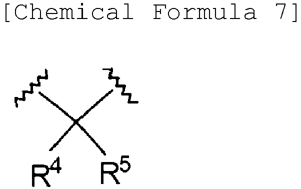
here, the wavy line represents a bonding position;
R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group; or R⁴, R⁵, and the carbon atom to which R⁴ and R⁵ are bonded may be joined together and form a 3- to 5-membered cycloalkyl group;
further, the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri(C₁₋₈ alkyl)silyl group, an acylamino group, an (N-acyl) (N-C₁₋₈ alkyl) amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group.

21. A medicament for treating or preventing pruritus, the medicament comprising a compound represented by the following General Formula (II), a tautomer of the compound, a stereoisomer of the compound, a pharmaceutically acceptable salt thereof, or a solvate thereof as an active ingredient: 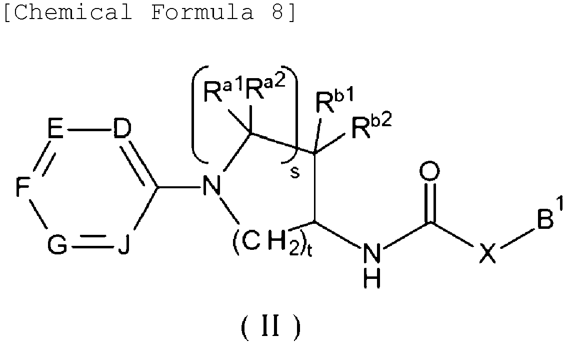
wherein D, E, F, G, and J are such that any two of them each represent N while the others represent CRs which may be identical or different, or any one of them represents N while the others represent CRs which may be identical or different, or all of them are CRs which may be identical or different;
here, R represents a hydrogen atom, a C₁₋₈ alkyl group, a C₁₋₈ alkoxy group, a C₁₋₈ alkyl group substituted with one to three halogen atoms, a C₁₋₈ alkoxy group which may be substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, or a C₁₋₈ alkoxy group substituted with a C₁₋₈ alkoxycarbonyl group;
R^{a1}, R^{a2}, R^{b1}, and R^{b2}, which may be identical or different, each represent a hydrogen atom, a halogen atom, a hydroxy group, a C₁₋₈ alkyl group, or a C₁₋₈ alkyl group substituted with one to three halogen atoms; or
R^{a1}, R^{a2}, and the carbon atom to which R^{a1} and R^{a2} are bonded may be joined together and form a 3- to 5-membered cycloalkyl group, or R^{b1}, R^{b2}, and the carbon atom to which R^{b1} and R^{b2} are bonded may be joined together and form a 3-to 5-membered cycloalkyl group;
s represents 0, 1, or 2;
t represents 1 or 2;
B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to adjacent X by mean of a carbon atom constituting these rings;
X represents: 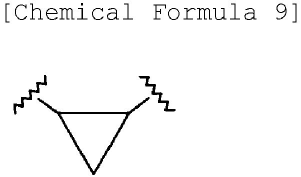
or 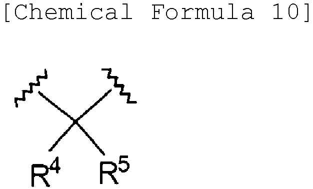
here, the wavy line represents a bonding position;
R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group; or
R⁴, R⁵, and the carbon atom to which R⁴ and R⁵ are bonded may be joined together and form a 3- to 5-membered cycloalkyl group;
further, the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri(C₁₋₈ alkyl)silyl group, an acylamino group, an (N-acyl) (N-C₁₋₈ alkyl) amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group;
provided that when a 6-membered ring composed of D to J may be a phenyl which may have a substituent, or a pyridazine which may have a substituent, and X represents the following: 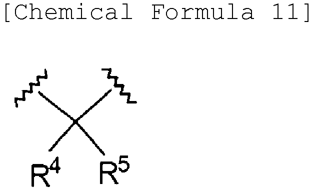
B¹ represents a heterocondensed ring which may have the above-mentioned substituent.

22. The medicament according to claim 20 or 21, wherein X represents:

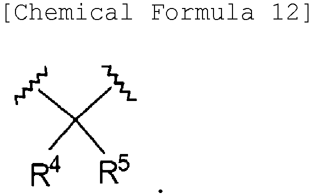

23. The medicament according to any one of claims 20 to 22, wherein D and J each represent CH.

24. The medicament according to any one of claims 20 to 22, wherein any one of D, E, F, G, and J represents N and the others each represent CR.

25. The medicament according to any one of claims 20 to 22, wherein D, E, F, and J represent CRs which may be identical or different, and G represents N.

26. The medicament according to any one of claims 20 to 25, wherein B¹ represents a phenyl which may have a substituent.

27. The medicament according to any one of claims 20 to 25, wherein B¹ represents a phenyl which may have a substituent, or a 5-membered or 6-membered heteroaryl ring composed of one to three identical or different heteroatoms selected from an oxygen atom, a sulfur atom, and a nitrogen atom and carbon atoms as ring-constituting atoms which may have a substituent.

28. The medicament according to claim 27, wherein the heteroaryl ring of B¹ is pyridine.

29. The medicament according to any one of claims 26 to 28, wherein when B¹ represents a phenyl which may have a substituent or a 6-membered heteroaryl ring which may have a substituent, the substituent is substituted at a 4-position (para-position).

30. The medicament according to any one of claims 20 to 25, wherein B¹ represents a heterocondensed ring composed of a 5-membered or 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.

31. The medicament according to claim 30, wherein the heterocondensed ring of B¹ represents indole, benzimidazole, indazole, benzoxazole, benzothiazole, benzo[d] [1,2,3] triazole, or quinoline.

32. The medicament according to claim 30, wherein the heterocondensed ring of B^{a} represents indazole.

33. The medicament according to any one of claims 20 to 32, wherein s represents 1.

34. The medicament according to any one of claims 20 to 33, wherein t represents 1.

35. The medicament according to any one of claims 20 to 34, wherein R^{a1}, R^{a2}, R^{b1}, and R^{b2} each represent a hydrogen atom.

36. A medicament for treating or preventing pruritus, the medicament comprising a compound represented by the following General Formula (III), a tautomer of the compound, a stereoisomer of the compound, a pharmaceutically acceptable salt thereof, or a solvate thereof as an active ingredient: 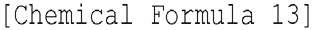 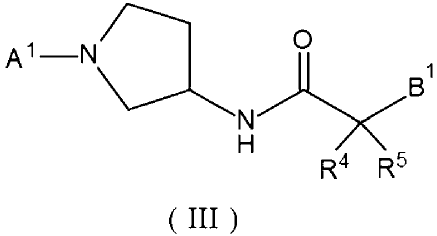
wherein A¹ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to a nitrogen atom of the adjacent pyrrolidine by means of a carbon atom constituting these rings; and
B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to adjacent C(R⁴)(R⁵) by means of a carbon atom constituting these rings;
R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group; or
R⁴, R⁵, and the carbon atom to which R⁴ and R⁵ are bonded may be joined together and form a 3- to 5-membered cycloalkyl group;
further, the substituent that may be carried by the phenyl of A¹, the heteroaryl ring of A¹, and the heterocondensed ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl)(C₁₋₈ alkoxy-substituted C₁₋₈ alkyl)amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group.

37. A medicament for treating or preventing pruritus, the medicament comprising a compound represented by the following General Formula (III), a tautomer of the compound, a stereoisomer of the compound, a pharmaceutically acceptable salt thereof, or a solvate thereof as an active ingredient: 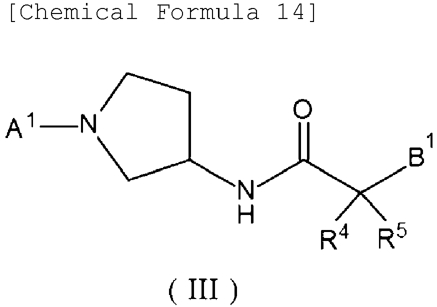
wherein A¹ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to a nitrogen atom of the adjacent pyrrolidine by means of a carbon atom constituting these rings; and
B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to adjacent C(R⁴) (R⁵) by means of a carbon atom constituting these rings;
R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group; or
R⁴, R⁵, and the carbon atom to which R⁴ and R⁵ are bonded may be joined together and form a 3- to 5-membered cycloalkyl group;
further, the substituent that may be carried by the phenyl of A¹, the heteroaryl ring of A¹, and the heterocondensed ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group;
provided that when A¹ represents a phenyl which may have a substituent, a pyridazine which may have a substituent, and a quinazoline which may have a substituent, B¹ represents a heterocondensed ring which may have the above-mentioned substituent.

38. The medicament according to claim 36 or 37, wherein A¹ represents a phenyl which may have a substituent.

39. The medicament according to claim 36 or 37, wherein A¹ represents a phenyl which may have a substituent, or a 4-membered to 6-membered heteroaryl ring composed of one to three identical or different heteroatoms selected from an oxygen atom, a sulfur atom, and a nitrogen atom and carbon atoms as ring-constituting atoms which may have a substituent.

40. The medicament according to claim 39, wherein the heteroaryl ring of A¹ is thiophene, oxazole, thiazole, pyridine, pyrazine, pyrimidine, or pyridazine.

41. The medicament according to claim 39, wherein the heteroaryl ring of A¹ is pyridine.

42. The medicament according to claim 36 or 37, wherein A¹ represents a heterocondensed ring composed of a 4-membered to 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.

43. The medicament according to claim 42, wherein the heterocondensed ring of A¹ is quinoline or benzo[d]thiazole.

44. The medicament according to any one of claims 36 to 43, wherein B¹ represents a phenyl which may have a substituent.

45. The medicament according to any one of claims 36 to 43, wherein B¹ represents a phenyl which may have a substituent, or a 5-membered or 6-membered heteroaryl ring composed of one to three identical or different heteroatoms selected from an oxygen atom, a sulfur atom, and a nitrogen atom and carbon atoms as ring-constituting atoms which may have a substituent.

46. The medicament according to claim 45, wherein the heteroaryl ring of B¹ is pyridine.

47. The medicament according to any one of claims 44 to 46, wherein when B¹ represents a phenyl which may have a substituent or a 6-membered heteroaryl ring which may have a substituent, the substituent is substituted at a 4-position (para-position).

48. The medicament according to any one of claims 36 to 43, wherein B¹ represents a heterocondensed ring composed of a 5-membered or 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.

49. The medicament according to claim 48, wherein the heterocondensed ring of B¹ represents indole, benzimidazole, indazole, benzoxazole, benzothiazole, benzo[d][1,2,3] triazole, or quinoline.

50. The medicament according to claim 48, wherein the heterocondensed ring of B¹ represents indazole.

51. The medicament according to any one of claims 36 to 50, wherein R⁴ and R⁵ each represent a hydrogen atom.

52. A medicament for treating or preventing pruritus, the medicament comprising a compound represented by the following General Formula (IV), a tautomer of the compound, a stereoisomer of the compound, a pharmaceutically acceptable salt thereof, or a solvate thereof as an active ingredient: 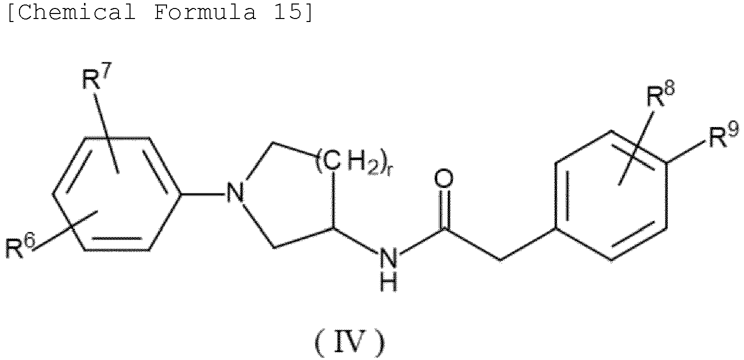
wherein R⁶, R⁷, and R⁸, which are identical or different, each represent a hydrogen atom, a halogen atom, a C₁₋₈ alkyl group, a C₁₋₈ alkyl group substituted with one to three halogen atoms, a C₁₋₈ alkoxy group, a C₁₋₈ alkoxy group substituted with one to three halogen atoms, a hydroxy group, or a C₁₋₈ alkoxy group substituted with a C₁₋₈ alkoxycarbonyl group;
R⁹ represents a C₁₋₈ alkyl substituted with one to three halogen atoms, a tert-butyl, or a cyclopropyl; and
r represents 0, 1, or 2.

53. The medicament according to claim 52, wherein R⁶, R⁷, and R⁸, which are different, each represent a hydrogen atom, a halogen atom, a C₁₋₈ alkyl group, a C₁₋₈ alkyl group substituted with one to three halogen atoms, or a C₁₋₈ alkoxy group.

54. The medicament according to claim 52 or 53, wherein R⁹ represents trifluoromethyl or cyclopropyl.

55. The medicament according to any one of claims 52 to 54, wherein r represents 1.

56. A medicament for treating or preventing pruritus, the medicament comprising a compound represented by the following General Formula (V), a tautomer of the compound, a stereoisomer of the compound, a pharmaceutically acceptable salt thereof, or a solvate thereof as an active ingredient: 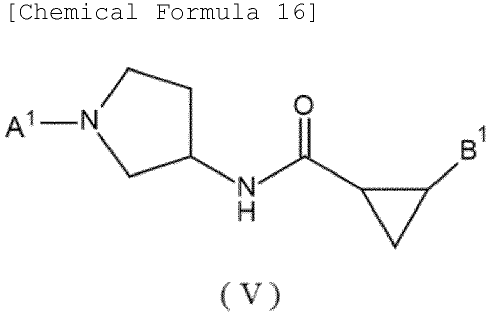
wherein A¹ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to a nitrogen atom of the adjacent pyrrolidine by means of a carbon atom constituting these rings; and
B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while here, the heteroaryl ring or the heterocondensed ring may have a substituent and is bonded to the adjacent cyclopropyl by means of a carbon atom constituting these rings;
further, the substituent that may be carried by the phenyl of A¹, the heteroaryl ring of A¹, and the heterocondensed ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, a 3- to 5-membered cycloalkyl group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri(C₁₋₈ alkyl)silyl group, an acylamino group, an (N-acyl) (N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
here, the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopiperidino, 2-, 3-, or 4-oxopiperidino, morpholino, 1,1-dioxide thiomorpholino, 1,4-dioxo-8-azaspiro[4.5]decan-8-yl, 2-oxo-6-azaspiro[3.3]heptan-6-yl, 3-oxo-8-azabicyclo[3.2.1]octan-8-yl, 2-oxo-5-azabicyclo[2.2.2]octan-5-yl, and 2-oxo-5-azabicyclo[2.2.1]heptan-5-yl, while such a cyclic amino group may be further substituted with a C₁₋₈ alkyl group, a halogen atom, or an acyl group.

57. The medicament according to claim 56, wherein A¹ represents a phenyl which may have a substituent.

58. The medicament according to claim 56, wherein A¹ represents a 4-membered to 6-membered heteroaryl ring composed of one to three identical or different heteroatoms selected from an oxygen atom, a sulfur atom, and a nitrogen atom and carbon atoms as ring-constituting atoms which may have a substituent.

59. The medicament according to claim 58, wherein the heteroaryl ring of A¹ is thiophene, oxazole, thiazole, pyridine, pyrazine, pyrimidine, or pyridazine.

60. The medicament according to claim 58, wherein the heteroaryl ring of A¹ is pyridine.

61. The medicament according to claim 56, wherein A¹ represents a heterocondensed ring composed of a 4-membered to 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.

62. The medicament according to claim 61, wherein the heterocondensed ring of A¹ is quinoline or benzo[d]thiazole.

63. The medicament according to any one of claims 56 to 62, wherein B¹ represents a phenyl which may have a substituent.

64. The medicament according to any one of claims 56 to 62, wherein B¹ represents a 5-membered or 6-membered heteroaryl ring composed of one to three identical or different heteroatoms selected from an oxygen atom, a sulfur atom, and a nitrogen atom and carbon atoms as ring-constituting atoms which may have a substituent.

65. The medicament according to claim 63, wherein the heteroaryl ring of B¹ is pyridine.

66. The medicament according to any one of claims 56 to 62, wherein B¹ represents a heterocondensed ring composed of a 5-membered or 6-membered heteroaryl ring composed of one to three identical or different heteroatoms selected from an oxygen atom, a sulfur atom, and a nitrogen atom and carbon atoms as ring-constituting atoms which may have a substituent, and a benzene ring.

67. The medicament according to claim 66, wherein the heterocondensed ring of B¹ represents indole, benzimidazole, indazole, benzoxazole, benzothiazole, benzo[d][1,2,3] triazole, or quinoline.

68. The medicament according to claim 66, wherein the heterocondensed ring of B¹ represents benzo[d]oxazole.

69. A medicament for treating or preventing pruritus, the medicament comprising a compound represented by the following General Formula (IV), a tautomer of the compound, a stereoisomer of the compound, a pharmaceutically acceptable salt thereof, or a solvate thereof as an active ingredient: 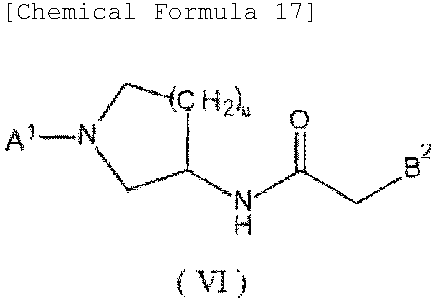
wherein A¹ represents a phenyl which may have a substituent or a 4- to 6-membered heteroaryl ring composed of one to three identical or different heteroatoms selected from an oxygen atom, a sulfur atom, and a nitrogen atom and carbon atoms as ring-constituting atoms, while here, the heteroaryl ring or the heterocondensed ring may have a substituent and is bonded to a nitrogen atom of the adjacent cyclic amine by means of a carbon atom constituting these rings; and
B² represents a heterocondensed ring composed of a 5- or 6-membered heteroaryl ring composed of one to three identical or different heteroatoms selected from an oxygen atom, a sulfur atom, and a nitrogen atom and carbon atoms as ring-constituting atoms, and either a benzene ring or a 6-membered heteroaryl ring composed of one to two nitrogen atoms and carbon atoms, while here, the heterocondensed ring may have a substituent and is bonded to the adjacent methylene group by means of a carbon atom constituting these rings;
further, the substituent that may be carried by the phenyl of A¹ and the heteroaryl ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the heterocondensed ring of B² is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri(C₁₋₈ alkyl)silyl group, an acylamino group, an (N-acyl) (N-C₁₋₈ alkyl) amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
here, the cyclic amino group of the substituent that may be carried by the heterocondensed ring of B² is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group, and
u represents 0, 1, or 2.

70. The medicament according to claim 69, wherein A¹ represents a phenyl which may have a substituent, or a 4-membered to 6-membered heteroaryl ring composed of one to three identical or different heteroatoms selected from an oxygen atom, a sulfur atom, and a nitrogen atom and carbon atoms as ring-constituting atoms which may have a substituent.

71. The medicament according to claim 70, wherein the heteroaryl ring of A¹ is thiophene, oxazole, thiazole, pyridine, pyrazine, pyrimidine, or pyridazine.

72. The medicament according to claim 70, wherein the heteroaryl ring of A¹ is pyridine.

73. A medicament for treating or preventing pruritus, the medicament comprising a compound selected from the following compounds, a tautomer of the compound, a stereoisomer of the compound, a pharmaceutically acceptable salt thereof, or a solvate thereof as an active ingredient.
(R)-2-(4-cyclopropylphenyl)-N-(1-(5-(trifluoromethyl) pyridin-3-yl) pyrrolidin-3-yl) acetamide,
(R)-2-(5-(trifluoromethyl) pyridin-2-yl)-N-(1-(5-(trifluoromethyl) pyridin-3-yl) pyrrolidin-3-yl) acetamide,
(R)-2-(4-isopropylphenyl)-N-(1-(2-(trifluoromethyl) pyrimidin-5-yl) pyrrolidin-3-yl) acetamide,
(R)-2-(4-isopropylphenyl)-N-(1-(5-(trifluoromethyl) pyridin-3-yl) pyrrolidin-3-yl) acetamide,
2-(4-cyclopropylphenyl)-N-((3S,4S)-4-hydroxy-1-(5-(trifluoromethyl) pyridin-3-yl) pyrrolidin-3-yl) acetamide,
(R)-2-(4-cyclopropylphenyl)-N-(5-(5-(trifluoromethyl) pyridin-3-yl)-5-azaspiro[2.4]heptan-7-yl) acetamide,
(R)-2-(4-cyclopropylphenyl)-N-(5-(6-(trifluoromethyl) pyridin-3-yl)-5-azaspiro[2.4]heptan-7-yl) acetamide,
(R)-2-(4-cyclopropylphenyl)-N-(5-(4-(trifluoromethyl) thiazol-2-yl)-5-azaspiro[2.4]heptan-7-yl) acetamide,
2-(4-cyclopropylphenyl)-N-((3R, 5S)-5-methyl-1-(5-(trifluoromethyl) pyridin-3-yl) pyrrolidin-3-yl) acetamide,
2-(4-cyclopropylphenyl)-N-((1S,5R)-3-(5-(trifluoromethyl) pyridin-3-yl)-3-azabicyclo[3.1.0]hexan-1-yl) acetamide,
2-(4-cyclopropylphenyl)-N-((1R,5S)-3-(5-(trifluoromethyl) pyridin-3-yl)-3-azabicyclo[3.1.0]hexan-1-yl) acetamide,
(R)-2-(4-(trifluoromethyl) phenyl)-N-(1-(6-(trifluoromethyl) pyrazin-2-yl) pyrrolidin-3-yl) acetamide,
(R)-N-(1-(6-cyano-5-(trifluoromethyl) pyridin-3-yl) pyrrolidin-3-yl)-2-(4-cyclopropylphenyl) acetamide,
(R)-3-(2-(4-cyclopropylphenyl) acetamide)-1-(5-(trifluoromethyl) pyridin-3-yl) pyrrolidine-3-methyl carboxylate,
(S)-3-(2-(4-cyclopropylphenyl) acetamide)-1-(5-(trifluoromethyl) pyridin-3-yl) pyrrolidine-3-methyl carboxylate,
(R)-3-(2-(4-cyclopropylphenyl) acetamide)-1-(5-(trifluoromethyl) pyridin-3-yl) pyrrolidine-3-carboxylic acid,
(S)-3-(2-(4-cyclopropylphenyl) acetamide)-1-(5-(trifluoromethyl) pyridin-3-yl) pyrrolidine-3-carboxylic acid,
(R)-2-(1H-indazol-6-yl)-N-(1-(5-(trifluoromethyl) pyridin-3-yl) pyrrolidin-3-yl) acetamide,
(R)-2-(4-cyclopropylphenyl)-N-(1-(4-(trifluoromethyl) thiazol-2-yl) pyrrolidin-3-yl) acetamide, (R)-2-(4-isobutylphenyl)-N-((R)-1-(5-(trifluoromethyl) pyridin-3-yl) pyrrolidin-3-yl) propanamide,
(S)-2-(4-isobutylphenyl)-N-((R)-1-(5-(trifluoromethyl) pyridin-3-yl) pyrrolidin-3-yl) propanamide,
(R)-2-(4-cyclopropylphenyl)-N-(1-6-methyl-5-(trifluoromethyl) pyridin-3-yl) pyrrolidin-3-yl) acetamide,
(R)-2-(4-cyclopropylphenyl)-N-(1-(5-(2-hydroxypropan-2-yl) pyridin-3-yl) pyrrolidin-3-yl) acetamide,
(R)-2-(4-cyclopropylphenyl)-N-(1-(8-(trifluoromethyl) imidazo[1,2-a] pyridin-6-yl) pyrrolidin-3-yl) acetamide,
(R)-3-(2-(4-cyclopropylphenyl) acetamide)-1-(5-(trifluoromethyl) pyridin-3-yl) pyrrolidine-3-carboxyamide,
(S)-3-(2-(4-cyclopropylphenyl) acetamide)-1-(5-(trifluoromethyl) pyridin-3-yl) pyrrolidine-3-carboxyamide,
(R)-2-(4-hydroxyphenyl)-N-(1-(5-(trifluoromethyl) pyridin-3-yl) pyrrolidin-3-yl) acetamide,
(R)-2-(4-(1,1-dioxidethiomorpholino) phenyl)-N-(1-(5-(trifluoromethyl) pyridin-3-yl) pyrrolidin-3-yl) acetamide,
(R)-1-(4-chlorophenyl)-N-(1-(5-(trifluoromethyl) pyridin-3-yl) pyrrolidin-3-yl) cyclopropane-1-carboxyamide,
(R)-2-(4-bromophenyl)-2-hydroxy-N-((R)-1-(5-(trifluoromethyl) pyridin-3-yl) pyrrolidin-3-yl) acetamide,
(S)-2-(4-bromophenyl)-2-hydroxy-N-((R)-1-(5-(trifluoromethyl) pyridin-3-yl) pyrrolidin-3-yl) acetamide,
(R)-N-(1-(5-(trifluoromethyl) pyridin-3-yl) pyrrolidin-3-yl)-2-(4-(trimethylsilyl) phenyl) acetamide,
(R)-2-(4-(2-hydroxy-2-methylpropoxy) phenyl)-N-(1-(5-(trifluoromethyl) pyridin-3-yl) pyrrolidin-3-yl) acetamide,
2-(4-cyclopropylphenyl)-N-((3S,4S)-4-fluoro-1-(5-(trifluoromethyl) pyridin-3-yl) pyrrolidin-3-yl) acetamide,
(R)-2-(4-(dimethylamino) phenyl)-N-(1-(5-(trifluoromethyl) pyridin-3-yl) pyrrolidin-3-yl) acetamide,
(R)-2-(4-nitrophenyl)-N-(1-(5-(trifluoromethyl) pyridin-3-yl) pyrrolidin-3-yl) acetamide,
(S)-2-(4-isobutylphenyl)-N-((R)-1-(5-(trifluoromethyl) pyridin-3-yl) pyrrolidin-3-yl) propanamide,
(R)-2-(3-fluoro-4-(trifluoromethyl) phenyl)-N-(1-(5-(trifluoromethyl) pyridin-3-yl) pyrrolidin-3-yl) acetamide,
(R)-2-(4-aminophenyl)-N-(1-(5-(trifluoromethyl) pyridin-3-yl) pyrrolidin-3-yl) acetamide,
(R)-1-(4-(trifluoromethyl) phenyl)-N-(1-(5-(trifluoromethyl) pyridin-3-yl) pyrrolidin-3-yl) cyclopropane-1-carboxyamide,
(R)-2-(4-acetamide phenyl)-N-(1-(5-(trifluoromethyl) pyridin-3-yl) pyrrolidin-3-yl) acetamide,
(R)-N-(3-cyano-1-(5-(trifluoromethyl) pyridin-3-yl) pyrrolidin-3-yl)-2-(4-cyclopropylphenyl) acetamide,
(S)-N-(3-cyano-1-(5-(trifluoromethyl) pyridin-3-yl) pyrrolidin-3-yl)-2-(4-cyclopropylphenyl) acetamide,
(R)-3-(2-(1H-indol-6-yl) acetamide)-1-(5-(trifluoromethyl) pyridin-3-yl) pyrrolidine-3-methyl carboxylate,
(S)-3-(2-(1H-indol-6-yl) acetamide)-1-(5-(trifluoromethyl) pyridin-3-yl) pyrrolidine-3-methyl carboxylate,
(R)-3-(2-(4-(trifluoromethyl) phenyl) acetamide)-1-(5-(trifluoromethyl) pyridin-3-yl) pyrrolidine-3-methyl carboxylate,
(S)-3-(2-(4-(trifluoromethyl) phenyl) acetamide)-1-(5-(trifluoromethyl) pyridin-3-yl) pyrrolidine-3-methyl carboxylate,
(1S,2S)-2-(benzo[d]oxazol-2-yl)-N-((R)-1-(5-(trifluoromethyl) pyridin-3-yl) pyrrolidin-3-yl) cyclopropane-1-carboxyamide,
(R)-2-(4-morpholinophenyl)-N-(1-(4-(trifluoromethyl) thiazol-2-yl) pyrrolidin-3-yl) acetamide,
3-(2-(4-morpholinophenyl) acetamide)-1-(5-(trifluoromethyl) pyridin-3-yl) pyrrolidine-3-methyl carboxylate,
(R)-2-(4-morpholinophenyl)-N-(1-(6-(trifluoromethyl) pyridin-3-yl) pyrrolidin-3-yl) acetamide,
(R)-2-(4-cyclopropylphenyl)-N-(3-ethyl-1-(5-(trifluoromethyl) pyridin-3-yl) pyrrolidin-3-yl) acetamide,
(S)-2-(4-cyclopropylphenyl)-N-(3-ethyl-1-(5-(trifluoromethyl) pyridin-3-yl) pyrrolidin-3-yl) acetamide,
(1S,2S)-2-(6-fluorobenzo[d]oxazol-2-yl)-N-((R)-1-(5-(trifluoromethyl) pyridin-3-yl) pyrrolidin-3-yl) cyclopropane-1-carboxyamide,
(1S,2S)-2-(5,6-difluorobenzo[d]oxazol-2-yl)-N-((R)-1-(5-(trifluoromethyl) pyridin-3-yl) pyrrolidin-3-yl) cyclopropane-1-carboxyamide,
(R)-2-(4-(2-oxa-6-azaspiro[3.3]heptan-6-yl) phenyl)-N-(1-(5-(trifluoromethyl) pyridin-3-yl) pyrrolidin-3-yl) acetamide,
(1S,2S)-2-(benzo[d]oxazol-2-yl)-N-((R)-1-(4-(trifluoromethyl) thiazol-2-yl) pyrrolidin-3-yl) cyclopropane-1-carboxyamide,
(1S,2S)-2-(6-fluorobenzo[d]oxazol-2-yl)-N-((R)-1-(6-(trifluoromethyl) pyridin-3-yl) pyrrolidin-3-yl) cyclopropane-1-carboxyamide,
(1S,2S)-2-(5-fluorobenzo[d]oxazol-2-yl)-N-((R)-1-(5-(trifluoromethyl) pyridin-3-yl) pyrrolidin-3-yl) cyclopropane-1-carboxyamide,
(1R,2R)-2-(benzo[d]oxazol-2-yl)-N-((R)-1-(5-(trifluoromethyl) pyridin-3-yl) pyrrolidin-3-yl) cyclopropane-1-carboxyamide,
(1S,2S)-2-(benzo[d]oxazol-2-yl)-N-((R)-1-(5-(trifluoromethyl) pyridin-3-yl) pyrrolidin-3-yl) cyclopropane-1-carboxyamide,
(1S,2S)-2-(1H-benzo[d]imidazol-2-yl)-N-((R)-1-(4-(trifluoromethyl) phenyl) pyrrolidin-3-yl) cyclopropane-1-carboxyamide,
(1R,2R)-2-(1H-benzo[d]imidazol-2-yl)-N-((R)-1-(4-(trifluoromethyl) phenyl) pyrrolidin-3-yl) cyclopropane-1-carboxyamide,
(1S,2S)-2-(1H-benzo[d]imidazol-2-yl)-N-((S)-1-(4-(trifluoromethyl) phenyl) pyrrolidin-3-yl) cyclopropane-1-carboxyamide,
(1R,2R)-2-(1H-benzo[d]imidazol-2-yl)-N-((R)-1-(6-(trifluoromethyl) pyridin-3-yl) pyrrolidin-3-yl)) cyclopropane-1-carboxyamide,
(1S,2S)-2-(1H-benzo[d]imidazol-2-yl)-N-((S)-1-(6-(trifluoromethyl) pyridin-3-yl) pyrrolidin-3-yl) cyclopropane-1-carboxyamide,
(1S,2S)-2-(1H-benzo[d]imidazol-2-yl)-N-((R)-1-(5-(trifluoromethyl) pyrimidin-2-yl) pyrrolidin-3-yl)) cyclopropane-1-carboxyamide,
(1S,2S)-2-(1H-benzo[d]imidazol-2-yl)-N-((R)-1-(4-fluoro-3-(trifluoromethyl) phenyl) pyrrolidin-3-yl)) cyclopropane-1-carboxyamide,
(1S,2S)-N-((R)-1-(4-fluoro-3-(trifluoromethyl) phenyl) pyrrolidin-3-yl)-2-(quinazolin-2-yl) cyclopropane-1-carboxamide,
(1S,2S)-2-(1H-benzo[d]imidazol-2-yl)-N-((R)-1-(3-(methylsulfonyl) phenyl) pyrrolidin-3-yl) cyclopropane-1-carboxamide,
(1S,2S)-2-(1H-benzo[d]imidazol-2-yl)-N-((R)-1-(3-cyanophenyl) pyrrolidin-3-yl) cyclopropane-1-carboxyamide,
(1S,2S)-2-(5-cyano-1H-benzo[d]imidazol-2-yl)-N-((R)-1-(6-(trifluoromethyl) pyridin-3-yl) pyrrolidin-3-yl) cyclopropane-1-carboxyamide,
(1R,2R)-2-(5-cyano-1H-benzo[d]imidazol-2-yl)-N-((R)-1-(6-(trifluoromethyl) pyridin-3-yl) pyrrolidin-3-yl) cyclopropane-1-carboxyamide,
(1S,2S)-2-(1H-benzo[d]imidazol-2-yl)-N-((R)-1-(2-(trifluoromethyl) pyridin-4-yl) pyrrolidin-3-yl)) cyclopropane-1-carboxyamide,
(1S,2S)-2-(1H-benzo[d]imidazol-2-yl)-N-((R)-1-(6-(trifluoromethyl) pyridin-3-yl) piperidin-3-yl)) cyclopropane-1-carboxyamide,
(1R,2R)-2-(1H-benzo[d]imidazol-2-yl)-N-((R)-1-(6-(trifluoromethyl) pyridin-3-yl) piperidin-3-yl)) cyclopropane-1-carboxyamide,
(1S,2S)-2-(1H-benzo[d]imidazol-2-yl)-N-((R)-1-(4-(trifluoromethyl) thiazol-2-yl) pyrrolidin-3-yl)) cyclopropane-1-carboxyamide,
(1S,2S)-2-(1H-benzo[d]imidazol-2-yl)-N-((R)-1-(4-(trifluoromethyl) phenyl) piperidin-3-yl) cyclopropane-1-carboxyamide,
(1S,2S)-2-(1H-indol-3-yl)-N-((R)-1-(6-(trifluoromethyl) pyridin-3-yl) pyrrolidin-3-yl) cyclopropane-1-carboxyamide,
(1R,2R)-2-(1H-indol-3-yl)-N-((R)-1-(6-(trifluoromethyl) pyridin-3-yl) pyrrolidin-3-yl) cyclopropane-1-carboxyamide,
(1S,2S)-2-(1H-benzo[d]imidazol-2-yl)-N-((R)-1-(6-methoxy-5-(trifluoromethyl) pyridin-3-yl) pyrrolidin-3-yl) cyclopropane-1-carboxyamide, (1S,2S)-2-(1H-benzo[d]imidazol-2-yl)-N-((R)-1-(4-(tert-butyl) thiazol-2-yl) pyrrolidin-3-yl) cyclopropane-1-carboxyamide,
(1S,2S)-2-(1H-benzo[d]imidazol-2-yl)-N-((R)-1-(6-(trifluoromethyl) pyridazin-3-yl) pyrrolidin-3-yl)) cyclopropane-1-carboxyamide,
(1S,2S)-2-(1H-benzo[d]imidazol-2-yl)-N-((R)-1-(2-(trifluoromethyl) pyrimidin-5-yl) pyrrolidin-3-yl)) cyclopropane-1-carboxyamide,
(1S,2S)-2-(1H-benzo[d]imidazol-2-yl)-N-((R)-1-(5-(trifluoromethyl) pyridin-3-yl) pyrrolidin-3-yl)) cyclopropane-1-carboxyamide,
(1S,2S)-2-(1H-benzo[d]imidazol-2-yl)-N-((R)-1-(6-fluorobenzo[d]thiazol-2-yl) pyrrolidin-3-yl) cyclopropane-1-carboxyamide,
(1S,2S)-2-(1-cyclopropyl-1H-benzo[d]imidazol-2-yl)-N-((R)-1-(6-(trifluoromethyl) pyridin-3-yl) pyrrolidin-3-yl) cyclopropane-1-carboxyamide,
(1S,2S)-2-(1H-benzo[d]imidazol-2-yl)-N-((R)-1-(6-fluoroquinolin-2-yl) pyrrolidin-3-yl) cyclopropane-1-carboxyamide,
(1S,2S)-2-(1-cyclopropyl-1H-benzo[d]imidazol-2-yl)-N-((R)-1-(3-trifluoromethyl) phenyl) pyrrolidin-3-yl) cyclopropane-1-carboxyamide,
(1S,2S)-2-(1H-benzo[d]imidazol-2-yl)-N-((R)-1-(5-bromopyridin-3-yl) pyrrolidin-3-yl) cyclopropane-1-carboxyamide,
(R)-2-(quinolin-6-yl)-N-(1-(5-(trifluoromethyl) pyridin-3-yl) pyrrolidin-3-yl) acetamide,
(R)-2-(1H-indol-3-yl)-N-(1-(5-(trifluoromethyl) pyridin-3-yl) pyrrolidin-3-yl) acetamide,
(R)-2-(quinolin-7-yl)-N-(1-(5-(trifluoromethyl) pyridin-3-yl) pyrrolidin-3-yl) acetamide,
(R)-2-(4-cyclopropylphenyl)-N-(1-(4-(trifluoromethyl) oxazol-2-yl) pyrrolidin-3-yl) acetamide,
(R)-2-(1-methyl-5-(trifluoromethyl)-1H-pyrazol-3-yl)-N-(1-(5-(trifluoromethyl) pyridin-3-yl) pyrrolidin-3-yl) acetamide,
(R)-2-(6-(trifluoromethyl) pyridin-3-yl)-N-(1-(5-(trifluoromethyl) pyridin-3-yl) pyrrolidin-3-yl) acetamide,
(R)-2-(4-(trifluoromethyl) phenyl)-N-(1-(5-(trifluoromethyl) pyridin-3-yl) pyrrolidin-3-yl) acetamide-2,2-d2,
(R)-2-(4-(3,3-difluoropyrrolidin-1-yl) phenyl)-N-(1-(6-(trifluoromethyl) pyridin-3-yl) pyrrolidin-3-yl) acetamide,
(R)-2-(1H-indol-6-yl)-N-(1-(6-(trifluoromethyl) pyridin-3-yl) pyrrolidin-3-yl) acetamide,
2-(4-((2R,6S)-2,6-dimethylmorpholino) phenyl)-N-((R)-1-(5-(trifluoromethyl) pyridin-3-yl) pyrrolidin-3-yl) acetamide,
(R)-2-(4-(pyrrolidin-1-yl) phenyl)-N-(1-(5-(trifluoromethyl) pyridin-3-yl) pyrrolidin-3-yl) acetamide,
(R)-2-(4-((2-methoxyethyl) (methyl) amino) phenyl)-N-(1-(5-(trifluoromethyl) pyridin-3-yl) pyrrolidin-3-yl) acetamide,
2-(4-(trifluoromethyl) phenyl)-N-(1-(5-(trifluoromethyl) pyridin-3-yl) azetidin-3-yl) acetamide,
2-(1H-indol-6-yl)-N-(1-(5-(trifluoromethyl) pyridin-3-yl) azetidin-3-yl) acetamide,
(R)-2-(1-methyl-1H-indol-6-yl)-N-(1-(5-(trifluoromethyl) pyridin-3-yl) pyrrolidin-3-yl) acetamide,
(R)-2-(1-methyl-1H-indol-6-yl)-N-(1-(6-(trifluoromethyl) pyridin-3-yl) pyrrolidin-3-yl) acetamide,
(R)-2-(4-(4-methylpiperazin-1-yl) phenyl)-N-(1-(5-(trifluoromethyl) pyridin-3-yl) pyrrolidin-3-yl) acetamide,
(R)-2-(4-(piperazin-1-yl) phenyl)-N-(1-(5-(trifluoromethyl) pyridin-3-yl) pyrrolidin-3-yl) acetamide,
(R)-2-(4-(4-acetylpiperazin-1-yl) phenyl)-N-(1-(5-(trifluoromethyl) pyridin-3-yl) pyrrolidin-3-yl) acetamide,
(R)-2-(4-(2-oxopiperidin-1-yl) phenyl)-N-(1-(5-(trifluoromethyl) pyridin-3-yl) pyrrolidin-3-yl) acetamide,
(R)-2-(6-chlorobenzo[d]oxazol-2-yl)-N-(1-(5-(trifluoromethyl) pyridin-3-yl) pyrrolidin-3-yl) acetamide,
(R)-2-(benzo[d]oxazol-2-yl)-N-(1-(5-(trifluoromethyl) pyridin-3-yl) pyrrolidin-3-yl) acetamide,
(1S,2S)-2-(3,5-dichlorophenyl)-N-((R)-1-(5-(trifluoromethyl) pyridin-3-yl) pyrrolidin-3-yl) cyclopropane-1-carboxamide,
(1R,2R)-2-(3,5-dichlorophenyl)-N-((R)-1-(5-(trifluoromethyl) pyridin-3-yl) pyrrolidin-3-yl) cyclopropane-1-carboxamide,
(1S,2S)-2-(4-bromophenyl)-N-((R)-1-(5-(trifluoromethyl) pyridin-3-yl) pyrrolidin-3-yl) cyclopropane-1-carboxyamide,
(1R,2R)-2-(4-bromophenyl)-N-((R)-1-(5-(trifluoromethyl) pyridin-3-yl) pyrrolidin-3-yl) cyclopropane-1-carboxyamide,
(R)-2-(4-(1,4-dioxa-8-azaspiro[4.5]decan-8-yl) phenyl)-N-(1-(5-(trifluoromethyl) pyridin-3-yl) pyrrolidin-3-yl) acetamide,
(R)-2-(4-(4-oxopiperidin-1-yl) phenyl)-N-(1-(5-(trifluoromethyl) pyridin-3-yl) pyrrolidin-3-yl) acetamide,
(R)-2-(4-cyclopropylphenyl)-N-(3-(hydroxymethyl)-1-(5-(trifluoromethyl) pyridin-3-yl) pyrrolidin-3-yl) acetamide,
(S)-2-(4-cyclopropylphenyl)-N-(3-(hydroxymethyl)-1-(5-(trifluoromethyl) pyridin-3-yl) pyrrolidin-3-yl) acetamide,
(R)-(3-(2-(4-cyclopropylphenyl)acetamido)-1-(5-(trifluoromethyl)pyridin-3-yl)pyrrolidin-3-yl)methyl acetate,
(S)-(3-(2-(4-cyclopropylphenyl)acetamido)-1-(5-(trifluoromethyl)pyridin-3-yl)pyrrolidin-3-yl)methyl acetate,
(R)-2-(4-cyclopropylphenyl)-N-(3-(methoxymethyl)-1-(5-(trifluoromethyl) pyridin-3-yl) pyrrolidin-3-yl) acetamide,
(S)-2-(4-cyclopropylphenyl)-N-(3-(methoxymethyl)-1-(5-(trifluoromethyl) pyridin-3-yl) pyrrolidin-3-yl) acetamide,
(1S,2S)-2-(4-cyclopropylphenyl)-N-((R)-1-(5-(trifluoromethyl) pyridin-3-yl) pyrrolidin-3-yl) cyclopropane-1-carboxamide,
(1R,2R)-2-(4-cyclopropylphenyl)-N-((R)-1-(5-(trifluoromethyl) pyridin-3-yl) pyrrolidin-3-yl) cyclopropane-1-carboxamide,
(1S,2S)-2-(3,4-difluorophenyl)-N-((R)-1-(5-(trifluoromethyl) pyridin-3-yl) pyrrolidin-3-yl) cyclopropane-1-carboxamide,
(1R,2R)-2-(3,4-difluorophenyl)-N-((R)-1-(5-(trifluoromethyl) pyridin-3-yl) pyrrolidin-3-yl) cyclopropane-1-carboxamide,
(1S,2S)-2-(4-chlorophenyl)-N-((R)-1-(5-(trifluoromethyl) pyridin-3-yl) pyrrolidin-3-yl) cyclopropane-1-carboxyamide,
(1R,2R)-2-(4-chlorophenyl)-N-((R)-1-(5-(trifluoromethyl) pyridin-3-yl) pyrrolidin-3-yl) cyclopropane-1-carboxyamide,
2-(4-isopropylphenyl)-N-(1-(5-(trifluoromethyl) pyridin-3-yl) piperidin-4-yl) acetamide,
(R)-2-(4-isopropylphenyl)-N-(1-(6-methoxy-5-(trifluoromethyl) pyridin-3-yl) pyrrolidin-3-yl) acetamide, (R)-2-(4-isopropylphenyl)-N-(1-(5-(trifluoromethyl) pyrimidin-2-yl) pyrrolidin-3-yl) acetamide,
(R)-2-(4-isopropylphenyl)-N-(1-(4-(trifluoromethyl) thiazol-2-yl) pyrrolidin-3-yl) acetamide,
(R)-2-(4-isopropylphenyl)-N-(1-(6-(trifluoromethyl) pyridin-3-yl) pyrrolidin-3-yl) acetamide,
(S)-2-(4-isopropylphenyl)-N-(1-(5-(trifluoromethyl) pyridin-3-yl) pyrrolidin-3-yl) acetamide,
(R)-2-(4-(trifluoromethoxy) phenyl)-N-(1-(5-(trifluoromethyl) pyridin-3-yl) pyrrolidin-3-yl) acetamide,
(R)-2-(4-(2,2,2-trifluoroethoxy) phenyl)-N-(1-(5-(trifluoromethyl) pyridin-3-yl) pyrrolidin-3-yl) acetamide,
2-(4-isopropylphenyl)-N-(1-(5-(trifluoromethyl) pyridin-3-yl) azetidin-3-yl) acetamide,
2-(4-isopropylphenyl)-N-(1-(6-(trifluoromethyl) pyridin-3-yl) azetidin-3-yl) acetamide,
(R)-2-(3,5-dichlorophenyl)-N-(1-(5-(trifluoromethyl) pyridin-3-yl) pyrrolidin-3-yl) acetamide,
(R)-2-(3-chloro-5-fluorophenyl)-N-(1-(5-(trifluoromethyl) pyridin-3-yl) pyrrolidin-3-yl) acetamide,
(R)-2-(4-cyclopropylphenyl)-N-(1-(6-(trifluoromethyl) pyridin-3-yl) pyrrolidin-3-yl) acetamide,
(R)-2-(4-isopropylphenyl)-N-(1-(5-(trifluoromethyl) thiophen-2-yl) pyrrolidin-3-yl) acetamide,
(R)-2-(4-(trifluoromethyl) phenyl)-N-(1-(5-(trifluoromethyl) pyridin-3-yl) pyrrolidin-3-yl) acetamide,
(R)-2-(1H-indol-6-yl)-N-(1-(5-(trifluoromethyl) pyridin-3-yl) pyrrolidin-3-yl) acetamide,
(R)-2-(4-cyclopropylphenyl)-N-(1-(6-(2,2,2-trifluoroethoxy) pyridin-3-yl) pyrrolidin-3-yl) acetamide,
(R)-2-(4-cyclopropylphenyl)-N-(1-(5-(2,2,2-trifluoroethoxy) pyridin-3-yl) pyrrolidin-3-yl) acetamide,
(R)-2-(4-cyclopropylphenyl)-N-(1-(4-(2,2,2-trifluoroethoxy) pyridin-2-yl) pyrrolidin-3-yl) acetamide,
(R)-2-(4-cyclopropylphenyl)-N-(1-(2-methoxy-5-(trifluoromethyl) pyridin-3-yl) pyrrolidin-3-yl) acetamide,
(R)-2-(4-cyclopropylphenyl)-N-(1-(5-(trifluoromethyl) pyridin-3-yl) piperidin-3-yl) acetamide,
(R)-2-(4-cyclopropylphenyl)-N-(1-(6-(trifluoromethyl) pyridin-3-yl) piperidin-3-yl) acetamide,
(R)-2-(4-(2-hydroxypropan-2-yl) phenyl)-N-(1-(5-(trifluoromethyl) pyridin-3-yl) pyrrolidin-3-yl) acetamide,
(R)-2-(4-(3-methylpyrazin-2-yl) phenyl)-N-(1-(5-(trifluoromethyl) pyridin-3-yl) pyrrolidin-3-yl) acetamide,
(R)-2-(4-(4-methyloxazol-5-yl) phenyl)-N-(1-(5-(trifluoromethyl) pyridin-3-yl) pyrrolidin-3-yl) acetamide,
(R)-3-(3-(2-(4-cyclopropylphenyl) acetamide) pyrrolidin-1-yl)-5-(trifluoromethyl) pyridine 1-oxide,
(3R)-3-(2-(4-cyclopropylphenyl) acetamide)-1-(5-(trifluoromethyl) pyridin-3-yl) pyrrolidine 1-oxide,
(R)-2-(4-cyclopropylphenyl)-2-methyl-N-(1-(5-(trifluoromethyl) pyridin-3-yl) pyrrolidin-3-yl) propanamide,
(R)-2-(4-cyclopropylphenyl)-N-(1-(3-fluoro-5-(trifluoromethyl) phenyl) pyrrolidin-3-yl) acetamide,
(R)-2-amino-2-(4-cyclopropylphenyl)-N-((R)-1-(5-(trifluoromethyl) pyridin-3-yl) pyrrolidin-3-yl) acetamide,
(S)-2-amino-2-(4-cyclopropylphenyl)-N-((R)-1-(5-(trifluoromethyl) pyridin-3-yl) pyrrolidin-3-yl) acetamide,
(R)-2-(4-cyclopropylphenyl)-2-(dimethylamino)-N-((R)-1-(5-(trifluoromethyl) pyridin-3-yl) pyrrolidin-3-yl) acetamide,
(S)-2-(4-cyclopropylphenyl)-2-(dimethylamino)-N-((R)-1-(5-(trifluoromethyl) pyridin-3-yl) pyrrolidin-3-yl) acetamide,
(R)-2-(1-methyl-1H-indol-5-yl)-N-(1-(5-(trifluoromethyl) pyridin-3-yl) pyrrolidin-3-yl) acetamide,
(R)-2-(2,4-bis(trifluoromethyl) phenyl)-N-(1-(5-(trifluoromethyl) pyridin-3-yl) pyrrolidin-3-yl) acetamide,
(R)-2-(2-fluoro-4-(trifluoromethyl) phenyl)-N-(1-(5-(trifluoromethyl) pyridin-3-yl) pyrrolidin-3-yl) acetamide,
(R)-2-(1-methyl-1H-indazol-5-yl)-N-(1-(5-(trifluoromethyl) pyridin-3-yl) pyrrolidin-3-yl) acetamide,
(R)-2-(1-methyl-1H-indazol-5-yl)-N-(1-(4-(trifluoromethyl) thiazol-2-yl) pyrrolidin-3-yl) acetamide,
(R)-2-(1-methyl-1H-indazol-5-yl)-N-(1-(6-(trifluoromethyl) pyridin-3-yl) pyrrolidin-3-yl) acetamide,
(R)-2-(4-(2,2-dimethylmorpholino) phenyl)-N-(1-(5-(trifluoromethyl) pyridin-3-yl) pyrrolidin-3-yl) acetamide,
(R)-2-(4-(1H-pyrazol-1-yl) phenyl)-N-(1-(5-(trifluoromethyl) pyridin-3-yl) pyrrolidin-3-yl) acetamide,
(R)-N-(3-methyl-1-(5-(trifluoromethyl) pyridin-3-yl) pyrrolidin-3-yl)-2-(4-morpholinophenyl) acetamide,
(R)-2-(4-cyclopropylphenyl)-N-(3-methyl-1-(5-(trifluoromethyl) pyridin-3-yl) pyrrolidin-3-yl) acetamide,
2-(4-(3-oxa-8-azabicyclo[3.2.1]octan-8-yl) phenyl)-N-((R)-1-(5-(trifluoromethyl) pyridin-3-yl) pyrrolidin-3-yl) acetamide,
2-(4-(3-oxa-8-azabicyclo[3.2.1]octan-8-yl) phenyl)-N-((R)-1-(6-(trifluoromethyl) pyridin-3-yl) pyrrolidin-3-yl) acetamide,
(R)-2-(benzo[d]oxazol-5-yl)-N-(1-(6-(trifluoromethyl) pyridin-3-yl) pyrrolidin-3-yl) acetamide,
(R)-2-(benzo[d]oxazol-5-yl)-N-(1-(5-(trifluoromethyl) pyridin-3-yl) pyrrolidin-3-yl) acetamide,
(R)-2-(2-methylbenzo[d]oxazol-5-yl)-N-(1-(5-(trifluoromethyl) pyridin-3-yl) pyrrolidin-3-yl) acetamide,
(R)-2-(2-methylbenzo[d]oxazol-5-yl)-N-(1-(6-(trifluoromethyl) pyridin-3-yl) pyrrolidin-3-yl) acetamide,
2-(4-((1S,4S)-2-oxa-5-azabicyclo[2.2.2]octan-5-yl) phenyl)-N-((R)-1-(5-(trifluoromethyl)) pyridin-3-yl) pyrrolidin-3-yl) acetamide,
2-(4-((1S,4S)-2-oxa-5-azabicyclo[2.2.2]octan-5-yl) phenyl)-N-((R)-1-(6-(trifluoromethyl)) pyridin-3-yl) pyrrolidin-3-yl) acetamide,
(R)-2-(benzo[d]thiazol-6-yl)-N-(1-(5-(trifluoromethyl) pyridin-3-yl) pyrrolidin-3-yl) acetamide,
(R)-2-(benzo[d]thiazol-6-yl)-N-(1-(6-(trifluoromethyl) pyridin-3-yl) pyrrolidin-3-yl) acetamide,
(R)-2-(1H-indazol-5-yl)-N-(1-(5-(trifluoromethyl) pyridin-3-yl) pyrrolidin-3-yl) acetamide,
(R)-2-(1H-indazol-5-yl)-N-(1-(6-(trifluoromethyl) pyridin-3-yl) pyrrolidin-3-yl) acetamide,
2-(4-cyclopropylphenyl)-N-(3-(trifluoromethyl)-1-(5-(trifluoromethyl) pyridin-3-yl) pyrrolidin-3-yl) acetamide,
(R)-N-(3-(trifluoromethyl)-1-(5-(trifluoromethyl) pyridin-3-yl) pyrrolidin-3-yl)-2-(4-(trifluoromethyl) phenyl) acetamide,
(S)-N-(3-(trifluoromethyl)-1-(5-(trifluoromethyl) pyridin-3-yl) pyrrolidin-3-yl)-2-(4-(trifluoromethyl) phenyl) acetamide,
(R)-2-(1H-benzo[d][1,2,3]triazol-5-yl)-N-(1-(5-(trifluoromethyl) pyridin-3-yl) pyrrolidin-3-yl) acetamide,
(R)-2-(1H-benzo[d][1,2,3]triazol-5-yl)-N-(1-(6-(trifluoromethyl) pyridin-3-yl) pyrrolidin-3-yl) acetamide,
2-(4-((1R,4R)-2-oxa-5-azabicyclo[2.2.1]heptan-5-yl) phenyl)-N-((R)-1-(5-(trifluoromethyl)) pyridin-3-yl) pyrrolidin-3-yl) acetamide,
2-(4-((1S,4S)-2-oxa-5-azabicyclo[2.2.1]heptan-5-yl) phenyl)-N-((R)-1-(5-(trifluoromethyl)) pyridin-3-yl) pyrrolidin-3-yl) acetamide,
2-(4-((1R,4R)-2-oxa-5-azabicyclo[2.2.1]heptan-5-yl) phenyl)-N-((R)-1-(6-(trifluoromethyl)) pyridin-3-yl) pyrrolidin-3-yl) acetamide,
2-(4-((1S,4S)-2-oxa-5-azabicyclo[2.2.1]heptan-5-yl) phenyl)-N-((R)-1-(6-(trifluoromethyl)) pyridin-3-yl) pyrrolidin-3-yl) acetamide,
(R)-2-(1-methyl-1H-benzo[d][1,2,3]triazol-5-yl)-N-(1-(5-(trifluoromethyl) pyridin-3-yl) pyrrolidin-3-yl) acetamide,
(R)-2-(1-methyl-1H-benzo[d][1,2,3]triazol-5-yl)-N-(1-(6-(trifluoromethyl) pyridin-3-yl) pyrrolidin-3-yl) acetamide,
2-(4-cyclopropylphenyl)-N-(1-(4-(trifluoromethyl) phenyl) azetidin-3-yl) acetamide,
2-(4-cyclopropylphenyl)-N-(1-(4-fluoro-3-(trifluoromethyl) phenyl) azetidin-3-yl) acetamide,
(R)-2-(4-bromophenyl)-N-(1-(5-(trifluoromethyl) pyridin-3-yl) pyrrolidin-3-yl) acetamide,
(R)-2-(4-cyclopropylphenyl)-N-(1-(6-fluorobenzo[d]thiazol-2-yl) pyrrolidin-3-yl) acetamide,
(R)-N-(1-(5-bromothiazol-2-yl) pyrrolidin-3-yl)-2-(4-cyclopropylphenyl) acetamide,
(R)-2-(4-cyclopropylphenyl)-N-(1-(4-fluoro-3-(trifluoromethyl) phenyl) pyrrolidin-3-yl) acetamide,
(R)-2-(4-cyclopropylphenyl)-N-(1-(3-methoxyphenyl) pyrrolidin-3-yl) acetamide,
(R)-2-(4-(3,3-difluoropyrrolidin-1-yl) phenyl)-N-(1-(5-(trifluoromethyl) pyridin-3-yl) pyrrolidin-3-yl) acetamide,
ethyl (R)-2-(3-(3-(2-(4-cyclopropylphenyl)acetamido)pyrrolidin-1-yl)phenoxy)-2-methylpropanoate,
(R)-2-(4-cyclopropylphenyl)-N-(1-(3-hydroxyphenyl)pyrrolidin-3-yl) acetamide,
(R)-2-(4-cyclopropylphenyl)-N-(1-(6-(trifluoromethyl)pyridin-2-yl)pyrrolidin-3-yl)acetamide,
(R)-2-(4-cyclopropylphenyl)-N-(1-(4,6-dimethoxypyrimidin-2-yl)pyrrolidin-3-yl) acetamide,
(R)-2-(4-morpholinophenyl)-N-(1-(5-(trifluoromethyl)pyridin-3-yl)pyrrolidin-3-yl)acetamide,
(R)-2-(4-cyclopropylphenyl)-N-(1-(4-(trifluoromethyl)pyridin-2-yl)pyrrolidin-3-yl)acetamide,
(R)-2-(4-trifluoromethyl) phenyl-N-(1-(6-(trifluoromethyl)pyridin-3-yl)pyrrolidin-3-yl)acetamide,
(R)-N-(1-(4-fluoro-3-(trifluoromethyl)phenyl)pyrrolidin-3-yl)-2-(4-(trifluoromethyl)phenyl)acetamide,
(R)-2-(4-(trifluoromethyl)phenyl)-N-((R)-1-(6-(trifluoromethyl)pyridin-3-yl)pyrrolidin-3-yl) propanamide,
(S)-2-(4-(trifluoromethyl)phenyl)-N-((R)-1-(6-(trifluoromethyl)pyridin-3-yl)pyrrolidin-3-yl) propanamide,
(1S,2S)-2-(1H-benzo[d]imidazol-2-yl)-N-((R)-1-(5-(trifluoromethyl)pyridin-3-yl) piperidin-3-yl)cyclopropane-1-carboxyamide,
(R)-2-(1H-indol-6-yl)-N-(1-(4-(trifluoromethyl) thiazol-2-yl)pyrrolidin-3-yl)acetamide, and
(R)-2-(1-methyl-1H-indol-5-yl)-N-(1-(6-(trifluoromethyl)pyridin-3-yl)pyrrolidin-3-yl)acetamide.

74. The medicament according to any one of claims 1 to 73, wherein the pruritus is histaminergic pruritus.

75. The medicament according to any one of claims 1 to 73, wherein the pruritus is non-histaminergic pruritus.

76. Use of the compound according to any one of claims 1 to 73, a tautomer of the compound, a stereoisomer of the compound, a pharmaceutically acceptable salt thereof, or a solvate thereof for producing a medicament for preventing or treating pruritus.

77. The use according to claim 76, wherein the pruritus is histaminergic pruritus.

78. The use according to claim 76, wherein the pruritus is non-histaminergic pruritus.

79. The compound according to any one of claims 1 to 73, a tautomer of the compound, a stereoisomer of the compound, a pharmaceutically acceptable salt thereof, or a solvate thereof, used for preventing or treating pruritus.

80. The compound according to any one of claims 1 to 73, a tautomer of the compound, a stereoisomer of the compound, a pharmaceutically acceptable salt thereof, or a solvate thereof, used for preventing or treating histaminergic pruritus.

81. The compound according to any one of claims 1 to 73, a tautomer of the compound, a stereoisomer of the compound, a pharmaceutically acceptable salt thereof, or a solvate thereof, used for preventing or treating non-histaminergic pruritus.

82. A method for treating pruritus in a human, the method comprising a step of administering an effective amount of the compound according to any one of claims 1 to 73, a tautomer of the compound, a stereoisomer of the compound, a pharmaceutically acceptable salt thereof, or a solvate thereof to a subject in need thereof.

83. The method according to claim 82, wherein the pruritus is histaminergic pruritus.

84. The method according to claim 82, wherein the pruritus is non-histaminergic pruritus.
